# EUROPEAN PATENT SPECIFICATION

(11) **EP 1 222 187 B1**
(45) Date of publication and mention of the grant of the patent: **22.09.2004**
(21) Application number: 00968713.8
(22) Date of filing: 05.10.2000
(51) Int. Cl.: C07D 471/04, C07D 513/04, C07D 498/04, A61K 31/437, A61K 31/4355, A61P 35/00, C07D 217/24, C07D 513/00

(54) **HETEROCYCLIC COMPOUNDS USEFUL AS INHIBITORS OF TYROSINE KINASES**
HETEROCYCLISCHE VERBINDUNGEN VERWENDBAR ALS TYROSINKINASE INHIBITOREN
COMPOSES HETEROCYCLIQUES UTILES COMME INHIBITEURS DE TYROSINE KINASES

(30) Priority: 06.10.1999 US 157922 P
(43) Date of publication of application: 17.07.2002
(73) Proprietor: BOEHRINGER INGELHEIM PHARMACEUTICALS INC., Ridgefield, Connecticut 06877-0368 (US)
(72) Inventor: SNOW, Roger, John, Danbury, CT 06811 (US); CARDOZO, Mario, Gustavo, Brookfield, CT 06804 (US); GOLDBERG, Daniel, Redding, CT 06896 (US); HAMMACH, Abdelhakim, Danbury, CT 06810 (US); MORWICK, Tina, New Milford, CT 06776 (US); MOSS, Neil, Ridgefield, CT 06877 (US); PATEL, Usha, R., Brookfield, CT 06804 (US); PROKOPOWICZ, Anthony, S., III, Patterson, NY 12563 (US); TAKAHASHI, Hidenori, LaGrangeville, NY 12540 (US); TSCHANTZ, Matt, Aaron, Newtown, CT 06470 (US); WANG, Xiao-jun, Danbury, CT 06811 (US)
(74) Representative: Kompter, Hans-Michael, Dr.
(86) International application number: PCT/US2000/027444
(87) International publication number: WO 2001/025238

(56) References cited:
- EP-A- 0 322 746
- WO-A-99/24035
- DE-A- 2 732 951
- US-A- 4 176 184
- US-A- 5 646 153

## Description

### Technical Field of the Invention

This invention relates to substituted compounds of formula (I): 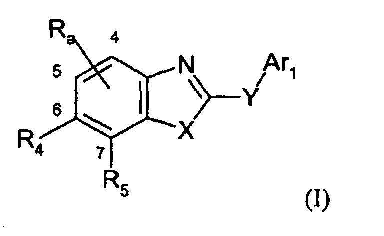 wherein Ar₁, Rₐ, R₄, R₅, X and Y are defined below, which are useful as inhibitors of certain protein tyrosine kinases and are thus useful for treating diseases resulting from inappropriate cell proliferation, which include autoimmune diseases, chronic inflammatory diseases, allergic diseases, transplant rejection and cancer. This invention also relates to processes for preparing these compounds and to pharmaceutical compositions comprising these compounds.

### Background of the Invention

Tyrosine kinases play an essential role in the regulation of cell signaling and cell proliferation by phosphorylating tyrosine residues of peptides and proteins. Inappropriate activation of tyrosine kinases is known to be involved in a variety of disease states, including immunologic and oncologic disorders.

It has been well established that T cells play an important role in regulating the immune response (F. Powrie and R.L. Coffman, *Immunol*. *Today,* **1993**, *14*, 270). Activation of T cells is often the initiating event in many inflammatory and autoimmune diseases. In addition to their role in immune surveillance, T cells can become autoreactive by recognizing self-antigens and thereby cause autoimmune disease such as rheumatoid arthritis and inflammatory bowel disease.

The T cell receptor (TCR) is the antigen-specific component of the T cell and is activated when the receptor is engaged with foreign or self-antigenic peptides. When the TCR is activated a series of enzyme-mediated signal transduction cascades is initiated which results in the production of pro-inflammatory cytokines such as interleukin-2 (IL-2).
The release of IL-2 is critically important since this lymphokine is required for T-lymphocyte proliferation, differentiation, and effector function. Clinical studies have shown that interference with IL-2 activity effectively suppresses immune response *in vivo* (T.A. Waldmann, *Immunol*. *Today,* 1993, *14*, 270). Accordingly, agents which inhibit T-lymphocyte activation and subsequent IL-2 production, or block the activity of IL-2 are therapeutically useful for selectively suppressing immune response in a patient in need of such immunosuppression.

The eight members of the src family of tyrosine kinases are src, lck, fyn, lyn, hck, fgr, blk and yes (J.B. Bolen, J.S. Brugge, *Ann. Rev. Immunol.,* **1997**, *15*, 371). These can be divided into 2 groups based on their pattern of tissue expression. Src, fyn and yes have a broad distribution while expression of lck, lyn, hck, fgr, and blk is largely limited to hemopoietic cells. The therapeutic effects of inhibiting kinases of the src family can be ascertained by linking functional defects seen in gene disruption studies in mice. Src(-/-) mice had severe abnormalities in bone remodeling. Inhibition of src may therefore be useful in treating osteoporosis. Lck(-/-) mice display a complete lack of CD4+ cells and are unable to mount antigen-dependent immune responses.

A kinase of particular interest is p561ck, which is only expressed in T-cells. Within the TCR signal transduction cascade the tyrosine kinase p561ck is a required element to initiate the activation response from the TCR intracellular domains to other signaling proteins. For example, T cells which lack the p561ck protein are unable to signal through the T cell receptor (D.B. Straus and A. Weiss, *Cell*, 1992, *70*, 585). Transfection of p561ck back into these cell lines restores TCR responsiveness. Also, it has been shown in mice that inactivation of the p561ck gene leads to lack of proper thymocyte development (T.J. Molina et al., *Nature,* 1992, *357*, 161).

The conclusion drawn from these studies is that p561ck plays a crucial role in T cell maturation and antigen-induced T-cell activation. Therefore, an agent blocking p561ck would effectively block T cell function, act as an immunosuppressive agent and have potential utility in autoimmune diseases, for example rheumatoid arthritis, multiple sclerosis, lupus, transplant rejection and allergic diseases (J.H. Hanke et al., *Inflamm*. *Res.,* **1995,** *44*, 357).

Inhibitors of other members of the src family of non-receptor tyrosine kinases are also useful for treating various disease states. Src is present in osteoclasts, and is important in bone remodeling. For example, inactivation of p60src diminishes bone resorption by osteoclasts (P. Soriano et al., *Cell* 1991, *64*, 693, B.F. Boyce et al. *J*. *Clin. Invest* **1992***, 90*, 1622), it is therefore possible that inhibitors of the kinase activity of p60src are useful in the treatment of osteoporosis, Paget's disease and inflammation of bones and joints.

Src kinases have been found to be activated in tumors, including breast and colon cancers, melanoma and sarcoma. For example, a number of primary tumors and tumor cell lines from patients with breast cancer, colon cancer, melanoma and sarcoma have been shown to have elevated src kinase activity, and activating src mutations are seen in some advanced colon cancers. Inhibitors of src kinase had significant antiproliferative activity against cancer cell lines (M.M. Moasser et al., *Cancer Res.,* 1999, *59,* 6145) and inhibited the transformation of cells to an oncogenic phenotype (R. Karni et al., *Oncogene,* **1999**, *18*, 4654) suggesting that src kinase inhibitors may be useful anti-cancer agents.

In addition, src family kinases participate in signal transduction in several cell types. For example, fyn, like lck, is involved in T-cell activation. Hck and fgr are involved in Fc gamma receptor mediated oxidative burst of neutrophils. Src and lyn are believed to be important in Fc epsilon induced degranulation of mast cells, and so may play a role in asthma and other allergic diseases. The kinase lyn is known to be involved in the cellular response to DNA damage induced by UV light (T. Hiwasa, *FEBS Lett.* **1999**, *444*, 173) or ionizing radiation (S. Kumar, *J*. *Biol Chem*, **1998**, *273*, 25654). Inhibitors of lyn kinase may thus be useful as potentiators in radiation therapy.

Platelet derived growth factor is a potent mitogen for smooth muscle cells. Its receptor (PDGFR) is a member of the receptor tyrosine kinase family (L. Claesson-Welsh, *J*. *Biol Chem,* **1994**, *269,* 32023). PDGF is involved in atherosclerosis and restenosis (K.E. Bornfeldt, *Trends Cardiovasc. Med*., **1996**, *6*, 143). In addition, receptor tyrosine kinases including PDGFR kinase have been implicated as contributing factors in cancer (A. Levitzki and A. Gazit, *Science,* 1995, *267*, 1782) including ovarian (M.B. Dabrow et al., *Gynecologic Oncology,* 1998, *71*, 29) and prostate (S.M. Sintich et al., *Endocrinology,* 1999, *140*, 3411) cancers and glioblastoma (B.J. Silver, *BioFactors*, **1992** *3*, 217). Inhibitors of PDGFR kinase are thus useful in the treatment of fibrotic diseases, restenosis and PDGF-dependent tumors.

Reports have appeared in the literature of agents that inhibit the kinase activity of p561ck kinase and thus inhibit T cell activation. These include the natural product lavendustin A, and analogs (M.S. Smyth, *J. Med*. *Chem.,* **1993**, *36,* 3010), the natural product damnacanthal (C.R. Faltynek et al., *Biochemistry,* **1995**, *34*, 12404), and a 1-methoxy agroclavine isolated from a fungal extract (R. Padmanabha et al. *Bioorganic and Med*. *Chem. Letters,* 1998, 8, 569). Other inhibitors reported include WIN 61651 *(J. Enzyme Inhibition,* **1995**, *9*, 111) pyrazolopyrimidines PP1 and PP2 (Hanke et al. *J*. *Biol Chem,* **1996**, *271,* 695) and indanone and indandione derivatives (J.L. Bullington et al., *Bioorganic and Med*. *Chem. Letters*, **1998**, *8*, 2489).

A.P. Spader et al. (WO 98/54157, 1998) describe quinoline and quinoxaline compounds that inhibit p561ck and PDGFR kinase. Fused polycyclic 2-aminopyrimidine derivatives that inhibit p561ck are reported by J.M. Davis et al. (WO 98/28281, 1998). J. Das et al. claim a series of benzothiazole amides as inhibitors of lck and other src family kinases (WO 99/24035, 1999). Inhibitors of PDGFR kinase and src-family kinases were reviewed by H.D.H. Showalter, A.J. Kraker, *Pharmacol. Ther*., **1997**, *76*, 55. Several patents on inhibitors of lck are reviewed in P.M. Traxler, *Exp. Opin. Ther*. *Patents,* 1997, 7, 571,and P.M. Traxler, *Exp. Opin. Ther. Patents,* 1998, 8, 1599.

U.S. Pat. No. 4,176,184 discloses imidazoisoquinoline-diones, which are described as being useful as cardiotonics, hypotensives, antithrombotics and antiarrhythmics. DE 3410168 A 1 discloses imidazoisoquinoline-dione derivatives, these compounds are described as being useful as cardiotonic agents in which the substituent on the fused imidazole ring is a pyridine ring bridged to the imidazole carbon by a C₁-C₄ alkyl group, a vinyl group or a chemical bond. EP 322 746 A 1 discloses heterocyclic lactam derivatives described as being useful as cardiotonic agents, antihypertensive agents and vasodilators.

The compounds of the present invention represent a novel structural class, which is distinct from previously reported tyrosine kinase inhibitors.

### Brief Summary of the Invention

The work cited above supports the principle that inhibition of the kinases mentioned above will be beneficial in the treatment of various disease states.

It is therefore an object of the invention to provide novel compounds which inhibit PDGFR kinase and the src-family kinases including lck, src, fyn, lyn, hck, fgr, blk and yes.

It is a further object of the invention to provide methods for treating diseases and pathological conditions mediated by src-family tyrosine kinases and PDGFR kinase such as autoimmune diseases, transplant rejection, psoriasis, osteoporosis, Paget's disease, cancer, including src-dependent tumors and PDGF-dependent tumors, atherosclerosis, restenosis and allergic diseases, using the novel compounds of the invention.

It is yet a further object of the invention to provide processes of preparation of the abovementioned novel compounds and pharmaceutical compositions comprising the same.

### Detailed Description of the Invention

The src-family tyrosine kinases and PDGFR kinase discussed above exhibit some homology in their amino acid structure. It is contemplated that due to structural differences between individual src-family kinases and PDGFR kinase, different compounds of the invention may have different inhibitory potencies against individual tyrosine kinases. Thus some of compounds of the invention may also be expected to be most effective in treating diseases mediated by tyrosine kinases that they inhibit most potently. Particular compounds disclosed herein have been shown to be active inhibitors of p561ck kinase, p60src kinase and PDGFR kinase. See the section entitled "Assessment of Biological Properties" disclosed herein.

In its broadest generic aspect, the invention provides novel compounds of the formula I: 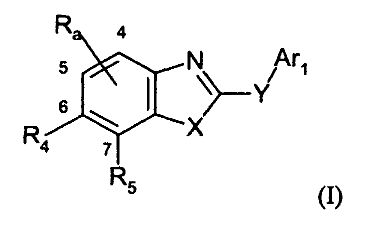 wherein:
Ar₁ is an aromatic or nonaromatic carbocycle, heteroaryl or heterocycle; wherein said carbocycle, heteroaryl or heterocycle is optionally substituted by one or more R₁, R₂ and R₃;
X is NH, N-C₁₋₃alkyl, N-cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl; wherein each Rₐ is independently optionally substituted with one or more C₁₋₆alkyl, C₁₋₆ alkoxy, halogen, OH, oxo, NR₁₀R₁₁, aryl or heteroaryl, each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, CONR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 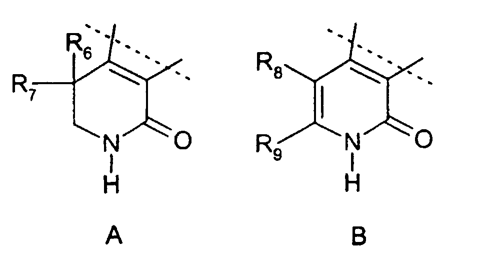
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁; or R₈ is phenyl or heteroaryl, each being optionally substituted with C₁₋₃alkyl, C₁₋₃alkoxy, OH, -SO₃H or halogen;
R₉ is H, CN or CONR₁₀R₁₁; or R₉ is C₁₋₁₀alkyl branched or unbranched, C₃₋₁₀cycloalkyl, C₅₋₇cycloalkenyl, C₂₋₆ alkenyl, C₂₋₆ alkynyl each being optionally substituted with one or more C₃₋₁₀cycloalkyl, C₃₋₁₀cycloalkylidene, C₅₋₇cycloalkenyl, halogen, OH, oxo, CN, C₁₋₃alkoxy, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl, heteroaryl, or heterocycle, wherein each aryl, heteroaryl or heterocycle is optionally substituted with one to three groups selected from C₁₋₃alkyl optionally substituted with phenyl or NR₁₀C(=NR₁₀)NR₁₀R₁₁, C₁₋₃alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by one or two C₁₋₃alkyl, OH, oxo or (CH₂)ₙNR₁₀R₁₁, or optionally spire-fused to a 1,3 dioxolane group or 1,3 dithiolane group, each 1,3 dioxolane group or 1,3 dithiolane group optionally substituted by C₁₋₆alkyl, C₁₋₆alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, CN, NO₂, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH, -(CH₂)ₙNR₁₃R₁₄, CONR₁₃R₁₄ or NR₁₃COR₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl or heterocycle, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
   or R₁₃ and R₁₄ together form a chain completing a ring, said chain is (CH₂)₄₋₅ or (CH₂)₂O(CH₂)₂;
R₁₅ is H or C₁₋₃ alkyl;
m is 1-4; n is 0-3 and p is 0-2; and
the pharmaceutically acceptable acid or salt derivatives thereof.

In one embodiment of the invention, there are provided compounds of the formula (I) described above, wherein:
Ar₁ is
   a) a cycloalkyl group selected from cyclopropyl, cyclobutyl, cyclopentanyl, cyclohexanyl, cycloheptanyl;
   b) a cycloalkenyl group selected from cyclopentenyl, cyclohexenyl, cycloheptenyl;
   c) phenyl, naphthyl; indanyl, indenyl, dihydronaphthyl, tetrahydronaphthyl, fluorenyl;
   d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, and indazolyl,or a fused heteroaryl selected from cyclopentenopyridine, cyclohexanopyridine, cyclopentanopyrimidine, cyclohexanopyrimidine, cyelopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
   e) a heterocycle selected from pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
   wherein each of the above Ar₁ are optionally substituted by one or more R₁, R₂ and R₃;
Rₐ is H, C₁₋₆alkyl, C₂₋₅alkenyl, C₂₋₅alkynyl, phenyl or heteroaryl selected from pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₁ and R₂ are as hereinabove defined;
R₃ is H, halogen, methyl, methoxy, hydroxymethyl or OH;
R₈ is H, C₁₋₃alkyl branched or unbranched, saturated or unsaturated, optionally substituted with OH; or R₈ is (CH₂)₂₋₃NR₁₀R₁₁, (CH₂)ₙCO₂R₁₂ or (CH₂)ₙCONR₁₀R₁₁;
R₉ is CN or CONR₁₀R_{11;} or R₉ is C₁₋₃alkyl branched or unbranched, C₂₋₄ alkenyl, C₂₋₄alkynyl each being optionally substituted with one or more C₅₋₇cycloalkyl, C₅₋₇cycloalkylidene, C₅₋₇cycloalkenyl,OH, CN, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, CONR₁₀R₁₁, CO₂R₁₂, C(R₁₀)=NNR₁₀R₁₁, C(R₁₀)=NNR₁₀CONR₁₀R₁₁, aryl or heteroaryl; wherein each aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl,heteroaryl or heterocycle, each optionally substituted with one to three groups selected from C₁₋₃alkyl optionally substituted with phenyl or NR₁₀C(=NR₁₀)NR₁₀R₁₁, C₁₋₃alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by C₁₋₃alkyl or OH, or optionally spiro-fused to a 1,3 dioxolane group or 1,3 dithiolane group, each 1,3 dioxolane group or 1,3 dithiolane group optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl, benzyl or phenyl are optionally substituted with OH, C₁₋₃alkoxy, C₁₋₃acyloxy, CN, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH, -(CH₂)ₙNR₁₃R₁₄, CONR₁₃R₁₄ or NR₁₃COR₁₄;;
R₁₂ is H, C₁₋₆alkyl or C₅₋₇cycloalkyl, each optionally substituted with phenyl, OH, C₁₋₃ alkoxy or NR₁₃R₁₄; or R₁₂ is phenyl or heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂; and
R₁₅ is H.

In another embodiment, there are provided compounds of the formula (I) described immediately above, wherein:
Ar₁ is phenyl, or pyridyl, wherein each is optionally substituted by one or more
R₁, R₂ and R₃ as defined below;
X is NH or N-CH₃;
Y is NH and
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
R₁ and R₂ are the same or different and selected from: H, halogen, C₁₋₃ alkyl, wherein the C₁₋₃ alkyl are optionally partially or fully halogenated, NO₂, NR₁₃R₁₄;
R₃ is H, halogen, methoxy or methyl;
R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is CN; or R₉ is methyl, C₂₋₃ alkenyl or C₂₋₃ alkynyl, each being optionally substituted with one or more C₅₋₇ cycloalkylidene, C₅₋₇cycloalkenyl, OH, CN, NR₁₀R₁₁, NR₁₀CONR₁₀R₁₁, NR₁₀COR₁₂, NR₁₀S(O)ₚR₁₂, CONR₁₀R₁₁, CO₂R₁₂, C(R₁₀)=NNR₁₀R₁₁ or heteroaryl;
or R₉ is aryl or heteroaryl optionally substituted with one to three groups selected from C₁₋₃alkyl optionally substituted with phenyl, C₁₋₃alkoxy, halogen, amino or CONH₂;
or R₈ and R₉ together form a cyclopentene ring spiro-fused to a 1,3 dioxolane group, said 1,3 dioxolane group being optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₃alkyl branched or unbranched, C₅₋₇cycloalkyl or phenyl, wherein said alkyl, cycloalkyl or phenyl are optionally substituted with OH, C₁₋₃alkoxy, C₁₋₃acyloxy, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy,OH, (CH₂)ₙNR₁₃R₁₄, CONR₁₃R₁₄ or NR₁₃COR₁₄;
R₁₂ is H, C₁₋₃alkyl or C₅₋₇cycloalkyl, each optionally substituted with phenyl, OH, C₁₋₃ alkoxy or NR₁₃R₁₄; or R₁₂ is phenyl or is a saturated, 4- to 6-membered nitrogen-containing heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R₁₁;
R₁₃ and R₁₄ are each independently selected from H and C₁₋₃alkyl optionally substituted with C₁₋₃alkoxy or OH;
   or R₁₃ and R₁₄ together form a chain completing a ring, said chain is (CH₂)₄₋₅ or (CH₂)₂O(CH₂)₂.
   In yet another embodiment, there are provided compounds of the formula (I) described immediately above, wherein:
   Ar₁ is phenyl;
   Rₐ is H or hydroxymethyl;
   R₁ and R₂ are the same or different and selected from: halogen, methyl optionally partially or fully halogenated, NO₂ and NH₂;
   R₃ is H, chloro, fluoro, bromo or methoxy;
   R₁₀ and R₁₁ may be the same or different and are each independently selected from H, OH, methoxy, C₁₋₃alkyl branched or unbranched or C₅₋₇cycloalkyl, wherein said alkyl or cycloalkyl are optionally substituted with OH, NR₁₃R₁₄ or phenyl;
   or R₁₀ and R₁₁ together form morpholino, pyrrolidinyl, piperazinyl or piperidinyl each optionally substituted by C₁₋₂ alkyl, NR₁₃R₁₄, CONR₁₃R₁₄ or NR₁₃COR₁₄; and
   R₁₂ is C₁₋₃alkyl optionally substituted with morpholino; or R₁₂ is phenyl or is azetidinyl, pyrrolidinyl or piperidinyl, each optionally substituted with one to three groups selected from C₁₋₃alkyl, C₁₋₃alkoxy and halogen.

In another subgeneric aspect, the invention provides novel compounds of the formula I: 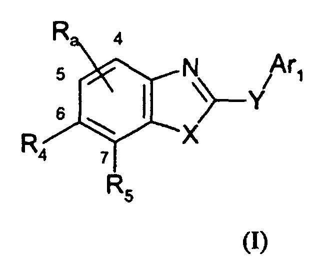 wherein:
Ar₁ is an aromatic or nonaromatic carbocycle, heteroaryl or heterocycle; wherein said carbocycle, heteroaryl or heterocyle is optionally substituted by one or more R₁, R₂ and R₃;
X is NH, N-C₁₋₃alkyl, N-cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl; wherein each Rₐ is independently optionally substituted with one or more C₁₋₃alkyl, C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the above mentioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 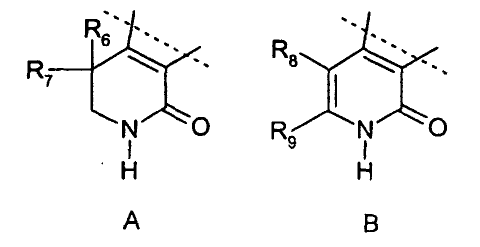
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁; or R₈ is phenyl or heteroaryl, each being optionally substituted with C₁₋₃alkyl, C₁₋₃alkoxy, OH, -SO₃H or halogen;
R₉ is H; or R₉ is C₁₋₁₀alkyl branched or unbranched, C₃₋₁₀ cycloalkyl, C₂₋₆ alkenyl, C₂₋₆ alkynyl each being optionally substituted with one or more halogen, OH, oxo, CN, C₁₋₃alkoxy, NR₁₀R₁₁, NR₁₀COR₁₂, SR₁₂, CONR₁₀R₁₁, CO₂R₁₂, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
   or R₉ is aryl or heteroaryl, wherein each aryl or heteroaryl is optionally substituted with one to three groups selected from C₁₋₃alkyl optionally substituted with phenyl, C₁₋₃alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by one or two OH, oxo or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
   or R₁₃ and R₁₄ together form a chain completing a ring, said chain is (CH₂)₄₋₅ or (CH₂)₂O(CH₂)₂;
R₁₅ is H or C₁₋₃ alkyl;
m is 1-4, n is 0-3 and p is 0-2; and
the pharmaceutically acceptable acid or salt derivatives thereof.

In one embodiment of the invention, there are provided compounds of the formula (I) as described immediately above, and wherein:
Ar₁ is
   a) a cycloalkyl group selected from cyclopropyl, cyclobutyl, cyclopentanyl, cyclohexanyl, cycloheptanyl;
   b) a cycloalkenyl group selected from cyclopentenyl, cyclohexenyl, cycloheptenyl;
   c) phenyl, naphthyl, indanyl, indenyl, dihydronaphthyl, tetrahydronaphthyl, fluorenyl;
   d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, and indazolyl,or a fused heteroaryl selected from cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
   e) a heterocycle selected from: pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
   wherein each of the above Ar₁ are optionally substituted by one or more R₁, R₂ and R₃ as hereinabove defined;
   Rₐ is H, C₁₋₆ alkyl, C₂₋₅ alkenyl, C₂₋₅ alkynyl, phenyl or heteroaryl selected from: pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₃ is H, halogen, methyl, methoxy, hydroxymethyl or OH;
R₈ is H, C₁₋₃alkyl branched or unbranched, saturated or unsaturated, optionally substituted with OH; or R₈ is (CH₂)₂₋₃NR₁₀R₁₁, (CH₂)ₙCO₂R₁₂ or (CH₂)ₙCONR₁₀R₁₁;
R₉ is C₁₋₃alkyl branched or unbranched, C₂₋₄ alkenyl, C₂₋₄alkynyl each being optionally substituted with one or more OH, CN, NR₁₀R₁₁, CONR₁₀R₁₁, CO₂R₁₂, aryl or heteroaryl; wherein each aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
   or R₉ is aryl or heteroaryl optionally substituted with one to three groups selected from C₁₋₃alkyl optionally substituted with phenyl, C₁₋₃alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by OH;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl, benzyl or phenyl are optionally substituted with OH, C₁₋₃ alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH or -(CH₂)ₙNR₁₃R₁₄;
R₁₂ is H or C₁₋₆alkyl optionally substituted with phenyl, OH, C₁₋₃alkoxy or NR₁₃R₁₄;
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂; and
R₁₅ is H.
   In another embodiment of the invention, there are provided compounds of the formula (I) as described immediately above, and wherein:
   Ar₁ is phenyl, or pyridyl;
   X is NH or N-CH₃;
   Y is NH and
   Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
   R₁ and R₂ are the same or different and selected from: halogen, C₁₋₃ alkyl, wherein the C₁₋₃ alkyl are optionally partially or fully halogenated, NO₂, NR₁₃R₁₄;
   R₃ is H, halogen, methoxy or methyl;
   R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is methyl or C₂₋₃ alkenyl each being optionally substituted with one or more OH, CN, NR₁₀R₁₁, CONR₁₀R₁₁ or CO₂R₁₂;
   or R₉ is heteroaryl optionally substituted with one to three groups selected from C₁₋₃alkyl optionally substituted with phenyl, C₁₋₃alkoxy, halogen or amino;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
   or R₁₃ and R₁₄ together form a chain completing a ring, said chain is (CH₂)₄₋₅ or (CH₂)₂O(CH₂)₂.
   In yet another embodiment of the invention there are provided compounds of the formula (I) as described immediately above, and wherein:
   Ar₁ is phenyl;
   Rₐ is H or hydroxymethyl;
   R₁ and R₂ are the same or different and selected from: halogen, methyl optionally partially or fully halogenated, NO₂ and NH₂;
   R₃ is H, chloro, fluoro, bromo or methoxy;
   R₁₀ and R₁₁ may be the same or different and are each independently selected from H, OH, methoxy, C₁₋₃alkyl branched or unbranched, optionally substituted with OH, NR₁₃R₁₄ or phenyl;
      or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

In still another embodiment of the invention there are provided compounds of the formula (Ia): 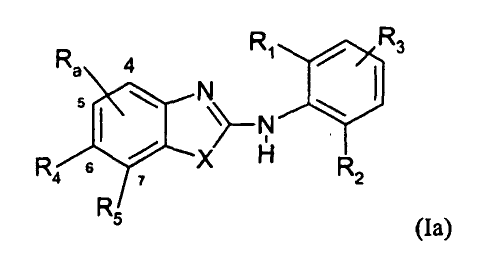 wherein:
X is NH, N-C₁₋₃alkyl, N-cyclopropyl, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
   wherein each Rₐ is independently optionally substituted with one or more C₁₋₆alkyl, C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃ alkoxy, hydroxyC₁₋₃alkyl and (CH₂)ₘNR₁₀R₁₁; and wherein Rₐ is attached at the 4- or 5-position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, CONR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 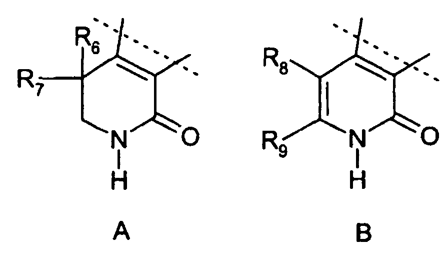
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁ or R₈ is phenyl or heteroaryl, each being optionally substituted with C₁₋₃alkyl, C₁₋₃alkoxy, OH, -SO₃H or halogen;
R₉ is H, CN or CONR₁₀R₁₁; or R₉ is C₁₋₁₀alkyl branched or unbranched, C₃₋₁₀cycloalkyl, C₅₋₇cycloalkenyl, C₂₋₆ alkenyl, C₂₋₆ alkynyl each being optionally substituted with one or more C₃₋₁₀cycloalkyl, C₃₋₁₀cycloalkylidene, C₅₋₇cycloalkenyl, halogen, OH, oxo, CN, C₁₋₃alkoxy, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl, heteroaryl, or heterocycle, wherein each aryl, heteroaryl or heterocycle is optionally substituted with one to three groups selected from C₁₋₃alkyl optionally substituted with phenyl or NR₁₀C(=NR₁₀)NR₁₀R₁₁, C₁₋₃alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by one or two C₁₋₃alkyl, OH, oxo or (CH₂)ₙNR₁₀R₁₁, or optionally spiro-fused to a 1,3 dioxolane group or 1,3 dithiolane group, each 1,3 dioxolane group or 1,3 dithiolane group optionally substituted by C₁₋₆alkyl, C₁₋₆alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, CN, NO₂, C₁₋₃acyloxy, CO₂R₁₂, NR₁₃R₁₄, O(CH₂)₂-₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH, -(CH₂)ₙNR₁₃R₁₄, CONR₁₃R₁₄ or NR₁₃COR₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl or heterocycle, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R₁₁;
R₁₃ and R₁₄ are each independently selected from H and C₁₋₆ alkyl optionally substituted with C₁₋₃alkoxy, OH or phenyl;
   or R₁₃ and R₁₄ together form a chain completing a ring, said chain is (CH₂)₄₋₅ or (CH₂)₂O(CH₂)₂;
m is 1-4, n is 0-3 and p is 0-2; and
   the pharmaceutically acceptable acid or salt derivatives thereof.
   In another embodiment of the invention, there are provided compounds of the formula (Ia) as described above, wherein:
   X is NH or N-CH₃;
   Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl; and wherein Rₐ is attached at the 4- position;
   R₁ and R₂ are the same or different and selected from: H, halogen, C₁₋₃ alkyl, wherein the C₁₋₃ alkyl is optionally partially or fully halogenated, NO₂, NR₁₃R₁₄;
   R₃ is H, halogen, methoxy or methyl;
   R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is CN; or R₉ is methyl, C₂₋₃ alkenyl or C₂₋₃ alkynyl, each being optionally substituted with one or more C₅₋₇ cycloalkylidene, C₅₋₇cycloalkenyl, OH, CN, NR₁₀R₁₁, NR₁₀CONR₁₀R₁₁, , NR₁₀COR₁₂, NR₁₀S(O)ₚR₁₂, CONR₁₀R_{11,} CO₂R_{12,} C(R₁₀)=NNR₁₀R₁₁ or heteroaryl;
or R₉ is aryl or heteroaryl optionally substituted with one to three groups selected from C₁₋₃alkyl optionally substituted with phenyl, C₁₋₃alkoxy, halogen, amino or CONH₂;
or R₈ and R₉ together form a cyclopentene ring spiro-fused to a 1,3 dioxolane group, said 1,3 dioxolane group being optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₃alkyl branched or unbranched, C₅₋₇cycloalkyl or phenyl, wherein said alkyl, cycloalkyl or phenyl are optionally substituted with OH, C₁₋₃alkoxy, C₁₋₃acyloxy, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy,OH, (CH₂)ₙNR₁₃R₁₄, CONR₁₃R₁₄ or NR₁₃COR₁₄;
R₁₂ is H, C₁₋₃alkyl or C₅₋₇cycloalkyl, each optionally substituted with phenyl, OH, C₁₋₃alkoxy or NR₁₃R₁₄; or R₁₂ is phenyl or is a saturated, 4- to 6-membered nitrogen-containing heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₃alkyl optionally substituted with C₁₋₃alkoxy or OH;
   or R₁₃ and R₁₄ together form a chain completing a ring, said chain is (CH₂)₄₋₅ or (CH₂)₂O(CH₂)₂.
   In yet another embodiment of the present invention, there are provided compounds of the formula (Ia) described immediately above, wherein:
   Rₐ is H or hydroxymethyl;
   R₁ and R₂ are the same or different and selected from: halogen, methyl optionally partially or fully halogenated, NO₂ and NH₂;
   R₃ is H, chloro, fluoro, bromo or methoxy;
   R₁₀ and R₁₁ may be the same or different and are each independently selected from H, OH, methoxy, C₁₋₃alkyl branched or unbranched or C₅₋₇cycloalkyl, wherein said alkyl or cycloalkyl are optionally substituted with OH, NR₁₃R₁₄ or phenyl;
   or R₁₀ and R₁₁ together form morpholino, pyrrolidinyl, piperazinyl or piperidinyl each optionally substituted by C₁₋₂ alkyl, NR₁₃R₁₄, CONR₁₃R₁₄ or NR₁₃COR₁₄; and
   R₁₂ is C₁₋₃alkyl optionally substituted with morpholino; or R₁₂ is phenyl or is azetidinyl, pyrrolidinyl or piperidinyl, each optionally substituted with one to three groups selected from C₁₋₃alkyl, C₁₋₃alkoxy and halogen.

In still another subgeneric embodiment of the invention there are provided compounds of the formula (Ia): 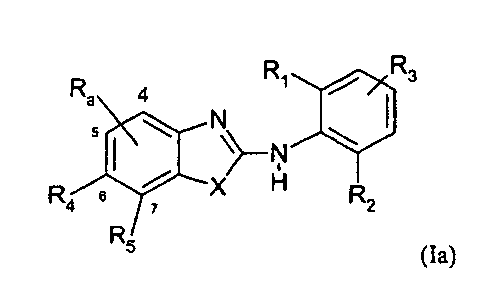 wherein:
X is NH, N-C₁₋₃alkyl, N-cyclopropyl, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
   wherein each Rₐ is independently optionally substituted with one or more C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀branched or unbranched acyl, C₁₋₁₀branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the above mentioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 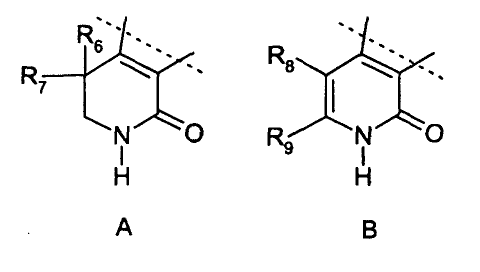
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁ or R₈ is phenyl or heteroaryl, each being optionally substituted with C₁₋₃alkyl, C₁₋₃alkoxy, OH, -SO₃H or halogen;
R₉ is H; or R₉ is C₁₋₁₀alkyl branched or unbranched, C₃₋₁₀cycloalkyl, C₂₋₆ alkenyl, C₂₋₆alkynyl each being optionally substituted with one or more halogen, OH, oxo, CN, C₁₋₃ alkoxy, NR₁₀R₁₁, NR₁₀COR₁₂, SR₁₂, CONR₁₀R₁₁, CO₂R₁₂, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl or heteroaryl, wherein each aryl or heteroaryl is optionally substituted with one to three groups selected from C₁₋₃alkyl optionally substituted with phenyl, C₁₋₃alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by one or two OH, oxo or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ and NR₁₃; and wherein said ring is optionally substituted by C₁₋₃ alkyl, C₁₋₃ alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₄NR₁₀R_{11;}
R₁₃ and R₁₄ are each independently selected from H and C₁₋₆ alkyl optionally substituted with C₁₋₃alkoxy, OH or phenyl;
   or R₁₃ and R₁₄ together form a chain completing a ring, said chain is (CH₂)₄₋₅ or (CH₂)₂O(CH₂)₂;
m is 1-4, n is 0-3 and p is 0-2; and
   the pharmaceutically acceptable acid or salt derivatives thereof.
   In another embodiment of the invention there are provided compounds of the formula (Ia) as described immediately above, and wherein:
   X is NH or N-CH₃;
   Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl; and wherein Rₐ is attached at the 4- position;
   R₁ and R₂ are the same or different and selected from: halogen, C₁₋₃ alkyl, wherein the C₁₋₃ alkyl is optionally partially or fully halogenated, NO₂, NR₁₃R₁₄;
   R₃ is H, halogen, methoxy or methyl;
   R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is methyl or C₂₋₄ alkenyl each being optionally substituted with one or more OH, CN, NR₁₀R₁₁, CONR₁₀R₁₁ or CO₂R₁₂;
   or R₉ is heteroaryl optionally substituted with one to three groups selected from C₁₋₃alkyl optionally substituted with phenyl, C₁₋₃alkoxy, halogen or (CH₂)ₙNR₁₀R₁₁;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
   or R₁₃ and R₁₄ together form a chain completing a ring, said chain is (CH₂)₄₋₅ or (CH₂)₂O(CH₂)₂.
   In still a further embodiment of the invention there are provided compounds of the formula (Ia) as described immediately above, and wherein:
   Rₐ is H or hydroxymethyl;
   R₁ and R₂ are the same or different and selected from: halogen, methyl optionally partially or fully halogenated, NO₂ and NH₂;
   R₃ is H, chloro, fluoro, bromo or methoxy;
   R₁₀ and R₁₁ may be the same or different and are each independently selected from H, OH, methoxy, C₁₋₃alkyl branched or unbranched, optionally substituted with OH, NR₁₃R₁₄ or phenyl;
      or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

In another aspect of the invention, there are provided intermediate compounds of the formula(III) useful in the synthetic schemes and examples set forth below. In yet another aspect of the invention are particular intermediate compounds of the formula(III), (representative examples shown Table 1 below) which possess physiological activity.

In their broadest generic aspect, intermediate compounds described above are represented by the formula (III): 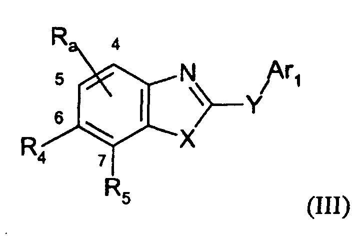 wherein:
Ar₁ is an aromatic or nonaromatic carbocycle, heteroaryl or heterocycle; wherein said carbocycle, heteroaryl or heterocycle is optionally substituted by one or more R₁, R₂ and R₃;
X is NH, N-C₁₋₃alkyl, N,cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
   wherein each Rₐ is independently optionally substituted with one or more C₁₋₃alkyl, C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5-position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formula C: 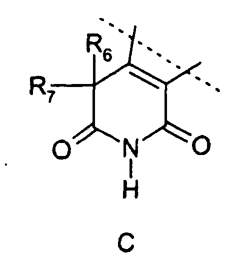
R₆ is C₁₋₃alkyl or H;
R₇ is C₁₋₆alkyl branched or unbranched or H;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ and NR₁₃; and wherein said ring is optionally substituted by C₁₋₃ alkyl, C₁₋₃alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with alkoxy, OH or phenyl;
   or R₁₃ and R₁₄ together form a chain completing a ring, said chain is (CH₂)₄₋₅ or (CH₂)₂O(CH₂)₂; and
m is 1-4, n is 0-3 and p is 0-2.
   One embodiment of the compounds of formula(III) are those wherein:
   Ar₁ is
      a) a cycloalkyl group selected from cyclopropyl, cyclobutyl, cyclopentanyl, cyclohexanyl, cycloheptanyl;
      b) a cycloalkenyl group selected from cyclopentenyl, cyclohexenyl, cycloheptenyl;
      c) phenyl, naphthyl; indanyl, indenyl, dihydronaphthyl, tetrahydronaphthyl, fluorenyl;
      d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, and indazolyl, or a fused heteroaryl selected from cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
      e) a heterocycle selected from: pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
      wherein each of the above Ar₁ are optionally substituted by one or more R₁, R₂ and R₃ as hereinabove defined;
   Rₐ is H, C₁₋₆alkyl, C₂₋₅alkenyl, C₂₋₅alkynyl, phenyl or heteroaryl selected from: pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₃ is H, halogen, methyl, methoxy, hydroxymethyl or OH;
R₆ is C₁₋₃alkyl or H;
R₇ is C₁₋₆alkyl branched or unbranched or H;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl or phenyl are optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH or -(CH₂)ₙNR₁₃R₁₄;
R₁₂ is H or C₁₋₆alkyl optionally substituted with phenyl, OH, C₁₋₃alkoxy or NR₁₃R₁₄;
R₁₃ and R₁₄ are each independently selected from H and C₁₋₆ alkyl optionally substituted with C₁₋₃alkoxy, OH or phenyl;
   and
   or R₁₃ and R₁₄ together form a chain completing a ring, said chain is (CH₂)₄₋₅ or (CH₂)₂O(CH₂)₂.
   Another embodiment of the compounds of the formula(III) are those described immediately above, and wherein:
   Ar₁ is phenyl, or pyridyl;
   X is NH or N-CH₃;
   Y is NH and
   Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
   R₁ and R₂ are the same or different and selected from: halogen, C₁₋₃ alkyl, wherein the C₁₋₃ alkyl are optionally partially or fully halogenated, NO₂, NR₁₃R₁₄;
   R₃ is H, halogen, methoxy or methyl;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
   or R₁₃ and R₁₄ together form a chain completing a ring, said chain is (CH₂)₄₋₅ or (CH₂)₂O(CH₂)₂.
   In yet another embodiment of the compounds of fonmula(III) are those described immediately above, and wherein:
   Ar₁ is phenyl;
   Rₐ is H or hydroxymethyl;
   R₁ and R₂ are the same or different and selected from: halogen, methyl optionally partially or fully halogenated, NO₂ and NH₂;
   R₃ is H, chloro, fluoro, bromo or methoxy;
   R₁₀ and R₁₁ may be the same or different and are each independently selected from H, OH, methoxy, C₁₋₃alkyl branched or unbranched, optionally substituted with OH, NR₁₃R₁₄ or phenyl;
      or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

In a further embodiment of the invention, there are provided the following compounds of the fomulas (I) and (Ia):
2-(2,6-Dichlorophenylamino)-6,7-dimethyl-1,8-dihydro-imidazo[4,5-*h*]isoquinoline-9-one;
2-(2,6-Dichlorophenylamino)-3,5-dihydro-imidazo[4,5-i]phenanthridin-4-one;
2-(2,6-Dichlorophenylamino)-1,6,7-trimethyl-1,8-dihydro-imidazo[4,5-*h*]isoquinoline-9-one;
2-(2,6-Dichlorophenylamino)-7-methyl-1,8-dihydro-imidazo[4,5-*h*]isoquinoline-9-one;
2-(2,6-Dichlorophenylamino) -1,7-dimethyl-9-oxo-1,8-dihydro-imidazo[4,5-*h*]isoquinolin-6-acetic acid ethyl ester;
3-[2-(2,6-Dichlorophenylamino)-1,6-dimethyl-9-oxo-8,9-dihydro-1H-imidazo[4,5-*h*]isoquinolin-7-yl]-acrylic acid methyl ester;
2-(2,6-Dichlorophenylamino)-1,7-dimethyl-6-(2-hydroxyethyl)-1,8-dihydro-imidazo[4,5-*h*]isoquinoline-9-one;
2-(2,6-Dichlorophenylamino)-1,7-dimethyl-9-oxo-1,8-dihydro-imidazo[4,5-*h*]isoquinoline-6- carboxylic acid methyl ester;
2-(2,6-Dichlorophenylamino)-1,7-dimethyl-1,8-dihydro-imidazo[4,5-*h*]isoquinoline-9-one;
3-[2-(2,6-Dichlorophenylamino)-1-methyl-9-oxo-8,9-dihydro-1H-imidazo[4,5-*h*]isoquinolin-7-yl]-acrylic acid methyl ester;
3-[2-(2,6-Dichlorophenylamino) -1,7-dimethyl-9-oxo-1,8-dihydro-imidazo[4,5-h]isoquinolin-6-yl]propionic acid ethyl ester
*N*-Benzyl-*N*-methyl-2-[(2,6-dichlorophenylamino)-1,7-dimethyl-9-oxo-1,8-dihydro-imidazo[4,5-*h*]isoquinolin-6-yl] acetamide;
2-(2,6-Dichlorophenylamino)-1,7-dimethyl-6-(2-morpholin-4-ylethyl)-1,8-dihydro-imidazo[4,5-*h*]isoquinoline-9-one;
2-(2-Chloro-6-methylphenylamino)-1,6,7-trimethyl-1,8-dihydro-imidazo[4,5-*h*]isoquinoline-9-one;
2-(4-Bromo-2-dichlorophenylamino)-1,6,7-trimethyl-1,8-dihydro-imidazo[4,5-*h*]isoquinoline-9-one;
3-[2-(2,6-Dichlorophenylamino)-1-methyl-9-oxo-8,9-dihydro-1H-imidazo[4,5-*h*]isoquinolin-7-yl]-N-methoxy-N-methylacrylamide;
2-(2-Chloro-6-nitrophenylamino)-1,6,7-trimethyl-1,8-dihydro-imidazo[4,5-*h*]isoquinoline-9-one;
N-Benzyl-3-[2-(2,6-Dichlorophenylamino)-1-methyl-9-oxo-8,9-dihydro-1H-imidazo[4,5-*h*]isoquinolin-7-yl]-acrylamide;
3-[2-(2,6-Dichlorophenylamino)-1-methyl-9-oxo-8,9-dihydro-1H-imidazo[4,5-*h*]isoquinolin-7-yl]-acrylic acid 4-morpholine amide;
2-(2,6-Dichlorophenylamino)-1,7-dimethyl -6-[3-(4-morpholino)propyl]-1,8-dihydro-imidazo[4,5-*h*]isoquinoline-9-one;
2-(2,6-Dichlorophenylamino)-4-hydroxymethyl-1,6,7-trimethyl-1,8-dihydro-imidazo[4,5-h]isoquinoline-9-one;
2-(2,6-Dimethylphenylamino)-1,6,7-trimethyl-1,8-dihydro-imidazo[4,5-*h*]isoquinoline-9-one;
2-(2-Ethyl-6-methylphenylamino)-1,6,7-trimethyl-1,8-dihydro-imidazo[4,5-*h*]isoquinoline-9-one;
2-(2,6-Dichlorophenylamino)-1,7-dimethyl-6-(3-phenylaminopropyl)-1,8-dihydro-imidazo[4,5-*h*]isoquinoline-9-one;
2-(2,6-Dichlorophenylamino-6-{3-[4-(2-diethylaminoethoxy)-phenylamino]propyl}-1,7-dimethyl-1,8-dihydro-imidazo[4,5-*h*]isoquinoline-9-one;
2-(2-Bromo-6-chloro-4-fluorophenylamino)-1,6,7-trimethyl-1,8-dihydro-imidazo[4,5-*h*]isoquinoline-9-one;
2-(2,6-Dichlorophenylamino)-4-(2-hydroxyethylaminomethyl)-1,6,7-trimethyl-1,8-dihydro-imidazo[4,5-*h*]isoquinoline-9-one;
2-(2,6-Dichlorophenylamino)-4-(4-methoxybenzylaminomethyl)-1,6,7-trimethyl-1,8-dihydro-imidazo[4,5-*h*]isoquinoline-9-one;
2-(2,6-Dichlorophenylamino)-1,6-dimethyl-7-vinyl-1,8-dthydro-imidazo[4,5-*h*]isoquinoline-9-one;
2-(2,6-Dichlorophenylamino)-4-(2,6-difluoropyridin-3yl)-1,6,7-trimethyl-1,8-dihydro-imidazo[4,5-*h*]isoquinoline-9-one;
2-(2,6-Dichlorophenylamino)-4-(3-methylphenyl)-1,6,7-trimethyl-1,8-dihydro-imidazo[4,5-*h*]isoquinoline-9-one;
2-(2,6-Dichlorophenylamino)-1,7-dimethyl-9-oxo-1,8-dihydro-imidazo[4,5-*h*]isoquinoline-6- carboxylic acid 2-(4-moropholino)ethyl ester;
2-(2,6-Dichlorophenylamino)-4-(3-hydroxy-2-oxo-propyl)-1,6,7-trimethyl-1,8-dihydro-imidazo[4,5-*h*]isoquinoline-9-one;
N-4-(2-Diethylaminoethoxy)phenyl-3-[2-(2,6-dichlorophenylamino)-1-methyl-9-oxo-8,9-dihydro-1H-imidazo[4,5-*h*]isoquinolin-7-yl]-acrylamide;
3-[2-(2,6-Dichlorophenylamino)-1-methyl-9-oxo-8,9-dihydro-1H-imidazo[4,5-h]isoquinolin-7-yl]-N-methyl acrylamide;
9- Hydroxy-2-(2,6-dichlorophenylamino)-3,5,6,7,8,9-hexahydro-imidazo[4,5-i]phenanthridin-4-one;
2-(2,6-Dichlorophenylamino)- 1,6-dimethyl-7-(3-hydroxypropen-1-yl)-1,8-dihydro-imidazo[4,5-*h*]-isoquinolin-9-one;
2-(2,6-Dichlorophenylamino)-1,6-dimethyl-7-(2-phenylethenyl)-1,8-dihydro-imidazo[4,5-*h*]-isoquinolin-9-one;
2-(2-Amino-6-chlorophenylamino)-1,6,7-trimethyl-1,8-dihydro-imidazo[4,5-*h*]isoquinoline-9-one;
2-(2,6-Dichlorophenylamino)-1,6,7-trimethyl-4-vinyl-1,8-dihydro-imidazo[4,5-*h*]isoquinoline-9-one;
2-(2,6-Dichlorophenylamino)-4-(3-methoxypropyn-1-yl)-1,6,7-trimethyl-1,8-dihydro-imidazo[4,5-*h*]isoquinoline-9-one;
2-(2,6-Dichlorophenylamino)-1,6,7-trimethyl-4-(5-phenylpent-1-ynyl)-1,8-dihydro-imidazo[4,5-*h*]isoquinoline-9-one;
2-(2,6-Dichlorophenylamino)- 1,6-dimethyl-7-oxazol-5-yl-1,8-dihydro-imidazo[4,5-*h*]isoquinolin-9-one;
2-(2,6-Dichlorophenylamino)-1-methyl-7-vinyl-1,8-dihydro-imidazo[4,5-*h*]isoquinoline-9-one;
2-(2,6-Dichlorophenylamino)-1,6-dimethyl-7-(3-morpholin-4-yl-propen-1-yl)- 1,8-dihydro-imidazo[4,5-*h*]-isoquinolin-9-one;
2-(2,6-Dichlorophenylamino)-1,7-dimethyl-6-[2-(2-hydroxyethyl)aminoethyl]-1,8-dihydro-imidazo[4,5-*h*]isoquinoline-9-one;
3-[2-(2,6-Dichlorophenylamino)-1,7-dimethyl-9-oxo-8,9-dihydro-1H-imidazo[4,5-*h*]isoquinolin-7-yl]-acrylonitrile;
2-(2-Chloro-6-methylphenylamino)-1,7-dimethyl-1,8-dihydro-imidazo[4,5-*h*]isoquinoline-9-one;
2-(2,6-Dichlorophenylamino)-1-methyl-7-oxazol-5-yl-1,8-dihydro-imidazo[4,5-*h*]isoquinolin-9-one;
2-(2,6-Dichlorophenylamino)-1,7-dimethyl-6-(3-hydroxypropyl)-1,8-dihydro-imidazo[4,5-*h*]isoquinoline-9-one;
2-(2,6-Dichlorophenylamino)-7-(3-hydroxypropen-1-yl)-1-methyl-1,8-dihydro-imidazo[4,5-*h*]-isoquinolin-9-one;
2-(2-Chloro-6-methylphenylamino)-7-(3-hydroxypropen-1-yl)-1-methyl-1,8-dihydro-imidazo[4,5-*h*]-isoquinolin-9-one;
2-(2,6-Dichlorophenylamino)-7-(3-diethylaminopropen-1-yl)-1,6-dimethyl-1,8-dihydro-imidazo[4,5-*h*]-isoquinolin-9-one;
7-(3-Aminopropen-1-yl)-2-(2,6-dichlorophenylamino)-1,6-dimethyl-1,8-dihydro-imidazo[4,5-*h*]-isoquinolin-9-one;
2-(2,6-Dichlorophenylamino)-1,6-dimethyl-7-(3-pyrrolidin-1-yl-propen-1-yl)-1,8-dihydro-imidazo[4,5-*h*]-isoquinolin-9-one;
7-(3-Benzylmethylaminopropen-1-yl)2-(2,6-dichlorophenylamino)-1,6-dimethyl-1,8-dihydro-imidazo[4,5-*h*]-isoquinolin-9-one;
2-(2,6-Dichloro-4-methoxyphenylamino)-1,6,7-trimethyl-1,8-dihydro-imidazo[4,5-*h*]isoquinoline-9-one;
2-(2,6-Dichloro-4-methoxyphenylamino)-1,6-dimethyl-7-oxazol-5-yl-1,8-dihydro-imidazo[4,5-*h*]isoquinolin-9-one;
2-(2,6-Dichlorophenylamino)-7-(3-diethylaminopropen-1-yl)-1-methyl-1,8-dihydro-imidazo[4,5-*h*]-isoquinolin-9-one;
2-(2,6-Dimethylphenylamino)-1,7-dimethyl-1,8-dihydro-imidazo[4,5-*h*]isoquinoline-9-one;
2-(2,6-Dichlorophenylamino)-1,6-dimethyl-7-(4-methylpiperazin-1-yl-propen-1-yl)-1,8-dihydro-imidazo[4,5-*h*]-isoquinolin-9-one;
2-(2,6-Dichlorophenylamino)-1,6-dimethyl-7-(3-piperidin-1-yl-propen-1-yl)- 1,8-dihydro-imidazo[4,5-*h*]-isoquinolin-9-one;
2-(2,6-Dichlorophenylamino)-1,6-dimethyl-7-{3-[ethyl(2-hydroxyethyl)amino]propen-1-yl}-1,8-dihydro-imidazo[4,5-*h*]-isoquinolin-9-one;
2-(2,6-Dichlorophenylamino)-1,6-dimethyl-7-[3-(3-hydroxypyrrolidin-1-yl)-propen-1-yl]- 1,8-dihydro-imidazo[4,5-*h*]-isoquinolin-9-one;
7-(3-Dibutylaminopropen-1-yl)-2-(2,6-dichlorophenylamino)-1,6-dimethyl-1,8-dihydro-imidazo[4,5-*h*]-isoquinolin-9-one;
2-(2,6-Dichlorophenylamino)-1,6-dimethyl-7-{3-[(2-methoxyethyl)methylamino]propen-1-yl}-1,8-dihydro-imidazo[4,5-*h*]-isoquinolin-9-one;
7-(3-Diethylaminopropen-1-yl)-1,6-dimethyl-2-(2,6-dimethylphenylamino)-1,8-dihydro-imidazo[4,5-*h*]-isoquinolin-9-one;
2-(2,6-Dichlorophenylamino)-7-{3-[(2-diethylaminoethyl)methylamino]-propen-1-yl}-1,6-dimethyl-1,8-dihydro-imidazo[4,5-*h*]-isoquinolin-9-one;
7-(3-Diethylaminopropen-1-yl)-1,6-dimethyl-2-(2,4,6-trichlorophenylamino)-1,8-dihydro-imidazo[4,5-h]-isoquinolin-9-one
2-(2,6-Dichlorophenylamino)-6-methyl-7-oxazol-5-yl-1,8-dihydro-imidazo[4,5-h]isoquinolin-9-one
2-(2,6-Dichlorophenylamino)-1,6-dimethyl-7-[3-(2-pyrrolidin-1-ylmethylpyrrolidin-1-yl)-propen-1-yl]-1,8-dihydro-imidazo[4,5-*h*]-isoquinolin-9-one
7-[3-(2*S*-Aminomethylpyrrolidin-1-yl)-propen-1-yl]-2-(2,6-dichlorophenylamino)-1,6-dimethyl-1,8-dihydro-imidazo[4,5-*h*]-isoquinolin-9-one
1-{3-[2-(2,6-Ddichlorophenylamino)-1,6-dimethyl-9-oxo-8,9-dihydro-1H-imidazo[4,5-h]isoquinolin-7-yl]-propenyl}-*L*-proline carboxamide
1-{3-[2-(2,6-dichlorophenylamino)-1,6-dimethyl-9-oxo-8,9-dihydro-1H-imidazo[4,5-h]isoquinolin-7-yl]-propenyl}-piperidine-3-carboxamide
2-(2,6-Dichlorophenylamino)-1,6-dimethyl-7-(methylhydrazonomethyl)-1,8-dihydro-imidazo[4,5-h]-isoquinolin-9-one
7-[3-(3-Aminopyrrolidin-1-yl)-propen-1-yl]-2-(2,6-dichlorophenylamino)-1,6-dimethyl-1,8-dihydro-imidazo[4,5-*h*]-isoquinolin-9-one
2-(2,6-Dichlorophenylamino)-1,6-dimethyl-7-[3-(3-acetamidopyrrolidin-1-yl)-propen-1-yl]- 1,8-dihydro-imidazo[4,5-*h*]-isoquinolin-9-one
2-(2,6-Dichlorophenylamino)-1,6-dimethyl-7-[3-(3-dimethylaminopyrrolidin-1-yl)-propen-1-yl]- 1,8-dihydro-imidazo[4,5-*h*]-isoquinolin-9-one
1-{3-[2-(2,6-Dichlorophenylamino)-1,6-dimethyl-9-oxo-8,9-dihydro-1H-imidazo[4,5-h]isoquinolin-7-yl]-propenyl}-piperidine-2-carboxamide
7-[3-(3-Aminomethylpiperidin-1-yl)-propen-1-yl]-2-(2,6-dichlorophenylamino)-1,6-dimethyl-1,8-dihydro-imidazo[4,5-*h*]-isoquinolin-9-one
1-{3-[2-(2,6-Dichlorophenylamino)-1,6-dimethyl-9-oxo-8,9-dihydro-1H-imidazo[4,5-h]isoquinolin-7-yl]-propenyl}-piperidine-3-carboxylic acid diethylamide
2-(2,6-Dichlorophenylamino)-1,6-dimethyl-7-ethynyl-1,8-dihydro-imidazo[4,5-h]-isoquinolin-9-one
1-{3-[2-(2,6-dichlorophenylamino)-1,6-dimethyl-9-oxo-8,9-dihydro-1H-imidazo[4,5-h]isoquinolin-7-yl]-propenyl}-3-methyl urea
Cyclohexane carboxylic acid {3-[2-(2,6-dichlorophenylamino)-1,6-dimethyl-9-oxo-8,9-dihydro-1H-imidazo[4,5-h]isoquinolin-7-yl]-propenyl}amide
2-(2,6-Dichlorophenylamino)-1-methyl-7-phenyl-1,8-dihydro-imidazo[4,5-h]isoquinolin-9-one
*N*-{3-[2-(2,6-Dichlorophenylamino)-1,6-dimethyl-9-oxo-8,9-dihydro-1*H*-imidazo[4,5-h]isoquinolin-7-yl]-propenyl}methanesulfonamide
3-[2-(2,6-dichlorophenylamino)-1,6-dimethyl-9-oxo-8,9-dihydro-1H-imidazo[4,5-h]isoquinolin-7-yl]-propenyl urea
1-Cyclohexyl-3-{3-[2-(2,6-dichlorophenylamino)-1,6-dimethyl-9-oxo-8,9-dihydro-1H-imidazo[4,5-h]isoquinolin-7-yl]-propenyl}-urea
*N*-{3-[2-(2,6-Dichlorophenylamino)-1,6-dimethyl-9-oxo-8,9-dihydro-1*H*-imidazo[4,5-h]isoquinolin-7-yl]-propenyl} benzenesulfonamide
2-(2,6-Dichlorophenylamino)-1,6-dimethyl-7-(3-ethylaminopropen-1-yl)-1,8-dihydro-imidazo[4,5-h]-isoquinolin-9-one
*N*-{3-[2-(2,6-Dichlorophenylamino)-1,6-dimethyl-9-oxo-8,9-dihydro-1*H*-imidazo[4,5-h]isoquinolin-7-yl]-propenyl}-guanidine
Piperidine-3-carboxylic acid {3-[2-(2,6-dichlorophenylamino)-1,6-dimethyl-9-oxo-8,9-dihydro-1H-imidazo[4,5-h]isoquinolin-7-yl]-propenyl}amide
*L*-Proline {3-[2-(2,6-dichlorophenylamino)-1,6-dimethyl-9-oxo-8,9-dihydro-1H-imidazo[4,5-h]isoquinolin-7-yl]-propenyl}amide
*D*-Proline {3-[2-(2,6-dichlorophenylamino)-1,6-dimethyl-9-oxo-8,9-dihydro-1H-imidazo[4,5-h]isoquinolin-7-yl]-propenyl}amide
3-[2-(2,6-Dichlorophenylamino)-1,6-dimethyl-9-oxo-8,9-dihydro-1H-imidazo[4,5-h]isoquinolin-7-yl]-benzamide
*L*-Azetidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl}amide
Piperidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl}amide; and
the pharmacuetically acceptable derivatives thereof.

In yet still a further embodiment of the invention, there are provided the following compounds of the fomulas (I) and (Ia):
2-(2,6-Dichlorophenylamino)-3,5-dihydro-imidazo[4,5-i]phenanthridin-4-one;
2-(2,6-Dichlorophenylamino)-1,6,7-trimethyl-1,8-dihydro-imidazo[4,5-h]isoquinoline-9-one;
2-(2,6-Dichlorophenylamino)-1,7-dimethyl-6-(2-hydroxyethyl)-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6- carboxylic acid methyl ester;
3-[2-(2,6-Dichlorophenylamino)-1-methyl-9-oxo-8,9-dihydro-1H-imidazo[4,5-h]isoquinolin-7-yl]-acrylic acid methyl ester;
2-(2-Chloro-6-methylphenylamino)-1,6,7-trimethyl-1,8-dihydro-imidazo[4,5-h]isoquinoline-9-one;
3-[2-(2,6-Dichlorophenylamino)-1-methyl-9-oxo-8,9-dihydro-1H-imidazo[4,5-h]isoquinolin-7-yl]-N-methoxy-N-methylacrylamide;
2-(2-Chloro-6-nitrophenylamino)-1,6,7-trimethyl-1,8-dihydro-imidazo[4,5-h]isoquinoline-9-one;
N-Benzyl-3-[2-(2,6-Dichlorophenylamino)-1-methyl-9-oxo-8,9-dihydro-1H-imidazo[4,5-h]isoquinolin-7-yl]-acrylamide;
3-[2-(2,6-Dichlorophenylamino)-1-methyl-9-oxo-8,9-dihydro-1H-imidazo[4,5-h]isoquinolin-7-yl]-acrylic acid 4-morpholine amide;
2-(2,6-Dichlorophenylamino)-4-hydroxymethyl-1,6,7-trimethyl-1,8-dihydro-imidazo[4,5-h]isoquinoline-9-one;
2-(2,6-Dichlorophenylamino)-1,6-dimethyl-7-vinyl-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6- carboxylic acid 2-(4-moropholino)ethyl ester;
2-(2,6-Dichlorophenylamino)-1,6-dimethyl-7-(3-hydroxypropen-1-yl)-1,8-dihydro-imidazo[4,5-h]-isoquinolin-9-one;
2-(2,6-Dichlorophenylamino)-1,6-dimethyl-7-oxazol-5-yl-1,8-dihydro-imidazo[4,5-h]isoquinolin-9-one;
2-(2,6-Dichlorophenylamino)-1-methyl-7-vinyl-1,8-dihydro-imidazo[4,5-h]isoquinoline-9-one;
2-(2,6-Dichlorophenylamino)-1,6-dirriethyl-7-(3-morpholin-4-yl-propen-1-yl)-1,8-dihydro-imidazo[4,5-h]-isoquinolin-9-one;
3-[2-(2,6-Dichlorophenylamino)-1,7-dimethyl-9-oxo-8,9-dihydro-1H-imidazo[4,5-h]isoquinolin-7-yl]-acrylonitrile;
2-(2-Chloro-6-methylphenylamino)-1,7-dimethyl-1,8-dihydro-imidazo[4,5-h]isoquinoline-9-one;
2-(2,6-Dichlorophenylamino)-1-methyl-7-oxazol-5-yl-1,8-dihydro-imidazo[4,5-h]isoquinolin-9-one;
2-(2,6-Dichlorophenylamino)-7-(3-hydroxypropen-1-yl)-1-methyl-1,8-dihydro-imidazo[4,5-h]-isoquinolin-9-one;
2-(2-Chloro-6-methylphenylamino)-7-(3-hydroxypropen-1-yl)-1-methyl-1,8-dihydro-imidazo[4,5-h]-isoquinolin-9-one;
2-(2,6-Dichlorophenylamino)-7-(3-diethylaminopropen-1-yl)-1,6-dimethyl-1,8-dihydro-imidazo[4,5-h]-isoquinolin-9-one;
2-(2,6-Dichlorophenylamino)-1,6-dimethyl-7-(3-pyrrolidin-1-yl-propen-1-yl)- 1,8-dihydro-imidazo[4,5-h]-isoquinolin-9-one;
2-(2,6-Dichlorophenylamino)-7-(3-diethylaminopropen-1-yl)-1-methyl-1,8-dihydro-imidazo[4,5-h]-isoquinolin-9-one;
2-(2,6-Dichlorophenylamino)-1,6-dimethyl-7-(4-methylpiperazin-1-yl-propen-1-yl)-1,8-dihydro-imidazo[4,5-h]-isoquinolin-9-one;
2-(2,6-Dichlorophenylamino)-1,6-dimethyl-7-(3-piperidin-1-yl-propen-1-yl)- 1,8-dihydro-imidazo[4,5-h]-isoquinolin-9-one;
2-(2,6-Dichlorophenylamino)-1,6-dimethyl-7-{3-[ethyl(2-hydroxyethyl)amino]propen-1-yl}-1,8-dihydro-imidazo[4,5-h]-isoquinolin-9-one;
7-(3-Diethylaminopropen-1-yl)-1,6-dimethyl-2-(2,6-dimethylphenylamino)-1,8-dihydro-imidazo[4,5-h]-isoquinolin-9-one;
2-(2,6-Dichlorophenylamino)-7-{3-[(2-diethylaminoethyl)methylamino]-propen-1-yl}-1,6-dimethyl-1,8-dihydro-imidazo[4,5-h]-isoquinolin-9-one;
7-(3-Diethylaminopropen-1-yl)-1,6-dimethyl-2-(2,4,6-trichlorophenylamino)-1,8-dihydro-imidazo[4,5-h]-isoquinolin-9-one
2-(2,6-Dichlorophenylamino)-6-methyl-7-oxazol-5-yl-1,8-dihydro-imidazo[4,5-h]isoquinolin-9-one
2-(2,6-Dichlorophenylamino)-1,6-dimethyl-7-[3-(2-pyrrolidin-1-ylmethylpyrrolidin-1-yl)-propen-1-yl]-1,8-dihydro-imidazo[4,5-*h*]-isoquinolin-9-one
7-[3-(2S-Aminomethylpyrrolidin-1-yl)-propen-1-yl]-2-(2,6-dichlorophenylamino)-1,6-dimethyl-1,8-dihydro-imidazo[4,5-*h*]-isoquinolin-9-one
1-{3-[2-(2,6-Ddichlorophenylamino)-1,6-dimethyl-9-oxo-8,9-dihydro-1H-imidazo[4,5-h]isoquinolin-7-yl]-propenyl}-*L*-proline carboxamide
1-{3-[2-(2,6-dichlorophenylamino)-1,6-dimethyl-9-oxo-8,9-dihydro-1H-imidazo[4,5-h]isoquinolin-7-yl]-propenyl}-piperidine-3-carboxamide
2-(2,6-Dichlorophenylamino)-1,6-dimethyl-7-(methylhydrazonomethyl)-1,8-dihydro-imidazo[4,5-h]-isoquinolin-9-one
7-[3-(3-Aminopyrrolidin-1-yl)-propen-1-yl]-2-(2,6-dichlorophenylamino)-1,6-dimethyl-1,8-dihydro-imidazo[4,5-*h*]-isoquinolin-9-one
2-(2,6-Dichlorophenylamino)-1,6-dimethyl-7-[3-(3-acetamidopyrrolidin-1-yl)-propen-1-yl]- 1,8-dihydro-imidazo[4,5-*h*]-isoquinolin-9-one
2-(2,6-Dichlorophenylamino)-1,6-dimethyl-7-[3-(3-dimethylaminopyrrolidin-1-yl)-propen-1-yl]-1,8-dihydro-imidazo[4,5-*h*]-isoquinolin-9-one
1- {3-[2-(2,6-Dichlorophenylamino)-1,6-dimethyl-9-oxo-8,9-dihydro-1H-imidazo[4,5-h]isoquinolin-7-yl]-propenyl}-piperidine-2-carboxamide
7-[3-(3-Aminomethylpiperidin-1-yl)-propen-1-yl]-2-(2,6-dichlorophenylamino)-1,6-dimethyl-1,8-dihydro-imidazo[4,5-*h*]-isoquinolin-9-one
1-{3-[2-(2,6-Dichlorophenylamino)-1,6-dimethyl-9-oxo-8,9-dihydro-1H-imidazo[4,5-h]isoquinolin-7-yl]-propenyl}-piperidine-3-carboxylic acid diethylamide
2-(2,6-Dichlorophenylamino)- 1,6-dimethyl-7-ethynyl-1,8-dihydro-imidazo[4,5-h]-isoquinolin-9-one
1-{3-[2-(2,6-dichlorophenylamino)-1,6-dimethyl-9-oxo-8,9-dihydro-1H-imidazo[4,5-h]isoquinolin-7-yl]-propenyl}-3-methyl urea
Cyclohexane carboxylic acid {3-[2-(2,6-dichlorophenylamino)-1,6-dimethyl-9-oxo-8,9-dihydro-1H-imidazo[4,5-h]isoquinolin-7-yl]-propenyl}amide
2-(2,6-Dichloropbenylamino)-1-methyl-7-phenyl-1,8-dihydro-imidazo[4,5-h]isoquinolin-9-one
*N*-{3-[2-(2,6-Dichlorophenylamino)-1,6-dimethyl-9-oxo-8,9-dihydro-1*H*-imidazo[4,5-h]isoquinolin-7-yl]-propenyl} methanesulfonamide
3-[2-(2,6-Dichlorophenylamino)-1,6-dimethyl-9-oxo-8,9-dihydro-1H-imidazo[4,5-h]isoquinolin-7-yl]-propenyl urea
1-Cyclohexyl-3-{3-[2-(2,6-dichlorophenylamino)-1,6-dimethyl-9-oxo-8,9-dihydro-1H-imidazo[4,5-h]isoquinolin-7-yl]-propenyl}-urea
*N*-{3-[2-(2,6-Dichlorophenylamino)-1,6-dimethyl-9-oxo-8,9-dihydro-1*H*-imidazo[4,5-h]isoquinolin-7-yl]-propenyl} benzenesulfonamide
2-(2,6-Dichlorophenylamino)-1,6-dimethyl-7-(3-ethylaminopropen-1-yl)-1,8-dihydro-imidazo[4,5-h]-isoquinolin-9-one
*N*-{3-[2-(2,6-Dichlorophenylamino)-1,6-dimethyl-9-oxo-8,9-dihydro-1*H*-imidazo[4,5-h]isoquinolin-7-yl]-propenyl}-guanidine
Piperidine-3-carboxylic acid {3-[2-(2,6-dichlorophenylamino)-1,6-dimethyl-9-oxo-8,9-dihydro-1H-imidazo[4,5-h]isoquinolin-7-yl]-propenyl}amide
*L*-Proline {3-[2-(2,6-dichlorophenylamino)-1,6-dimethyl-9-oxo-8,9-dihydro-1H-imidazo[4,5-h]isoquinolin-7-yl]-propenyl}amide
*D*-Proline {3-[2-(2,6-dichlorophenylamino)-1,6-dimethyl-9-oxo-8,9-dihydro-1H-imidazo[4,5-h]isoquinolin-7-yl]-propenyl}amide
3-[2-(2,6-Dichlorophenylamino)-1,6-dimethyl-9-oxo-8,9-dihydro-1H-imidazo[4,5-h]isoquinolin-7-yl]-benzamide
*L*-Azetidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl}amide
Piperidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl}amide; and
the pharmacuetically acceptable derivatives thereof.

Any compounds of this invention containing one or more asymmetric carbon atoms may occur as racemates and racemic mixtures, single enantiomers, diastereomeric mixtures and individual diastereomers. All such isomeric forms of these compounds are expressly included in the present invention. Each stereogenic carbon may be in the R or S configuration, or a combination of configurations.

Some of the compounds of the invention can exist in more than one tautomeric form. The invention includes all such tautomers.

The compounds of the invention are only those which are contemplated to be 'chemically stable' as will be appreciated by those skilled in the art. For example, a compound which would have a 'dangling valency', or a 'carbanion' are not compounds contemplated by the invention.

All terms as used herein in this specification, unless otherwise stated, shall be understood in their ordinary meaning as known in the art. For example, "C₁₋₆alkoxy" is a C₁₋₆alkyl with a terminal oxygen, such as methoxy, ethoxy, propoxy, pentoxy and hexoxy. All alkyl, alkylene or alkynyl groups shall be understood as being branched or unbranched unless otherwise specified. Other more specific definitions are as follows:

The term "halogen" as used in the present specification shall be understood to mean bromine, chlorine, fluorine or iodine.

The term "heteroaryl" refers to a stable 5-8 membered (but preferably, 5 or 6 membered) monocyclic or 8-11 membered bicyclic aromatic heterocycle radical. Each heterocycle consists of carbon atoms and from 1 to 4 heteroatoms chosen from nitrogen, oxygen and sulfur. The heterocycle may be attached by any atom of the cycle, which results in the creation of a stable structure. Example "heteroaryl" radicals include, pyridinyl, pyrimidinyl, pyrazinyl, pyridazinyl, pyrrolyl, imidazolyl, pyrazolyl, thienyl, furyl, isoxazolyl, isothiazolyl, oxazolyl, thiazolyl, oxadiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, 2,4-dioxo-quinazolinyl, imidazo[4,5-*c*]pyridinyl and indazolyl, or a fused heteroaryl such as cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene;

The term "heterocycle" refers to a stable 4-8 membered (but preferably, 5 or 6 membered) monocyclic or 8-11 membered bicyclic heterocycle radical which may be either saturated or unsaturated, and is non-aromatic. Each heterocycle consists of carbon atoms and from 1 to 4 heteroatoms chosen from nitrogen, oxygen and sulfur. The heterocycle may be attached by any atom of the cycle, which results in the creation of a stable structure. Example "heterocycle" radicals include azetidinyl, pyrrolinyl, pyrrolidinyl, pyrazolinyl, pyrazolidinyl, piperidinyl, morpholinyl, thiomorpholinyl, pyranyl, thiopyranyl, piperazinyl,indolinyl, 2,3-dihydrobenzimidazolyl and 2,3-dihydro-1*H*-imidazo[4,5-*c*] pyridinyl. As used herein and throughout this specification, the terms "nitrogen" and "sulfur" and their respective elements symbols include any oxidized form of nitrogen and sulfur and the quaternized form of any basic nitrogen.

The term "aryl" shall be understood to mean a 6-10 membered aromatic carbocycle, "aryl" includes, for example, phenyl and naphthyl; other terms comprising "aryl" will have the same definition for the aryl component, examples of these moieties include: arylalkyl, aryloxy or arylthio.

The term "carbocycle" shall be understood to mean a 3-10 membered aromatic or nonaromatic cyclic carbon chain. Examples of nonaromatic carbocycles include cyclopropyl, cyclobutyl, cyclopentyl and the like. Examples of aromatic carbocycles include the "aryl" compounds as described hereinabove.

The term "acyl" shall be understood to mean an R-(C=O)- moiety wherein R is an alkyl. Examples of R can be a C₁₋₁₀alkyl, saturated or unsaturated, branched or unbranched, or R can be "aryl" as defined hereinabove. "Acyloxy" shall be understood to mean an R-CO₂- group wherein R is as defined in this paragraph.

The invention includes pharmaceutically acceptable derivatives of compounds of the invention. A "pharmaceutically acceptable derivative" refers to any pharmaceutically acceptable salt or ester of a compound of this invention, or any other compound which, upon administration to a patient, is capable of providing (directly or indirectly) a compound of this invention, a pharmacologically active metabolite or pharmacologically active residue thereof.

Pharmaceutically acceptable salts of the compounds of this invention include those derived from pharmaceutically acceptable inorganic and organic acids and bases. Examples of suitable acids include hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, formic, benzoic, malonic, naphthalene-2-sulfonic and benzenesulfonic acids. Other acids, such as oxalic acid, while not themselves pharmaceutically acceptable, may be employed in the preparation of salts useful as intermediates in obtaining the compounds of this invention and their pharmaceutically acceptable acid addition salts. Salts derived from appropriate bases include alkali metal (*e.g.*, sodium), alkaline earth metal (*e.g.*, magnesium), ammonium and N-(C₁-C₄ alkyl)₄⁺ salts.

In addition, the compounds of this invention include prodrugs of compounds of the invention. Prodrugs include those compounds that, upon simple chemical transformation, are modified to produce a compound of the invention. Simple chemical transformations include hydrolysis, oxidation and reduction, enzymatically, metabolically or otherwise.

Specifically, when a prodrug of this invention is administered to a patient, the prodrug may be transformed into a compound of the invention, thereby imparting the desired pharmacological effect.

### General Synthetic Methods

The compounds of the invention may be prepared by the methods described below. Optimum reaction conditions and reaction times may vary depending on the particular reactants used. Unless otherwise specified, solvents, temperatures, pressures and other reaction conditions may be readily selected by one of ordinary skill in the art. Specific procedures are provided in the Synthetic Examples section. Typically, reaction progress may be monitered by thin layer chromatography (TLC) if desired. If desired, intermediates and products may be purified by chromatography on silica gel and/or recrystallization. Starting materials and reagents are either commercially available or may be prepared by one skilled in the art using methods described in the chemical literature.

A general procedure (Method A) that may be used to synthesize compounds of formula (I) is illustrated in Scheme I.

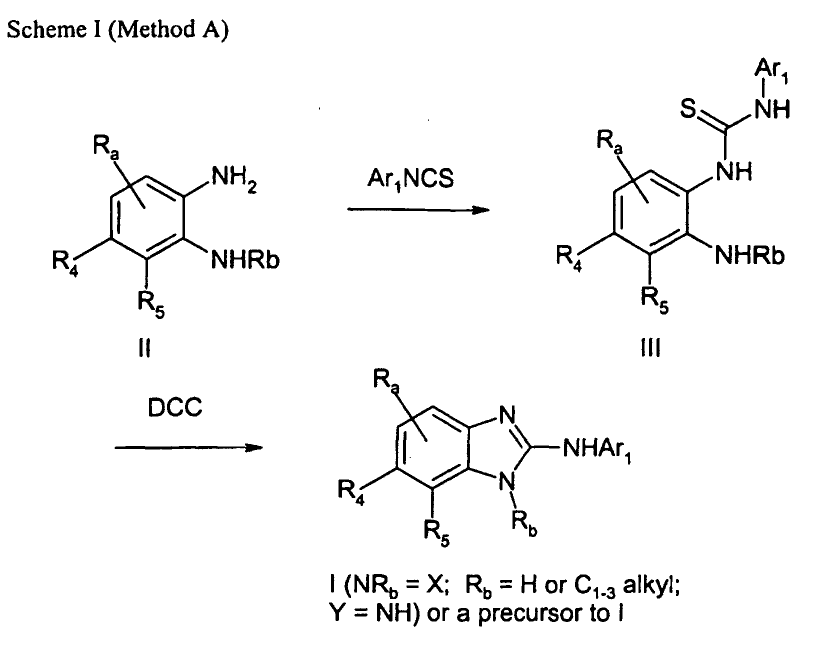

An optionally substituted diamine II is reacted with an aryl isothiocyanate in a suitable solvent such as EtOAc, DMF or THF at about ambient to reflux temperature for about 3 to 24 hr to provide thiourea III. Alternately, one can begin with a salt of II and react with an aryl isothiocyanate in pyridine or in a neutral solvent such as THF in the presence of a suitable base such as triethylamine. Reaction of the thiourea with a suitable activating agent such as 1,3-dicyclohexylcarbodiimide (DCC) or mercuric oxide in a suitable solvent such as THF or DMF at about ambient to reflux temperature provides I or a precursor to I which may undergo further chemical transformation to obtain the desired compound. If desired, one may perform the two steps without isolating the thiourea, by adding DCC or mercuric oxide to the reaction of II and the aryl isothiocyanate.

One may also prepare benzothiazoles (formula I, X = S) by Method A, starting with the analogous aminothiophenol. Preferably, one may also use Method B illustrated in Scheme II and described below.

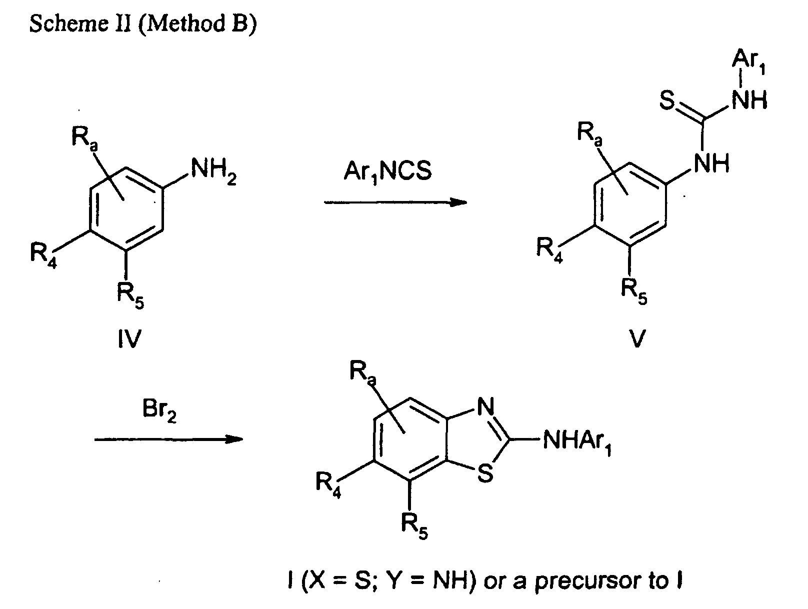

In this method, an appropriately substituted aniline is reacted with an aryl isothiocyanate as in Method A to provide thiourea V. Reaction of V under cyclizing conditions, such as in the presence of bromine in a suitable solvent such as chloroform at about reflux temperature, provides I (X = S) or a precursor to I.

The starting diamine (II) in Method A may be prepared by reduction of a nitroaniline, for example under hydrogen atmosphere in the presence of a suitable catalyst such as palladium on carbon in a suitable solvent, such as EtOAc or HOAc.

One procedure (Method C) for preparing starting nitroanilines is illustrated in Scheme III and described below.

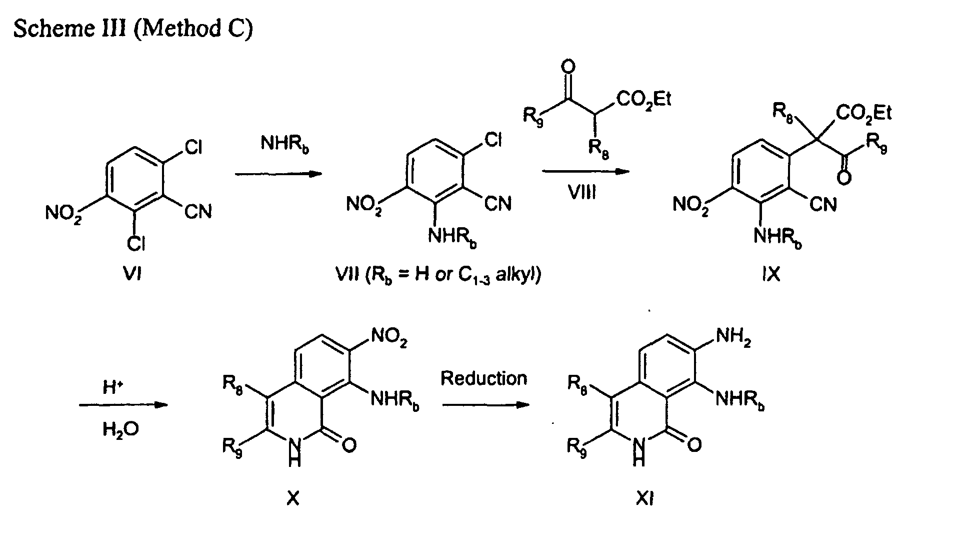

In Method C, 2,6-dichloro-3-nitrobenzonitrile (VI) is reacted with an amine in a suitable solvent, such as EtOH, THF or EtOAc, optionally in a pressure flask and at about 0 to 80 °C, to provide VII. Reaction of VII with keto-ester VIII in the presence of a suitable base, such as K₂CO₃, potassium *t*-butoxide or 1,8-diazabicyclo[5.4.0]undec-7-ene (DBU) in a suitable solvent, such as DMF or DMSO at about ambient temperature provides IX. Hydrolysis and cyclization of IX to provide X is accomplished by reaction with aqueous acid, for example a mixture of acetic acid, sulfuric acid and water at about reflux temperature. Reduction of nitroaniline X, in a suitable solvent, preferably acetic acid and/or trifluoroacetic acid, as described above, provides XI.

In a variation of Method C, one may reduce intermediate IX as described above, to the corresponding diamine and form the benzimidazole by Method A prior to formation of the isoquinolinone.

A procedure for introducing R_{A} into compounds of formula (I) is illustrated in Scheme IV.

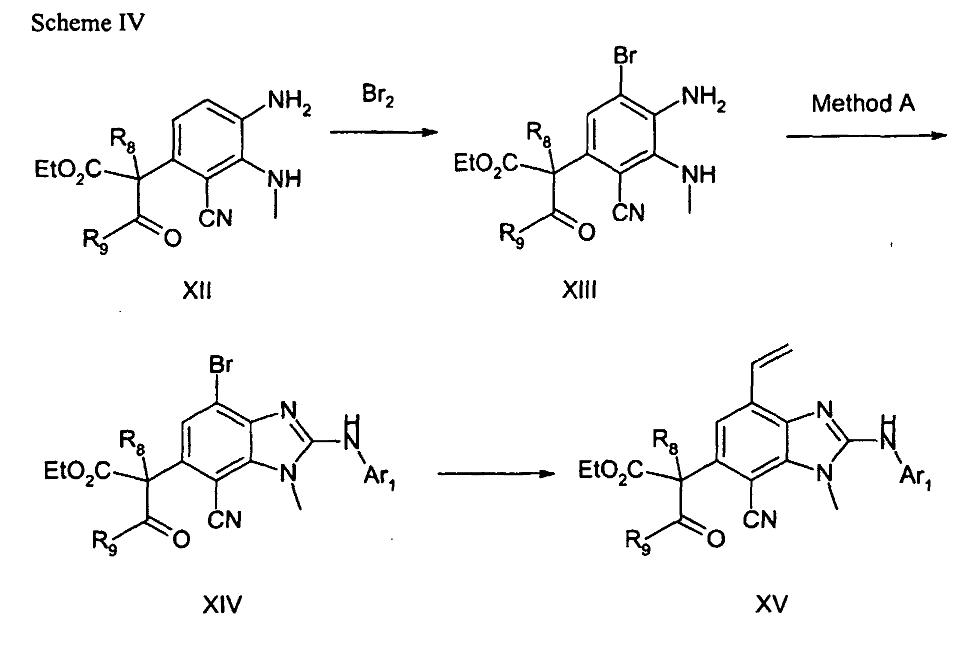

Intermediate XII (prepared as described in Scheme III for preparation of IX, followed by reduction) is reacted with bromine in a suitable solvent, such as chloroform at ambient temperature to provide XIII. Intermediate XIII is converted to XIV according to Method A. Cross-coupling chemistry can be used to introduce carbon in place of bromine. For example, reaction with vinyl tributyltin in the presence of a suitable catalyst, such as (PPh₃)₂PdCl₂, in a suitable solvent, such as 1-methyl-2-pyrrolidinone (NMP) at about 100 °C, provides XV. Alternately, reaction with a terminal alkyne in the presence of a suitable catalyst, such as (PPh₃)₂PdCl₂, and CuI, and a suitable base, such as triethylamine in a solvent such as THF at about ambient temperature provides an alkyne as Rₐ. Other Rₐ may be obtained by transformation of these Rₐ by methods known to those skilled in the art. Several of these transformations are exemplified below.

A method for preparing compounds of the invention in which R₄ and R₅ represent ring B, which is based on the procedure described in *J*. *Heterocyclic Chem.,* **1970**, *7*, 615, is shown in Scheme V.

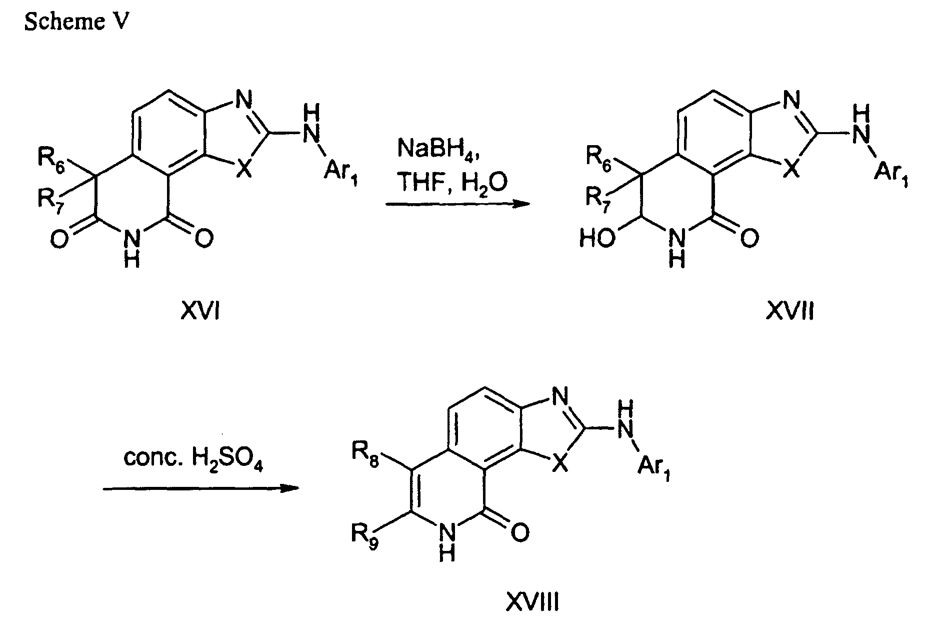

Intermediate XVI (prepared according to Method A or Method B) is reacted with a reducing agent such as sodium borohydride, in a suitable solvent, such as THF or dioxane, at about 0°C to ambient temperature, to give intermediate XVII, in which one carbonyl of the imide has been reduced selectively. Treatment of XVII with a strong acid, such as sulfuric acid, at ambient temperature, causes rearrangement to the isoquinolone XVIII. It will be appreciated that this method is most suitable for compounds where R₆, R₇, R₈ and R₉ are all the same group, preferably methyl. In a variation of this method, the reduction of the imide and rearrangement to the isoquinolone can be carried out prior to forming the benzimidazole ring.

Functional groups at R₈ or R₉ on compounds of formula (I) or intermediates prepared as illustrated in the Schemes above may also be transformed by methods known to those skilled in the art to prepare additional compounds of the invention. Several of these transformations are also exemplified below.

### Methods of Therapeutic Use

The compounds of the invention are useful in inhibiting the activity of src-family kinases and PDGFR kinase. In doing so, the compounds are effective in blocking disease processes mediated by these kinases. For example, by inhibiting p56 lck, the compounds block downstream signaling events following T cell activation by antigen. Activation of antigen-specific T cells is necessary for the induction and progression of diseases, including autoimmune diseases, allergic diseases and transplant rejection (J.H. Hanke et al., *Inflamm*. *Res.,* 1995, *44*, 357). Therefore the compounds of the invention are useful for treating such diseases. These include but are not limited to rheumatoid arthritis, multiple sclerosis, Guillain-Barre syndrome, Crohn's disease, ulcerative colitis, psoriasis, graft versus host disease, systemic lupus erythematosus, insulin-dependent diabetes mellitus and asthma.

In view of their inhibitory effect on src-family kinases and PDGFR kinase, the compounds of the invention are useful in treating cancer. For example, the compounds of the invention are useful in treating src-dependent tumors, such as in mammary carcinoma, colon carcinoma, melanoma and sarcoma, and are also useful in treating PDGF-dependent tumors, such as ovarian cancer, prostate cancer and glioblastoma.

By inhibiting p60src, compounds of the invention may also be useful in treating osteoporosis, Paget's disease, bone inflammation and joint inflammation. By inhibiting PDGFR kinase, compounds of the invention may also be useful in treating fibrotic diseases, restenosis and atherosclerosis. By inhibiting lyn kinase, the compounds of the invention may also be useful in enhancing or potentiating the effectiveness of radiation therapy.

For therapeutic use, the compounds of the invention may be administered in any conventional dosage form in any conventional manner. Routes of administration include, but are not limited to, intravenously, intramuscularly, subcutaneously, intrasynovially, by infusion, sublingually, transdermally, orally, rectally, topically or by inhalation. The preferred modes of administration are oral and intravenous. Compositions comprising the compounds of the invention for each of the aforementioned routes of administration will be apparent to the skilled artisan. For example, one embodiment of the invention provides for pharmaceutical compositions including a pharmaceutically effective amount of the compounds according to the invention. Such pharmaceutical compositions will include pharmaceutically acceptable carriers and adjuvants as further described below.

The compounds of this invention may be administered alone or in combination with adjuvants that enhance stability of the inhibitors, facilitate administration of pharmaceutic compositions containing them in certain embodiments, provide increased dissolution or dispersion, increase inhibitory activity, provide adjunct therapy, and the like, including other active ingredients. Advantageously, such combination therapies utilize lower dosages of the conventional therapeutics, thus avoiding possible toxicity and adverse side effects incurred when those agents are used as monotherapies. Compounds of the invention may be physically combined with the conventional therapeutics or other adjuvants into a single pharmaceutical composition. Advantageously, the compounds may then be administered together in a single dosage form. In some embodiments, the pharmaceutical compositions comprising such combinations of compounds contain at least about 5%, but more preferably at least about 20%, of a compound of formula (I) (w/w) or a combination thereof. The optimum percentage (w/w) of a compound of formula(I) may vary and is within the purview of those skilled in the art. Alternatively, the compounds may be administered separately (either serially or in parallel). Separate dosing allows for greater flexibility in the dosing regime.

As mentioned above, dosage forms of the compounds of this invention include pharmaceutically acceptable carriers and adjuvants known to those of ordinary skill in the art. These carriers and adjuvants include, for example, ion exchangers, alumina, aluminum stearate, lecithin, serum proteins, buffer substances, water, salts or electrolytes and cellulose-based substances. Preferred dosage forms include, tablet, capsule, caplet, liquid, solution, suspension, emulsion, lozenges, syrup, reconstitutable powder, granule, suppository and transdermal patch. Methods for preparing such dosage forms are known (see, for example, H.C. Ansel and N.G. Popovish, *Pharmaceutical Dosage Forms and Drug Delivery Systems,* 5th ed., Lea and Febiger (1990)). Dosage levels and requirements are well-recognized in the art and may be selected by those of ordinary skill in the art from available methods and techniques suitable for a particular patient. In some embodiments, dosage levels range from about 1-1000 mg/dose for a 70 kg patient. Although one dose per day may be sufficient, up to 5 doses per day may be given. For oral doses, up to 2000 mg/day may be required. As the skilled artisan will appreciate, lower or higher doses may be required depending on particular factors. For instance, specific dosage and treatment regimens will depend on factors such as the patient's general health profile, the severity and course of the patient's disorder or disposition thereto, and the judgment of the treating physician.

### SYNTHETIC EXAMPLES

### Example 1: Synthesis of 2-(2,6-Dichlorophenylamino)-6,6-dimethyl-1H,6H-imidazo[4,5-h]isoquinoline-7,9-dione.

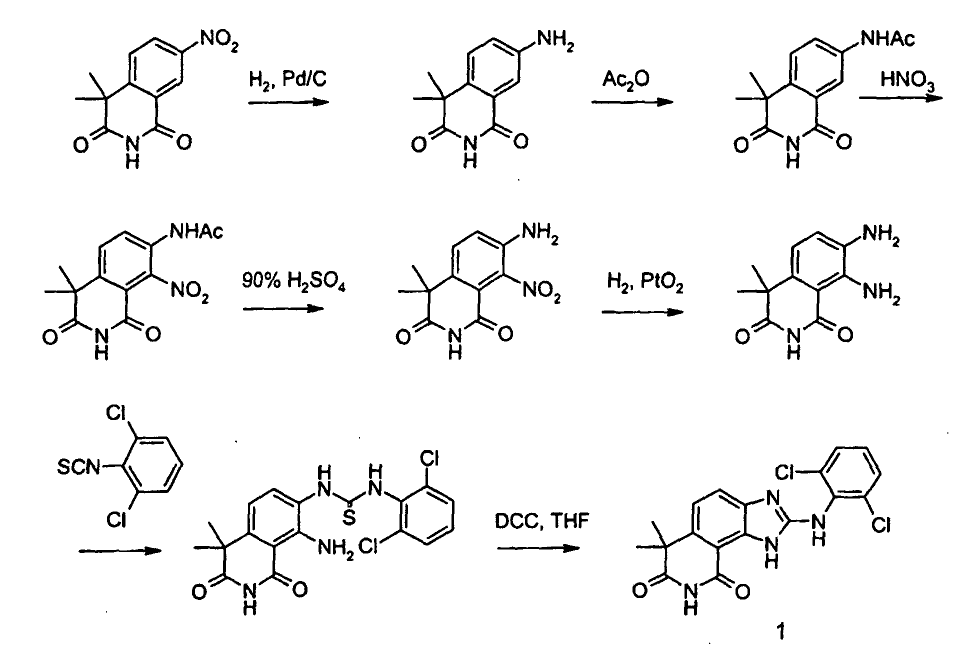

4,4-Dimethyl-7-nitro-2*H*,4*H*-isoquinoline-1,3-dione, prepared as described in US 4666923 (1987), (1.0g, 4.5mmol) in methanol (50mL) was hydrogenated over 10% Pd/C (30mg) at 50psi for 1.5h. The catalyst was removed by filtration and the solvent removed to give 8-amino-4,4-dimethyl-2*H*,4*H*-isoquinoline-1,3-dione (0.90g, 98%).

The above amine (1.5g, 7.35mmol) was stirred in acetic anhydride (9mL) at room temperature for 3h, then poured on to ice. The precipitate was filtered, washed with water and dried to give 7-acetamido-4,4-dimethyl-2*H*,4*H*-isoquinoline-1,3-dione (1.55g, 86%)

The above amide was converted to 7-acetamido-4,4-dimethyl-8-nitro-2*H*,4*H*-isoquinoline-1,3-dione as described in US 4176184 (1979).

7-Acetamido-4,4-dimethyl-8-nitro-2*H*,4*H*-isoquinoline-1,3-dione (4.0g, 13.7mmol) was added to 90% H₂SO₄ and heated at 70°C for 8h. The cooled mixture was poured onto ice. The precipitate was collected, dissolved in ethyl acetate, washed with water, dried and evaporated to give 7-amino-4,4-dimethyl-8-nitro-2*H*,4*H*-isoquinoline-1,3-dione (3.38g, 99%), mp 259-263°C; MS (CI) 250 (MH+).

A solution of the above amine (1.5g, 6.0mmol) in methanol (50mL) was hydrogenated over platinum oxide (30mg) at 50 psi for 1.25h. The mixture was filtered through diatomaceous earth and evaporated to provide 7,8-diamino-4,4-dimethyl-2*H*,4*H*-isoquinoline-1,3-dione. (1.31g, 100%). MS (CI) 220 (MH+).

As described in Method A, 2,6-dichlorophenylisothiocyanate (1.16g, 5.7mmol) was added to a suspension of 7,8-diamino-4,4-dimethyl-2*H*,4*H*-isoquinoline-1,3-dione (1.31 g, 6.0mmol) in ethyl acetate (40mL) and the mixture stirred overnight. The solid was filtered and dried to yield the thiourea (1.55g, 61%). mp >300°C; MS (CI) 423, 425 (MH+). A solution of the thiourea (2.23g, 5.28mmol) in THF (50mL) and dicyclohexylcarbodiimide (1.11g, 5.4mmol) was heated under reflux with stirring for 4h. The cooled solution was stirred overnight, filtered, and the crystals washed with CH₂Cl₂ to give the title compound (1.20g). The filtrate was evaporated and triturated with CH₂Cl₂ to give more product (0.7g, 93% combined yield), mp 290-292°C; MS (EI) 388, 390 (M+).

### Example 2: Synthesis of 2-(2,6-Dichlorophenylamino)-6,6-dimethyl-7,8-dihydro-1H,6H-imidazo[4,5-h]isoquinoline-9-one.

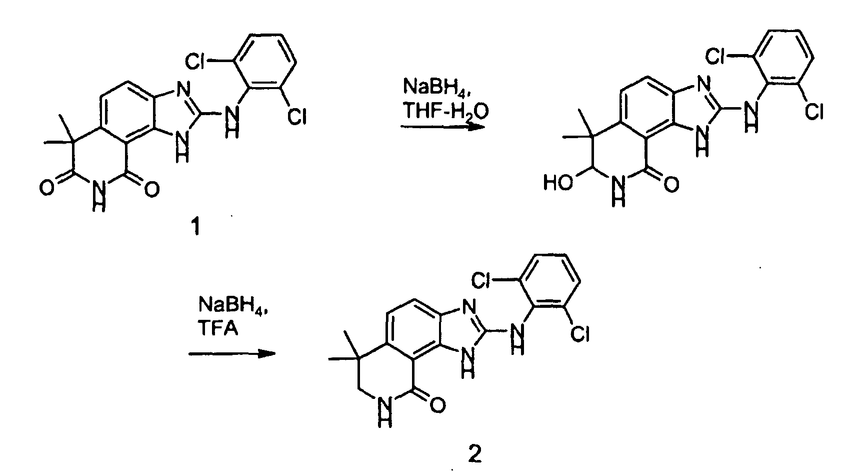

To a solution of the product of Example 1 (90mg, .23mmol) in THF (5mL) was added NaBH₄ (90mg, 2.3mmol) followed by water (4 drops). The reaction mixture was stirred at ambient temperature for 1 h. Subsequently, 1N HCl (5mL) was added dropwise and the reaction mixture was stirred an additional 15 minutes, neutralized with NaHCO₃ and extracted with EtOAc. The extract was washed with brine, dried and evaporated yielding the alcohol 2-(2,6-dichlorophenylamino)-6,6-dimethyl-7-hydroxy-7,8-dihydro-1*H*,6*H*-imidazo[4,5-*h*]isoquinoline-9-one (90mg 99%). This intermediate was used immediately due to its instability. It was characterized as the methyl ether, which was prepared by dissolving the product in MeOH/HCl and stirring for several hours. After evaporation, the residue was partitioned between EtOAc/aq NaHCO₃. The organic phase was washed with brine, dried and evaporated to the methyl ether derivative, mp 278-280°C(dec); MS (ES) 405 (MH+).

The alcohol from above (100mg, .26mmol) was dissolved in TFA (2 mL) and this solution was subsequently added to a solution of sodium tristrifluoroacetoxyborohydride (generated in-situ from 160mg, 4.2mmol of sodium borohydride and 3mL TFA) at 0°C. The reaction mixture was stirred at ambient temperature for 4h, the solvent was evaporated, the residue was triturated with water and the resultant mixture was neutralized with NaHCO₃ and filtered yielding the title compound (85mg, 92%). This product was purified by flash chromatography on SiO₂ using 4% MeOH/CH₂Cl₂ as eluant and recrystallization from EtOAc, mp 287-290°C; MS (CI) 375 (MH⁺).

### Example 3a : Synthesis of 2-(2,6-Dichlorophenylamino)-6,7-dimethyl-1,8-dihydro-imidazo[4,5-h]isoquinoline-9-one.

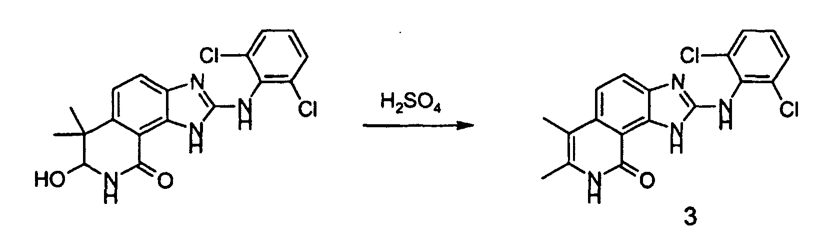

2-(2,6-Dichlorophenylamino)-6,6-dimethyl-7-hydroxy-7,8-dihydro-1*H*,6*H*-imidazo[4,5-*h*]isoquinoline-9-one (from Example 2) (45mg, 0.11mmol) was suspended in conc. H₂SO₄ (1mL) and the resultant mixture was stirred at ambient temperature for 15 min. The solution was poured over ice, neutralized with NaHCO₃ and filtered. The filtrate was triturated with water (10mL) and centrifuged. The liquid was decanted, and the residual solid was triturated with methanol and centrifuged. The supernatant was decanted and the residue dried to give the title compound (35mg, 84%). Mp >300°C; MS (ES) 373 (MH⁺).

### Example 3b: Synthesis of 2-(2,6-Dichlorophenylamino)-6,7-dimethyl-1,8-dihydro-imidazo[4,5-h]isoquinoline-9-one (Method C).

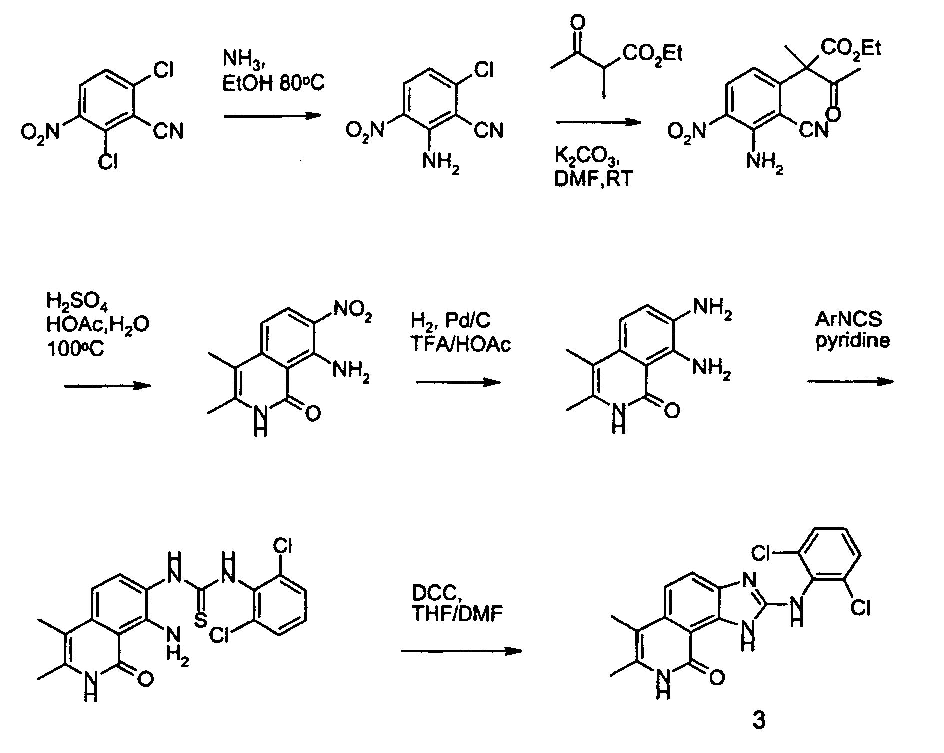

A 500mL pressure flask was charged with 2,6-dichloro-3-nitrobenzonitrile (30.0g, 138mmol) and a 5.1M solution of ammonia in EtOH (170mL). The flask was sealed and heated in an oil bath at 80°C with stirring for 1.5h. The cooled solution was filtered, the crystals washed with water and dried to yield 2-amino-6-chloro-3-nitrobenzonitrile (18.4g, 68%), mp 181-184°C; MS (ES⁻) 196, 198 (M-H⁻).

To a solution of 2-amino-6-chloro-3-nitrobenzonitrile (1.97g, 10mmol) and ethyl 2-methylacetoacetate (3.6g, 25mmol) in DMF (10mL) was added finely powdered K₂CO₃, and the mixture stirred vigorously for 24h. The deep red mixture was diluted with EtOAc and washed in turn with 2M HCl, water and brine. The residue after evaporation was purified by flash chromatography in hexane/EtOAc 3:1 to yield 2-(3-amino-2-cyano-4-nitrophenyl)-2-methyl-3-oxobutyric acid ethyl ester as an oil (1.39g, 46%), MS (NH₃ CI) 323 (M+NH₄⁺), 293 (M+NH₄-NO⁺).

The ester from above (1.39g, 4.56mmol) was added to a mixture of acetic acid (20mL), H₂SO₄ (3mL) and water (2mL), and the solution heated at 100°C for 3h. The cooled solution was diluted with water (30mL), the precipitate was collected, washed with water and MeOH and dried to give 8-amino-3,4-dimethyl-7-nitro-2*H*-isoquinolin-1-one (0.76g, 72%). mp >300°C.

A solution of the amino isoquinolin-1-one from above (0.20g, 0.86mmol) in trifluoroacetic acid (11mL) and acetic acid (7mL) was hydrogenated over 10% palladium on carbon (21mg) at 50psi for 2h. The solution was filtered through diatomaceous earth, washing with acetic acid, and the filtrate evaporated to give 7,8-diamino-3,4-dimethyl-2*H*-isoquinolin-1-one ditrifluoroacetate salt (310mg, 83%).

A suspension of the diamino isoquinolin-1-one ditrifluoroacetate salt from above (1.15g, 2.67mmol) and 2,6-dichlorophenylisothiocyanate (0.60g, 2.93mmol) in pyridine (16mL) was stirred for 18h at room temperature (Method A). The solution was diluted with toluene and evaporated, and remaining pyridine removed with a toluene azeotrope. The residue was triturated with EtOAc to give the thiourea (1.17g). A portion of this material (0.50g, 1.23mmol) and dicyclohexylcarbodiimide (0.375g, 1.84mmol) were heated together in DMF under argon at 80°C for 4h. The cooled solution was evaporated and triturated first with cold MeOH, then with boiling MeOH, to leave the title compound as a light tan solid, (0.309g, 73%), identical with the sample obtained in Example 3a.

### Example 4: Synthesis of 2-(2,6-Dichlorophenylamino)-1,6,7-trimethyl-1,8-dihydro-imidazo[4,5-h]isoquinoline-9-one.

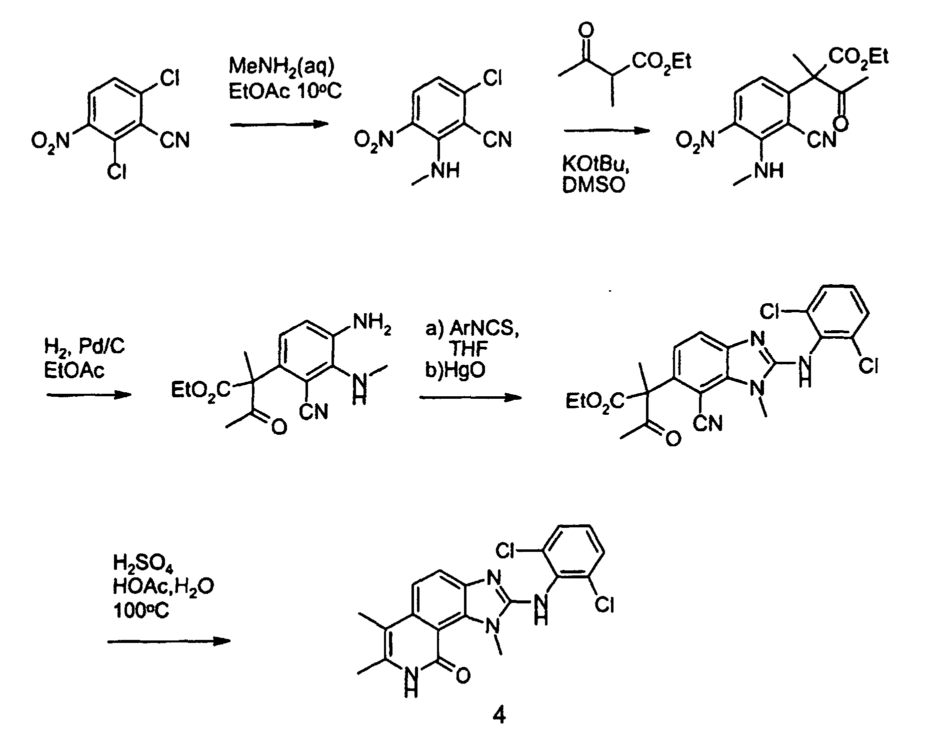

A solution of 2,6-dichloro-3-nitrobenzonitrile (98.7g, 0.455mol) in EtOAc (910mL) was cooled to 5°C. 40% Aqueous methylamine (79.5mL, 1.14mol) was added with vigorous mechanical stirring, keeping the temperature at 10-15°C. After addition was complete, stirring was continued for 3h at the same temperature. More methylamine (16mL, 0.23mol) was added, and the mixture stirred for a further 1.5h at room temperature. Water (300mL) was added, followed by hexane (450mL). The mixture was stirred for 15min, filtered, and the solid washed with water and MeOH, to give 6-chloro-2-methylamino-3-nitrobenzonitrile (80.3g, 83%), mp 167-170°C.

To a stirred solution of potassium t-butoxide (24.3g, 206mmol) in DMSO (500mL) was added ethyl 2-methylacetoacetate (34.3g, 233mmol), dropwise over 5min. The temperature rose to 30°C. 6-Chloro-2-methylamino-3-nitrobenzonitrile (43.6g, 190mmol) was added in portions over 15min. The temperature rose to 40°C. The solution was stirred for 1h with no external heating or cooling. The mixture was poured into 10% NH₄Cl (500mL), and extracted with EtOAc (2 x 500mL). The combined extracts were washed with water (2 x 250mL) and brine, and evaporated. MeOH (200mL) was added to the residue and stirred for 1.5h. The yellow solid was filtered, washed with cold MeOH (25mL) and dried to give 2-(2-cyano-3-methylamino-4-nitrophenyl)-2-methyl-3-oxobutyric acid ethyl ester (36.2g, 60%), mp 87-91°C.

A solution of the above ester (10.5g, 32.5mmol) in EtOAc (130mL) was hydrogenated over 10% palladium on carbon (0.5g) at 50psi for 24h. The catalyst was removed by filtration through diatomaceous earth, and the filtrate was evaporated. A mixture of EtOAc/hexane (1:1, 10mL) was added to the residue and the resulting mixture was stirrec for 0.5h. The crystals were filtered and washed with hexane to give 2-(4-amino-2-cyano-3-methylaminophenyl)-2-methyl-3-oxobutyric acid ethyl ester (7.74g, 81%), mp 118-123°C.

A solution of the amino ester from above (7.7g, 26.6mmol) and 2,6-dichlorophenylisothiocyanate (5.43g, 26.6mmol) in THF (150mL) was stirred at room temperature for 5h. Mercuric oxide (6.34g, 29.3 mmol) was then added in one portion, and stirring continued overnight. The mixture was filtered through diatomaceous earth, washing well with THF. The filtrate was evaporated, and the residue triturated with ether to give 2-[4-cyano-2-(2,6-dichlorophenylamino)-3-methyl-3*H*-benzimiazol-5-yl]-2-methyl-3-oxobutyric acid ethyl ester as an off-white solid (7.7g, 63%).

To a stirred mixture of conc. H₂SO₄ (40mL), HOAc (40mL) and water (40mL) at 60°C was added 2-[4-cyano-2-(2,6-dichlorophenylamino)-3-methyl-3*H*-benzimiazol-5-yl]-2-methyl-3-oxobutyric acid ethyl ester (7.4g, 16mmol) in one portion. The solution was heated at 100°C for 2.5h, then stirred overnight at room temperature. The reaction mixture was poured onto ice, and neutralized with conc. NH₄OH, with ice cooling. The precipitate was filtered and washed well with water. The solid was slurried in MeOH, stirred well, filtered, washed with MeOH until washings were colorless, and dried. The title compound was obtained as a grey solid (5.48g, 88%), mp >300°C; MS (NH₃ CI) 387,389 (MH+).

### Example 5: Synthesis of 2-(2,6-Dichlorophenylamino)-6,6-dimethyl-6H-thiazolo[4,5-h]isoquinoline-7,9-dione (Method B).

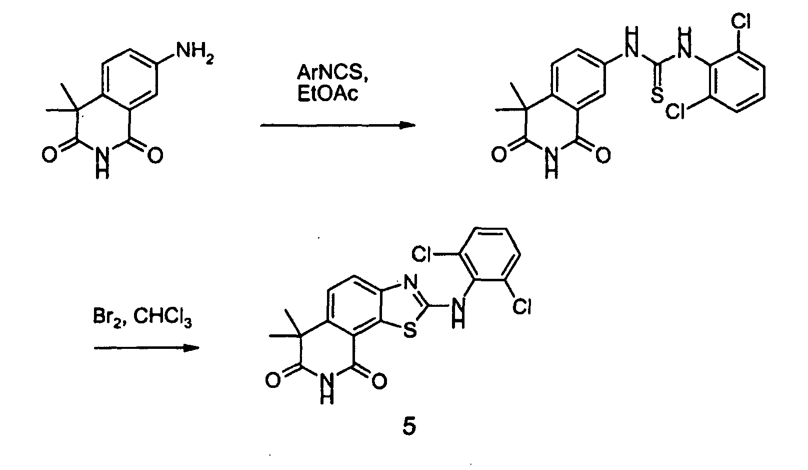

To a suspension of 7-amino-4,4-dimethyl-2*H*,4*H*-isoquinoline-1,3-dione (204mg, 1mmol) in EtOAc (25mL) was added 2,6-dichlorophenylisothiocyanate (223mg, 1.1mmol) in three portions, and the mixture was stirred overnight. The solid was filtered and dried to yield the thiourea (380mg, 93%), mp 142-144°C; MS (CI) 408(MH⁺). To a suspension of the thiourea (140mg, 0.34mmol) in CHCl₃ (20mL) was added Br₂ (60mg, 0.37mmol) in CHCl₃ (2mL) dropwise. The solution was heated to reflux for 1h. The solvent was evaporated and the residue triturated with saturated NaHCO₃ (50mL). The solid was filtered, washed with water, and dried, to yield the title compound (114mg, 82%), mp >300°C; MS (CI) 406(MH+).

### Example 6: Synthesis of 2-(2,6-Dichlorophenylamino)-6,7-dimethyl-8H-thiazolo[4,5-h]isoquinoline-9-one (Method B).

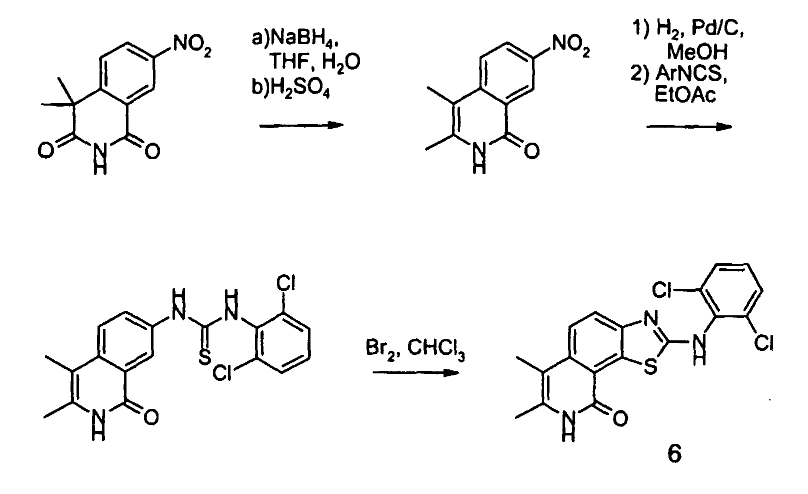

To a solution of 7-nitro-4,4-dimethyl-2*H*,4*H*-isoquinoline-1,3-dione from Example 1 (1.0g, 4.3mmol) in THF (50mL) was added NaBH₄ (330mg, 8.7mmol) followed by water (10 drops). The reaction mixture was stirred at room temperature for 2.5h, cooled in an ice bath and treated with 1N HCl until a pale yellow color was maintained. After 10 min. the reaction mixture was neutralized with saturated NaHCO₃ and extracted with EtOAc. The extract was washed with brine, dried and evaporated to the alcohol, which was immediately taken up in conc. H₂SO₄ (8mL). This mixture was stirred until completely dissolved (10 min.), poured over ice, neutralized with 10% NH₄OH, allowed to stand several hours, filtered and dried to yield 3,4-dimethyl-7-nitro-isoquinoline-1-one (780mg, 83%). MS (CI) 219(MH⁺).

A solution of 3,4-dimethyl-7-nitro-isoquinoline-1-one (750mg, 3.4mmol) in MeOH (250mL) was hydrogenated over Pd/C (25mg) at 60psi for 24h. The reaction mixture was filtered through diatomaceous earth, washing well with MeOH. Evaporation of the filtrate provided the amine (554mg, 85%) which was immediately dissolved in EtOAc (60mL) and treated with 2,6-dichlorophenylisothiocyanate (663mg, 3.3mmol). The mixture was stirred at ambient temperature for 48h, refluxed for 4h and stirred at ambient temperature an additional 72h. The thiourea was filtered and washed with EtOAc (850mg, 74%). mp 220°C (dec). A portion of the thiourea (490mg, 1.25mmol) was suspended in CHCl₃ (50mL) and treated with a solution of Br₂ (200mg, 1.25mmol) in CHCl₃ (5mL), and the resulting mixture was refluxed for 1h. The solvent was evaporated, the residue was suspended in a saturated solution of NaHSO₃, filtered, then treated analogously with a saturated solution of NaHCO₃. Purification by silica column chromatography (0 to 5% MeOH in CH₂Cl₂ eluant) yielded the title compound (281mg, 58%), mp >300°C, MS (CI) 390 (MH⁺).

### Example 7: Synthesis of 2-(2,6-Dichlorophenylamino)-1,7-dimethyl-6-(2-morpholin-4-yl-2-oxoethyl)-1,8-dihydro-imidazo[4,5-h]isoquinoline-9-one.

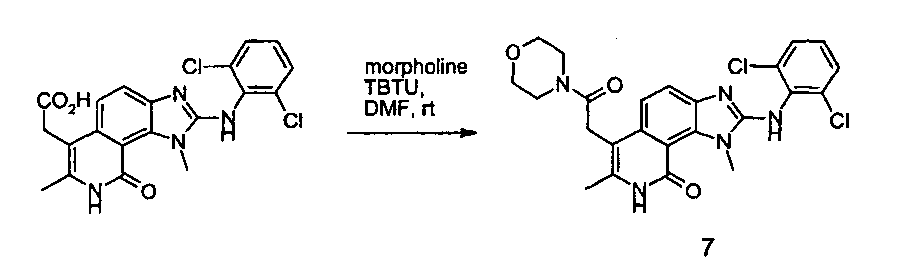

To a solution of 2-(2,6-dichlorophenylamino)-1,7-dimethyl-9-oxo-1,8-dihydro-imidazo[4,5-*h*]isoquinolin-6-yl acetic acid (prepared using Methods C and A) (1.0g, 2.3mmol) in DMF (7mL) was added *O*-benzotriazol-1-yl-*N*,*N*,*N'*,*N'*-tetramethyluronium tetrafluoroborate (TBTU) (0.82g, 2.6mmol) and morpholine (0.24mL, 2.8mmol), and the mixture stirred 18h at room temperature. Ice water was added, the precipitate collected, washed with water and dried to give the title compound, 0.97g, 84%, mp >300°C; MS (ES) 500, 502 (MH+).

### Example 8: Synthesis of 2-(2,6-Dichlorophenylamino)-1,7-dimethyl-6-(2-morpholin-4-yl-ethyl)-1,8-dihydro-imidazo[4,5-h]isoquinoline-9-one.

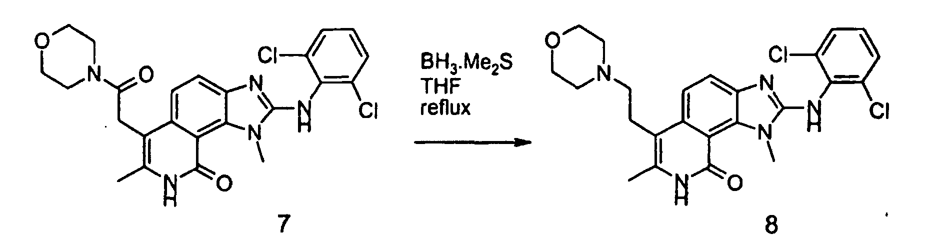

A stirred suspension of the product of Example 7 (85mg, 0.17mmol) in THF (9mL) was heated to reflux and borane-methylsulfide (0.09mL, 0.9mmol) added. Stirring was continued for 3.5h at reflux and overnight at room temperature. 6M HCl was added and the solution stirred for 2h. The solution was applied to a Varian SCX column, washed with MeOH/CH₂Cl₂ 50:50, then the product eluted with MeOH/CH₂Cl₂/NH₄OH 50:50:1. The product was further purified on a silica column eluting with CH₂Cl₂/MeOH 98:2 to give the title compound 32mg, 39%, mp 285-290°C; MS (ES) 486, 488 (MH+).

### Example 9: Synthesis of 2-(2,6-Dichlorophenylamino)-1,7-dimethyl-9-oxo-1,8-dihydro-imidazo[4,5-h]isoquinolin-6-yl acetic acid ethyl ester.

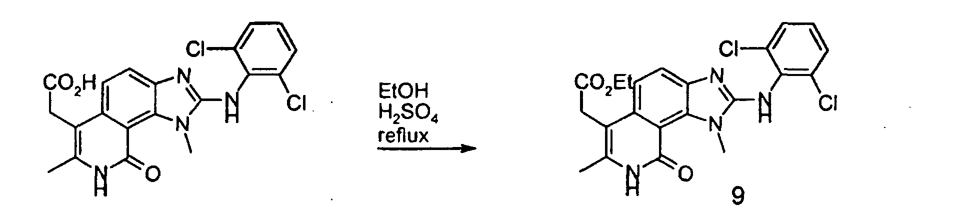

Prepared from 2-(2,6-dichlorophenylamino)-1,7-dimethyl-9-oxo-1,8-dihydro-imidazo[4,5-*h*]isoquinolin-6-yl acetic acid by refluxing in ethanol and H₂SO₄. Mp 280-285°C (dec); MS(CI) 459, 461 (MH+).

### Example 10: Synthesis 2-(2,6-Dichlorophenylamino)-1,7-dimethyl-6-(2-hydroxyethyl)-1,8-dihydro-imidazo[4,5-h]isoquinoline-9-one.

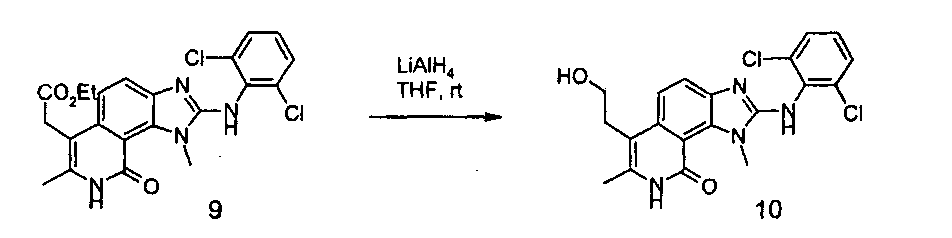

To a stirred solution of the product of Example 9 (25mg, 0.05mmol), in THF (2mL), under nitrogen, was added a solution of lithium aluminum hydride (1M in THF, 0.25mL, 0.25mmol). The mixture was stirred for 30min at room temperature. Ethyl acetate was added, followed by water, and then acidified with 1N HCl. The whole mixture was applied to a Varian SCX cartridge, and washed in turn with 1N HCl, water, acetone, MeOH, and MeOH/CH₂Cl₂(1:1). The product was then eluted with MeOH/CH₂Cl₂ /NH₄OH (49:49:2). Evaporation of the eluent gave the title compound (15mg, 72%). Mp >300°C; MS(ES) 417, 419 (MH+).

### Example 11: Synthesis of 2-(2,6-Dichlorophenylamino)-1,6-dimethyl-9-oxo-8,9-dihydro-1H-imidazo[4,5-h]isoquinoline-7-carbaldehyde.

The method described below is useful for preparing intermediate compounds such as 11, which possess an aldehyde moiety at the 7-position.

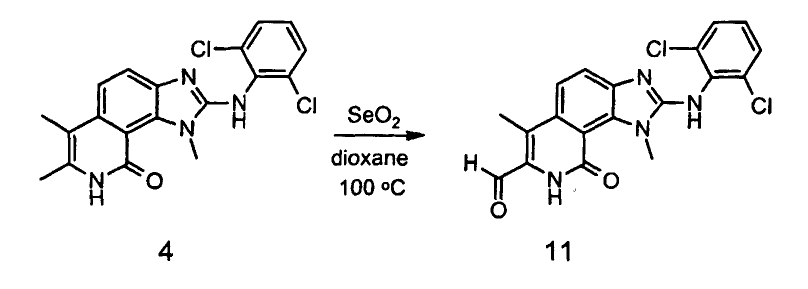

To a suspension of the product from Example 4 (521mg, 1.3mmol) in dioxane (30mL) was added selenium dioxide (430mg, 3.9mmol) and the mixture was heated at 100°C for 5h. The reaction was then cooled to room temperature, filtered through diatomaceous earth with 10% MeOH-CH₂Cl₂ and then concentrated in vacuo. The crude material was triturated with CH₂Cl₂ to provide the title compound (476mg, 92%), mp: >300°C; MS (CI) 401, 403 (MH+).

### Example 12: Synthesis of 3-[2-(2,6-Dichlorophenylamino)-1,6-dimethyl-9-oxo-8,9-dihydro-1H-imidazo[4,5-h]isoquinolin-7-yl]-acrylic acid methyl ester.

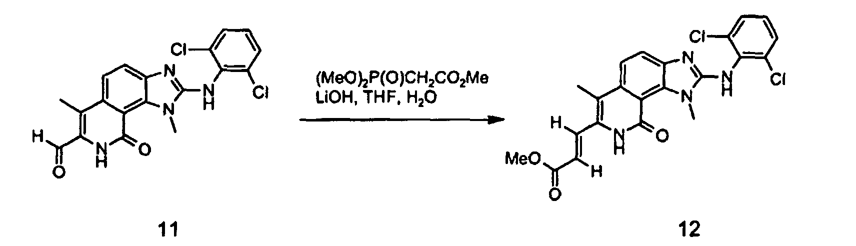

To a suspension of the product of Example 11 (329mg, 0.82mmol) in THF (5mL) was added sequentially, trimethyl phosphonoacetate (164mg, 0.90mmol), lithium hydroxide monohydrate (76mg, 1.8mmol) and water (0.9mL). The blood red solution was stirred for 2h, quenched with water, and the resulting solid was collected and dried in vacuo. Column chromatography (5% MeOH-CH₂Cl₂) provided the title compound (300mg, 80%), mp >300°C; MS (ES) 457, 459 (MH+).

### Example 13: Synthesis of 3-[2-(2,6-Dichlorophenylamino)-1,6-dimethyl-9-oxo-8,9-dihydro-1H-imidazo[4,5-h]isoquinolin-7-yl]-propionic acid methyl ester.

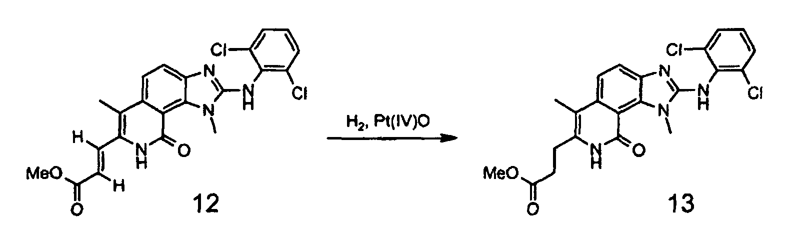

To a solution of the product of Example 12 (30mg, 0.06mmol) in EtOH (3mL) and AcOH (4mL) in a Parr reactor was added PtO₂ (2mg, 0.007mmol). The Parr reactor was charged with 50psi of H₂ and shaken for 12h. The crude reaction was filtered through diatomaceous earth with EtOH and concentrated in vacuo. Column chromatography (2% MeOH-CH₂Cl₂) provided the title compound (9mg, 30%), mp 268°C(dec); MS(ES) 459, 46 1 (MH+).

### Example 14: Synthesis of 2-(2,6-Dichlorophenylamino)-1,6-dimethyl-7-(3-hydroxy-propen-1-yl)-1,8-dihydro-imidazo[4,5-h]isoquinolin-9-one.

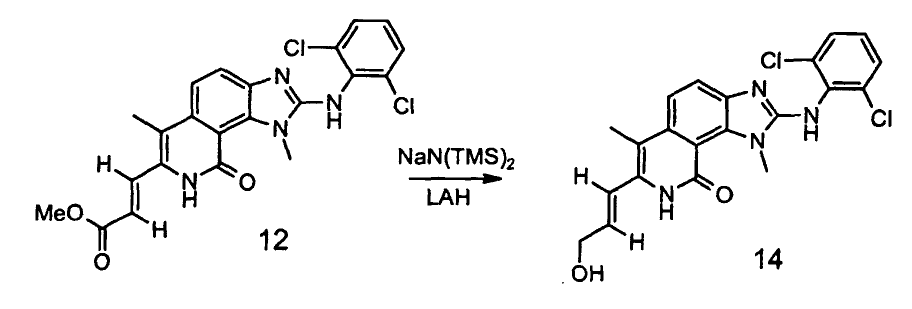

A suspension of the product of Example 12 (100mg, 0.22mmol) in THF (7mL) was cooled to -78°C. Sodium bis(trimethylsilyl)amide (1M in THF, 0.44mmol) was added dropwise. The bright red solution was warmed to 0°C for 15 minutes, then lithium aluminum hydride (1M in THF, 2.6mmol) was added and the orange solution was warmed to room temperature for 0.5h. The mixture was cooled to 0°C, quenched with saturated ammonium chloride and extracted with ethyl acetate. Column chromatography (3-6% MeOH-CH₂Cl₂) provided the title compound (32 mg, 34%), mp 298-300°C; MS(ES) 429, 431(MH+).

### Example 15: Synthesis of 2-(2,6-Dichlorophenylamino)-1,6-dimethyl-7-vinyl-1,8-dihydro-imidazo[4,5-h]isoquinoline-9-one.

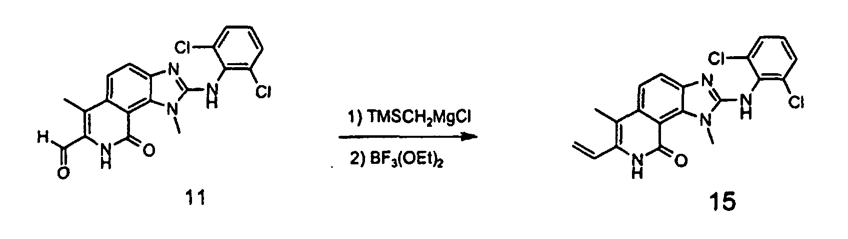

To a suspension of the product of Example 11 (100mg, 0.25mmol) in THF (5mL) was added trimethylsilylmethyl magnesium chloride (2mL, 2mmol) at -78°C. The reaction was warmed to room temperature for 1h, then cooled to 0°C and quenched with water and extracted with ethyl acetate to provide the silyl alcohol (85 mg, 70%). The crude silyl alcohol was suspended in CH₂Cl₂ and cooled to 0°C. Borontrifluoride etherate (42µL, 0.32mmol) was added and the slurry was warmed to room temperature for 1 h. The reaction was quenched with water, and the CH₂Cl₂ was removed in vacuo. Collection of the resulting solid followed by CH₂Cl₂ trituration provided the title compound (17mg, 61%), mp > 300°C; MS(Es) 399, 401(MH+).

### Example 16: Synthesis of 2-(2,6-Dichlorophenylamino)-1,6-dimethyl-7-(1-hydroxyprop-2-en-1-yl)-1,8-dihydro-imidazo[4,5-h]isoquinolin-9-one.

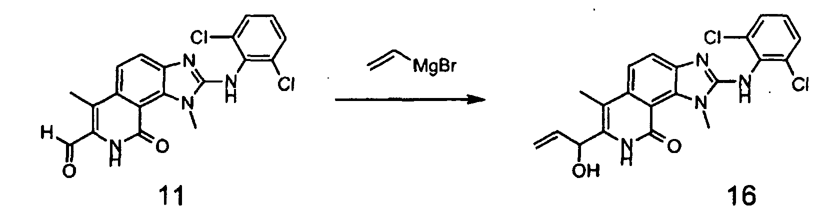

A suspension of 2-(2,6-dichlorophenylamino)-1,6-dimethyl-9-oxo-8,9-dihydro-1*H*-imidazo[4,5-*h*]isoquinoline-7-carbaldehyde (2) (100mg, 0.25mmol) in THF (3ml) was cooled to -78°C. Vinylmagnesium bromide (1M in THF, 2.0mmol) was added dropwise, and the brown suspension was warmed gradually to -10°C over 2h. The solution was quenched with saturated ammonium chloride and extracted with ethyl acetate, and concentrated in vacuo to provide the title compound, which was used in the next step without purification, mp 235-236°C, MS (ES) 429 (MH+).

### Example 17: Synthesis of 2-(2,6-Dichlorophenylamino)-7-(1-acetoxyprop-3-en-1-yl)-1,6-dimethyl-1,8-dihydro-imidazo[4,5-h]isoquinolin-9-one.

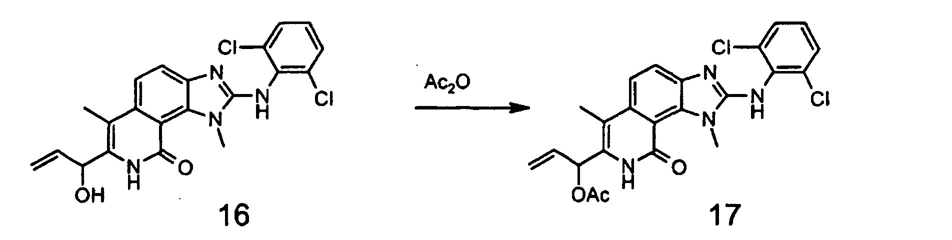

To a solution of the product of Example 16 (106mg, 0.25mmol) in THF (1ml) was added acetic anhydride (1ml). Triethylamine (35µL, 0.25mmol) was added, and the reaction was stirred for 14h, then concentrated in vacuo. Column chromatography (2% MeOH-CH₂Cl₂) provided the title compound (85mg, 79%), mp 169-171°C; MS (ES) 471 (MH+).

### Example 18: Synthesis of 2-(2,6-Dichlorophenylamino)-1,6-dimethyl-7-(3-morpholin-4-yl-propen-1-yl)- 1,8-dihydro-imidazo[4,5-h]-isoquinolin-9-one.

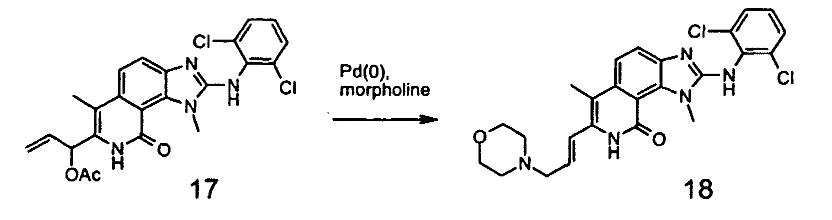

Tris(dibenzylideneacetone) dipalladium(0) (1.8mg, 0.002mmol) and triphenylphosphine (1.6mg, 0.006mmol) were stirred in THF (0.5ml) for 20min under inert atmosphere until the red solution turned yellow. To this solution was added sequentially, the product of Example 17 (20mg, 0.04mmol) in THF (0.5 ml), triethylamine (17µL, 0.12mmol) and morpholine (11µL, 0.12mmol). The solution was stirred 14h, then concentrated to an oil.

Column chromatography (10% MeOH-CH₂Cl₂) provided the title compound (10 mg, 50%), mp 175-177°C; MS (ES) 498 (MH+).

### Example 19: Synthesis of 7-Benzylaminomethyl-2-(2,6-dichlorophenylamino)-1,6-dimethyl-1,8-dihydro-imidazo[4,5-h]-isoquinolin-9-one.

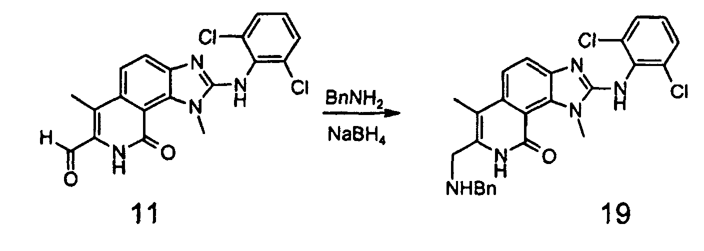

To a suspension of the product of Example 11 (50mg, 0.12mmol) in THF (4mL) was added benzylamine (54mg, 0.50mmol). The reaction was stirred for 12h, then concentrated in vacuo. The crude imine was suspended in MeOH (2mL), sodium borohydride (21mg, 0.55mmol) was added, and the reaction was stirred for 3h. The reaction was quenched with water, and the resulting solid was collected and dried to provide the title compound (11mg, 39%), mp 231-234°C; MS (ES) 492(MH+).

### Example 20: Synthesis of 2-(2,6-Dichlorophenylamino)-1,6-dimethyl-7-oxazol-5-yl-1,8-dihydro-imidazo[4,5-h]isoquinolin-9-one.

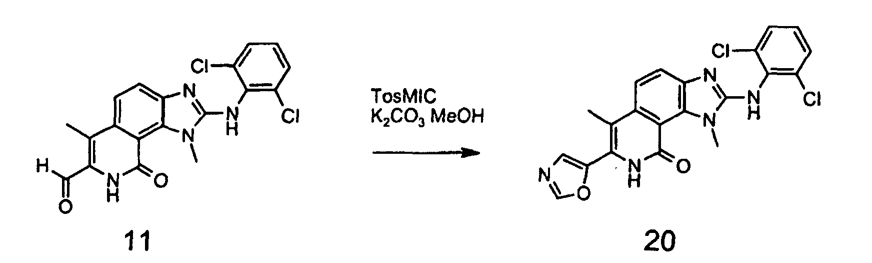

A suspension of the product of Example 11 (40mg, 0.10mmol), tosylmethyl isocyanide (21mg, 0.11mmol) and K₂CO₃ in methanol (2mL) was heated to 40°C for 90min. The mixture was diluted with water (3mL) and the solid collected by filtration to obtain the title compound (26mg, 60 %), mp >300°C; MS (ES) 440, 442(MH+).

### Example 21: Synthesis of 2-(2,6-Dichlorophenylamino)-1,6-dimethyl-7-(3-methyl-3H-imidazol-4-yl)-1,8- dihydro-imidazo[4,5-h]isoquinolin-9-one.

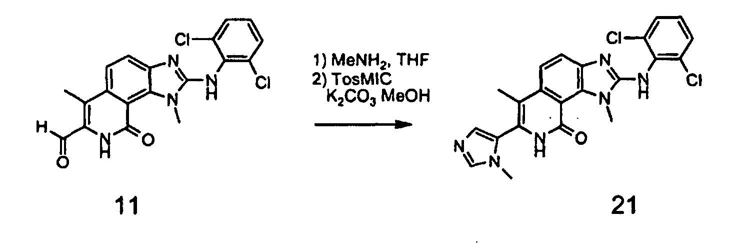

A suspension of the product of Example 11 (50mg, 0.13mmol) and methylamine (2M in THF, 2mL, 4mmol) in dry THF (2mL) was stirred at room temperature for 12h. The THF was evaporated and the resulting imine was mixed with tosylmethyl isocyanide (27mg, 0.14mmol), K₂CO₃ (3 1 mg, 0.23mmol) and dry DMSO (2mL). This suspension was stirred for five days at room temperature. Water (5mL) was added and the precipitate collected by filtration. Flash chromatography in CH₂Cl₂/MeOH (98:2) gave the title compound (8mg, 14%), mp >300°C; MS(ES) 453, 455 (MH+).

### Example 22: Synthesis of 2-{2,6-Dichlorophenylamino)-1,6,7-trimethyl-4-vinyl-1,8-dihydro-imidazo[4,5-h]isoquinoline-9-one.

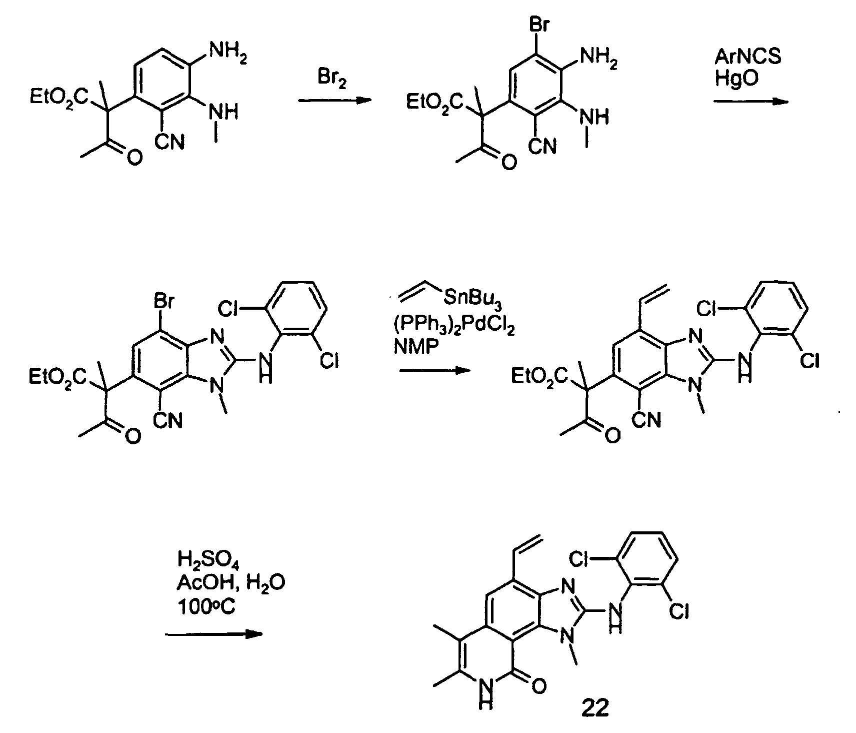

To a solution of 2-(4-amino-2-cyano-3-methylaminophenyl)-2-methyl-3-oxobutyric acid ethyl ester from Example 4 (9.02g, 31.2mmol) in CHCl₃ (90mL) was added bromine (4.98g, 31.2mmol) dropwise at ambient temperature. After the addition of bromine, the reaction mixture was diluted with ethyl acetate (800mL). This solution was washed successively with sat. NaHCO₃ solution and brine and dried. The residue after evaporation was purified by flash chromatography in hexanes/EtOAc 2:1 to yield 2-(4-amino-5-bromo-2-cyano-3-methylaminophenyl)-2-methyl-3-oxobutyric acid ethyl ester as an oil (6.06g, 53%).

To a solution of the above ester (3.32g, 9.02mmol) in 1,4-dioxane (45mL) was added 2,6-dichlorophenylisothiocyanate (2.02g, 9.92mmol) and mercuric oxide (2.54g, 11.7mmol) under nitrogen atmosphere. The resulting mixture was stirred and heated at 95°C overnight. The reaction mixture was cooled to room temperature and filtered though a short pad of diatomaceous earth and SiO₂. The filtrate was concentrated and the residue was purified by flash chromatography in hexanes/EtOAc 2:1 to yield 2-[8-bromo-4-cyano-2-(2,6-dichlorophenylamino)-3-methyl-3*H*-benzimiazol-5-yl]-2-methyl-3-oxobutyric acid ethyl ester as a brown solid (3.44g, 71%).

A mixture of the above bromo ester (600mg, 1.11mmol), (PPh₃)₂PdCl₂ (78mg, 0.11mmmol) and tributyl(vinyl)tin (0.49mL, 1.67mmol) in NMP (4mL) was degassed and heated at 100°C for 3 days under argon. The mixture was concentrated and the residue was purified by flash chromatography in hexanes/EtOAc 3:1 to yield 2-[4-cyano-2-(2,6-dichlorophenylamino)-3-methyl-8-vinyl-3*H*-benzimiazol-5-yl]-2-methyl-3-oxobutyric acid ethyl ester as an oil (530mg, 98%).

A solution of the above keto ester (66mg, 0.14mmol) in a mixture of H₂SO₄ (0.6mL), acetic acid (0.6mL) and water (0.6mL) was heated at 100°C for 2h. The resulting mixture was cooled to room temperature and diluted with water (10mL). The solution was adjusted to pH 8 with 10% NaOH solution. The precipitated brown solid was filtered and purified by flash chromatography in CH₂Cl₂/MeOH 30:1 to yield the title compound (15mg, 27%), mp decomp. above 250°C; MS (CI) 413(MH+).

### Example 23: Synthesis of 2-(2,6-Dichlorophenylamino)-1,6,7-trimethyl-9-oxo-1,8-dihydro-imidazo[4,5-h]isoquinoline-4-carbaldehyde.

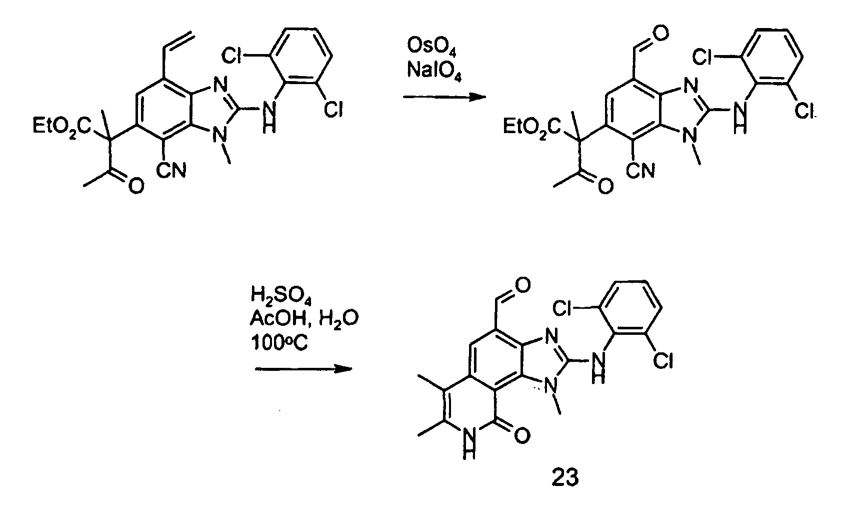

A solution of 2-[4-cyano-2-(2,6-dichlorophenylamino)-3-methyl-8-vinyl-3*H*-benzimiazol-5-yl]-2-methyl-3-oxobutyric acid ethyl ester (see Example 22) (538mg, 1.11mmol) in THF (30mL) was treated with 2.5% OsO₄ solution in tBuOH (3.0mL), NaIO₄ (712mg, 3.33mmol) and water (3mL). After stirring for 1.5h at room temperature, the mixture was diluted with EtOAc. The organic solution was washed with brine, dried and concentrated. The residue was purified by flash chromatography in hexanes/EtOAc 4:1 to yield 2-[4-cyano-2-(2,6-dichlorophenylamino)-8-formyl-3-methyl-3*H*-benzimiazol-5-yl]-2-methyl-3-oxobutyric acid ethyl ester as an oil 345mg, 64%).

A solution of the above aldehyde (35mg, 0.07mmol) in a mixture of H₂SO₄ (0.6mL), acetic acid (0.6mL) and water (0.6mL) was heated at 100°C for 1.5h and cooled to room temperature. The resulting mixture was diluted water (10mL) and the pH adjusted to 7 with ammonium hydroxide solution. The precipitated orange powder was filtered to give the title compound as an orange solid (18mg, 60%).

### Example 24: Synthesis of 2-(2,6-Dichlorophenylamino)-4-(2-hydroxyethylaminomethyl)-1,6,7-trimethyl-1,8-dihydro-imidazo[4,5-h]isoquinoline-9-one.

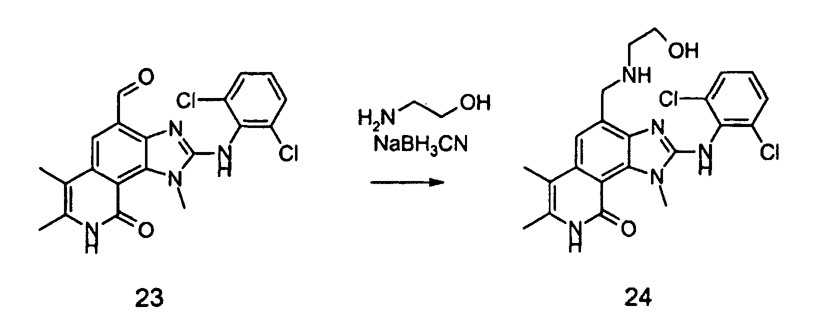

A suspension of the product of Example 23 (30mg, 0.07mmol) in MeOH (5mL) was treated with ethanolamine (44µL, 0.72mmol) and NaBH₃CN (14 mg, 0.22mmol), and stirred at room temperature for 16h. The resulting mixture was concentrated and the residue was diluted with water. The precipitated solid was filtered to give the title compound (12mg, 36%). mp decomp. above 250°C; MS (CI) 460 (MH+).

### Example 25: Synthesis of 2-(2,6-Dechlorophenylamino)-4-(3-methoxypropyn-1-yl)-1,6,7-trimethyl-1,8-dihydro-imidazo[4,5-h]isoquinoline-9-one.

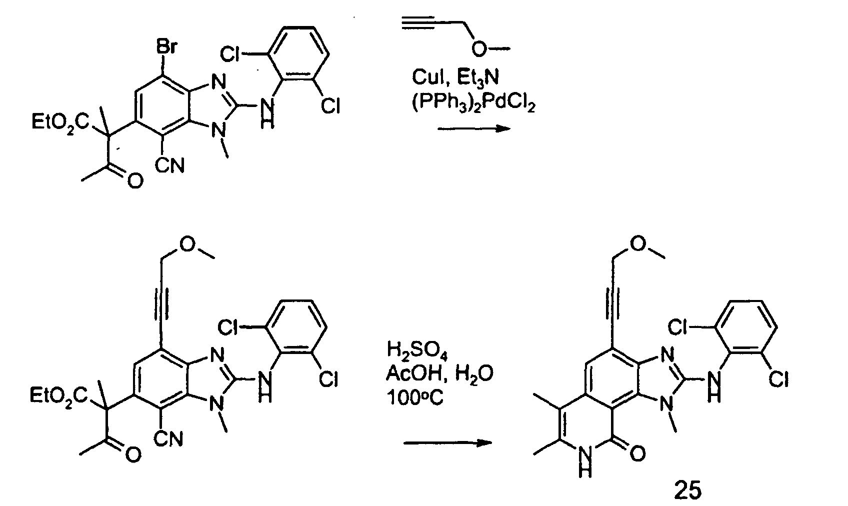

A mixture of 2-[8-bromo-4-cyano-2-(2,6-dichlorophenylamino)-3-methyl-3*H*-benzimidazol-5-yl]-2-methyl-3-oxobutyric acid ethyl ester (see Example 22) (50mg, 0.09mmol), methyl propargyl ether (16µL, 0.19mmol), (PPh₃)₂PdCl₂ (6.5mg, 0.009mmol) and Cul (3.5mg, 0.02mmol) in Et₃N (1mL) and THF(1mL) was stirred at room temperature for 5 days under argon. The resulting mixture was concentrated and the residue was purified by flash chromatography in hexanes/EtOAc 3:1 to give 2-[4-cyano-2-(2,6-dichlorophenylamino)-8-(4-methoxypropyn-1-yl)-3-methyl-3*H*-benzimidazol-5-yl]-2-methyl-3-oxobutyric acid ethyl ester as an oil (30mg, 61%).

A solution of the above ketoester (29mg, 0.006mmol) in a mixture of H₂SO₄ (0.4mL), acetic acid (0.4mL) and water (0.4mL) was heated at 100°C for 2h. The resulting mixture was cooled to room temperature and diluted with water (10mL). The pH of this solution was adjusted to 8 with 10% NaOH solution. The precipitated solid was filtered to give the title compound (17mg, 68%), mp decomp. above 250°C; MS(CI) 455 (MH+).

### Example 26: Synthesis of 2-(2,6-Dichlorophenylamino)-3,5-dihydro-imidazo[4,5-i]phenanthridin-4-one.

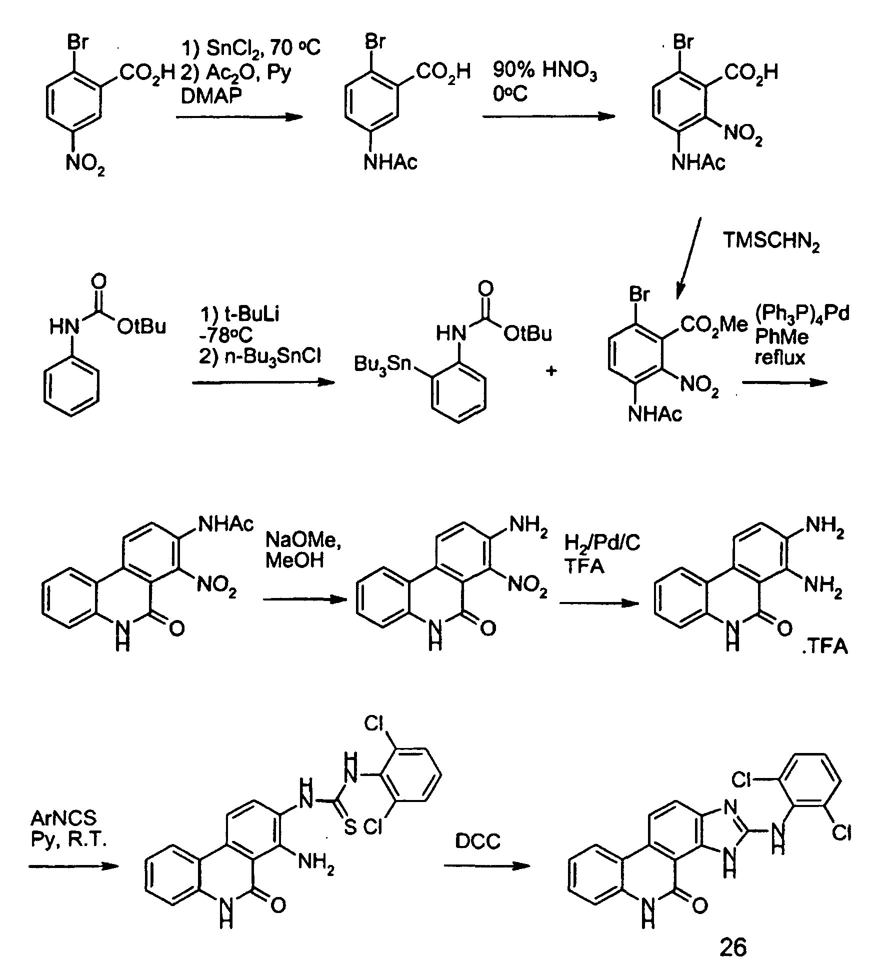

To a solution of N-(*t*-butoxycarbonyl)aniline (2.0g, 10.35mmol) in dry THF (50mL) at -78°C, a solution of *t*-BuLi (2.7M in pentane, 26mL, 25.88mmol) was added dropwise over 30min. The resulting yellow solution was warmed to -20°C and stirred at this temperature for 2.5h. *n*-Bu₃SnCl (4.2mL, 15.52mmol) in dry THF (10mL) was added over 20min, and the solution stirred at -20°C for 2h, then at room temperature for 12h. The reaction mixture was poured into NaHCO₃ solution and extracted with ether. The extract was washed with water, brine, and dried over MgSO₄. The solvent was evaporated and the resulting oil purified by flash chromatography in hexanes/ether (20:1) to give 2-tributylstannylphenylcarbamic acid *t*-butyl ester (2.42g, 54%).

Tin(II) chloride (46.67g, 206.84mmol) was added portionwise to a solution of 2-bromo-5-nitro benzoic acid (12.71g, 49.9mmol) in dry ethanol (200mL). The mixture was heated at 70°C for 45min, then ethanol was evaporated. The residue was cooled to 0°C, and acetic anhydride (43mL) and pyridine (26mL) were added. The solution was stirred at room temperature for 14h and evaporated. The residue was partitioned between aq. 2M HCl and ethyl acetate (400mL). The organic phase was washed with brine and dried over MgSO₄. The residue from evaporation was crystallized from water to give 5-acetylamino-2-bromobenzoic acid (12.4 g, 96 %).

5-Acetylamino-2-bromobenzoic acid (6.81 g, 26.39 mmol) was added portionwise to fuming nitric acid (90%, 11mL) at 0°C (as described by H. Goldstin, G. Preitner. *Helv*. *Chim. Acta* **1944,** *27*, 888). The ice bath was removed and the solution stirred at room temperature for 1.5h, then poured into ice water. 3-Acetylamino-6-bromo-2-nitro-benzoic acid was collected by filtration (5.07g, 63 %).

To a solution of 3-acetylamino-6-bromo-2-nitro-benzoic acid (3.86g, 14.96mmol) in dry THF (42mL) and dry methanol (18mL) was added a solution of (trimethylsilyl)diazomethane in hexane (2M, 24mL, 48mmol). The solution was stirred at room temperature for 3h and evaporated. The residue was purified by flash chromatography in hexanes/ethyl acetate (6:1) to give 3-acetylamino-6-bromo-2-nitrobenzoic acid methyl ester (2.45g, 52%).

A solution of 3-acetylamino-6-bromo-2-nitrobenzoic acid methyl ester (1.3g, 4. 11mmol) and Pd(PPh₃)₄ (0.31 g, 0.27 mmol) in dry toluene (15 mL) was stirred at room temperature for 10 min. To this orange solution was added a solution of 2-tributylstannylphenylcarbamic acid *t*-butyl ester (2.10 g, 4.94 mmol) in dry toluene (10 mL) and the mixture was heated to reflux for 14 h, during which time a precipitate formed. 8-Acetamido-7-nitro-6-oxo-5,6-dihydro-phenanthridin-6-one was collected by filtration as an off-white solid (1.07g, 88%).

A suspension of 8-acetamido-7-nitro-6-oxo-5,6-dihydro-phenanthridin-6-one (770mg, 2.66mmol) and NaOMe (25% w/w solution in MeOH, 3.5mL, 6.64mmol) in dry methanol (15mL) was heated to reflux for 3h. The methanol was evaporated and the residue triturated with water and filtered to yield 8-amino-7-nitro-*5H*-phenanthridin-6-one (500mg, 74%).

A mixture of 8-amino-7-nitro-*5H*-phenanthridin-6-one (412mg, 1.62mmol), Pd/C (10 wt %, 234 mg) in TFA was hydrogenated at 50 psi for 50min. The catalyst was filtered through a plug of diatomaceous earth and rinsed with ethanol. The solvent was evaporated to obtain 7,8-diamino-*5H*-phenanthridin-6-one trifluoroacetate salt (520 mg, 71 %).

To a solution of 7,8-diamino-*5H*-phenanthridin-6-one trifluoroacetate salt (200mg, 0.44mmol) in pyridine (3mL) was added 2,6-dichlorophenylisothiocyanate (93mg, 0.46mmol). The suspension was stirred at room temperature for 14h. The pyridine was evaporated using a toluene azeotrope. The residue was triturated with ethanol to obtain the thiourea (150mg, 79%). A mixture of thiourea (146mg, 0.34mmol) and dicyclohexylcarbodiimide (83mg, 40.80mmol) in dry THF (2mL) and dry DMF (0.9mL) was heated to 80°C for 8h. The solvent was removed under high vacuum and the residue triturated with hot ethanol to give the title compound (82mg, 61%), mp >300°C; MS(CI) 395, 397(MH+).

### Example 27: Synthesis of 2-(2,6-Dichlorophenylamino)-3-methyl-5,6,7,8-tetrahydro-3H-1,3,5-triaza-dicyclopenta[a,f]naphthalen-4-one.

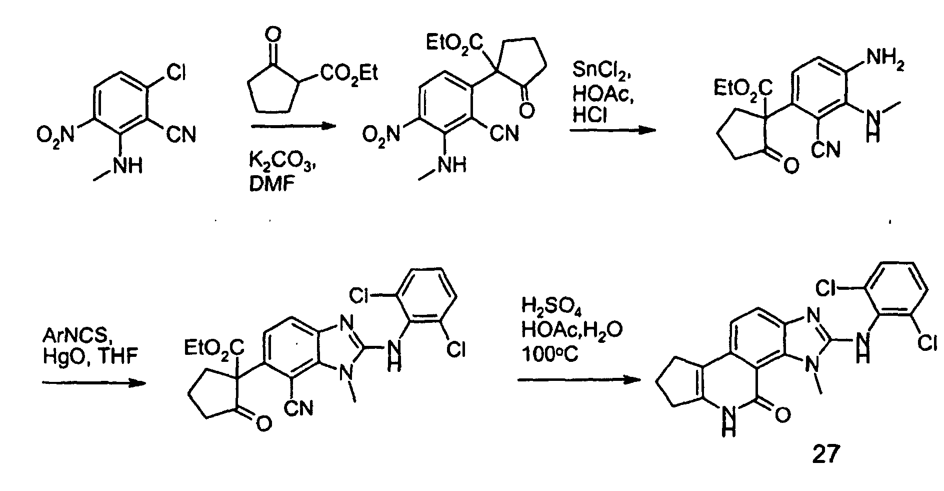

A mixture of 6-chloro-2-methylamino-3-nitrobenzonitrile (200mg, 0.95mmol) (Example 4), ethyl 2-oxocyclopentanecarboxylate (177mg, 1.13mmol) and K₂CO₃ (287mg, 2.07mmol) in DMF (5mL) was stirred at room temperature for 60h. The mixture was diluted with sat. NH₄Cl solution and extracted with ether. The ethereal layer was washed with water and brine, dried (Na₂SO₄), filtered and concentrated. The residue was purified by chromatography on silica (hexanes: EtOAc = 3:1) to give ethyl 1-(2-cyano-3-methylamino-4-nitrophenyl)-2-oxo-cyclopentanecarboxylate (127mg, 40%) as a yellow solid.

To a solution of the above compound (100mg, 0.30mmol) in acetic acid (1mL) was added a solution of tin (II) chloride dihydrate (681mg, 3.0mmol) in c. HCl (0.5mL) at room temperature. The resulting mixture was stirred at room temperature for 2h and quenched with sat. NaHCO₃ solution. The pH of the mixture was adjusted to 8 with NaHCO₃. The product was extracted into EtOAc and the organic layer was washed with water and brine, dried (Na₂SO₄), and concentrated to give ethyl 1-(4-amino-2-cyano-3-methylaminophenyl)-2-oxo-cyclopentanecarboxylate (76mg, 84%) as a yellow oil.

A mixture of the above diamine (140mg, 0.46mmol), 2,6-dichlorophenyl isothiocyanate (104mg, 0.51mmol) and HgO (110mg, 0.51mmol) in THF (5mL) was refluxed for 8h. The cooled mixture was filtered through diatomaceous earth and concentrated. The residue was diluted with EtOAc, washed with water and brine, dried (Na₂SO₄), filtered and evaporated. The residue was purified by chromatography on silica in hexanes/EtOAc, (1:1) to give ethyl 1-[4-cyano-2-(2,6-dichlorophenylamino)-3-methyl-3*H*-benzimidazol-5-yl]-2-oxo-cyclopentanecarboxylate (200mg, 92%) as a light yellow solid.

A solution of the above benzimidazole (195mg, 0.41mmol) in a 1:1:1 mixture of water, acetic acid and H₂SO₄ (1.5mL) was heated at 100 °C for 3h. The reaction mixture was cooled and diluted with water. The precipitate was collected and washed with water. The collected yellow solid was purified by chromatography on SiO₂ in CH₂Cl₂ /MeOH (20:1) to give the title compound (70mg, 43%) as a light yellow solid. Mp >300°C (dec.), MS (CI) m/z 399 (M⁺+H).

### Example 28: Synthesis of 7-(3-Aminopropen-1-yl)-2-(2,6-dichlorophenylamino)-1,6-dimethyl-1,8-dihydro-imidazo[4,5-h]-isoquinolin-9-one.

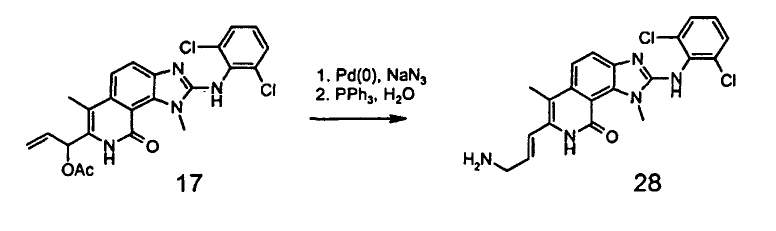

A suspension of tris(dibenzylideneacetone) dipalladium(0) (185mg, 0.25mmol) and triphenylphosphine (320mg, 1.2mmol) in THF (40mL) was stirred for 20 min under N₂. A solution of the product from Example 17 (1.88g, 4.0mmol) in THF (5 mL) was added and the mixture stirred for 20 min. Sodium azide (280mg, 4.4mmol) and water (4.0mL) were added and the reaction was heated at 60°C for 3h. The solution was cooled to rt and triphenylphosphine (1.0g, 3.8mmol) was added. After stirring for 45 min., ammonium hydroxide (4 mL) was added and stirring continued overnight. The resulting solution was dried over MgSO₄, then concentrated to an oil. Column chromatography on silica eluting with CH₂Cl₂/MeOH (90:10 increasing to 50:50) provided the title compound (1.2 g, 70%), mp >300°C; MS (ES) 428 (MH+).

### Example 29: Synthesis of 1-{3-[2-(2,6-dichlorophenylamino)-1,6-dimethyl-9-oxo-8,9-dihydro-1H-imidazo[4,5-h]isoquinolin-7-yl]-propenyl}-3-phenyl urea.

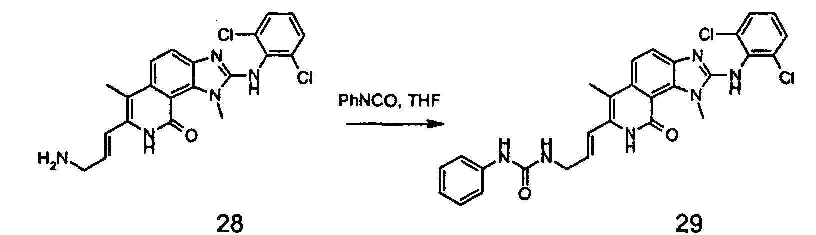

A solution of the product of Example 28 (50mg, 0.12mmol) and phenyl isocyanate (17mg, 0.13mmol) in DMF (2mL) was heated at 60°C for 10h. The resulting precipitate was filtered, triturated with MeOH/CH₂Cl₂ (90:10), and dried to provide the title compound (57 mg, 89%) mp >290°C; MS (ES) 547 (MH+).

### Other Examples

Using methods analogous to those described above, the following compounds of this invention (Tables 1-3) were prepared:

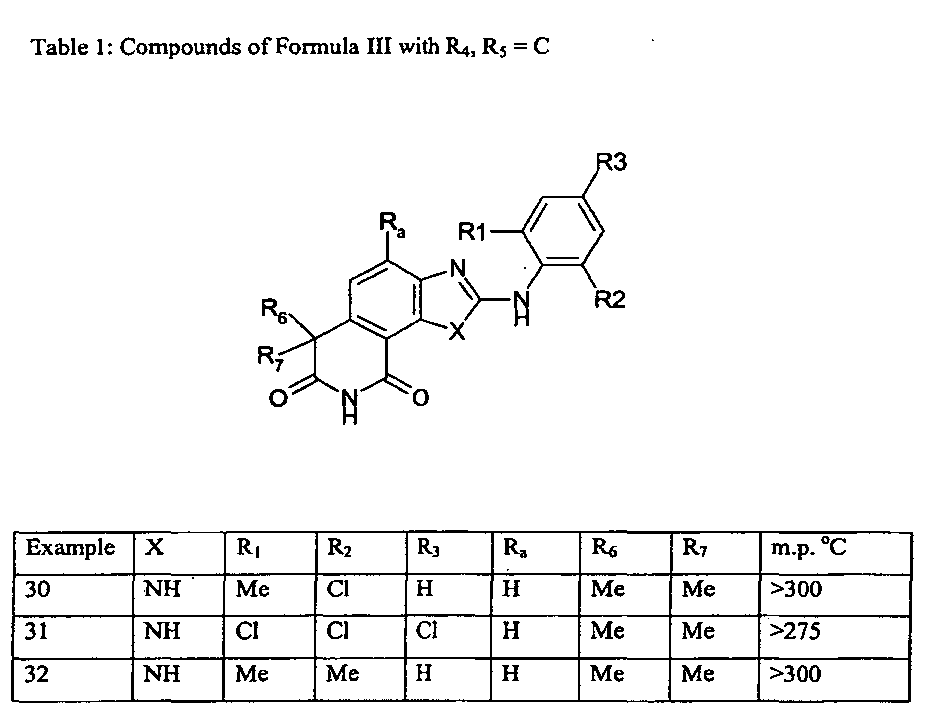

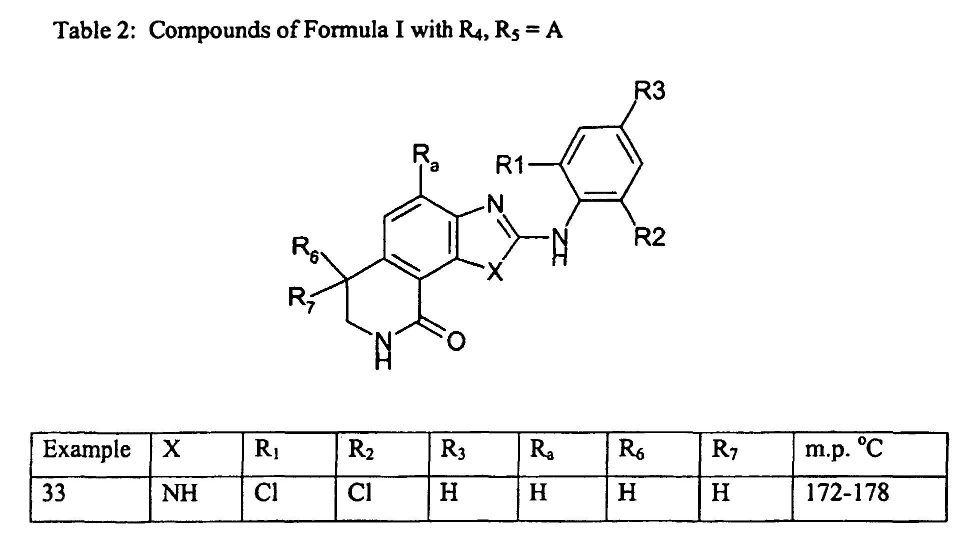

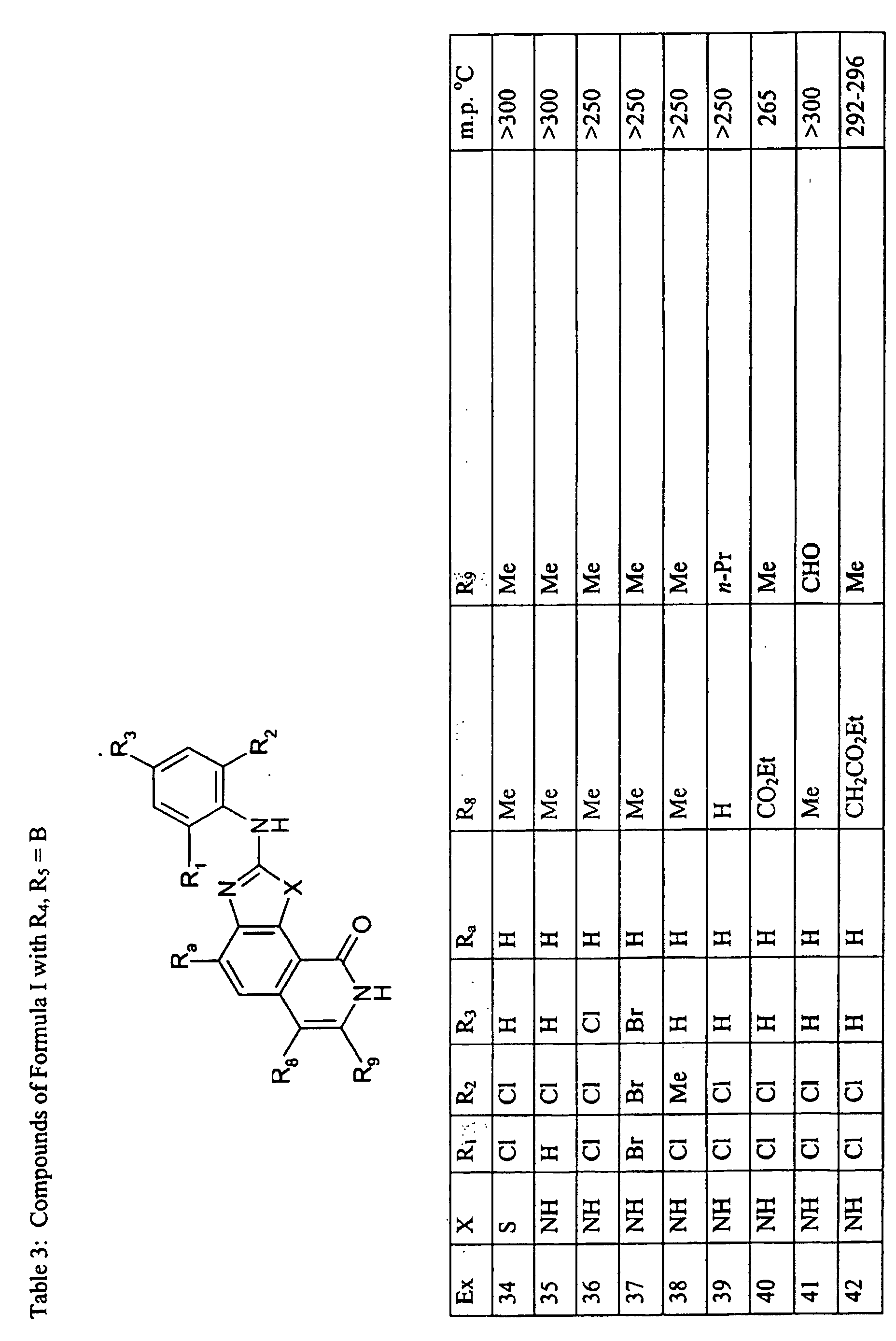

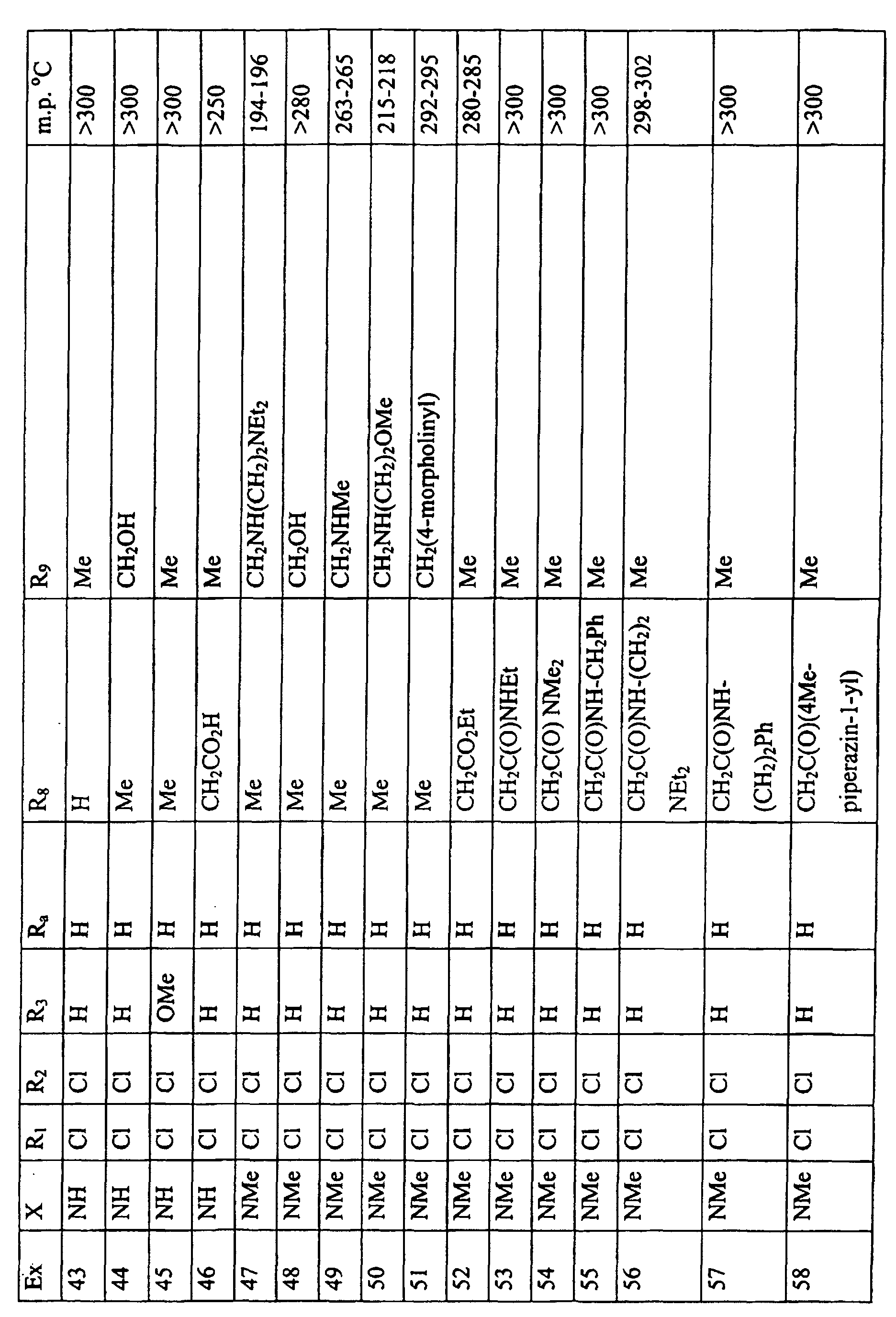

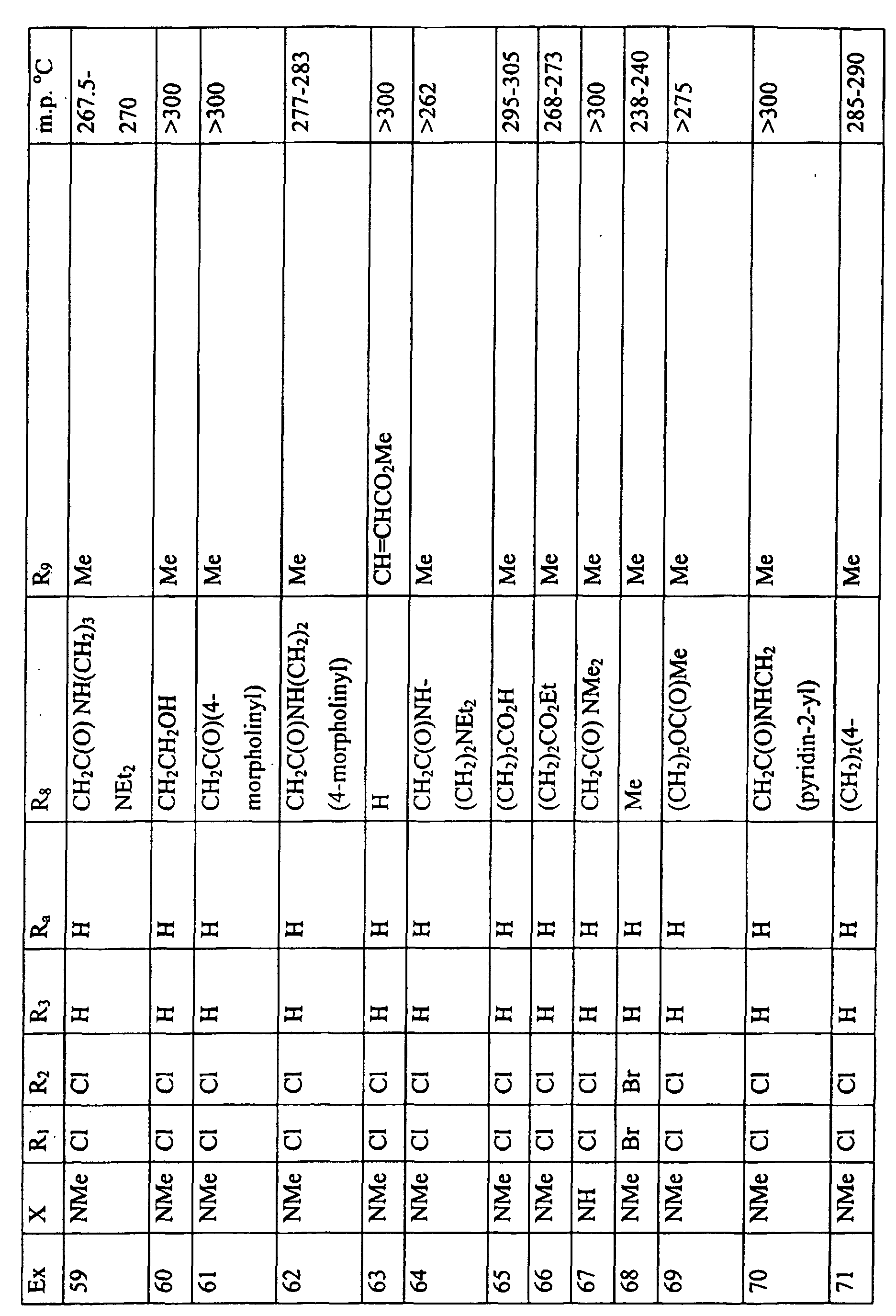

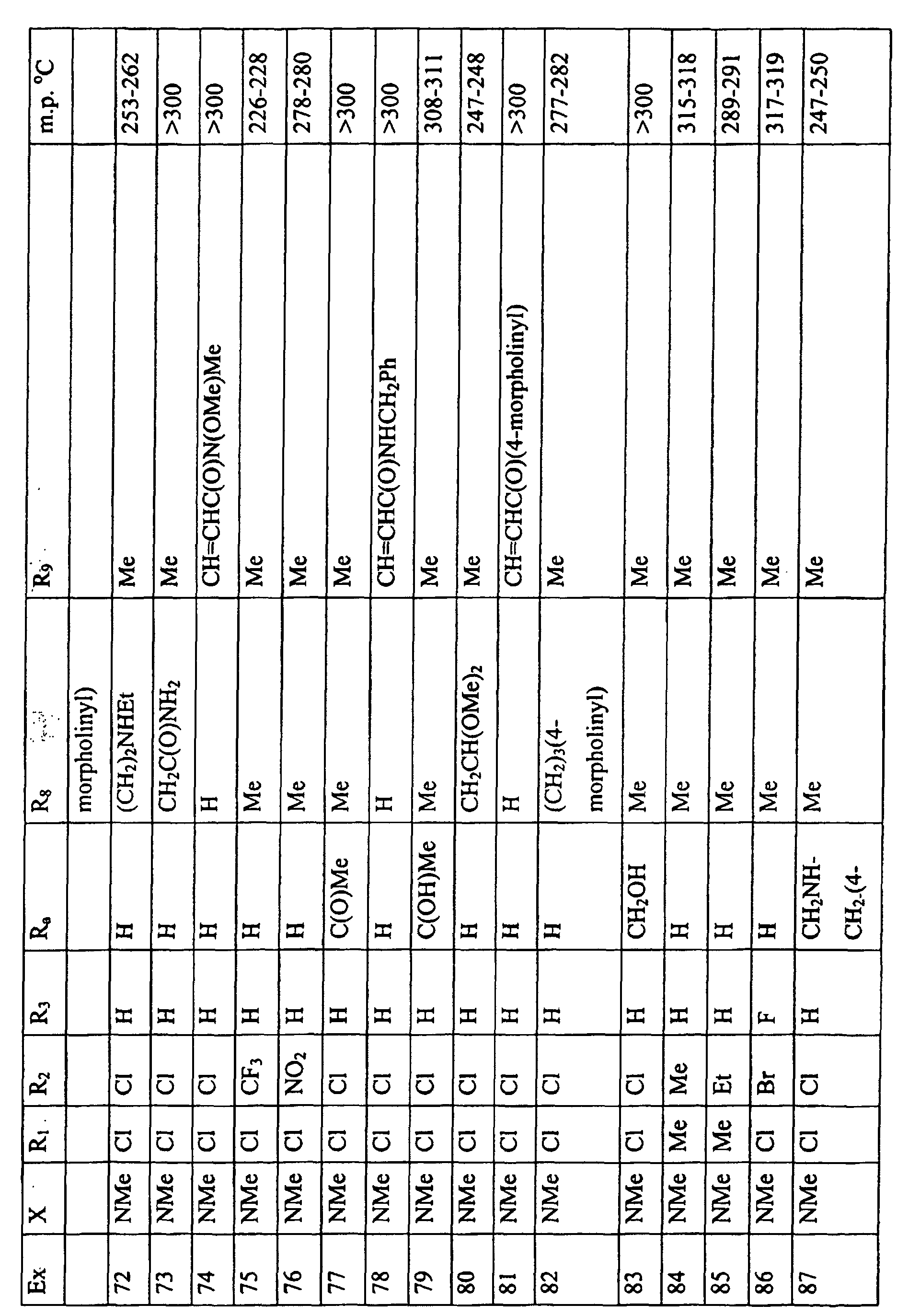

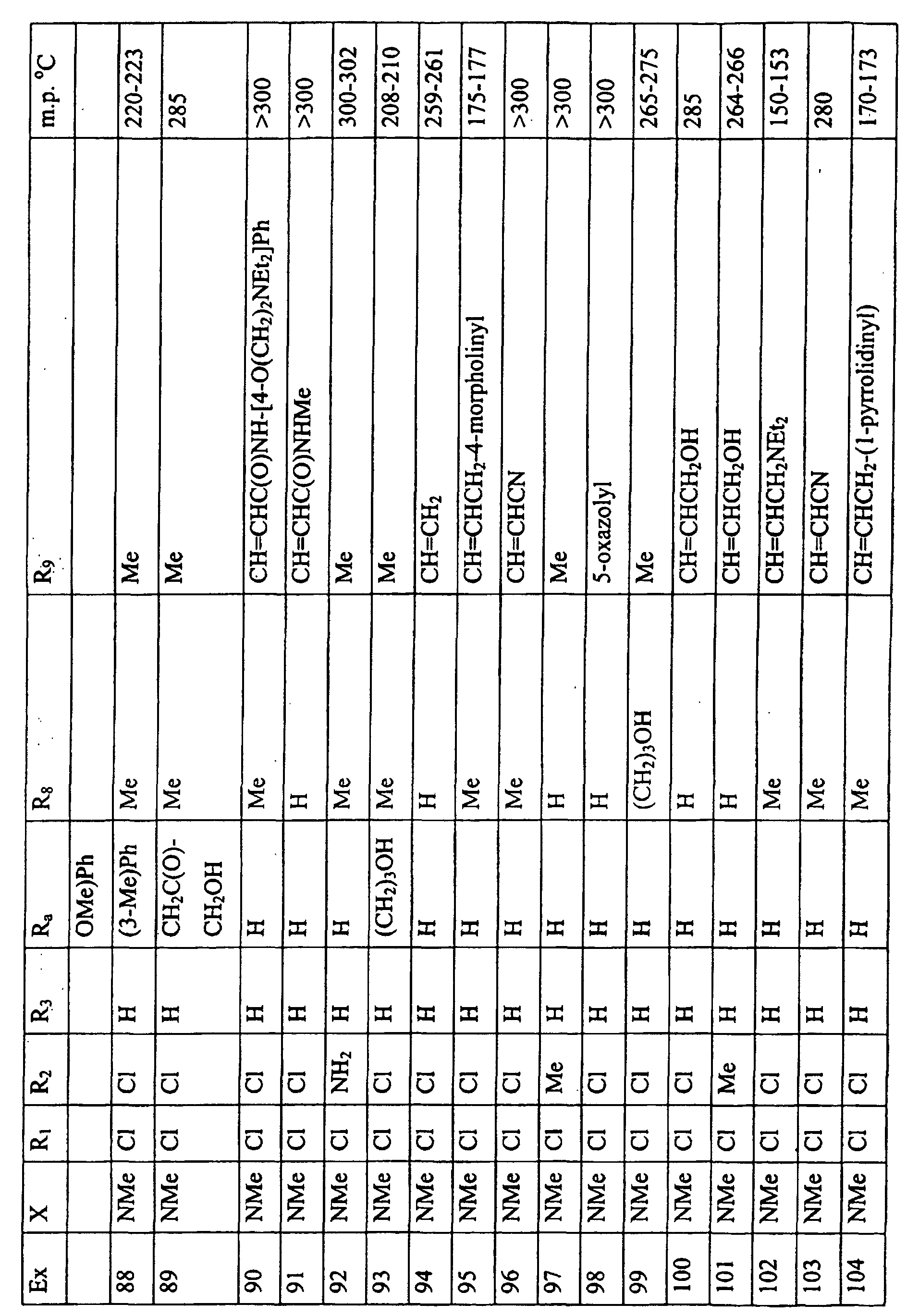

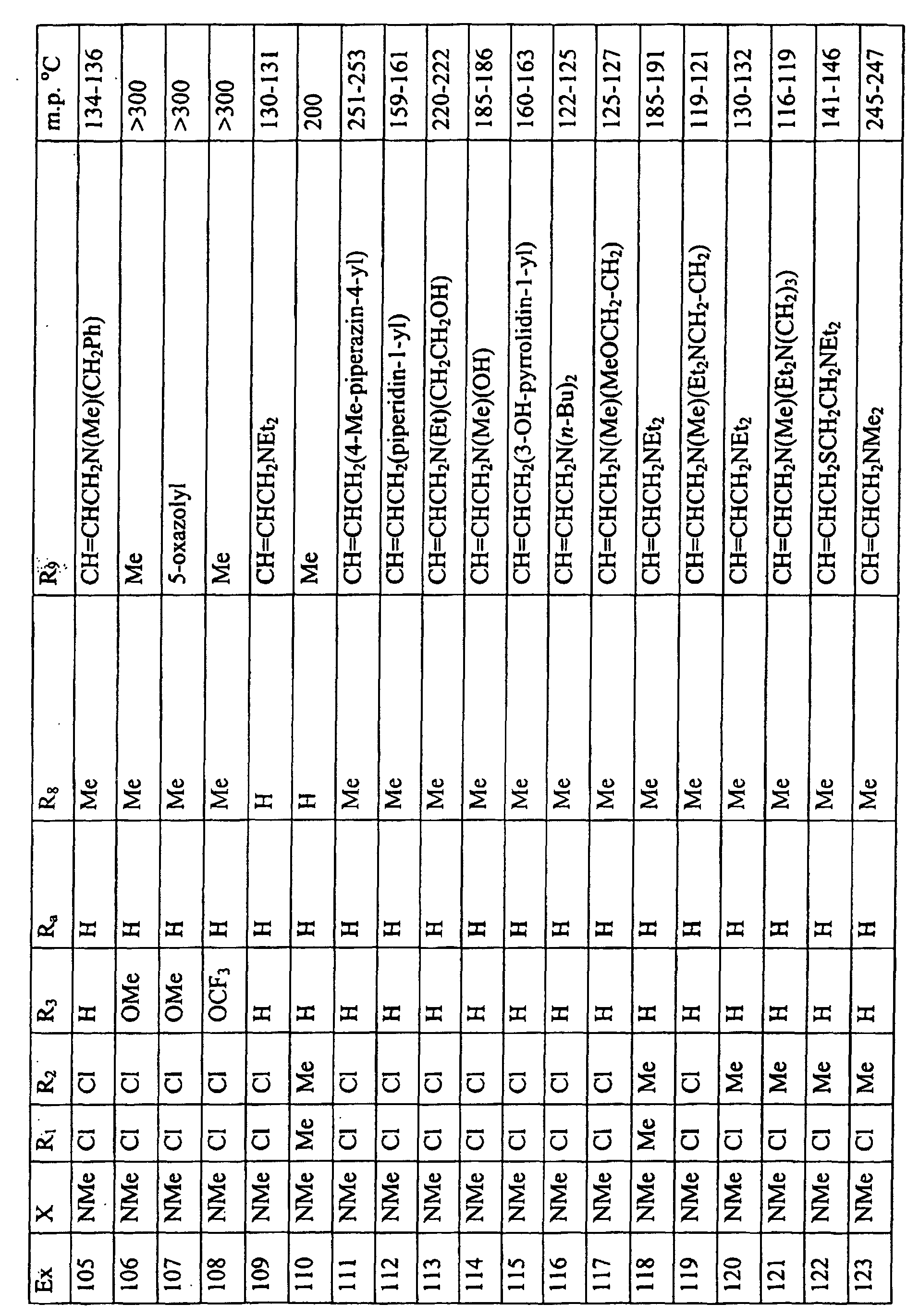

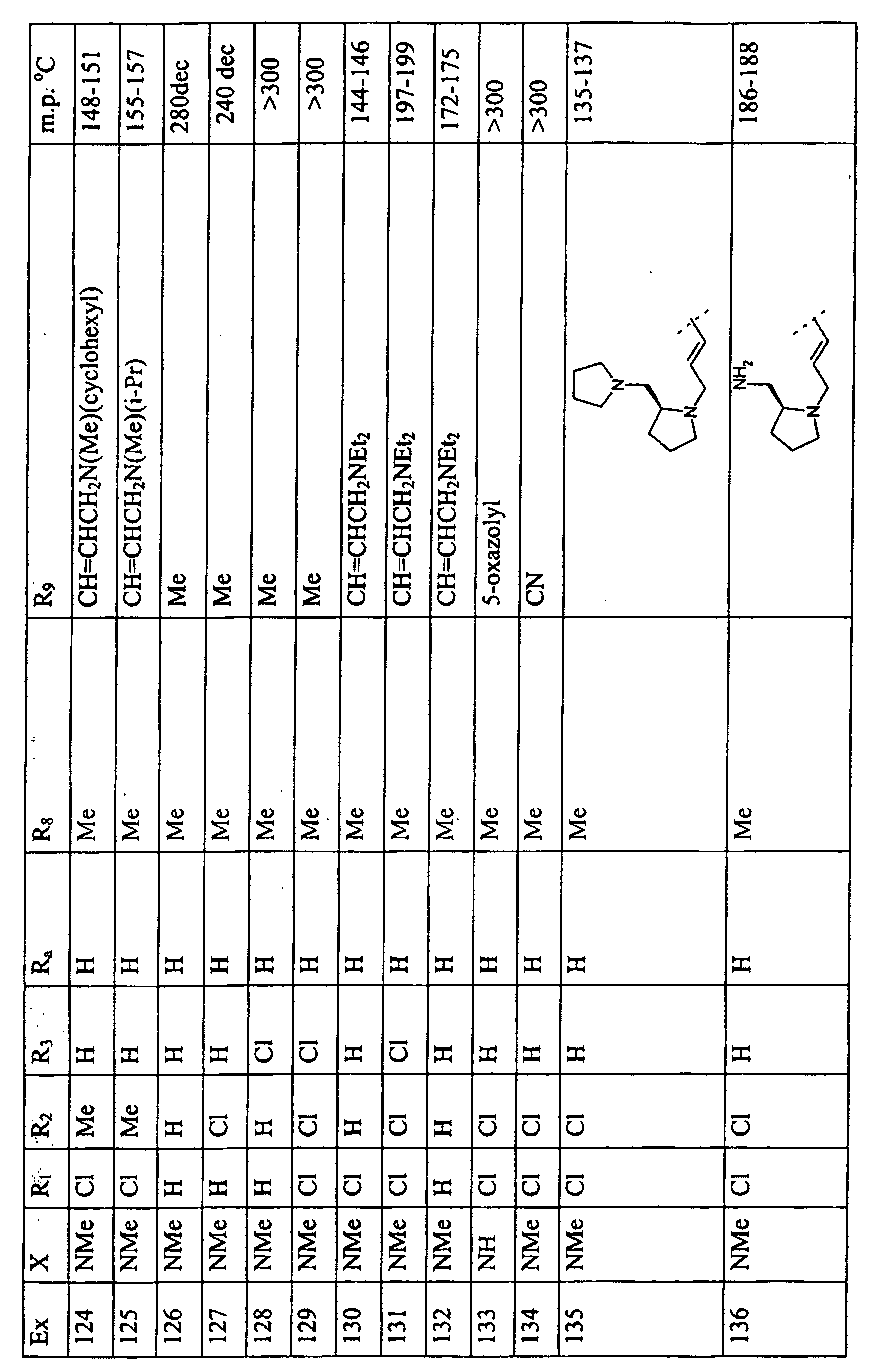

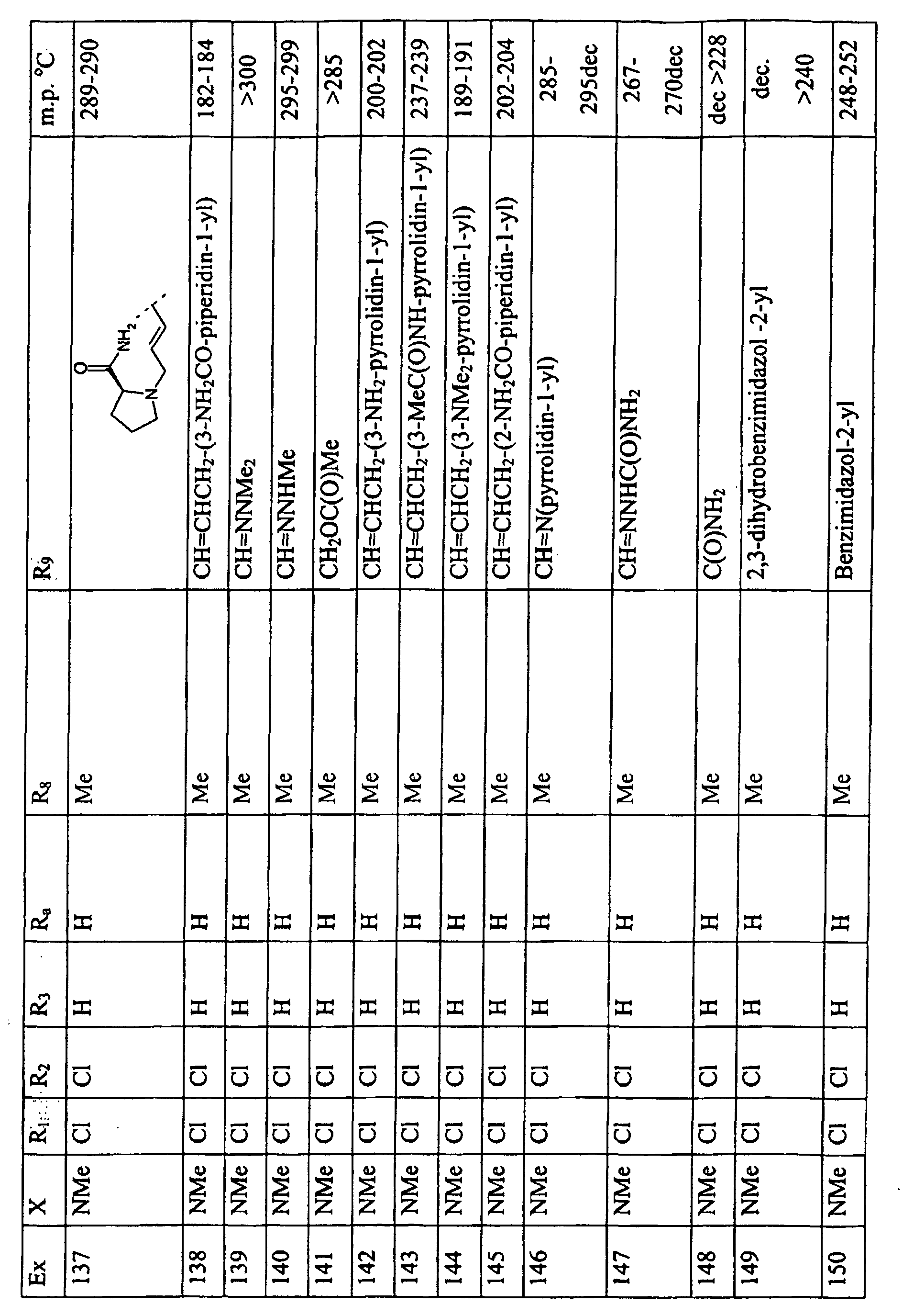

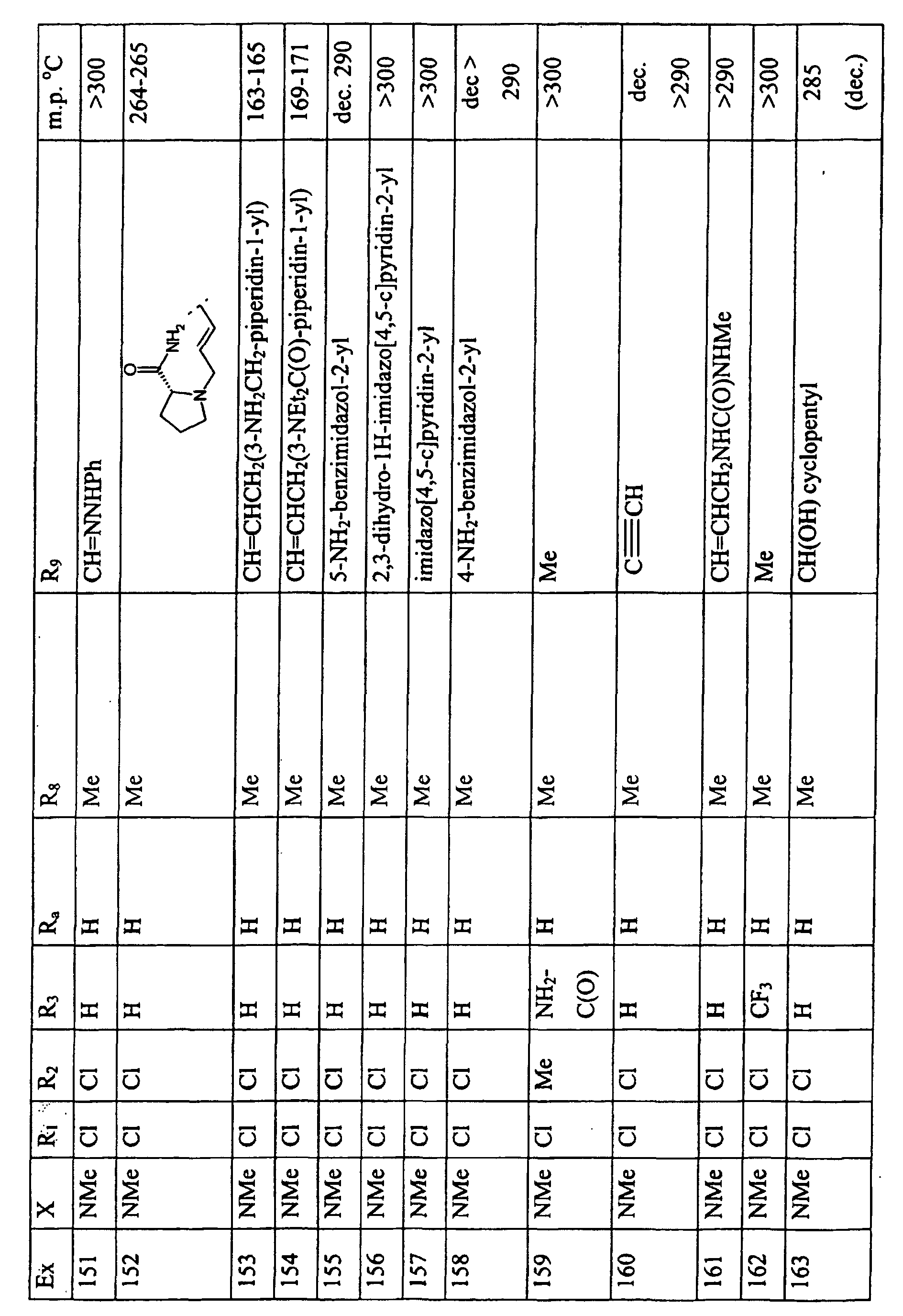

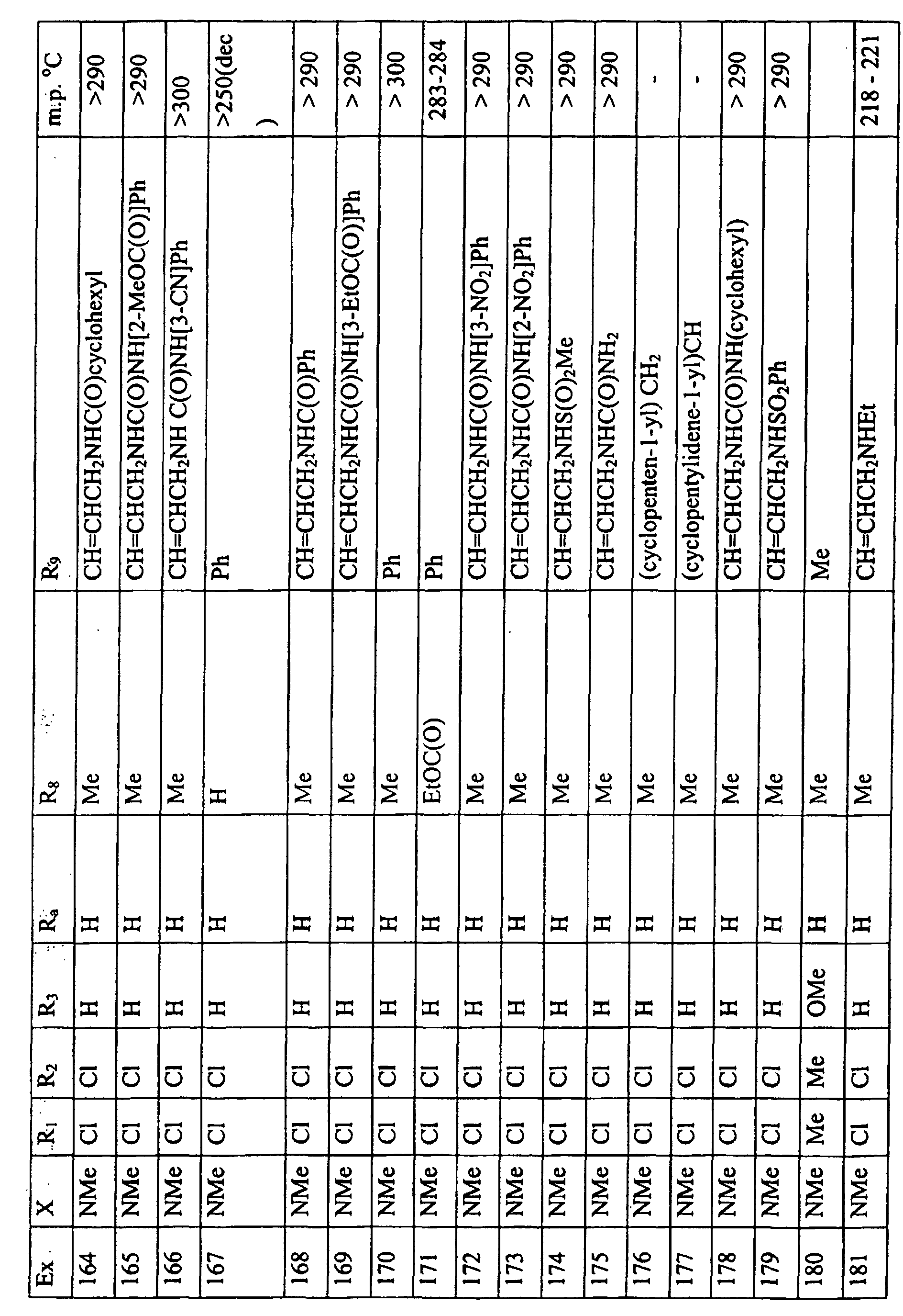

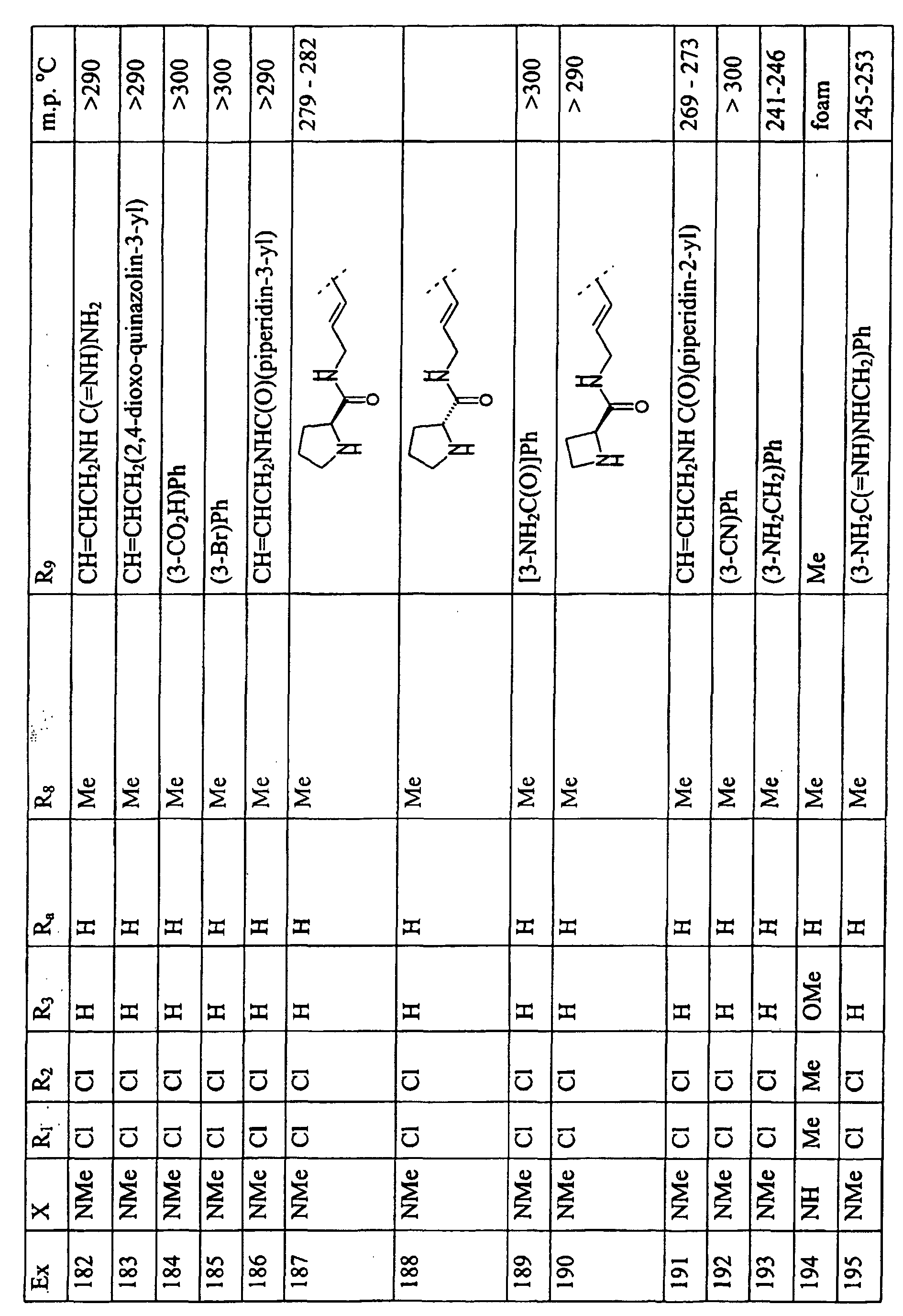

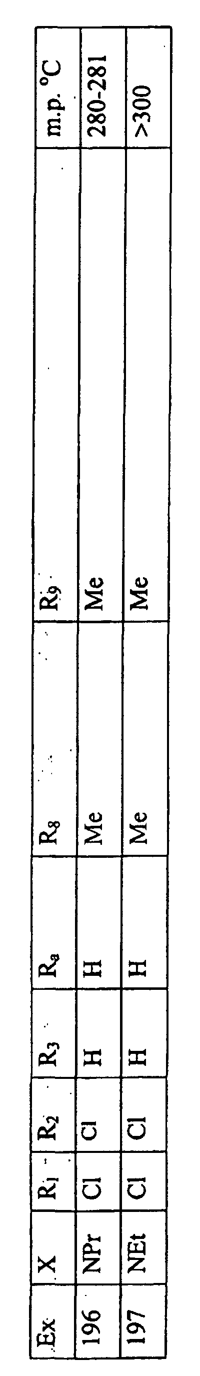

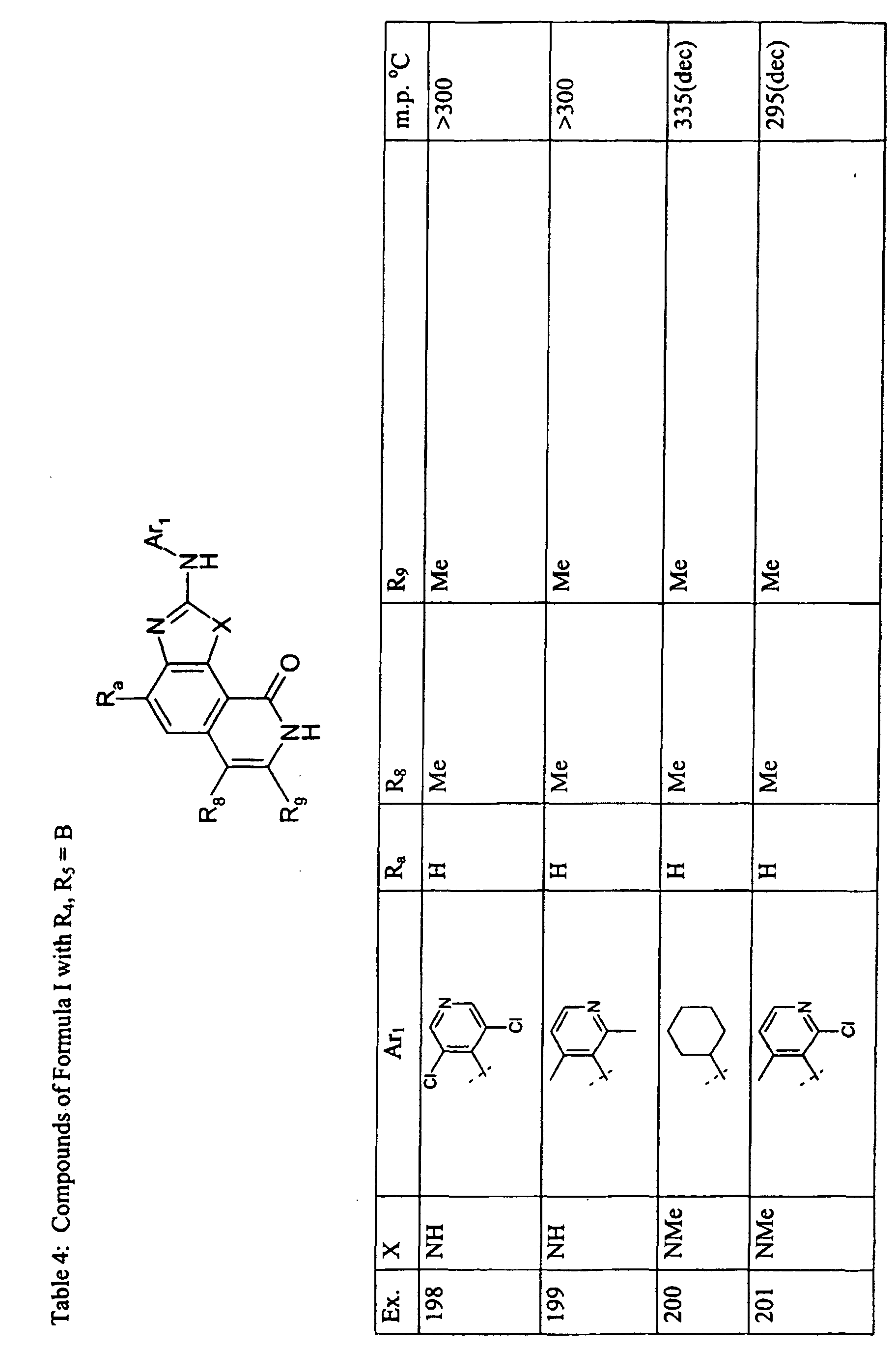

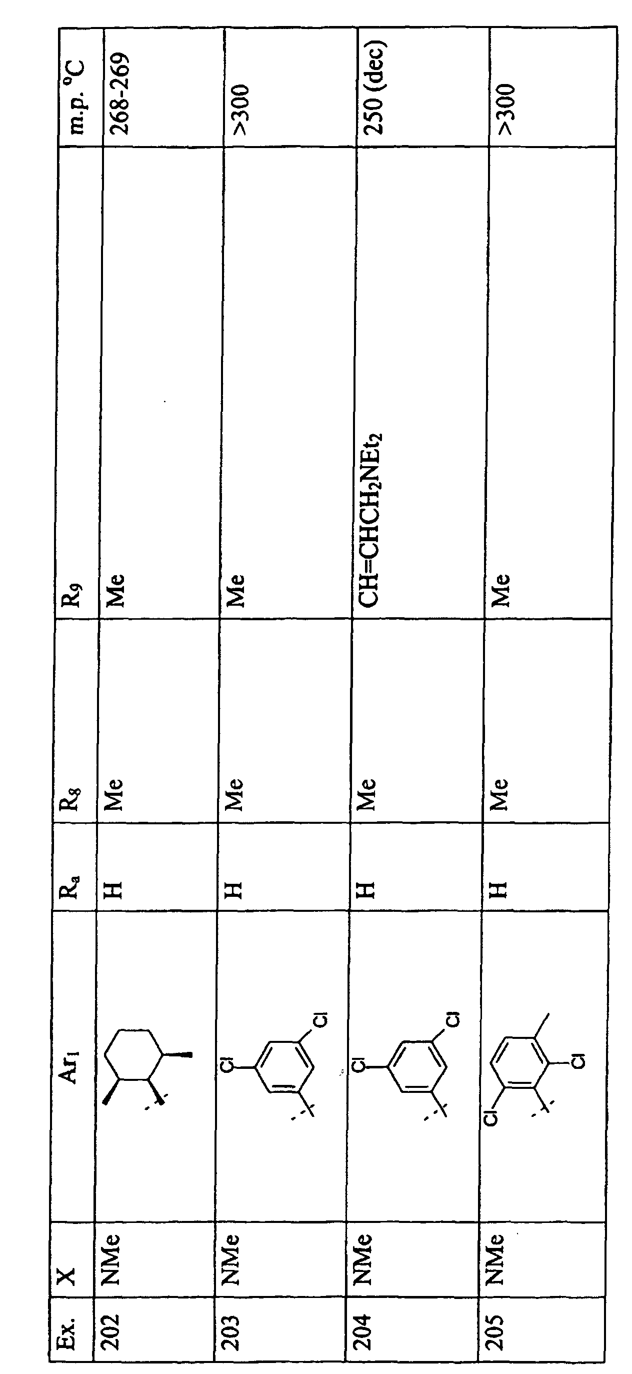

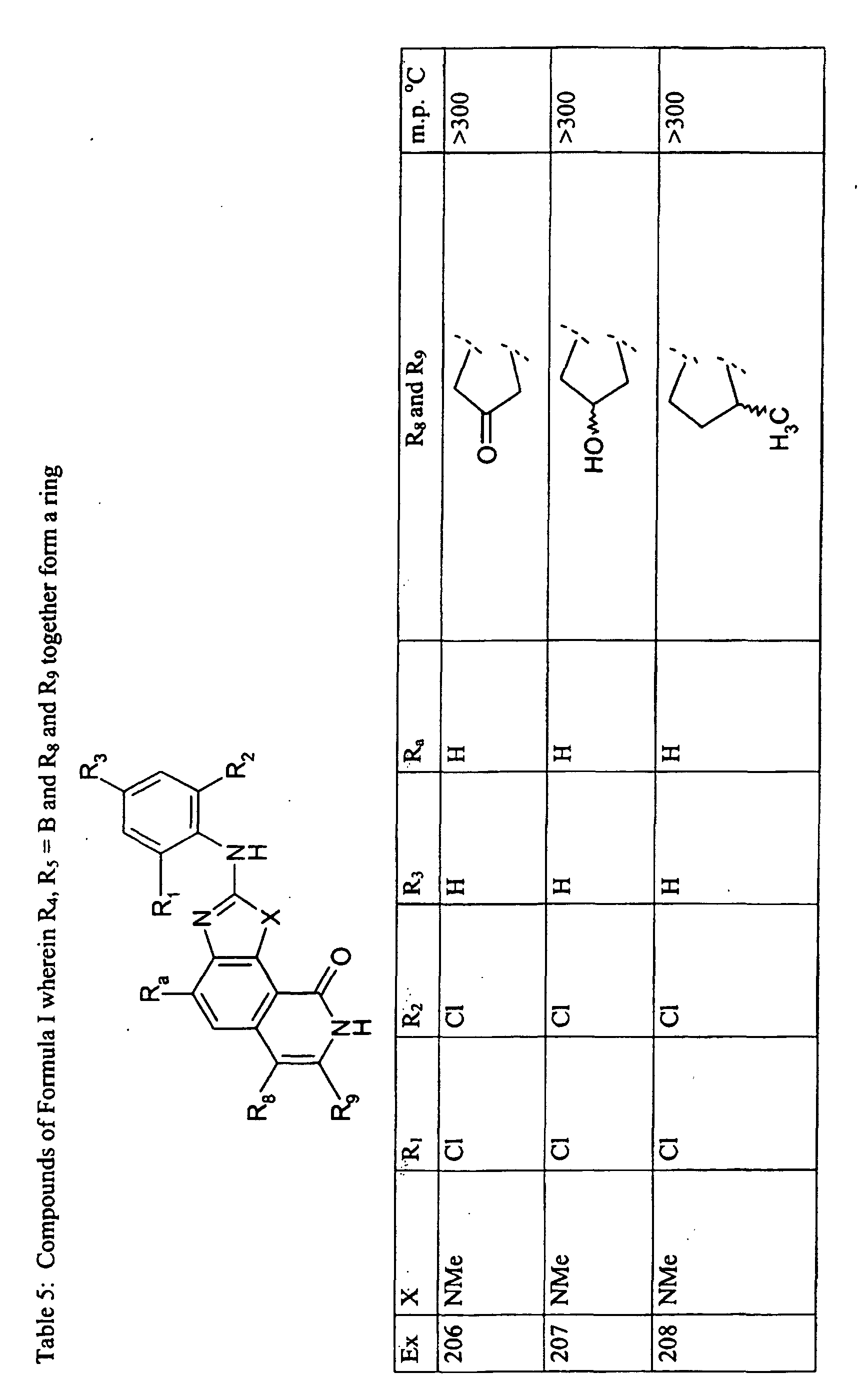

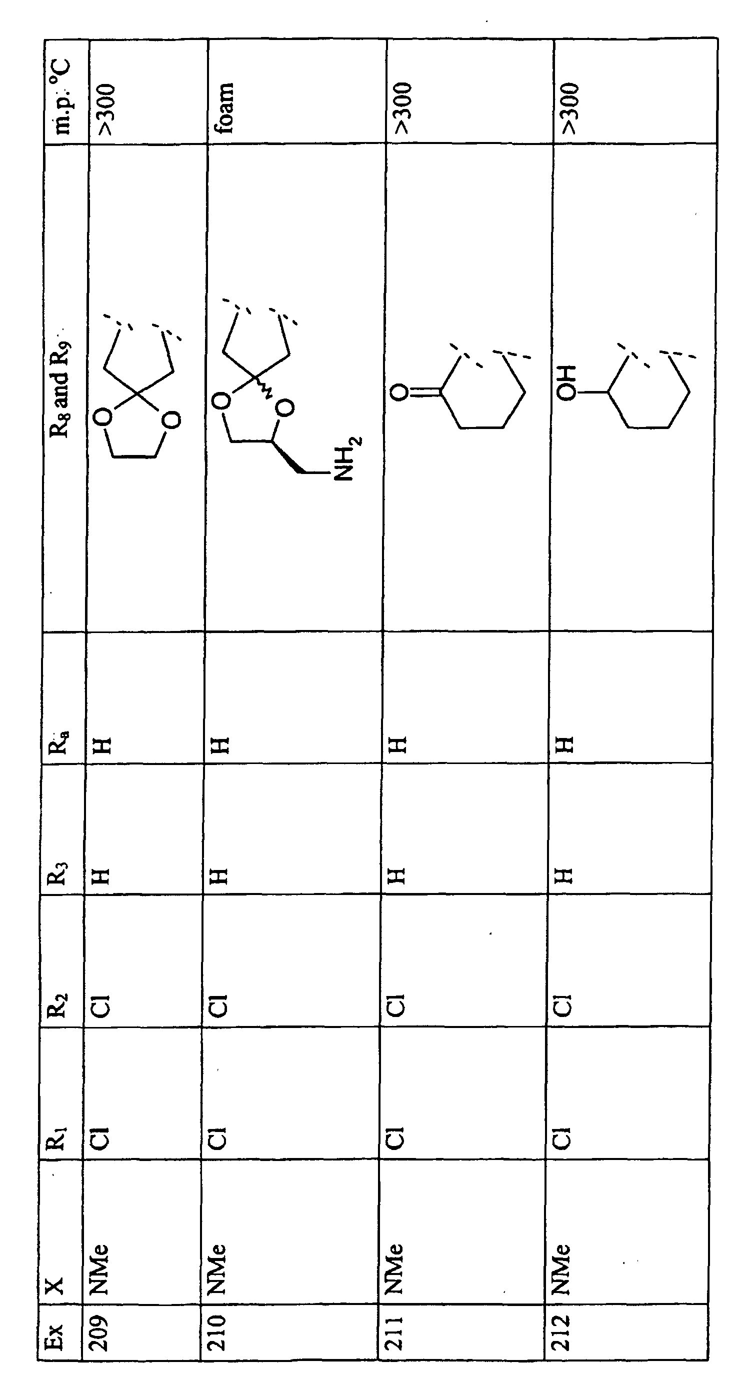

### Assessment of Biological Properties

### Tyrosine Kinase Inhibition Assay

The inhibition of tyrosine kinases by the compounds of the invention was measured with the following assay.

Kinase Reaction Buffer 50mM Hepes, pH 7.5, 50mM KCl, 25mM MgCl₂, 5mM MnCl₂, 100 µM, Na₃VO₄, .01% CHAPS, 1mM DTT, and 50mg/mL BSA, Adenosine 5'-Triphosphate (ATP) solution at 100mM, pH 7.5 -γ33P-ATP, 2000 Ci/mmol at 10µCi/µl, - Poly(L-glutamic acid-L-tyrosine, 4:1) or (E4Y)ₙ at 10mg/mL in water.

Assay: Test compounds, obtained routinely at 5mg/mL in 100% DMSO were diluted appropriately into complete Kinase assay buffer with 10% DMSO, 10µl of the 6Xcompound solution was distributed into each assay well, the final compound concentration for IC₅₀ determinations ranged from 200 to 1µg/mL. [γ33P]-ATP label was prepared as a 10 Ci/mmol working solution in complete Kinase assay buffer. Protein kinase was initiated by adding 10 to 50ηg of diluted enzyme stock.

Plates were incubated at 30 °C for 30 min. During the incubation period, the MultiScreen harvest plates were pre-wetted with 10% TCA/5% Ppi. 150µl of TCA/PPi was added to all MultiScreen plate wells after pre-wetting. The kinase reaction was stopped via replica transfer of the polypropylene reaction wells into the MultiScreen plates. The plates were incubated at room temperature for 5 min then vacuum harvested and washed with 200 µl TCA/PPi 3-4 times per well, then 100 µl of cocktail per well was added.

Experimental data consisted of eight (8) compound doses in duplicate with ten (10) enzyme control reaction wells (so-called totals) and six (6) background wells. The results were obtained as percent inhibition (mean with S.D.) over the full compound dose range. IC₅₀ potency estimates are determined using a floating inhibition maximum (Imax).

All compounds in the synthetic examples and Tables above were evaluated in the tyrosine kinase assay above using a kinase such as p56 lck and were found to have IC₅₀'s less than 10 µM.

Representative compounds from the examples above were evaluated in the tyrosine kinase assay above using p60 src and were found to have IC₅₀'s less than 10 µM.

Representative compounds from the examples above were evaluated in the tyrosine kinase assay above using PDGFR kinase and were found to have IC₅₀'s less than 10 µM.

### Inhibition of IL-2 Production

Inactivation of T cells resulting from inhibition of the tyrosine kinase p56 lck can be measured by inhibition of IL-2 production in Jurkat cells. 96-well flat bottom plates were coated with anti-CD3, clone UCHT1, (Immunotech cat. # 1304) at 4 µg/ml in Phosphate Buffered Saline (PBS), 100 µl/well. The solution was prepared by taking 200 µl of 200 µg/ml anti-CD3 stock/ 10ml PBS. The plate was then incubated at 37°C for 2h. Jurkat cells were pelleted and counted. The cells were resuspended at 2.5 x 10⁶ cells/ml in RPMI, 10 % FBS (complete media). Test compounds were diluted from a 5mg/ml DMSO stock directly into complete media.

10 µl of 20 X compound/ well was added to a separate plate, followed by 100µl of cell suspension in triplicate and this plate was preincubated at 37°C for 30min. The 96-well plate containing anti-CD3 was aspirated, and the cells and compound transferred to this plate. 100 µl of PMA (Phorbol 12-Myristate 13-Acetate, Sigma cat.# P-8139) at 20 ng/ml was added, and the plate was incubated overnight at 37° C. (PMA stock at 1 mg/ml in ethanol, dilute 10 µl/ml in complete media, then 20 µl/10 mls. in complete media. 100 µl/well = 10 ng/ml. final concentration). The next day, the plate was centrifuged at 1500 rpm for 5 min. at room temperature and the supernatants were removed. The supernatants were tested using R&D Systems Quantikine Human IL-2 Kit (cat.#2050). Samples were diluted 1:5 in RPMI1640, and 100 µl/well used in the ELISA. The optical density of each well was determined using a microplate reader set to 450 nm. EC₅₀ values were determined using Origin (non-linear regression) or SAS by plotting absorbance vs. concentration of compound.

Representatives from the synthetic examples and the Tables above were screened in this assay and had IC₅₀·s below 10 µM.

## Claims

1. A Compound of the formula (I): 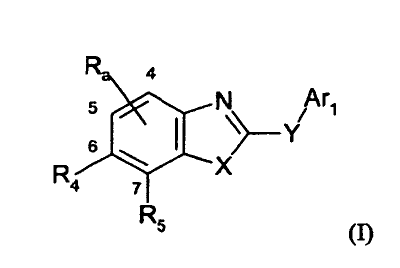 wherein:
Ar₁ is an aromatic or nonaromatic carbocycle, heteroaryl or heterocycle; wherein said carbocycle, heteroaryl or heterocycle is optionally substituted by one or more R₁, R₂ and R₃;
X is NH, N-C₁₋₃alkyl, N-cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl; wherein each Rₐ is independently optionally substituted with one or more C₁₋₆alkyl, C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, CONR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 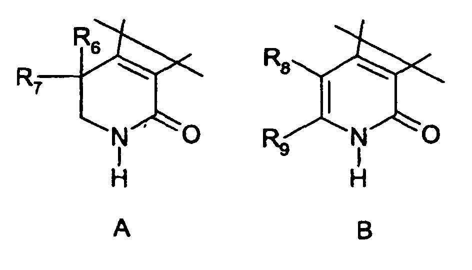
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁; or R₈ is phenyl or heteroaryl, each being optionally substituted with C₁₋₃alkyl, C₁₋₃alkoxy, OH, -SO₃H or halogen;
R₉ is H, CN or CONR₁₀R₁₁; or R₉ is C₁₋₁₀alkyl branched or unbranched, C₃₋₁₀cycloalkyl, C₅₋₇cycloalkenyl, C₂₋₆ alkenyl, C₂₋₆ alkynyl each being optionally substituted with one or more C₃₋₁₀cycloalkyl, C₃₋₁₀cycloalkylidene, C₅₋₇cycloalkenyl, halogen, OH, oxo, CN, C₁₋₃ alkoxy, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl, heteroaryl, or heterocycle, wherein each aryl, heteroaryl or heterocycle is optionally substituted with one to three groups selected from C₁₋₃alkyl optionally substituted with phenyl or NR₁₀C(=NR₁₀)NR₁₀R₁₁, C₁₋₃alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by one or two C₁₋₃alkyl, OH, oxo or (CH₂)ₙNR₁₀R₁₁, or optionally spiro-fused to a 1,3 dioxolane group or 1,3 dithiolane group, each 1,3 dioxolane group or 1,3 dithiolane group optionally substituted by C₁₋₆alkyl, C₁₋₆ alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, CN, NO₂, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH, -(CH₂)ₙNR₁₃R₁₄, CONR₁₃R₁₄ or NR₁₃COR₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl or heterocycle, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R₁₁;
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂;
R₁₅ is H or C₁₋₃ alkyl;
m is 1-4; n is 0-3 and p is 0-2;
wherein the term heteroaryl refers to a stable 5-8 membered monocyclic or 8-11 membered bicyclic aromatic heterocycle radical consisting of carbon atoms and from 1 to 4 heteroatoms chosen from nitrogen, oxygen and sulfur;
the term heterocycle refers to a stable 4-8 membered monocyclic or 8-11 membered bicyclic heterocycle radical which may be either saturated or unsaturated, and is non-aromatic consisting of carbon atoms and from 1 to 4 heteroatoms chosen from nitrogen, oxygen and sulfur;
the term aryl refers to a 6-10 membered aromatic carbocycle;
the term carbocycle refers to a 3-10 membered aromatic or nonaromatic cyclic carbon chain; and
the pharmaceutically acceptable acid or salt derivatives thereof.

2. The compound according to claim 1 wherein
Ar₁ is
a) a cycloalkyl group selected from cyclopropyl, cyclobutyl, cyclopentanyl, cyclohexanyl, cycloheptanyl;
b) a cycloalkenyl group selected from cyclopentenyl, cyclohexenyl, cycloheptenyl;
c) phenyl, naphthyl; indanyl, indenyl, dihydronaphthyl, tetrahydronaphthyl, fluorenyl;
d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, and indazolyl,or a fused heteroaryl selected from cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
e) a heterocycle selected from pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
wherein each of the above Ar₁ are optionally substituted by one or more R₁, R₂ and R₃;
Rₐ is H, C₁₋₆alkyl, C₂₋₅alkenyl, C₂₋₅alkynyl, phenyl or heteroaryl selected from pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₁ and R₂ are as hereinabove defined;
R₃ is H, halogen, methyl, methoxy, hydroxymethyl or OH;
R₈ is H, C₁₋₃alkyl branched or unbranched, saturated or unsaturated, optionally substituted with OH; or R₈ is (CH₂)₂₋₃NR₁₀R₁₁, (CH₂)ₙCO₂R₁₂ or (CH₂)ₙCONR₁₀R₁₁;
R₉ is CN or CONR₁₀R_{11;} or R₉ is C₁₋₃alkyl branched or unbranched, C₂₋₄ alkenyl, C₂₋₄ alkynyl each being optionally substituted with one or more C₅₋₇cycloalkyl, C₅₋₇ cycloalkylidene, C₅₋₇cycloalkenyl,OH, CN, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, CONR₁₀R₁₁, CO₂R₁₂, C(R₁₀)=NNR₁₀R₁₁, C(R₁₀)=NNR₁₀CONR₁₀R₁₁, aryl or heteroaryl; wherein each aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl,heteroaryl or heterocycle, each optionally substituted with one to three groups selected from C₁₋₃alkyl optionally substituted with phenyl or NR₁₀C(=NR₁₀)NR₁₀R₁₁, C₁₋₃ alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by C₁₋₃alkyl or OH, or optionally spiro-fused to a 1,3 dioxolane group or 1,3 dithiolane group, each 1,3 dioxolane group or 1,3 dithiolane group optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl, benzyl or phenyl are optionally substituted with OH, C₁₋₃alkoxy, C₁₋₃ acyloxy, CN, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH, -(CH₂)ₙNR₁₃R₁₄, CONR₁₃R₁₄ or NR₁₃COR₁₄;;
R₁₂ is H, C₁₋₆alkyl or C₅₋₇cycloalkyl, each optionally substituted with phenyl, OH, C₁₋₃ alkoxy or NR₁₃R₁₄; or R₁₂ is phenyl or heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂; and
R₁₅ is H
wherein the term heteroaryl refers to a stable 5-8 membered monocyclic or 8-11 membered bicyclic aromatic heterocycle radical consisting of carbon atoms and from 1 to 4 heteroatoms chosen from nitrogen, oxygen and sulfur;
the term heterocycle refers to a stable 4-8 membered monocyclic or 8-11 membered bicyclic heterocycle radical which may be either saturated or unsaturated, and is non-aromatic consisting of carbon atoms and from 1 to 4 heteroatoms chosen from nitrogen, oxygen and sulfur;
the term aryl refers to a 6-10 membered aromatic carbocycle;
the term carbocycle refers to a 3-10 membered aromatic or nonaromatic cyclic carbon chain;.

3. The compound according to claim 2 wherein:
Ar₁ is phenyl, or pyridyl, wherein each is optionally substituted by one or more
R₁, R₂ and R₃ as defined below;
X is NH or N-CH₃;
YisNHand
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
R₁ and R₂ are the same or different and selected from: H, halogen, C₁₋₃ alkyl, wherein the C₁₋₃ alkyl are optionally partially or fully halogenated, NO₂, NR₁₃R₁₄;
R₃ is H, halogen, methoxy or methyl;
R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is CN; or R₉ is methyl, C₂₋₃ alkenyl or C₂₋₃ alkynyl, each being optionally substituted with one or more C₅₋₇cycloalkylidene, C₅₋₇cycloalkenyl, OH, CN, NR₁₀R₁₁, NR₁₀CONR₁₀R₁₁, NR₁₀COR₁₂, NR₁₀S(O)ₚR₁₂, CONR₁₀R₁₁, CO₂R_{12,} C(R₁₀)=NNR₁₀R₁₁ or heteroaryl;
or R₉ is aryl or heteroaryl optionally substituted with one to three groups selected from C₁₋₃alkyl optionally substituted with phenyl, C₁₋₃alkoxy, halogen, amino or CONH₂;
or R₈ and R₉ together form a cyclopentene ring spiro-fused to a 1,3 dioxolane group, said 1,3 dioxolane group being optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₃alkyl branched or unbranched, C₅₋₇cycloalkyl or phenyl, wherein said alkyl, cycloalkyl or phenyl are optionally substituted with OH, C₁₋₃alkoxy, C₁₋₃acyloxy, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy,OH, (CH₂)ₙNR₁₃R₁₄, CONR₁₃R₁₄ or NR₁₃COR₁₄;
R₁₂ is H, C₁₋₃alkyl or C₅₋₇cycloalkyl, each optionally substituted with phenyl, OH, C₁₋₃ alkoxy or NR₁₃R₁₄; or R₁₂ is phenyl or is a saturated, 4- to 6-membered nitrogen-containing heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₃alkyl optionally substituted with C₁₋₃alkoxy or OH;
or R₁₃ and R₁₄ together form a chain completing a ring, said chain is (CH₂)₄₋₅ or (CH₂)₂O(CH₂)₂.

4. The compound according to claim 3 wherein:
Ar₁ is phenyl;
Rₐ is H or hydroxymethyl;
R₁ and R₂ are the same or different and selected from: halogen, methyl optionally partially or fully halogenated, NO₂ and NH₂;
R₃ is H, chloro, fluoro, bromo or methoxy;
R₁₀ and R₁₁ may be the same or different and are each independently selected from H, OH, methoxy, C₁₋₃alkyl branched or unbranched or C₅₋₇cycloalkyl, wherein said alkyl or cycloalkyl are optionally substituted with OH, NR₁₃R₁₄ or phenyl;
or R₁₀ and R₁₁ together form morpholino, pyrrolidinyl, piperazinyl or piperidinyl each optionally substituted by C₁₋₂ alkyl, NR₁₃R₁₄, CONR₁₃R₁₄ or NR₁₃COR₁₄; and
R₁₂ is C₁₋₃alkyl optionally substituted with morpholino; or R₁₂ is phenyl or is azetidinyl, pyrrolidinyl or piperidinyl, each optionally substituted with one to three groups selected from C₁₋₃alkyl, C₁₋₃alkoxy and halogen.

5. A compound according to claim 1, wherein:
Ar₁ is an aromatic or nonaromatic carbocycle, heteroaryl or heterocycle; wherein said carbocycle, heteroaryl or heterocycle is optionally substituted by one or more R₁, R₂ and R₃;
X is NH, N-C₁₋₃alkyl, N-cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl; wherein each Rₐ is independently optionally substituted with one or more C₁₋₃alkyl, C₁₋₆ alkoxy, halogen, OH, oxo, NR₁₀R₁₁, aryl or heteroaryl, each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 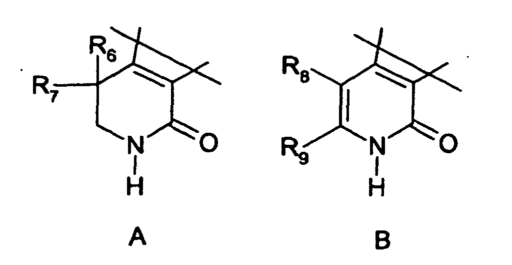
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁; or R₈ is phenyl or heteroaryl, each being optionally substituted with C₁₋₃alkyl, C₁₋₃alkoxy, OH, -SO₃H or halogen;
R₉ is H; or R₉ is C₁₋₁₀alkyl branched or unbranched, C₃₋₁₀cycloalkyl, C₂₋₆ alkenyl, C₂₋₆ alkynyl each being optionally substituted with one or more halogen, OH, oxo, CN, C₁₋₃ alkoxy, NR₁₀R₁₁, NR₁₀COR₁₂, SR₁₂, CONR₁₀R₁₁, CO₂R₁₂, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl or heteroaryl, wherein each aryl or heteroaryl is optionally substituted with one to three groups selected from C₁₋₃alkyl optionally substituted with phenyl, C₁₋₃ alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by one or two OH, oxo or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂;
R₁₅ is H or C₁₋₃ alkyl;
m is 1-4; n is 0-3 and p is 0-2; and
the pharmaceutically acceptable acid or salt derivatives thereof.

6. A compound according to claim 5, wherein:
Ar₁ is
a) a cycloalkyl group selected from cyclopropyl, cyclobutyl, cyclopentanyl, cyclohexanyl, cycloheptanyl;
b) a cycloalkenyl group selected from cyclopentenyl, cyclohexenyl, cycloheptenyl;
c) phenyl, naphthyl; indanyl, indenyl, dihydronaphthyl, tetrahydronaphthyl, fluorenyl;
d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl, and indazolyl,or a fused heteroaryl selected from cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
e) a heterocycle selected from: pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
wherein each of the above Ar₁ are optionally substituted by one or more R₁, R₂ and R₃ as hereinabove defined;
Rₐ is H, C₁₋₆alkyl, C₂₋₅alkenyl, C₂₋₅alkynyl, phenyl or heteroaryl selected from: pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₃ is H, halogen, methyl, methoxy, hydroxymethyl or OH;
R₈ is H, C₁₋₃alkyl branched or unbranched, saturated or unsaturated, optionally substituted with OH; or R₈ is (CH₂)₂₋₃NR₁₀R₁₁, (CH₂)ₙCO₂R₁₂ or (CH₂)ₙCONR₁₀R₁₁;
R₉ is C₁₋₃alkyl branched or unbranched, C₂₋₄ alkenyl, C₂₋₄alkynyl each being optionally substituted with one or more OH, CN, NR₁₀R₁₁, CONR₁₀R₁₁, CO₂R₁₂, aryl or heteroaryl;
wherein each aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl or heteroaryl optionally substituted with one to three groups selected from C₁₋₃ alkyl optionally substituted with phenyl, C₁₋₃alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by OH;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl, benzyl or phenyl are optionally substituted with OH, C₁₋₃alkoxy, C₁₋₃ acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH or -(CH₂)ₙNR₁₃R₁₄;
R₁₂ is H or C₁₋₆alkyl optionally substituted with phenyl, OH, C₁₋₃alkoxy or NR₁₃R₁₄;
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂; and
R₁₅ is H.

7. The compound according to claim 6 wherein:
Ar₁ is phenyl, or pyridyl;
X is NH or N-CH₃;
Y is NH and
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
R₁ and R₂ are the same or different and selected from: halogen, C₁₋₃ alkyl, wherein the C₁₋₃ alkyl are optionally partially or fully halogenated, NO₂, NR₁₃R₁₄;
R₃ is H, halogen, methoxy or methyl;
R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is methyl or C₂₋₃ alkenyl each being optionally substituted with one or more OH, CN, NR₁₀R₁₁, CONR₁₀R₁₁ or CO₂R₁₂;
or R₉ is heteroaryl optionally substituted with one to three groups selected from C₁₋₃alkyl optionally substituted with phenyl, C₁₋₃alkoxy, halogen or amino;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
or R₁₃ and R₁₄ together form a chain completing a ring, said chain is (CH₂)₄₋₅ or (CH₂)₂O(CH₂)₂.

8. The compound according to claim 7 wherein:
Ar₁ is phenyl;
Rₐ is H or hydroxymethyl;
R₁ and R₂ are the same or different and selected from: halogen, methyl optionally partially or fully halogenated, NO₂ and NH₂;
R₃ is H, chloro, fluoro, bromo or methoxy;
R₁₀ and R₁₁ may be the same or different and are each independently selected from H, OH, methoxy, C₁₋₃alkyl branched or unbranched, optionally substituted with OH, NR₁₃R₁₄ or phenyl;
or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

9. A compound according to claim 1 of the formula (Ia): 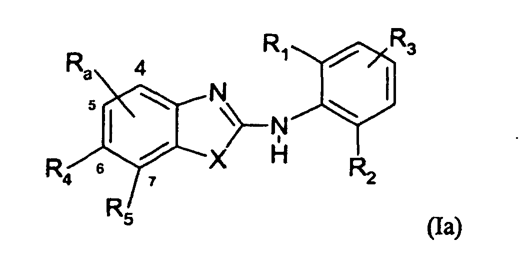 wherein:
X is NH, N-C₁₋₃alkyl, N-cyclopropyl, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
wherein each Rₐ is independently optionally substituted with one or more C₁₋₆alkyl, C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃ alkoxy, hydroxyC₁₋₃alkyl and (CH₂)ₘNR₁₀R₁₁; and wherein Rₐ is attached at the 4- or 5-position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, CONR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 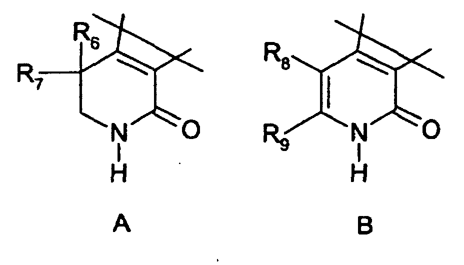
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁ or R₈ is phenyl or heteroaryl, each being optionally substituted with C₁₋₃alkyl, C₁₋₃alkoxy, OH, -SO₃H or halogen;
R₉ is H, CN or CONR₁₀R₁₁; or R₉ is C₁₋₁₀alkyl branched or unbranched, C₃₋₁₀cycloalkyl, C₅₋₇cycloalkenyl, C₂₋₆ alkenyl, C₂₋₆ alkynyl each being optionally substituted with one or more C₃₋₁₀cycloalkyl, C₃₋₁₀cycloalkylidene, C₅₋₇cycloalkenyl, halogen, OH, oxo, CN, C₁₋₃ alkoxy, C₁₋₃acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl, heteroaryl, or heterocycle, wherein each aryl, heteroaryl or heterocycle is optionally substituted with one to three groups selected from C₁₋₃alkyl optionally substituted with phenyl or NR₁₀C(=NR₁₀)NR₁₀R₁₁, C₁₋₃alkoxy, halogen, CN, oxo, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 5 or 6 membered aromatic or nonaromatic carbocyclic ring optionally substituted by one or two C₁₋₃alkyl, OH, oxo or (CH₂)ₙNR₁₀R₁₁, or optionally spiro-fused to a 1,3 dioxolane group or 1,3 dithiolane group, each 1,3 dioxolane group or 1,3 dithiolane group optionally substituted by C₁₋₆alkyl, C₁₋₆alkoxy, OH or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, CN, NO₂, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH, -(CH₂)ₙNR₁₃R₁₄, CONR₁₃R₁₄ or NR₁₃COR₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl or heterocycle, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂;
m is 1-4, n is 0-3 and p is 0-2; and
the pharmaceutically acceptable acid or salt derivatives thereof.

10. The compound according to claim 9 wherein:
X is NH or N-CH₃;
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl; and wherein Rₐ is attached at the 4- position;
R₁ and R₂ are the same or different and selected from: H, halogen, C₁₋₃ alkyl, wherein the C₁₋₃ alkyl is optionally partially or fully halogenated, NO₂, NR₁₃R₁₄;
R₃ is H, halogen, methoxy or methyl;
R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is CN; or R₉ is methyl, C₂₋₃ alkenyl or C₂₋₃ alkynyl, each being optionally substituted with one or more C₅₋₇ cycloalkylidene, C₅₋₇cycloalkenyl, OH, CN, NR₁₀R₁₁, NR₁₀CONR₁₀R₁₁,, NR₁₀COR₁₂, NR₁₀S(O)ₚR₁₂, CONR₁₀R₁₁, CO₂R₁₂, C(R₁₀)=NNR₁₀R₁₁ or heteroaryl;
or R₉ is aryl or heteroaryl optionally substituted with one to three groups selected from C₁₋₃ alkyl optionally substituted with phenyl, C₁₋₃alkoxy, halogen, amino or CONH₂;
or R₈ and R₉ together form a cyclopentene ring spiro-fused to a 1,3 dioxolane group, said 1,3 dioxolane group being optionally substituted by C₁₋₃alkyl, C₁₋₃alkoxy, OH or (CH₂)ₙNR₁₀R₁₁;
R₁₀ and R₁₁ may be the same or different and are each independently selected from H, OH, C₁₋₃alkoxy, C₁₋₃alkyl branched or unbranched, C₅₋₇cycloalkyl or phenyl, wherein said alkyl, cycloalkyl or phenyl are optionally substituted with OH, C₁₋₃alkoxy, C₁₋₃acyloxy, NO₂, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy,OH, (CH₂)ₙNR₁₃R₁₄, CONR₁₃R₁₄ or NR₁₃COR₁₄;
R₁₂ is H, C₁₋₃alkyl or C₅₋₇cycloalkyl, each optionally substituted with phenyl, OH, C₁₋₃ alkoxy or NR₁₃R₁₄; or R₁₂ is phenyl or is a saturated, 4- to 6-membered nitrogen-containing heterocycle, each optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₃alkyl optionally substituted with C₁₋₃alkoxy or OH;
or R₁₃ and R₁₄ together form a chain completing a ring, said chain is (CH₂)₄₋₅ or (CH₂)₂O(CH₂)₂.

11. The compound according to claim 10 wherein:
Rₐ is H or hydroxymethyl;
R₁ and R₂ are the same or different and selected from: halogen, methyl optionally partially or fully halogenated, NO₂ and NH₂;
R₃ is H, chloro, fluoro, bromo or methoxy;
R₁₀ and R₁₁ may be the same or different and are each independently selected from H, OH, methoxy, C₁₋₃alkyl branched or unbranched or C₅₋₇cycloalkyl, wherein said alkyl or cycloalkyl are optionally substituted with OH, NR₁₃R₁₄ or phenyl;
or R₁₀ and R₁₁ together form morpholino, pyrrolidinyl, piperazinyl or piperidinyl each optionally substituted by C₁₋₂ alkyl, NR₁₃R₁₄, CONR₁₃R₁₄ or NR₁₃COR₁₄; and
R₁₂ is C₁₋₃alkyl optionally substituted with morpholino; or R₁₂ is phenyl or is azetidinyl, pyrrolidinyl or piperidinyl, each optionally substituted with one to three groups selected from C₁₋₃alkyl, C₁₋₃alkoxy and halogen.

12. The compound according to claim 9, wherein:
X is NH, N-C₁₋₃alkyl, N-cyclopropyl, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
wherein each Rₐ is independently optionally substituted with one or more C₁₋₆ alkoxy, halogen, OH, oxo, NR₁₀R₁₁, aryl or heteroaryl each aryl or heteroaryl being optionally substituted with one or more groups selected from halogen, OH, C₁₋₃alkyl, C₁₋₃alkoxy, hydroxyC₁₋₃alkyl and (CH₂)ₘNR₁₀R₁₁; and wherein Rₐ is attached at the 4- or 5- position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formulas A or B: 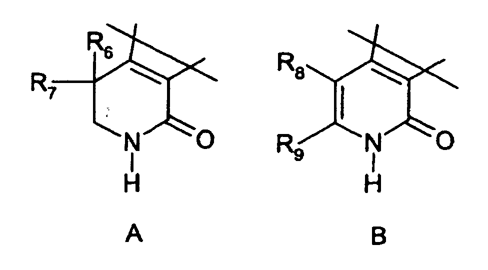
R₆ is C₁₋₃alkyl or H;
R₇ is C₁₋₆alkyl branched or unbranched or H;
R₈ is H, C₁₋₆alkyl branched or unbranched, saturated or unsaturated, optionally substituted with phenyl, OH or C₁₋₃alkoxy; or R₈ is (CH₂)ₘNR₁₀R₁₁, (CH₂)ₘNR₁₀COR₁₂, (CH₂)ₙCO₂R₁₂, (CH₂)ₙCONR₁₀R₁₁ or R₈ is phenyl or heteroaryl, each being optionally substituted with C₁₋₃alkyl, C₁₋₃alkoxy, OH, -SO₃H or halogen;
R₉ is H; or R₉ is C₁₋₁₀alkyl branched or unbranched, C₃₋₁₀cycloalkyl, C₂₋₆ alkenyl, C₂₋₆alkynyl each being optionally substituted with one or more halogen, OH, oxo, CN, C₁₋₃ alkoxy, NR₁₀R₁₁, NR₁₀COR₁₂, SR₁₂, CONR₁₀R₁₁, CO₂R₁₂, aryloxy, arylthio, aryl or heteroaryl; wherein each aryloxy, arylthio, aryl or heteroaryl is optionally substituted with C₁₋₃alkyl, C₁₋₃alkoxy, halogen, (CH₂)ₙNR₁₀R₁₁ or O(CH₂)₂₋₄NR₁₀R₁₁;
or R₉ is aryl or heteroaryl, wherein each aryl or heteroaryl is optionally substituted with one to three groups selected from C₁₋₃alkyl optionally substituted with phenyl, C₁₋₃alkoxy, halogen, (CH₂)ₙNR₁₀R₁₁, (CH₂)ₙCO₂R₁₂; (CH₂)ₙCONR₁₀R₁₁ and O(CH₂)₂₋₄NR₁₀R₁₁;
or R₈ and R₉ together form a saturated or unsaturated 6 membered aromatic or nonaromatic carbocyclic ring optionally substituted by one or two OH, oxo or (CH₂)ₙNR₁₀R₁₁;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ, and NR₁₃; and wherein said ring is optionally substituted by C₁₋₃ alkyl, C₁₋₃alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with C₁₋₃alkoxy, OH or phenyl;
or R₁₃ and R₁₄ together form a chain completing a ring, said chain is (CH₂)₄₋₅ or (CH₂)₂O(CH₂)₂;
m is 1-4, n is 0-3 and p is 0-2; and
the pharmaceutically acceptable acid or salt derivatives thereof.

13. The compound according to claim 12 wherein:
X is NH or N-CH₃;
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl; and wherein Rₐ is attached at the 4- position;
R₁ and R₂ are the same or different and selected from: halogen, C₁₋₃ alkyl, wherein the C₁₋₃ alkyl is optionally partially or fully halogenated, NO₂, NR₁₃R₁₄;
R₃ is H, halogen, methoxy or methyl;
R₄ and R₅ together complete a fused ring of formula B;
R₈ is H, C₁₋₃alkyl optionally substituted with OH; or R₈ is (CH₂)₂₋₃NR₁₀R₁₁ or CO₂R₁₂;
R₉ is methyl or C₂₋₃ alkenyl each being optionally substituted with one or more OH, CN, NR₁₀R₁₁, CONR₁₀R₁₁ or CO₂R₁₂;
or R₉ is heteroaryl optionally substituted with one to three groups selected from C₁₋₃alkyl optionally substituted with phenyl, C₁₋₃alkoxy, halogen or (CH₂)ₙNR₁₀R₁₁;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
or R₁₃ and R₁₄ together form a chain completing a ring, said chain is (CH₂)₄₋₅ or (CH₂)₂O(CH₂)₂.

14. The compound according to claim 13 wherein:
Rₐ is H or hydroxymethyl;
R₁ and R₂ are the same or different and selected from: halogen, methyl optionally partially or fully halogenated, NO₂ and NH₂;
R₃ is H, chloro, fluoro, bromo or methoxy;
R₁₀ and R₁₁ may be the same or different and are each independently selected from H, OH, methoxy, C₁₋₃alkyl branched or unbranched, optionally substituted with OH, NR₁₃R₁₄ or phenyl;
or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

15. An intermediate compound of the formula (III): 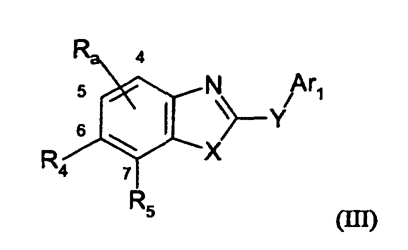 wherein:
Ar₁ is an aromatic or nonaromatic carbocycle, heteroaryl or heterocycle; wherein said carbocycle, heteroaryl or heterocycle is optionally substituted by one or more R₁, R₂ and R₃;
X is NH, N-C₁₋₃alkyl, N-cyclopropyl, S or O;
Y is NR₁₅, S or O;
Rₐ is H, C₁₋₁₀alkyl, C₂₋₁₀alkenyl or C₂₋₁₀alkynyl, each of which may be branched or cyclic; or Rₐ is aryl or heteroaryl;
wherein each Rₐ is independently optionally substituted with one or more C₁₋₃alkyl, C₁₋₆ alkoxy, halogen, OH, oxo, NR₁₀R₁₁, aryl or heteroaryl, each aryl or heteroaryl being optionally substituted with one or more groups selected from halogen, OH, C₁₋₃alkyl, C₁₋₃ alkoxy, hydroxyC₁₋₃alkyl and (CH₂)ₘNR₁₀R₁₁; and wherein Rₐ is attached at the 4- or 5-position;
R₁ and R₂ are the same or different and selected from H, halogen, CN, NO₂, C₁₋₁₀ branched or unbranched saturated or unsaturated alkyl, C₁₋₁₀ branched or unbranched alkoxy, C₁₋₁₀ branched or unbranched acyl, C₁₋₁₀ branched or unbranched acyloxy, C₁₋₁₀ branched or unbranched alkylthio, aminosulfonyl, di-(C₁₋₃)alkylaminosulfonyl, NR₁₀R₁₁, aryl, aroyl, aryloxy, arylsulfonyl, heteroaryl and heteroaryloxy; wherein the abovementioned R₁ and R₂ are optionally partially or fully halogenated or optionally substituted with one to three groups independently selected from oxo, OH, NR₁₀R₁₁, C₁₋₆ branched or unbranched alkyl, C₃₋₇cycloalkyl, phenyl, naphthyl, heteroaryl, aminocarbonyl and mono- or di(C₁₋₃)alkylaminocarbonyl;
R₃ is H, halogen, OH, (CH₂)ₙNR₁₀R₁₁, (CH₂)ₙCO₂R₁₂; C₁₋₃alkyl optionally substituted with OH, C₁₋₃ alkoxy optionally halogenated or C₁₋₃ alkylthio;
R₄ and R₅ together with the atoms to which they are attached complete a fused ring system of the formula C: 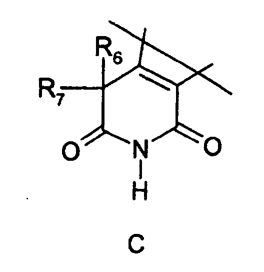
R₆ is C₁₋₃alkyl or H;
R₇ is C₁₋₆alkyl branched or unbranched or H;
R₁₀ and R₁₁ may be the same or different and are each independently selected from H, OH, C₁₋₃alkoxy, C₁₋₆alkyl branched or unbranched, C₃₋₈cycloalkyl, aryl, arylC₁₋₃alkyl and heteroaryl; wherein said alkyl, cycloalkyl, aryl, arylC₁₋₃alkyl or heteroaryl are optionally substituted with OH, C₁₋₃alkoxy, C₁₋₃acyloxy, CO₂R₁₂, NR₁₃R₁₄, O(CH₂)₂₋₄NR₁₃R₁₄, aryl or heteroaryl;
or R₁₀ and R₁₁ together form a 3-7 member alkylene chain completing a ring about the N atom to which they are attached; wherein said alkylene chain is optionally interrupted by O, S(O)ₚ and NR₁₃; and wherein said ring is optionally substituted by C₁₋₃ alkyl, C₁₋₃alkoxy, OH or -(CH₂)ₙNR₁₃R₁₄;
R₁₂ is H, C₁₋₆alkyl or C₃₋₈cycloalkyl wherein each alkyl or cycloalkyl is optionally substituted with phenyl, OH, C₁₋₃alkoxy or NR₁₃R₁₄; or R₁₂ is phenyl, optionally substituted with one to three groups selected from C₁₋₃alkyl, C₁₋₃alkoxy, halogen, (CH₂)ₘNR₁₀R₁₁, (CH₂)ₙCONR₁₀R₁₁ and O(CH₂)₂₋₄NR₁₀R_{11;}
R₁₃ and R₁₄ are each independently selected from H and C₁₋₆ alkyl optionally substituted with alkoxy, OH or phenyl;
or R₁₃ and R₁₄ together form a chain completing a ring, said chain is (CH₂)₄₋₅ or (CH₂)₂O(CH₂)₂; and
m is 1-4, n is 0-3 and p is 0-2;
wherein the term heteroaryl refers to a stable 5-8 membered monocyclic or 8-11 membered bicyclic aromatic heterocycle radical consisting of carbon atoms and from 1 to 4 heteroatoms chosen from nitrogen, oxygen and sulfur;
the term heterocycle refers to a stable 4-8 membered monocyclic or 8-11 membered bicyclic heterocycle radical which may be either saturated or unsaturated, and is non-aromatic consisting of carbon atoms and from 1 to 4 heteroatoms chosen from nitrogen, oxygen and sulfur;
the term aryl refers to a 6-10 membered aromatic carbocycle;
the term carbocycle refers to a 3-10 membered aromatic or nonaromatic cyclic carbon chain.

16. The compound according to claim 15 wherein
Ar₁ is
a) a cycloalkyl group selected from cyclopropyl, cyclobutyl, cyclopentanyl, cyclohexanyl, cycloheptanyl;
b) a cycloalkenyl group selected from cyclopentenyl, cyclohexenyl, cycloheptenyl;
c) phenyl, naphthyl; indanyl, indenyl, dihydronaphthyl, tetrahydronaphthyl, fluorenyl;
d) heteroaryl selected from pyridinyl, pyrimidinyl, pyrazinyl, pyridazinyl, pyrrolyl, imidazolyl, pyrazolyl, thienyl, furyl, isoxazolyl, isothiazolyl, oxazolyl, oxadiazolyl, thiazolyl, thiadiazolyl, quinolinyl, isoquinolinyl, indolyl, benzimidazolyl, benzofuranyl, benzoxazolyl, benzisoxazolyl, benzpyrazolyl, benzothiofuranyl, benzothiazolyl, quinazolinyl and indazolyl, or a fused heteroaryl selected from cyclopentenopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoline, cyclohexanoquinoline, cyclopentanoisoquinoline, cyclohexanoisoquinoline, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophene and cyclohexanothiophene; or
e) a heterocycle selected from: pyrrolinyl, pyrrolidinyl, pyrazolinyl, pyrazolidinyl, piperidinyl, morpholinyl, thiomorpholinyl, pyranyl, thiopyranyl, piperazinyl and indolinyl;
wherein each of the above Ar₁ are optionally substituted by one or more R₁, R₂ and R₃ as hereinabove defined;
Rₐ is H, C₁₋₆alkyl, C₂₋₅alkenyl, C₂₋₅alkynyl, phenyl or heteroaryl selected from: pyridinyl, pyridazinyl, pyrimidinyl, pyrazinyl, oxazolyl, pyrazolyl, imidazolyl, furyl, thiazolyl and thienyl; each Rₐ being optionally substituted with one or more phenyl, halogen, C₁₋₃alkyl, C₁₋₃ alkoxy, OH, oxo, or NR₁₀R₁₁; wherein Rₐ is at the 4- position;
R₃ is H, halogen, methyl, methoxy, hydroxymethyl or OH;
R₆ is C₁₋₃alkyl or H;
R₇ is C₁₋₆alkyl branched or unbranched or H;
R₁₀ and R₁₁ may be the same or different and are each independently selected from H, OH, C₁₋₃alkoxy, C₁₋₆alkyl branched or unbranched, C₃₋₈cycloalkyl, benzyl and phenyl; wherein said alkyl, cycloalkyl or phenyl are optionally substituted with OH, C₁₋₃alkoxy, C₁₋₃ acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy, OH or -(CH₂)ₙNR₁₃R₁₄;
R₁₂ is H or C₁₋₆alkyl optionally substituted with phenyl, OH, C₁₋₃alkoxy or NR₁₃R₁₄;
R₁₃ and R₁₄ are each independently selected from H and C₁₋₆ alkyl optionally substituted with C₁₋₃alkoxy, OH or phenyl;
and
or R₁₃ and R₁₄ together form a chain completing a ring, said chain is (CH₂)₄₋₅ or (CH₂)₂O(CH₂)₂.

17. The compound according to claim 16 wherein:
Ar₁ is phenyl, or pyridyl;
X is NH or N-CH₃;
Y is NH and
Rₐ is H, hydroxyC₁₋₂alkyl, 2-hydroxyethylaminomethyl, methoxybenzylaminomethyl, pyridinyl optionally halogenated, phenyl, 3-hydroxy-2-oxo-propyl, vinyl or C₃₋₅alkynyl substituted by C₁₋₃alkoxy or phenyl;
R₁ and R₂ are the same or different and selected from: halogen, C₁₋₃ alkyl, wherein the C₁₋₃ alkyl are optionally partially or fully halogenated, NO₂, NR₁₃R₁₄;
R₃ is H, halogen, methoxy or methyl;
R₁₀ and R₁₁ may be the same or different and are each independently selected from H, OH, C₁₋₃alkoxy, C₁₋₃alkyl branched or unbranched, optionally substituted with OH, C₁₋₃alkoxy, C₁₋₃acyloxy, CO₂R₁₂, NR₁₃R₁₄, O(CH₂)₂₋₄NR₁₃R₁₄ or phenyl;
or R₁₀ and R₁₁ together form morpholino, pyrrolidinyl, piperazinyl or piperidinyl each optionally substituted by C₁₋₃ alkyl, C₁₋₃alkoxy or OH;
R₁₂ is H or C₁₋₃alkyl optionally substituted with phenyl, OH, C₁₋₃alkoxy or NR₁₃R₁₄;
R₁₃ and R₁₄ are each independently selected from H and C₁₋₃alkyl optionally substituted with C₁₋₃alkoxy or OH;
or R₁₃ and R₁₄ together form a chain completing a ring, said chain is (CH₂)₄₋₅ or (CH₂)₂O(CH₂)₂.

18. The compound according to claim 17 wherein:
Ar₁ is phenyl;
Rₐ is H or hydroxymethyl;
R₁ and R₂ are the same or different and selected from: halogen, methyl optionally partially or fully halogenated, NO₂ and NH₂;
R₃ is H, chloro, fluoro, bromo or methoxy;
R₁₀ and R₁₁ may be the same or different and are each independently selected from H, OH, methoxy, C₁₋₃alkyl branched or unbranched, optionally substituted with OH, NR₁₃R₁₄ or phenyl;
or R₁₀ and R₁₁ together form morpholino, pyrrolidinyl, piperazinyl or piperidinyl each optionally substituted by C₁₋₂ alkyl; and
R₁₂ is C₁₋₃alkyl optionally substituted with morpholino.

19. A compound according to claim 9 selected from the group consisting of:
2-(2,6-Dichlorophenylamino)-3,5-dihydro-imidazo[4,5-i]phenanthridin-4-one;
2-(2,6-Dichlorophenylamino)-1,6,7-trimethyl-1,8-dihydro-imidazo[4,5-h]isoquinoline-9-one;
2-(2,6-Dichlorophenylamino)-1,7-dimethyl-6-(2-hydroxyethyl)-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6-carboxylic acid methyl ester;
3-[2-(2,6-Dichlorophenylamino)-1-methyl-9-oxo-8,9-dihydro-1H-imidazo[4,5-h]isoquinolin-7-yl]-acrylic acid methyl ester;
2-(2-Chloro-6-methylphenylamino)-1,6,7-trimethyl-1,8-dihydro-imidazo[4,5-h]isoquinoline-9-one;
3-[2-(2,6-Dichlorophenylamino)-1-methyl-9-oxo-8,9-dihydro-1H-imidazo[4,5-h]isoquinolin-7-yl]-N-methoxy-N-methylacrylamide;
2-(2-Chloro-6-nitrophenylamino)-1,6,7-trimethyl-1,8-dihydro-imidazo[4,5-h]isoquinoline-9-one;
N-Benzyl-3-[2-(2,6-Dichlorophenylamino)-1-methyl-9-oxo-8,9-dihydro-1H-imidazo[4,5-h]isoquinolin-7-yl]-acrylamide;
3-[2-(2,6-Dichlorophenylamino)-1-methyl-9-oxo-8,9-dihydro-1H-imidazo[4,5-h]isoquinolin-7-yl]-acrylic acid 4-morpholine amide;
2-(2,6-Dichlorophenylamino)-4-hydroxymethyl-1,6,7-trimethyl-1,8-dihydro-imidazo[4,5-h]isoquinoline-9-one;
2-(2,6-Dichlorophenylamino)-1,6-dimethyl-7-vinyl-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6- carboxylic acid 2-(4-moropholino)ethyl ester;
2-(2,6-Dichlorophenylamino)-1,6-dimethyl-7-(3-hydroxypropen-1-yl)-1,8-dihydro-imidazo[4,5-h]-isoquinolin-9-one;
2-(2,6-Dichlorophenylamino)-1,6-dimethyl-7-oxazol-5-yl-1,8-dihydro-imidazo[4,5-h]isoquinolin-9-one;
2-(2,6-Dichlorophenylamino)-1-methyl-7-vinyl-1,8-dihydro-imidazo[4,5-h]isoquinoline-9-one;
2-(2,6-Dichlorophenylamino)-1,6-dimethyl-7-(3-morpholin-4-yl-propen-1-yl)- 1,8-dihydro-imidazo[4,5-h]-isoquinolin-9-one;
3-[2-(2,6-Dichlorophenylamino)-1,7-dimethyl-9-oxo-8,9-dihydro-1H-imidazo[4,5-h]isoquinolin-7-yl]-acrylonitrile;
2-(2-Chloro-6-methylphenylamino)-1,7-dimethyl-1,8-dihydro-imidazo[4,5-h]isoquinoline-9-one;
2-(2,6-Dichlorophenylamino)-1-methyl-7-oxazol-5-yl-1,8-dihydro-imidazo[4,5-h]isoquinolin-9-one;
2-(2,6-Dichlorophenylamino)-7-(3-hydroxypropen-1-yl)-1-methyl-1,8-dihydro-imidazo[4,5-h]-isoquinolin-9-one;
2-(2-Chloro-6-methylphenylamino)-7-(3-hydroxypropen-1-yl)-1-methyl-1,8-dihydro-imidazo[4,5-h]-isoquinolin-9-one;
2-(2,6-Dichlorophenylamino)-7-(3-diethylaminopropen-1-yl)-1,6-dimethyl-1,8-dihydro-imidazo[4,5-h]-isoquinolin-9-one;
2-(2,6-Dichlorophenylamino)-1,6-dimethyl-7-(3-pyrrolidin-1-yl-propen-1-yl)- 1,8-dihydro-imidazo[4,5-h]-isoquinolin-9-one;
2-(2,6-Dichlorophenylamino)-7-(3-diethylaminopropen-1-yl)-1-methyl-1,8-dihydro-imidazo[4,5-h]-isoquinolin-9-one;
2-(2,6-Dichlorophenylamino)-1,6-dimethyl-7-(4-methylpiperazin-1-yl-propen-1-yl)-1,8-dihydro-imidazo[4,5-hJ-isoquinolin-9-one;
2-(2,6-Dichlorophenylamino)-1,6-dimethyl-7-(3-piperidin-1-yl-propen-1-yl)- 1,8-dihydro-imidazo[4,5-h]-isoquinolin-9-one;
2-(2,6-Dichlorophenylamino)-1,6-dimethyl-7-{3-[ethyl(2-hydroxyethyl)amino]propen-1-yl}-1,8-dihydro-imidazo[4,5-h]-isoquinolin-9-one;
7-(3-Diethylaminopropen-1-yl)-1,6-dimethyl-2-(2,6-dimethylphenylamino)-1,8-dihydro-imidazo[4,5-h]-isoquinolin-9-one;
2-(2,6-Dichlomphenylamino)-7-{3-[(2-diethylaminoethyl)methylamino]-propen-1-yl}-1,6-dimethyl-1,8-dihydro-imidazo[4,5-h]-isoquinolin-9-one;
7-(3-Diethylaminopropen-1-yl)-1,6-dimethyl-2-(2,4,6-trichlorophenylamino)-1,8-dihydro-imidazo[4,5-h]-isoquinolin-9-one;
2-(2,6-Dichlorophenylamino)-6-methyl-7-oxazol-5-yl-1,8-dihydro-imidazo[4,5-h]isoquinolin-9-one;
2-(2,6-Dichlorophenylamino)-1,6-dimethyl-7-[3-(2-pyrrolidin-1-ylmethylpyrrolidin-1-yl)-propen-1-yl]-1,8-dihydro-imidazo[4,5-*h*]-isoquinolin-9-one;
7-[3-(2*S*-Aminomethylpyrrolidin-1-yl)-propen-1-yl]-2-(2,6-dichlorophenylamino)-1,6-dimethyl-1,8-dihydro-imidazo[4,5-*h*]-isoquinolin-9-one;
1-{3-[2-(2,6-Ddichlorophenylamino)-1,6-dimethyl-9-oxo-8,9-dihydro-1H-imidazo[4,5-h]isoquinolin-7-yl]-propenyl}-*L*-proline carboxamide;
1-{3-[2-(2,6-dichlorophenylamino)-1,6-dimethyl-9-oxo-8,9-dihydro-1H-imidazo[4,5-h]isoquinolin-7-yl]-propenyl}-piperidine-3-carboxamide;
2-(2,6-Dichlorophenylamino)-1,6-dimethyl-7-(methylhydrazonomethyl)-1,8-dihydro-imidazo[4,5-h]-isoquinolin-9-one;
7-[3-(3-Aminopyrrolidin-1-yl)-propen-1-yl]-2-(2,6-dichlorophenylamino)-1,6-dimethyl-1,8-dihydro-imidazo[4,5-*h*]-isoquinolin-9-one;
2-(2,6-Dichlorophenylamino)-1,6-dimethyl-7-[3-(3-acetamidopyrrolidin-1-yl)-propen-1-yl]- 1,8-dihydro-imidazo[4,5-*h*]-isoquinolin-9-one;
2-(2,6-Dichlorophenylamino)-1,6-dimethyl-7-[3-(3-dimethylaminopyrrolidin-1-yl)-propen-1-yl]- 1,8-dihydro-imidazo[4,5-*h*]-isoquinolin-9-one;
1-{3-[2-(2,6-Dichlorophenylamino)-1,6-dimethyl-9-oxo-8,9-dihydro-1H-imidazo[4,5-h]isoquinolin-7-yl]-propenyl}-piperidine-2-carboxamide;
7-[3-(3-Aminomethylpiperidin-1-yl)-propen-1-yl]-2-(2,6-dichlorophenylamino)-1,6-dimethyl-1,8-dihydro-imidazo[4,5-*h*]-isoquinolin-9-one;
1-{3-[2-(2,6-Dichlorophenylamino)-1,6-dimethyl-9-oxo-8,9-dihydro-1H-imidazo[4,5-h]isoquinolin-7-yl]-propenyl}-piperidine-3-carboxylic acid diethylamide;
2-(2,6-Dichlorophenylamino)-1,6-dimethyl-7-ethynyl-1,8-dihydro-imidazo[4,5-h]-isoquinolin-9-one;
1-{3-[2-(2,6-dichlorophenylamino)-1,6-dimethyl-9-oxo-8,9-dihydro-1H-imidazo[4,5-h]isoquinolin-7-yl]-propenyl}-3-methyl urea;
Cyclohexane carboxylic acid {3-[2-(2,6-dichlorophenylamino)-1,6-dimethyl-9-oxo-8,9-dihydro-1H-imidazo[4,5-h]isoquinolin-7-yl]-propenyl}amide;
2-(2,6-Dichlorophenylamino)-1-methyl-7-phenyl-1,8-dihydro-imidazo[4,5-h]isoquinolin-9-one;
*N*-{3-[2-(2,6-Dichlorophenylamino)-1,6-dimethyl-9-oxo-8,9-dihydro-1*H*-imidazo[4,5-h]isoquinolin-7-yl]-propenyl}methanesulfonamide;
3-[2-(2,6-dichlorophenylamino)-1,6-dimethyl-9-oxo-8,9-dihydro-1H-imidazo[4,5-h]isoquinolin-7-yl]-propenyl urea;
1-Cyclohexyl-3-{3-[2-(2,6-dichlorophenylamino)-1,6-dimethyl-9-oxo-8,9-dihydro-1H-imidazo[4,5-h]isoquinolin-7-yl]-propenyl}-urea;
*N*-{3-[2-(2,6-Dichlorophenylamino)-1,6-dimethyl-9-oxo-8,9-dihydro-1*H*-imidazo[4,5-h]isoquinolin-7-yl]-propenyl} benzenesulfonamide;
2-(2,6-Dichlorophenylamino)-1,6-dimethyl-7-(3-ethylaminopropen-1-yl)-1,8-dihydro-imidazo[4,5-h]-isoquinolin-9-one;
*N*-{3-[2-(2,6-Dichlorophenylamino)-1,6-dimethyl-9-oxo-8,9-dihydro-1*H*-imidazo[4,5-h]isoquinolin-7-yl]-propenyl}-guanidine;
Piperidine-3-carboxylic acid {3-[2-(2,6-dichlorophenylamino)-1,6-dimethyl-9-oxo-8,9-dihydro-1H-imidazo[4,5-h]isoquinolin-7-yl]-propenyl}amide;
*L*-Proline {3-[2-(2,6-dichlorophenylamino)-1,6-dimethyl-9-oxo-8,9-dihydro-1H-imidazo[4,5-h]isoquinolin-7-yl]-propenyl}amide;
*D*-Proline {3-[2-(2,6-dichlorophenylamino)-1,6-dimethyl-9-oxo-8,9-dihydro-1H-imidazo[4,5-h]isoquinolin-7-yl]-propenyl}amide;
3-[2-(2,6-Dichlorophenylamino)-1,6-dimethyl-9-oxo-8,9-dihydro-1H-imidazo[4,5-h]isoquinolin-7-yl]-benzamide;
*L*-Azetidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl]amide;
Piperidine-2-carboxylic acid {3-[2-(2,6-dichlorophenylamino)-1,6-dimethyl-9-oxo-8,9-dihydro-1H-imidazo[4,5-h]isoquinolin-7-yl]-propenyl}amide; and
the pharmacuetically acceptable derivatives thereof.

20. A compound according to claim 19 selected from the group consisting of:
2-(2,6-Dichlorophenylamino)-3,5-dihydro-imidazo[4,5-i]phenanthridin-4-one;
2-(2,6-Dichlorophenylamino)-1,6,7-trimethyl-1,8-dihydro-imidazo[4,5-h]isoquinoline-9-one;
2-(2,6-Dichlorophenylamino)-1,7-dimethyl-6-(2-hydroxyethyl)-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6- carboxylic acid methyl ester;
3-[2-(2,6-Dichlorophenylamino)-1-methyl-9-oxo-8,9-dihydro-1H-imidazo[4,5-h]isoquinolin-7-yl]-acrylic acid methyl ester;
2-(2-Chloro-6-methylphenylamino)-1,6,7-trimethyl-1,8-dihydro-imidazo[4,5-h]isoquinoline-9-one;
3-[2-(2,6-Dichlomphenylamino)-1-methyl-9-oxo-8,9-dihydro-1H-imidazo[4,5-h]isoquinolin-7-yl]-N-methoxy-N-methylacrylamide;
2-(2-Chloro-6-nitrophenylamino)-1,6,7-trimethyl-1,8-dihydro-imidazo[4,5-h]isoquinoline-9-one;
N-Benzyl-3-[2-(2,6-Dichlorophenylamino)-1-methyl-9-oxo-8,9-dihydro-1H-imidazo[4,5-h]isoquinolin-7-yl]-acrylamide;
3-[2-(2,6-Dichlorophenylamino)-1-methyl-9-oxo-8,9-dihydro-1H-imidazo[4,5-h]isoquinolin-7-yl]-acrylic acid 4-morpholine amide;
2-(2,6-Dichlorophenylamino)-4-hydroxymethyl-1,6,7-trimethyl-1,8-dihydro-imidazo[4,5-h]isoquinoline-9-one;
2-(2,6-Dichlorophenylamino)-1,6-dimethyl-7-vinyl-1,8-dihydro-imidazo[4,5-h]isoquinoline-9-one;
2-(2,6-Dichlorophenylamino)-1,7-dimethyl-9-oxo-1,8-dihydro-imidazo[4,5-h]isoquinoline-6- carboxylic acid 2-(4-moropholino)ethyl ester;
2-(2,6-Dichlorophenylamino)-1,6-dimethyl-7-(3-hydroxypropen-1-yl)-1,8-dihydro-imidazo[4,5-h]-isoquinolin-9-one;
2-(2,6-Dichlorophenylamino)-1,6-dimethyl-7-oxazol-5-yl-1,8-dihydro-imidazo[4,5-h]isoquinolin-9-one;
2-(2,6-Dichlorophenylamino)-1-methyl-7-vinyl-1,8-dihydro-imidazo[4,5-h]isoquinoline-9-one;
2-(2,6-Dichlorophenylamino)-1,6-dimethyl-7-(3-morpholin-4-yl-propen-1-yl)- 1,8-dihydro-imidazo[4,5-h]-isoquinolin-9-one;
3-[2-(2,6-Dichlorophenylamino)-1,7-dimethyl-9-oxo-8,9-dihydro-1H-imidazo[4,5-h]isoquinolin-7-yl]-acrylonitrile;
2-(2-Chloro-6-methylphenylamino)-1,7-dimethyl-1,8-dihydro-imidazo[4,5-h]isoquinoline-9-one;
2-(2,6-Dichlorophenylamino)-1-methyl-7-oxazol-5-yl-1,8-dihydro-imidazo[4,5-h]isoquinolin-9-one;
2-(2,6-Dichlorophenylamino)-7-(3-hydroxypropen-1-yl)-1-methyl-1,8-dihydro-imidazo[4,5-h]-isoquinolin-9-one;
2-(2-Chloro-6-methylphenylamino)-7-(3-hydroxypropen-1-yl)-1-methyl-1,8-dihydro-imidazo[4,5-h]-isoquinolin-9-one;
2-(2,6-Dichlorophenylamino)-7-(3-diethylaminopropen-1-yl)-1,6-dimethyl-1,8-dihydro-imidazo[4,5-h]-isoquinolin-9-one;
2-(2,6-Dichlorophenylamino)-1,6-dimethyl-7-(3-pyrrolidin-1-yl-propen-1-yl)-1,8-dihydro-imidazo[4,5-h]-isoquinolin-9-one;
2-(2,6-Dichlorophenylamino)-7-(3-diethylaminopropen-1-yl)-1-methyl-1,8-dihydro-imidazo[4,5-h]-isoquinolin-9-one;
2-(2,6-Dichlorophenylamino)-1,6-dimethyl-7-(4-methylpiperazin-1-yl-propen-1-yl)-1,8-dihydro-imidazo[4,5-h]-isoquinolin-9-one;
2-(2,6-Dichlorophenylamino)-1,6-dimethyl-7-(3-piperidin-1-yl-propen-1-yl)- 1,8-dihydro-imidazo[4,5-h]-isoquinolin-9-one;
2-(2,6-Dichlorophenylamino)-1,6-dimethyl-7-{3-[ethyl(2-hydroxyethyl)amino]propen-1-yl}-1,8-dihydro-imidazo[4,5-h]-isoquinolin-9-one;
7-(3-Diethylaminopropen-1-yl)-1,6-dimethyl-2-(2,6-dimethylphenylamino)-1,8-dihydro-imidazo[4,5-h]-isoquinolin-9-one;
2-(2,6-Dichlorophenylamino)-7-{3-[(2-diethylaminoethyl)methylamino]-propen-1-yl}-1,6-dimethyl-1,8-dihydro-imidazo[4,5-h]-isoquinolin-9-one; and
the pharmaceutically acceptable derivatives thereof.

21. A pharmaceutical composition comprising a therapeutically effective amount of a compound according to claims 1, 9, 15 or 19.

22. Use of a compound according to claims 1, 9, 15, or 19 for the manufacture of a medicament for the treatment of an autoimmune disease or cancer,.

23. The use according to claim 22, wherein the autoimmune disease is selected from rheumatoid arthritis, multiple sclerosis, Guillain-Barre syndrome, Crohn's disease, ulcerative colitis, psoriasis, graft versus host disease, systemic lupus erythematosus, insulin-dependent diabetes mellitus and asthma.

24. The use according to claim 22, wherein the cancer is selected from a src-dependent tumor or a PDGF-dependent tumor.

25. The use according to claim 24, wherein the src-dependent tumor is selected from mammary carcinoma, colon carcinoma, melanoma and sarcoma.

26. The use according to claim 24, wherein the PDGF-dependent tumor is selected from ovarian cancer, prostate cancer and glioblastoma.

27. Use of a compound according to claims 1, 9, 15, or 19 for the manufacture of a medicament for the treatment of a disease selected from osteoporosis, Paget's disease, bone inflammation, and joint inflammation.

28. Use of a compound according to claims 1, 9, 15, or 19 for the manufacture of a medicament for the treatment of a disease selected from fibrotic diseases, restenosis and atherosclerosis.

29. Use of a compound according to claims 1, 9, 15, or 19 for the manufacture of a medicament for enhancing or potentiating the effectiveness of radiation therapy.

30. A method of making a compound of the formula (I) 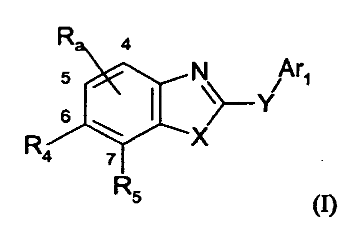 wherein X is N-R₁₅ and Ar₁, R₄, R₅, R₁₅ and Rₐ are as defined in claim 1, said process comprising:
a) reacting a compound of the formula (II) with Ar₁NCS in a suitable solvent at about ambient to reflux temperature for about 3 to 24 hr to provide a compound of the formula (III); 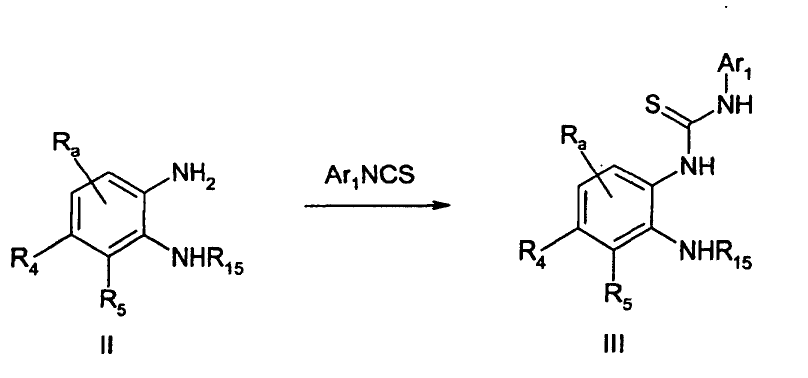
b) reacting the product (II) of step a) with a suitable activating agent chosen from 1,3-dicyclohexylcarbodiimide (DCC) and mercuric oxide in a suitable solvent at about ambient to reflux temperature to form a compound of the formula (I) as shown above or precursors thereof.

31. A method of making a compound of the formula (I) 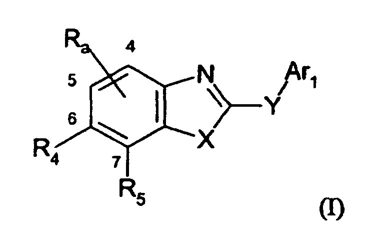 wherein X is S, Y is NH and Ar₁, R₄, R₅ and Rₐ are as defined in claim 1, said process comprising:
a) reacting a compound of the formula (IV) with Ar₁NCS in a suitable solvent at about ambient to reflux temperature for about 3 to 24 hr to form a compound of the formula (V); 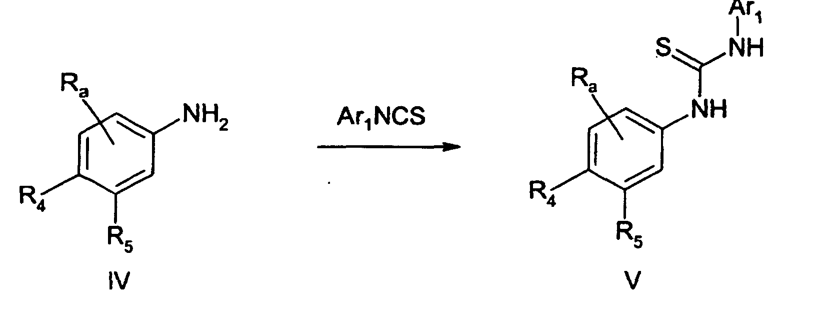
b) reacting the product (V) of step a) under cyclizing conditions in a suitable solvent at about reflux temperature to form a compound of the formula (I) or a precursor thereof.

32. A method of making a compound of the formula (XI) wherein R₁₅, R₈ and R₉ are as described in claim 1: 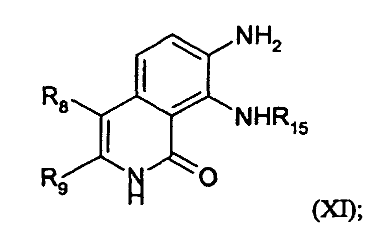 said method comprising:
a) reacting a compound of the formula (VI) with NHR₁₅ in a suitable solvent optionally in a pressure flask and at about 0 to 80 °C, to provide VII, and subsequently reacting compound VII with keto-ester VIII in the presence of a suitable base in a suitable solvent, at about ambient temperature to form a compound of the formula (IX): 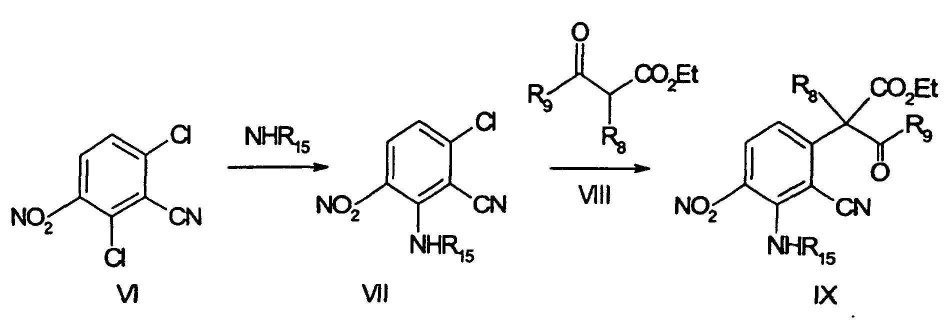
b) hydrolyzing the product of step a) by reacting with aqueous acid, and cyclizing at about reflux temperature; followed subsequently reducing the cyclized product in a suitable solvent to form a compound of the formula (XI).

33. A method of making a compound of the formula: 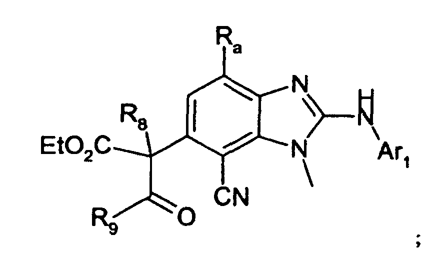 wherein Rₐ, R₈, R₉ and Ar₁ are as described in claim 1;
said method comprising:
a) reacting a compound of the formula (XII) with bromine in a suitable solvent at ambient temperature to provide XIII. 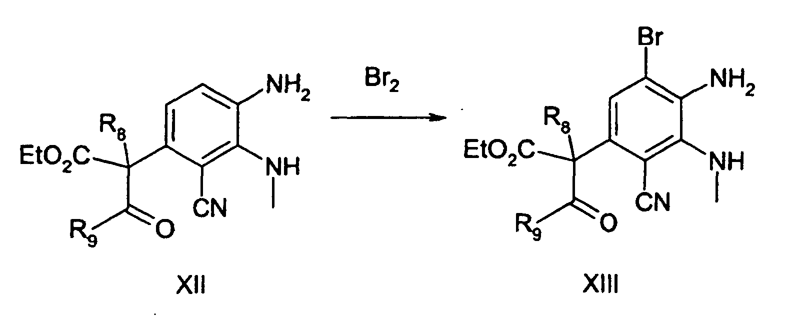
b) reacting a compound of the formula (XIII) with Ar₁NCS in a suitable solvent at about. ambient to reflux temperature for about 3 to 24 hr and subsequently reacting the product with a suitable activating agent chosen from 1,3-dicyclohexylcarbodiimide (DCC) and mercuric oxide in a suitable solvent at about ambient to reflux temperature to form a compound of the formula (XIV);
c) cross-coupling to introduce Rₐ in place of bromine in the presence of a suitable catalyst in a suitable solvent at about 100 °C, to form the product compound shown below:

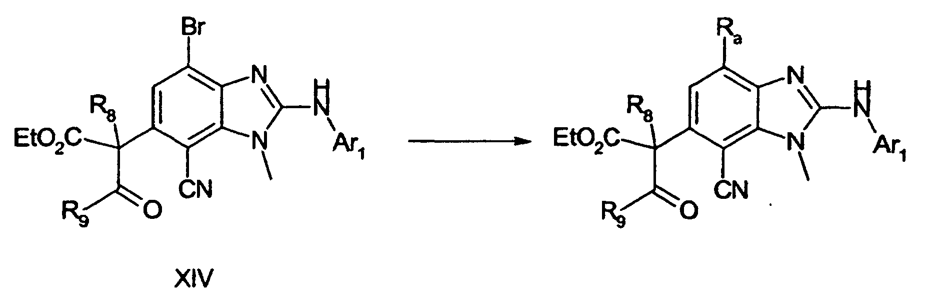

## Patentansprüche

1. Verbindung der Formel (I): 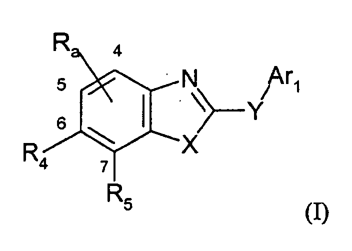 worin:
Ar₁ ein aromatischer(s) oder nichtaromatischer(s) Carbozyklus, Heteroaryl oder Heterozyklus ist; wobei besagter(s) Carbozyklus, Heteroaryl oder Heterozyklus gegebenenfalls mit einem oder mehreren R₁, R₂ und R₃ substituiert ist;
X ein NH, N-C₁₋₃alkyl, N-Cyclopropyl, S oder O ist;
Y ein NR₁₅, S oder O ist;
Rₐ ein H, C₁₋₁₀Alkyl, C₂₋₁₀Alkenyl oder C₂₋₁₀Alkinyl ist, wobei jedes verzweigt oder zyklisch sein kann; oder Rₐ ein Aryl oder Heteroaryl ist; worin jedes Rₐ unabhängig gegebenenfalls mit einem oder mehreren C₁₋₆Alkyl, C₁₋₆Alkoxy, Halogen, OH, Oxo, NR₁₀R₁₁, Aryl oder Heteroaryl substituiert ist, wobei jedes Aryl oder Heteroaryl gegebenenfalls mit einer oder mehreren Gruppen ausgewählt aus Halogen, OH, C₁₋₃Alkyl, C₁₋₃Alkoxy, HydroxyC₁₋₃alkyl und (CH₂)ₘNR₁₀R₁₁ substituiert ist; und wobei Rₐ an der 4- oder 5-Stellung gebunden ist;
R₁ und R₂ gleich oder verschieden sind und ausgewählt sind aus H, Halogen, CN, NO₂, verzweigtem oder unverzweigtem, gesättigtem oder ungesättigtem C₁₋₁₀Alkyl, verzweigtem oder unverzweigtem C₁₋₁₀Alkoxy, verzweigtem oder unverzweigtem C₁₋₁₀Acyl, verzweigtem oder unverzweigtem C₁₋₁₀Acyloxy, verzweigtem oder unverzweigtem C₁₋₁₀Alkylthio, Aminosulfonyl, Di-(C₁₋₃)alkylaminosulfonyl, NR₁₀R₁₁, Aryl, Aroyl, Aryloxy, Arylsulfonyl, Heteroaryl und Heteroaryloxy; wobei die oben erwähnten R₁ und R₂ gegebenenfalls teilweise oder vollständig halogeniert oder gegebenenfalls mit einer bis drei unabhängig aus Oxo, OH, NR₁₀R₁₁, verzweigtem oder unverzweigtem C₁₋₆Alkyl, C₃₋₇Cycloalkyl, Phenyl, Naphthyl, Heteroaryl, Aminocarbonyl und Mono- oder Di(C₁₋₃)alkylaminocarbonyl ausgewählten Gruppen substituiert sind;
R₃ ein H, Halogen, OH, (CH₂)ₙNR₁₀R₁₁, CONR₁₀R₁₁, (CH₂)ₙCO₂R₁₂; ein gegebenenfalls mit OH substituiertes C₁₋₃Alkyl, ein gegebenenfalls halogeniertes C₁₋₃Alkoxy oder ein C₁₋₃Alkylthio ist;
R₄ und R₅ zusammen mit den Atomen, an die sie gebunden sind, ein fusioniertes Ringsystem der Formeln A oder B vervollständigen: 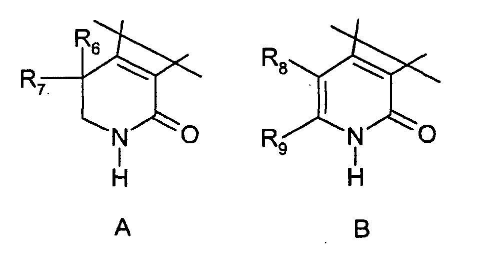
R₆ ein C₁₋₃Alkyl oder H ist;
R₇ ein verzweigtes oder unverzweigtes C₁₋₆Alkyl oder H ist;
R₈ ein H, ein verzweigtes oder unverzweigtes, gesättigtes oder ungesättigtes, gegebenenfalls mit Phenyl, OH oder C₁₋₃Alkoxy substituiertes C₁₋₆Alkyl ist; oder R₈ ein (CH₂)ₘNR₁₀R₁₁, (CH₂)ₘNR₁₀COR₁₂, (CH₂)ₙCO₂R₁₂, (CH₂)ₙCONR₁₀R₁₁ ist; oder R₈ ein Phenyl oder Heteroaryl ist, wovon jedes gegebenenfalls mit C₁₋₃Alkyl, C₁₋₃Alkoxy, OH, -SO₃H oder Halogen substituiert ist;
R₉ ein H, CN oder CONR₁₀R₁₁ ist; oder R₉ ein verzweigtes oder unverzweigtes C₁₋₁₀Alkyl, ein C₃₋₁₀Cycloalkyl, C₅₋₇Cycloalkenyl, C₂₋₆Alkenyl, C₂₋₆Alkinyl ist, wovon jedes gegebenenfalls mit einer oder mehreren C₃₋₁₀Cycloalkyl, C₃₋₁₀Cycloalkyliden, C₅₋₇Cycloalkenyl, Halogen, OH, Oxo, CN, C₁₋₃Alkoxy, C₁₋₃Acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, Aryloxy, Arylthio, Aryl oder Heteroaryl substituiert ist; wobei jedes Aryloxy, Arylthio, Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist;
oder R₉ ein Aryl, Heteroaryl, oder Heterozyklus ist, wobei jedes(r) Aryl, Heteroaryl oder Heterozyklus gegebenenfalls mit einer bis drei Gruppen substituiert ist, die ausgewählt sind aus gegebenenfalls mit Phenyl oder NR₁₀C(=NR₁₀)NR₁₀R₁₁ substituiertem C₁₋₃Alkyl, C₁₋₃Alkoxy, Halogen, CN, Oxo, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 5- oder 6-gliedrigen aromatischen oder nichtaromatischen carbozyklischen Ring bilden, der gegebenenfalls mit einem oder zwei C₁₋₃Alkyl, OH, Oxo oder (CH₂)ₙNR₁₀R₁₁ substituiert ist oder gegebenenfalls mit einer 1,3-Dioxolan-Gruppe oder 1,3-Dithiolan-Gruppe spiro-verknüpft ist, wobei jede 1,3-Dioxolan-Gruppe oder 1,3-Dithiolan-Gruppe gegebenenfalls mit C₁₋₆Alkyl, C₁₋₆Alkoxy, OH oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Aryl, ArylC₁₋₃alkyl und Heteroaryl; wobei besagte Alkyl, Cycloalkyl, Aryl, ArylC₁₋₃alkyl oder Heteroaryl gegebenenfalls mit OH, C₁₋₃Alkoxy, CN, NO₂, C₁₋₃Acyloxy, CO₂R₁₂, NR₁₃R₁₄, O(CH₂)₂₋₄NR₁₃R₁₄, Aryl oder Heteroaryl substituiert sind;
oder R₁₀ und R₁₁ zusammen eine 3-7-gliedrige Alkylen-Kette bilden, welche über das N Atom, an dem sie angebracht sind, einen Ring vervollständigt; wobei besagte Alkylen-Kette gegebenenfalls von O, S(O)ₚ, und NR₁₃ unterbrochen ist; und wobei besagter Ring gegebenenfalls mit C₁₋₃Alkyl, C₁₋₃Alkoxy, OH, -(CH₂)ₙNR₁₃R₁₄, CONR₁₃R₁₄ oder NR₁₃COR₁₄ substituiert ist;
R₁₂ ein H, C₁₋₆Alkyl oder C₃₋₈Cycloalkyl ist, worin jedes Alkyl oder Cycloalkyl gegebenenfalls mit Phenyl, OH, C₁₋₃Alkoxy oder NR₁₃R₁₄ substituiert ist; oder R₁₂ ein gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NP₁₀R₁₁ ausgewählten Gruppen substituiertes(r) Phenyl oder Heterozyklus ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist;
R₁₅ ein H oder C₁₋₃ Alkyl ist;
m 1-4 ist; n 0-3 ist und p 0-2 ist;
wobei sich der Begriff Heteroaryl auf ein stabiles 5-8-gliedriges monozyklisches oder 8-11-gliedriges bizyklisches aromatisches heterozyklisches Radikal bezieht, das aus Kohlenstoffatomen und 1 bis 4 aus Stickstoff, Sauerstoff und Schwefel ausgewählten Heteroatomen besteht;
sich der Begriff Heterozyklus auf ein stabiles 4-8-gliedriges monozyklisches oder 8-11-gliedriges bizyklisches heterozyklisches Radikal bezieht, das entweder gesättigt oder ungesättigt sein kann und nichtaromatisch ist und aus Kohlenstoffatomen sowie 1 bis 4 aus Stickstoff, Sauerstoff und Schwefel ausgewählten Heteroatomen besteht;
sich der Begriff Aryl auf einen 6-10-gliedrigen aromatischen Carbozyklus bezieht;
sich der Begriff Carbozyklus auf eine 3-10-gliedrige aromatische oder nichtaromatische zyklische Kohlenstoffkette bezieht; und
deren pharmazeutisch annehmbare Säure- oder Salzderivate.

2. Verbindung nach Anspruch 1, worin
Ar₁ ist:
a) eine aus Cyclopropyl, Cyclobutyl, Cyclopentanyl, Cyclohexanyl, Cycloheptanyl ausgewählte Cycloalkyl-Gruppe;
b) eine aus Cyclopentenyl, Cyclohexenyl, Cycloheptenyl ausgewählte Cycloalkenyl-Gruppe;
c) Phenyl, Naphthyl; Indanyl, Indenyl, Dihydronaphthyl, Tetrahydronaphthyl, Fluorenyl;
d) ein aus Pyridinyl, Pyrimidinyl, Pyrazinyl, Pyridazinyl, Pyrrolyl, Imidazolyl, Pyrazolyl, Thienyl, Furyl, Isoxazolyl, Isothiazolyl, Oxazolyl, Oxadiazolyl, Thiazolyl, Thiadiazolyl, Chinolinyl, Isochinolinyl, Indolyl, Benzimidazolyl, Benzofuranyl, Benzoxazolyl, Benzisoxazolyl, Benzpyrazolyl, Benzothiofuranyl, Benzothiazolyl, Chinazolinyl und Indazolyl ausgewähltes Heteroaryl, oder ein aus Cyclopentenopyridin, Cyclohexanopyridin, Cyclopentanopyrimidin, Cyclohexanopyrimidin, Cyclopentanopyrazin, Cyclohexanopyrazin, Cyclopentanopyridazin, Cyclohexanopyridazin, Cyclopentanochinolin, Cyclohexanochinolin, Cyclopentanoisochinolin, Cyclohexanoisochinolin, Cyclopentanoindol, Cyclohexanoindol, Cyclopentanobenzimidazol, Cyclohexanobenzimidazol, Cyclopentanobenzoxazol, Cyclohexanobenzoxazol, Cyclopentanoimidazol, Cyclohexanoimidazol, Cyclopentanothiophen und Cyclohexanothiophen ausgewähltes fusioniertes Heteroaryl; oder
e) ein aus Pyrrolinyl, Pyrrolidinyl, Pyrazolinyl, Pyrazolidinyl, Piperidinyl, Morpholinyl, Thiomorpholinyl, Pyranyl, Thiopyranyl, Piperazinyl und Indolinyl ausgewählter Heterozyklus;
wobei jedes der obigen Ar₁ gegebenenfalls mit einem oder mehreren R₁, R₂ und R₃ substituiert ist;
Rₐ ein H, C₁₋₆Alkyl, C₂₋₅Alkenyl, C₂₋₅Alkinyl, Phenyl oder ein aus Pyridinyl, Pyridazinyl, Pyrimidinyl, Pyrazinyl, Oxazolyl, Pyrazolyl, Imidazolyl, Furyl, Thiazolyl und Thienyl ausgewähltes Heteroaryl ist; wobei jedes Rₐ gegebenenfalls mit einem oder mehreren Phenyl, Halogen, C₁₋₃Alkyl, C₁₋₃Alkoxy, OH, Oxo oder NR₁₀R₁₁ substituiert ist; worin Rₐ sich an der 4-Stellung befindet;
R₁ und R₂ wie hier oben definiert sind;
R₃ ein H, Halogen, Methyl, Methoxy, Hydroxymethyl oder OH ist;
R₈ ein H, ein verzweigtes oder unverzweigtes, gesättigtes oder ungesättigtes, gegebenenfalls mit OH substituiertes C₁₋₃Alkyl ist; oder R₈ ein (CH₂)₂₋₃NR₁₀R₁₁, (CH₂)ₙCO₂R₁₂ oder (CH₂)ₙCONR₁₀R₁₁ ist;
R₉ ein CN oder CONR₁₀R₁₁ ist; oder R₉ ein verzweigtes oder unverzweigtes C₁₋₃Alkyl, ein C₂₋₄Alkenyl oder C₂₋₄Alkinyl ist, wovon jedes gegebenenfalls mit einem oder mehreren C₅₋₇Cycloalkyl, C₅₋₇ Cycloalkyliden, C₅₋₇Cycloalkenyl, OH, CN, C₁₋₃Acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, CONR₁₀R₁₁, CO₂R₁₂, C(R₁₀)=NNR₁₀R₁₁, C(R₁₀)=NNR₁₀CONR₁₀R₁₁, Aryl oder Heteroaryl substituiert ist; wobei jedes Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist;
oder R₉ ein Aryl, Heteroaryl oder Heterozyklus ist, die je gegebenenfalls mit einer bis drei Gruppen substituiert sind, die ausgewählt sind aus gegebenenfalls mit Phenyl oder NR₁₀C(=NR₁₀)NR₁₀R₁₁ substituiertem C₁₋₃Alkyl, C₁₋₃Alkoxy, Halogen, CN, Oxo, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 5-oder 6-gliedrigen aromatischen oder nichtaromatischen carbozyklischen Ring bilden, der gegebenenfalls mit C₁₋₃Alkyl oder OH substituiert ist oder gegebenenfalls mit einer 1,3-Dioxolan-Gruppe oder 1,3-Dithiolan-Gruppe spiro-verknüpft ist, wobei jede 1,3-Dioxolan-Gruppe oder 1,3-Dithiolan-Gruppe gegebenenfalls mit C₁₋₃Alkyl, C₁₋₃Alkoxy, OH oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Benzyl und Phenyl; wobei besagtes Alkyl, Cycloalkyl, Benzyl oder Phenyl gegebenenfalls mit OH, C₁₋₃Alkoxy, C₁₋₃Acyloxy, CN, NO₂, CO₂R₁₂, NR₁₃R₁₄, O(CH₂)₂₋₄NR₁₃R₁₄ oder Phenyl substituiert ist;
oder R₁₀ und R₁₁ zusammen Morpholino, Pyrrolidinyl, Piperazinyl oder Piperidinyl bilden, wovon jedes(r) gegebenenfalls mit C₁₋₃Alkyl, C₁₋₃Alkoxy, OH, -(CH₂)ₙNR₁₃R₁₄, CONR₁₃R₁₄ oder NR₁₃COR₁₄ substituiert ist;
R₁₂ ein H, C₁₋₆Alkyl oder C₅₋₇Cycloalkyl ist, wobei jedes gegebenenfalls mit Phenyl, OH, C₁₋₃Alkoxy oder NR₁₃R₁₄ substituiert ist; oder R₁₂ ein Phenyl oder Heterozyklus ist, wobei jedes gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiert ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist; und
R₁₅ ein H ist,
worin sich der Begriff Heteroaryl auf ein stabiles 5-8-gliedriges monozyklisches oder 8-11 gliedriges bizyklisches aromatisches heterozyklisches Radikal bezieht, das aus Kohlenstoffatomen und 1 bis 4 aus Stickstoff, Sauerstoff und Schwefel ausgewählten Heteroatomen besteht;
sich der Begriff Heterozyklus auf ein stabiles 4-8 gliedriges monozyklisches oder 8-11 gliedriges bizyklisches heterozyklisches Radikal bezieht, das entweder gesättigt oder ungesättigt sein kann, und nichtaromatisch ist und aus Kohlenstoffatomen sowie 1 bis 4 aus Stickstoff, Sauerstoff und Schwefel ausgewählten Heteroatomen besteht;
sich der Begriff Aryl auf einen 6-10-gliedrigen aromatischen Carbozyklus bezieht;
sich der Begriff Carbozyklus auf eine 3-10-gliedrige aromatische oder nichtaromatische zyklische Kohlenstoffkette bezieht;.

3. Verbindung nach Anspruch 2, worin:
Ar₁ ein Phenyl oder Pyridyl ist, worin jedes gegebenenfalls mit einem oder mehreren
wie folgt definierten R₁, R₂ und R₃ substituiert ist;
X ein NH oder N-CH₃ ist;
Y ein NH ist und
Rₐ ein H, HydroxyC₁₋₂alkyl, 2-Hydroxyethylaminomethyl, Methoxybenzylaminomethyl, ein gegebenenfalls halogeniertes Pyridinyl, ein Phenyl, 3-Hydroxy-2-oxo-propyl, Vinyl oder ein mit C₁₋₃Alkoxy oder Phenyl substituiertes C₃₋₅Alkinyl ist;
R₁ und R₂ gleich oder verschieden sind und ausgewählt sind aus: H, Halogen, C₁₋₃Alkyl, wobei die C₁₋₃Alkyl gegebenenfalls teilweise oder vollständig halogeniert sind, NO₂, NR₁₃R₁₄;
R₃ ein H, Halogen, Methoxy oder Methyl ist;
R₄ und R₅ zusammen einen fusionierten Ring der Formel B vervollständigen;
R₈ ein H, ein gegebenenfalls mit OH substituiertes C₁₋₃Alkyl ist; oder R₈ ein (CH₂)₂₋₃NR₁₀R₁₁ oder CO₂R₁₂ ist;
R₉ ein CN ist; oder R₉ ein Methyl, C₂₋₃Alkenyl oder C₂₋₃Alkinyl ist, wovon jedes gegebenenfalls mit einem oder mehreren C₅₋₇Cycloalkyliden, C₅₋₇Cycloalkenyl, OH, CN, NR₁₀R₁₁, NR₁₀CONR₁₀R₁₁, NR₁₀COR₁₂, NR₁₀S(O)ₚR₁₂, CONR₁₀R₁₁, CO₂R₁₂, C(R₁₀)=NNR₁₀R₁₁ oder Heteroaryl substituiert ist;
oder R₉ ein Aryl oder Heteroaryl ist, das gegebenenfalls mit einer bis drei Gruppen substituiert ist, die ausgewählt sind aus gegebenenfalls mit Phenyl substitutiertem C₁₋₃Alkyl, C₁₋₃Alkoxy, Halogen, Amino oder CONH₂;
oder R₈ und R₉ zusammen einen Cyclopenten-Ring bilden, der mit einer 1,3-Dioxolan Gruppe spiroverknüpft ist, wobei besagte 1,3-Dioxolan Gruppe gegebenenfalls mit C₁₋₃Alkyl, C₁₋₃alkoxy, OH oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₃Alkyl, C₅₋₇Cycloalkyl oder Phenyl, worin besagte Alkyl, Cycloalkyl oder Phenyl gegebenenfalls mit OH, C₁₋₃Alkoxy, C₁₋₃Acyloxy, NO₂, CO₂R₁₂, NR₁₃R₁₄, O(CH₂)₂₋₄NR₁₃R₁₄ oder Phenyl substituiert sind; oder R₁₀ und R₁₁ zusammen ein Morpholino, Pyrrolidinyl, Piperazinyl oder Piperidinyl bilden, wovon jedes gegebenenfalls mit C₁₋₃Alkyl, C₁₋₃Alkoxy, OH, (CH₂)ₙNR₁₃R₁₄, CONR₁₃R₁₄ oder NR₁₃COR₁₄ substituiert ist;
R₁₂ ein H, C₁₋₃Alkyl oder C₅₋₇Cycloalkyl ist, wovon jedes gegebenenfalls mit Phenyl, OH, C₁₋₃Alkoxy oder NR₁₃R₁₄ substituiert ist; oder R₁₂ ein Phenyl oder ein gesättigter, 4- bis 6-gliedriger Stickstoff-enthaltender Heterozyklus ist, wovon jeder(s) gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NP₁₀R₁₁ ausgewählten Gruppen substituiert ist;
R₁₃ und R₁₄ je unabhängig aus H und C₁₋₃Alkyl, das gegebenenfalls mit C₁₋₃Alkoxy oder OH substituiert ist, ausgewählt sind;
oder R₁₃ und R₁₄ zusammen eine ringschliessende Kette bilden, wobei besagtes Kette (CH₂)₄₋₅ oder (CH₂)₂O(CH₂)₂ ist.

4. Verbindung nach Anspruch 3, worin:
Ar₁ ein Phenyl ist;
Rₐ ein H oder Hydroxymethyl ist;
R₁ und R₂ gleich oder verschieden sind und ausgewählt sind aus: Halogen, einem gegebenenfalls teilweise oder vollständig halogeniertem Methyl, NO₂ und NH₂;
R₃ ein H, Chloro, Fluoro, Bromo oder Methoxy ist;
R₁₀ und R₁₁ gleich oder verschieden sein können und jedes unabhängig ausgewählt ist aus H, OH, Methoxy, verzweigtem oder unverzweigtem C₁₋₃Alkyl oder C₅₋₇Cycloalkyl, worin besagte Alkyl oder Cycloalkyl gegebenenfalls mit OH, NR₁₃R₁₄ oder Phenyl substituiert sind;
oder R₁₀ und R₁₁ zusammen ein Morpholino, Pyrrolidinyl, Piperazinyl oder Piperidinyl bilden, wovon jedes gegebenenfalls mit C₁₋₂ Alkyl, NR₁₃R₁₄, CONR₁₃R₁₄ oder NR₁₃COR₁₄ substituiert ist; und
R₁₂ ein gegebenenfalls mit Morpholino substituiertes C₁₋₃Alkyl ist; oder R₁₂ ein Phenyl oder ein Azetidinyl, Pyrrolidinyl oder Piperidinyl ist, wovon jedes gegebenenfalls mit einer bis drei aus C₁₋₃Alkyl, C₁₋₃Alkoxy und Halogen ausgewählten Gruppen substituiert ist.

5. Verbindung nach Anspruch 1, worin:
Ar₁ ein aromatischer oder nichtaromatischer(s) Carbozyklus, Heteroaryl oder Heterozyklus ist; worin besagter(s) Carbozyklus, Heteroaryl oder Heterozyklus gegebenenfalls mit einem oder mehreren R₁, R₂ und R₃ substituiert ist;
X ein NH, N-C₁₋₃Alkyl, N-Cyclopropyl, S oder O ist;
Y ein NR₁₅, S oder O ist;
Rₐ ein H, C₁₋₁₀Alkyl, C₂₋₁₀Alkenyl oder C₂₋₁₀Alkinyl ist, wovon jedes verzweigt oder zyklisch sein kann; oder Rₐ ein Aryl oder Heteroaryl ist; wobei jedes Rₐ unabhängig gegebenenfalls mit einem oder mehreren C₁₋₃Alkyl, C₁₋₆Alkoxy, Halogen, OH, Oxo, NR₁₀R₁₁, Aryl oder Heteroaryl substituiert ist, wobei jedes Aryl oder Heteroaryl gegebenenfalls mit einer oder mehreren Gruppen ausgewählt aus Halogen, OH, C₁₋₃Alkyl, C₁₋₃Alkoxy, HydroxyC₁₋₃alkyl und (CH₂)ₘNR₁₀R₁₁ substituiert ist; und wobei Rₐ in der 4- oder 5-Stellung gebunden ist;
R₁ und R₂ gleich oder verschieden sind und ausgewählt sind aus H, Halogen, CN, NO₂, verzweigtem oder unverzweigtem gesättigtem oder ungesättigtem C₁₋₁₀Alkyl, verzweigtem oder unverzweigtem C₁₋₁₀Alkoxy, verzweigtem oder unverzweigtem C₁₋₁₀Acyl, verzweigtem oder unverzweigtem C₁₋₁₀Acyloxy, verzweigtem oder unverzweigtem C₁₋₁₀ Alkylthio, Aminosulfonyl, Di-(C₁₋₃)alkylaminosulfonyl, NR₁₀R₁₁, Aryl, Aroyl, Aryloxy, Arylsulfonyl, Heteroaryl und Heteroaryloxy; wobei die oben erwähnten R₁ und R₂ gegebenenfalls teilweise oder vollständig halogeniert oder gegebenenfalls mit einer bis drei unabhängig aus Oxo, OH, NR₁₀R₁₁, verzweigtem oder unverzweigtem C₁₋₆Alkyl, C₃₋₇Cycloalkyl, Phenyl, Naphthyl, Heteroaryl, Aminocarbonyl und Mono- oder Di(C₁₋₃)alkylaminocarbonyl ausgewählten Gruppen substituiert sind;
R₃ ein H, Halogen, OH, (CH₂)ₙNR₁₀R₁₁, (CH₂)ₙCO₂R₁₂; ein gegebenenfalls mit OH substituiertes C₁₋₃Alkyl, ein gegebenenfalls halogeniertes C₁₋₃Alkoxy oder C₁₋₃Alkylthio ist;
R₄ und R₅ zusammen mit den Atomen, an die sie gebunden sind, ein fusioniertes Ringsystem der Formeln A oder B vervollständigen: 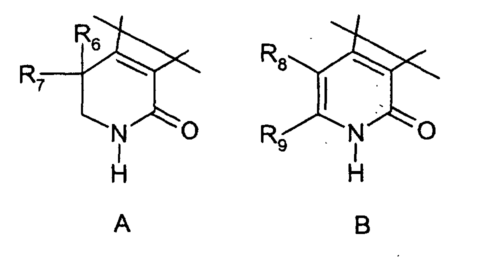
R₆ ein C₁₋₃Alkyl oder H ist;
R₇ ein verzweigtes oder unverzweigtes C₁₋₆Alkyl oder H ist;
R₈ ein H, ein verzweigtes oder unverzweigtes, gesättigtes oder ungesättigtes, gegebenenfalls mit Phenyl, OH oder C₁₋₃Alkoxy substituiertes C₁₋₆Alkyl ist; oder R₈ ein (CH₂)ₘNR₁₀R₁₁, (CH₂)ₘNR₁₀COR₁₂, (CH₂)ₙCO₂R₁₂, (CH₂)ₙCONR₁₀R₁₁ ist; oder R₈ ein Phenyl oder Heteroaryl ist, wovon jedes gegebenenfalls mit C₁₋₃Alkyl, C₁₋₃Alkoxy, OH, -SO₃H oder Halogen substituiert ist;
R₉ ein H ist; oder R₉ ein verzweigtes oder unverzweigtes C₁₋₁₀Alkyl, ein C₃₋₁₀Cycloalkyl, C₂₋₆Alkenyl, C₂₋₆Alkinyl ist, wovon jedes gegebenenfalls mit einem oder mehreren Halogen, OH, Oxo, CN, C₁₋₃Alkoxy, NR₁₀R₁₁, NR₁₀COR₁₂, SR₁₂, CONR₁₀R₁₁, CO₂R₁₂, Aryloxy, Arylthio, Aryl oder Heteroaryl substituiert ist; worin jedes Aryloxy, Arylthio, Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist;
oder R₉ ein Aryl oder Heteroaryl ist, wobei jedes Aryl oder Heteroaryl gegebenenfalls mit einer bis drei Gruppen substituiert ist, die ausgewählt sind aus gegebenenfalls mit Phenyl substituiertem C₁₋₃Alkyl, C₁₋₃Alkoxy, Halogen, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 6-gliedrigen aromatischen oder nichtaromatischen carbozyklischen Ring bilden, der gegebenenfalls mit einem oder zwei OH, Oxo oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Aryl, ArylC₁₋₃alkyl und Heteroaryl; worin besagte Alkyl, Cycloalkyl, Aryl, ArylC₁₋₃alkyl oder Heteroaryl gegebenenfalls mit OH, C₁₋₃Alkoxy, C₁₋₃Acyloxy, CO₂R₁₂, NR₁₃R₁₄, O(CH₂)₂₋₄NR₁₃R₁₄, Aryl oder Heteroaryl substituiert sind;
oder R₁₀ und R₁₁ zusammen einen 3-7-gliedrige Alkylen-Kette bilden, welche über das N Atom, an dem sie angebracht sind, einen Ring vervollständigen; wobei besagtes Alkylen-Kette gegebenenfalls von O, S(O)ₚ, und NR₁₃ unterbrochen ist; und wobei besagter Ring gegebenenfalls mit C₁₋₃Alkyl, C₁₋₃Alkoxy, OH oder -(CH₂)ₙNR₁₃R₁₄ substituiert ist;
R₁₂ ein H, C₁₋₆Alkyl oder C₃₋₈Cycloalkyl ist, worin jedes Alkyl oder Cycloalkyl gegebenenfalls mit Phenyl, OH, C₁₋₃Alkoxy oder NR₁₃R₁₄ substituiert ist; oder R₁₂ ein gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiertes Phenyl ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist;
R₁₅ ein H oder C₁₋₃ Alkyl ist;
m 1-4 ist; n 0-3 ist und p 0-2 ist; und
deren pharmazeutisch annehmbare Säure- oder Salzderivate.

6. Verbindung nach Anspruch 5, worin: ist:
Ar₁ ist:
a) eine aus Cyclopropyl, Cyclobutyl, Cyclopentanyl, Cyclohexanyl, Cycloheptanyl ausgewählte Cycloalkyl-Gruppe;
b) eine aus Cyclopentenyl, Cyclohexenyl, Cycloheptenyl ausgewählte Cycloalkenyl-Gruppe;
c) Phenyl, Naphthyl; Indanyl, Indenyl, Dihydronaphthyl, Tetrahydronaphthyl; Fluorenyl;
d) ein aus Pyridinyl, Pyrimidinyl, Pyrazinyl, Pyridazinyl, Pyrrolyl, Imidazolyl, Pyrazolyl, Thienyl, Furyl, Isoxazolyl, Isothiazolyl, Oxazolyl, Oxadiazolyl, Thiazolyl, Thiadiazolyl, Chinolinyl, Isochinolinyl, Indolyl, Benzimidazolyl, Benzofuranyl, Benzoxazolyl, Benzisoxazolyl, Benzpyrazolyl, Benzothiofuranyl, Benzothiazolyl, Chinazolinyl, und Indazolyl ausgewähltes Heteroaryl, oder ein aus Cyclopentenopyridin, Cyclohexanopyridin, Cyclopentanopyrimidin, Cyclohexanopyrimidin, Cyclopentanopyrazin, Cyclohexanopyrazin, Cyclopentanopyridazin, Cyclohexanopyridazin, Cyclopentanochinolin, Cyclohexanochinolin, Cyclopentanoisochinolin, Cyclohexanoisochinolin, Cyclopentanoindol, Cyclohexanoindol, Cyclopentanobenzimidazol, Cyclohexanobenzimidazol, Cyclopentanobenzoxazol, Cyclohexanobenzoxazol, Cyclopentanoimidazol, Cyclohexanoimidazol, Cyclopentanothiophen und Cyclohexanothiophen ausgewähltes fusioniertes Heteroaryl; oder
e) ein aus: Pyrrolinyl, Pyrrolidinyl, Pyrazolinyl, Pyrazolidinyl, Piperidinyl, Morpholinyl, Thiomorpholinyl, Pyranyl, Thiopyranyl, Piperazinyl und Indolinyl ausgewählter Heterozyklus;
wobei jedes der obigen Ar₁ gegebenenfalls mit einem oder mehreren wie oben definierten R₁, R₂ und R₃ substituiert ist;
Rₐ ein H, C₁₋₆Alkyl, C₂₋₅Alkenyl, C₂₋₅Alkinyl, Phenyl oder ein aus: Pyridinyl, Pyridazinyl, Pyrimidinyl, Pyrazinyl, Oxazolyl, Pyrazolyl, Imidazolyl, Furyl, Thiazolyl und Thienyl ausgewähltes Heteroaryl ist; wobei jedes Rₐ gegebenenfalls mit einem oder mehreren Phenyl, Halogen, C₁₋₃Alkyl, C₁₋₃Alkoxy, OH, Oxo oder NR₁₀R₁₁ substituiert ist; wobei Rₐ in der 4-Stellung gebunden ist;
R₃ ein H, Halogen, Methyl, Methoxy, Hydroxymethyl oder OH ist;
R₈ ein H, ein verzweigtes oder unverzweigtes, gesättigtes oder ungesättigtes, gegebenenfalls mit OH substituiertes C₁₋₃Alkyl ist; oder R₈ ein (CH₂)₂₋₃NR₁₀R₁₁, (CH₂)ₙCO₂R₁₂ oder (CH₂)ₙCONR₁₀R₁₁ ist;
R₉ ein verzweigtes oder unverzweigtes C₁₋₃Alkyl, C₂₋₄Alkenyl, C₂₋₄Alkinyl ist, wovon jedes gegebenenfalls mit einem oder mehreren OH, CN, NR₁₀R₁₁, CONR₁₀R₁₁, CO₂R₁₂, Aryl oder Heteroaryl substituiert ist; wobei jedes Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist; oder R₉ ein Aryl oder Heteroaryl ist, das gegebenenfalls mit einer bis drei Gruppen substituiert ist, die ausgewählt sind aus gegebenenfalls mit Phenyl substituiertem C₁₋₃Alkyl, C₁₋₃Alkoxy, Halogen, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 6-gliedrigen aromatischen oder nichtaromatischen carbozyklischen Ring bilden, der gegebenenfalls mit OH substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Benzyl und Phenyl; wobei besagte Alkyl, Cycloalkyl, Benzyl oder Phenyl gegebenenfalls mit OH, C₁₋₃Alkoxy, C₁₋₃Acyloxy, CO₂R₁₂, NR₁₃R₁₄, O(CH₂)₂₋₄NR₁₃R₁₄ oder Phenyl substituiert sind;
oder R₁₀ und R₁₁ zusammen Morpholino, Pyrrolidinyl, Piperazinyl oder Piperidinyl bilden, wobei jedes gegebenenfalls mit C₁₋₃Alkyl, C₁₋₃Alkoxy, OH oder -(CH₂)ₙNR₁₃R₁₄ substituiert ist;
R₁₂ ein H oder ein gegebenenfalls mit Phenyl, OH, C₁₋₃Alkoxy oder NR₁₃R₁₄ substituiertes C₁₋₆Alkyl ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist; und
R₁₅ ein H ist.

7. Verbindung nach Anspruch 6, worin:
Ar₁ ein Phenyl, oder Pyridyl ist;
X ein NH oder N-CH₃ ist;
Y ein NH ist und
Rₐ ein H, HydroxyC₁₋₂alkyl, 2-Hydroxyethylaminomethyl, Methoxybenzylaminomethyl, ein gegebenenfalls halogeniertes Pyridinyl, ein Phenyl, 3-Hydroxy-2-oxo-propyl, Vinyl oder ein mit C₁₋₃Alkoxy oder Phenyl substituiertes C₃₋₅Alkinyl ist;
R₁ und R₂ gleich oder verschieden sind und ausgewählt sind aus: Halogen, C₁₋₃Alkyl, wobei die C₁₋₃Alkyl gegebenenfalls teilweise oder vollständig halogeniert sind, NO₂, NR₁₃R₁₄;
R₃ ein H, Halogen, Methoxy oder Methyl ist;
R₄ und R₅ zusammen einen fusionierten Ring der Formel B vervollständigen;
R₈ ein H, ein gegebenenfalls mit OH substituiertes C₁₋₃Alkyl ist; oder R₈ ein (CH₂)₂₋₃NR₁₀R₁₁ oder CO₂R₁₂ ist;
R₉ ein Methyl oder C₂₋₃Alkenyl ist, wovon jedes gegebenenfalls mit einem oder mehreren OH, CN, NR₁₀R₁₁, CONR₁₀R₁₁ oder CO₂R₁₂ substituiert ist;
oder R₉ ein Heteroaryl ist, das gegebenenfalls mit einer bis drei Gruppen substituiert ist, die ausgewählt sind aus gegebenenfalls mit Phenyl substituiertem C₁₋₃Alkyl, C₁₋₃Alkoxy, Halogen oder Amino;
R₁₀ und R₁₁ gleich oder verschieden sein können und jedes unabhängig ausgewählt ist aus H, OH, C₁₋₃Alkoxy, verzweigtem oder unverzweigtem, gegebenenfalls mit OH, C₁₋₃Alkoxy, C₁₋₃Acyloxy, CO₂R₁₂, NR₁₃R₁₄, O(CH₂)₂₋₄NR₁₃R₁₄ oder Phenyl substituiertem C₁₋₃Alkyl;
oder R₁₀ und R₁₁ zusammen Morpholino, Pyrrolidinyl, Piperazinyl oder Piperidinyl bilden, wobei jedes gegebenenfalls mit C₁₋₃Alkyl, C₁₋₃Alkoxy oder OH substituiert ist;
R₁₂ ein H oder ein gegebenenfalls mit Phenyl, OH, C₁₋₃Alkoxy oder NR₁₃R₁₄ substituiertes C₁₋₃Alkyl ist;
R₁₃ und R₁₄ je unabhängig aus H und C₁₋₃Alkyl, das gegebenenfalls mit C₁₋₃Alkoxy oder OH substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist.

8. Verbindung nach Anspruch 7, worin:
Ar₁ ein Phenyl ist;
Rₐ ein H oder Hydroxymethyl ist;
R₁ und R₂ gleich oder verschieden sind und ausgewählt sind aus: Halogen, gegebenenfalls teilweise oder vollständig halogeniertem Methyl, NO₂ und NH₂;
R₃ ein H, Chloro, Fluoro, Bromo oder Methoxy ist;
R₁₀ und R₁₁ gleich oder verschieden sein können und jedes unabhängig ausgewählt ist aus H, OH, Methoxy, verzweigtem oder unverzweigtem, gegebenenfalls mit OH, NR₁₃R₁₄ oder Phenyl substituiertem C₁₋₃Alkyl; oder R₁₀ und R₁₁ zusammen Morpholino, Pyrrolidinyl, Piperazinyl oder Piperidinyl bilden, wovon jedes gegebenenfalls mit C₁₋₂Alkyl substituiert ist; und
R₁₂ ein gegebenenfalls mit Morpholino substituiertes C₁₋₃Alkyl ist.

9. Verbindung nach Anspruch 1 der Formel (Ia): 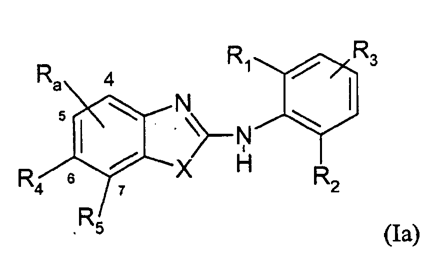 worin:
X ein NH, N-C₁₋₃Alkyl, N-Cyclopropyl, S oder O ist;
Rₐ ein H, C₁₋₁₀Alkyl, C₂₋₁₀Alkenyl oder C₂₋₁₀Alkinyl ist, wovon jedes verzweigt oder zyklisch sein kann; oder Rₐ ein Aryl oder Heteroaryl ist;
wobei jedes Rₐ unabhängig gegebenenfalls mit einem oder mehreren C₁₋₆Alkyl, C₁₋₆ Alkoxy, Halogen, OH, Oxo, NR₁₀R₁₁, Aryl oder Heteroaryl substituiert ist, wobei jedes Aryl oder Heteroaryl gegebenenfalls mit einer oder mehreren Gruppen ausgewählt aus Halogen, OH, C₁₋₃Alkyl, C₁₋₃Alkoxy, HydroxyC₁₋₃alkyl und (CH₂)ₘNR₁₀R₁₁ substituiert ist; und wobei Rₐ an der 4- oder 5-Stellung gebunden ist;
R₁ und R₂ gleich oder verschieden sind und ausgewählt sind aus H, Halogen, CN, NO₂, verzweigtem oder unverzweigtem, gesättigtem oder ungesättigtem C₁₋₁₀Alkyl, verzweigtem oder unverzweigtem C₁₋₁₀Alkoxy, verzweigtem oder unverzweigtem C₁₋₁₀Acyl, verzweigtem oder unverzweigtem C₁₋₁₀Acyloxy, verzweigtem oder unverzweigtem C₁₋₁₀Alkylthio, Aminosulfonyl, Di-(C₁₋₃)alkylaminosulfonyl, NR₁₀R₁₁, Aryl, Aroyl, Aryloxy, Arylsulfonyl, Heteroaryl und Heteroaryloxy; wobei die oben erwähnten R₁ und R₂ gegebenenfalls teilweise oder vollständig halogeniert oder gegebenenfalls mit einer bis drei unabhängig aus Oxo, OH, NR₁₀R₁₁, C₁₋₆ verzweigtem oder unverzweigtem Alkyl, C₃₋₇Cycloalkyl, Phenyl, Naphthyl, Heteroaryl, Aminocarbonyl und Mono- oder Di(C₁₋₃)alkylaminocarbonyl ausgewählten Gruppen substituiert sind;
R₃ ein H, Halogen, OH, (CH₂)ₙNR₁₀R₁₁, CONR₁₀R₁₁, (CH₂)ₙCO₂R₁₂; ein gegebenenfalls mit OH substituiertes C₁₋₃Alkyl, ein gegebenenfalls halogeniertes C₁₋₃Alkoxy oder ein C₁₋₃ Alkylthio ist;
R₄ und R₅ zusammen mit den Atomen, an die sie gebunden sind, ein fusioniertes Ringsystem der Formel A oder B vervollständigen: 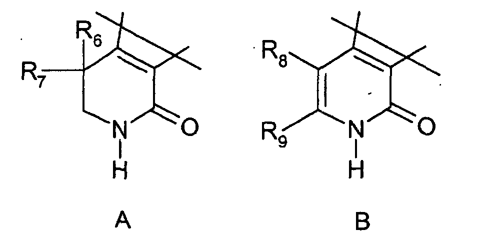
R₆ ein C₁₋₃Alkyl oder H ist;
R₇ ein verzweigtes oder unverzweigtes C₁₋₆Alkyl oder H ist;
R₈ ein H, ein verzweigtes oder unverzweigtes, gesättigtes oder ungesättigtes, gegebenenfalls mit Phenyl, OH oder C₁₋₃Alkoxy substituiertes C₁₋₆Alkyl ist; oder R₈ ein (CH₂)ₘNR₁₀R₁₁, (CH₂)ₘNR₁₀COR₁₂, (CH₂)ₙCO₂R₁₂, (CH₂)ₙCONR₁₀R₁₁ ist; oder R₈ ein Phenyl oder Heteroaryl ist, wovon jedes gegebenenfalls mit C₁₋₃Alkyl, C₁₋₃Alkoxy, OH, -SO₃H oder Halogen substituiert ist;
R₉ ein H, CN oder CONR₁₀R₁₁ ist; oder R₉ ein verzweigtes oder unverzweigtes C₁₋₁₀Alkyl, ein C₃₋₁₀Cycloalkyl, C₅₋₇Cycloalkenyl, C₂₋₆Alkenyl, C₂₋₆Alkinyl ist, wovon jedes gegebenenfalls mit einem oder mehreren C₃₋₁₀Cycloalkyl, C₃₋₁₀Cycloalkyliden, C₅₋₇Cycloalkenyl, Halogen, OH, Oxo, CN, C₁₋₃Alkoxy, C₁₋₃Acyloxy, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, Aryloxy, Arylthio, Aryl oder Heteroaryl substituiert ist; wobei jedes Aryloxy, Arylthio, Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist;
oder R₉ ein Aryl, Heteroaryl, oder Heterozyklus ist, wobei jedes(r) Aryl, Heteroaryl oder Heterozyklus gegebenenfalls mit einer bis drei Gruppen substituiert ist, die ausgewählt sind aus gegebenenfalls mit Phenyl oder NP₁₀C(=NR₁₀)NR₁₀R₁₁ substituiertem C₁₋₃Alkyl, C₁₋₃Alkoxy, Halogen, CN, Oxo, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 5- oder 6-gliedrigen aromatischen oder nichtaromatischen carbozyklischen Ring bilden, der gegebenenfalls mit einem oder zwei C₁₋₃Alkyl, OH, Oxo oder (CH₂)ₙNR₁₀R₁₁ substituiert ist oder gegebenenfalls mit einer 1,3-Dioxolan-Gruppe oder 1,3-Dithiolan-Gruppe spiro-verknüpft ist, wobei jede 1,3-Dioxolan-Gruppe oder 1,3-Dithiolan-Gruppe gegebenenfalls mit C₁₋₆Alkyl, C₁₋₆Alkoxy, OH oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Aryl, ArylC₁₋₃alkyl und Heteroaryl; wobei besagte Alkyl, Cycloalkyl, Aryl, ArylC₁₋₃alkyl oder Heteroaryl gegebenenfalls mit OH, C₁₋₃Alkoxy, CN, NO₂, C₁₋₃Acyloxy, CO₂R₁₂, NR₁₃R₁₄, O(CH₂)₂₋₄NR₁₃R₁₄, Aryl oder Heteroaryl substituiert sind;
oder R₁₀ und R₁₁ zusammen eine 3-7-gliedrige Alkylen-Kette bilden, welche über das N Atom, an dem sie angebracht sind, einen Ring vervollständigen; wobei besagte Alkylen-Kette gegebenenfalls von O, S(O)ₚ, und NR₁₃ unterbrochen ist; und wobei besagter Ring gegebenenfalls mit C₁₋₃Alkyl, C₁₋₃Alkoxy, OH, -(CH₂)ₙNR₁₃R₁₄, CONR₁₃R₁₄ oder NR₁₃COR₁₄ substituiert ist;
R₁₂ ein H, C₁₋₆Alkyl oder C₃₋₈Cycloalkyl ist, worin jedes Alkyl oder Cycloalkyl gegebenenfalls mit Phenyl, OH, C₁₋₃Alkoxy oder NR₁₃R₁₄ substituiert ist; oder R₁₂ ein gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiertes(r) Phenyl oder Heterozyklus ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette ein (CH₂)₄₋₅ oder (CH₂)₂O(CH₂)₂ ist;
m 1-4 ist, n 0-3 ist und p 0-2 ist; und
deren pharmazeutisch annehmbare Säure- oder Salzderivate.

10. Verbindung nach Anspruch 9, worin:
X ein NH oder N-CH₃ ist;
Rₐ ein H, HydroxyC₁₋₂alkyl, 2-Hydroxyethylaminomethyl, Methoxybenzylaminomethyl, ein gegebenenfalls halogeniertes Pyridinyl, ein Phenyl, 3-Hydroxy-2-oxo-propyl, Vinyl oder ein mit C₁₋₃Alkoxy oder Phenyl substituiertes C₃₋₅ Alkinyl ist; und worin Rₐ an der 4-Stellung gebunben ist;
R₁ und R₂ gleich oder verschieden sind und ausgewählt sind aus: H, Halogen, C₁₋₃Alkyl, wobei das C₁₋₃Alkyl gegebenenfalls teilweise oder vollständig halogeniert ist, NO₂, NR₁₃R₁₄;
R₃ ein H, Halogen, Methoxy oder Methyl ist;
R₄ und R₅ zusammen einen fusionierten Ring der Formel B vervollständigen;
R₈ ein H, ein gegebenenfalls mit OH substituiertes C₁₋₃Alkyl ist; oder R₈ ein (CH₂)₂₋₃NR₁₀R₁₁ oder CO₂R₁₂ ist;
R₉ ein CN ist; oder R₉ ein Methyl, C₂₋₃Alkenyl oder C₂₋₃Alkinyl ist, wovon jedes gegebenenfalls mit einem oder mehreren C₅₋₇Cycloalkyliden, C₅₋₇Cycloalkenyl, OH, CN, NR₁₀R₁₁, NR₁₀CONR₁₀R₁₁, NR₁₀COR₁₂, NR₁₀S(O)ₚR₁₂, CONR₁₀R₁₁, CO₂R₁₂, C(R₁₀)=NNR₁₀R₁₁ oder Heteroaryl substituiert ist;
oder R₉ ein Aryl oder Heteroaryl ist, die gegebenenfalls mit einer bis drei Gruppen substituiert sind, die ausgewählt sind aus gegebenenfalls mit Phenyl substituiertem C₁₋₃ Alkyl, C₁₋₃Alkoxy, Halogen, Amino oder CONH₂;
oder R₈ und R₉ zusammen einen Cyclopenten-Ring bilden, der mit einer 1,3-Dioxolan-Gruppe spiro-verknüpft ist, wobei besagte 1,3-Dioxolan-Gruppe gegebenenfalls mit C₁₋₃Alkyl, C₁₋₃Alkoxy, OH oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₃Alkyl, C₅₋₇Cycloalkyl oder Phenyl, wobei besagte Alkyl, Cycloalkyl oder Phenyl gegebenenfalls mit OH, C₁₋₃Alkoxy, C₁₋₃Acyloxy, NO₂, CO₂R₁₂, NR₁₃R₁₄, O(CH₂)₂₋₄NR₁₃R₁₄ oder Phenyl substituiert sind;
oder R₁₀ und R₁₁ zusammen Morpholino, Pyrrolidinyl, Piperazinyl oder Piperidinyl bilden, wovon jedes gegebenenfalls mit C₁₋₃Alkyl, C₁₋₃Alkoxy, OH, (CH₂)ₙNR₁₃R₁₄, CONR₁₃R₁₄ oder NR₁₃COR₁₄ substituiert ist;
R₁₂ ein H, C₁₋₃Alkyl oder C₅₋₇Cycloalkyl ist, wovon jedes gegebenenfalls mit Phenyl, OH, C₁₋₃Alkoxy oder NR₁₃R₁₄ substituiert ist; oder R₁₂ ein Phenyl oder ein gesättigter 4-bis 6-gliedriger Stickstoff enthaltender Heterozyklus ist, wovon jeder gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiert ist;
R₁₃ und R₁₄ je unabhängig ausgewählt sind aus H und C₁₋₃Alkyl, das gegebenenfalls mit C₁₋₃Alkoxy oder OH substituiert ist;
oder R₁₃ und R₁₄ zusammen eine ringschliessende Kette bilden, worin besagte Kette (CH₂)₄₋₅ oder (CH₂)₂O(CH₂)₂ ist.

11. Verbindung nach Anspruch 10 worin:
Rₐ ein H oder Hydroxymethyl ist;
R₁ und R₂ gleich oder verschieden sind und ausgewählt sind aus: Halogen, gegebenenfalls teilweise oder vollständig halogeniertem Methyl, NO₂ und NH₂;
R₃ ein H, Chloro, Fluoro, Bromo oder Methoxy ist;
R₁₀ und R₁₁ gleich oder verschieden sein können und jedes unabhängig ausgewählt ist aus H, OH, Methoxy, verzweigtem oder unverzweigtem C₁₋₃Alkyl oder C₅₋₇Cycloalkyl, wobei besagte Alkyl oder Cycloalkyl gegebenenfalls mit OH, NR₁₃R₁₄ oder Phenyl substituiert sind;
oder R₁₀ und R₁₁ zusammen Morpholino, Pyrrolidinyl, Piperazinyl oder Piperidinyl bilden, wobei jedes gegebenenfalls mit C₁₋₂Alkyl, NR₁₃R₁₄, CONR₁₃R₁₄ oder NR₁₃COR₁₄ substituiert ist; und
R₁₂ ein gegebenenfalls mit Morpholino substituiertes C₁₋₃Alkyl ist; oder R₁₂ ein Phenyl oder ein Azetidinyl, Pyrrolidinyl oder Piperidinyl ist, wobei jedes gegebenenfalls mit einer bis drei aus C₁₋₃Alkyl, C₁₋₃Alkoxy und Halogen ausgewählten Gruppen substituiert ist.

12. Verbindung nach Anspruch 9, worin:
X ein NH, N-C₁₋₃Alkyl, N-Cyclopropyl, S oder O ist;
Rₐ ein H, C₁₋₁₀Alkyl, C₂₋₁₀Alkenyl oder C₂₋₁₀Alkinyl ist, wovon jedes verzweigt oder zyklisch sein kann; oder Rₐ ein Aryl oder Heteroaryl ist;
wobei jedes Rₐ unabhängig gegebenenfalls mit einem oder mehreren C₁₋₆Alkoxy, Halogen, OH, Oxo, NR₁₀R₁₁, Aryl oder Heteroaryl substituiert ist, wobei jedes Aryl oder Heteroaryl gegebenenfalls mit einer oder mehreren Gruppen ausgewählt aus Halogen, OH, C₁₋₃Alkyl, C₁₋₃Alkoxy, HydroxyC₁₋₃alkyl und (CH₂)ₘNR₁₀R₁₁ substituiert ist; und wobei Rₐ an die 4-oder 5-Stellung gebunden ist;
R₁ und R₂ gleich oder verschieden sind und ausgewählt sind aus H, Halogen, CN, NO₂, verzweigtem oder unverzweigtem, gesättigten oder ungesättigten C₁₋₁₀ Alkyl, verzweigtem oder unverzweigtem C₁₋₁₀Alkoxy, verzweigtem oder unverzweigtem C₁₋₁₀ Acyl, verzweigtem oder unverzweigtem C₁₋₁₀Acyloxy, verzweigtem oder unverzweigtem C₁₋₁₀Alkylthio, Aminosulfonyl, Di-(C₁₋₃)alkylaminosulfonyl, NR₁₀R₁₁, Aryl, Aroyl, Aryloxy, Arylsulfonyl, Heteroaryl und Heteroaryloxy; wobei die oben erwähnten R₁ und R₂ gegebenenfalls teilweise oder vollständig halogeniert oder gegebenenfalls mit einer bis drei unabhängig aus Oxo, OH, NR₁₀R₁₁, verzweigtem oder unverzweigtem C₁₋₆Alkyl, C₃₋₇Cycloalkyl, Phenyl, Naphthyl, Heteroaryl, Aminocarbonyl und Mono- oder Di(C₁₋₃)alkylaminocarbonyl ausgewählten Gruppen substituiert sind;
R₃ ein H, Halogen, OH, (CH₂)ₙNR₁₀R₁₁, (CH₂)ₙCO₂R₁₂; ein gegebenenfalls mit OH substituiertes C₁₋₃Alkyl, ein gegebenenfalls halogeniertes C₁₋₃ Alkoxy oder C₁₋₃ Alkylthio ist;
R₄ und R₅ zusammen mit den Atomen, an die sie gebunden sind, ein fusioniertes Ringsystem der Formeln A oder B vervollständigen: 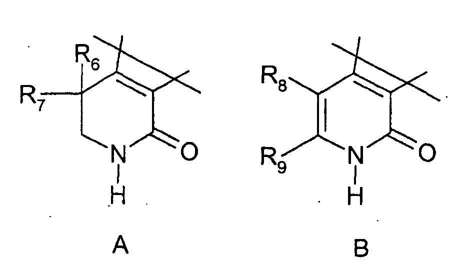
R₆ ein C₁₋₃Alkyl oder H ist;
R₇ ein verzweigtes oder unverzweigtes C₁₋₆Alkyl oder H ist;
R₈ ein H, ein verzweigtes oder unverzweigtes, gesättigtes oder ungesättigtes, gegebenenfalls mit Phenyl, OH oder C₁₋₃Alkoxy substituiertes C₁₋₆Alkyl ist; oder R₈ ein (CH₂)ₘNR₁₀R₁₁, (CH₂)ₘNR₁₀COR₁₂, (CH₂)ₙCO₂R₁₂, (CH₂)ₙCONR₁₀R₁₁ ist oder R₈ ein Phenyl oder Heteroaryl ist, wovon jedes gegebenenfalls mit C₁₋₃Alkyl, C₁₋₃Alkoxy, OH, -SO₃H oder Halogen substituiert ist;
R₉ ein H ist; oder R₉ ein verzweigtes oder unverzweigtes C₁₋₁₀Alkyl, ein C₃₋₁₀Cycloalkyl, C₂₋₆Alkenyl, C₂₋₆Alkinyl ist, wovon jedes gegebenenfalls mit einem oder mehreren Halogen, OH, Oxo, CN, C₁₋₃Alkoxy, NR₁₀R₁₁, NR₁₀COR₁₂, SR₁₂, CONR₁₀R₁₁, CO₂R₁₂, Aryloxy, Arylthio, Aryl oder Heteroaryl substituiert ist; wobei jedes Aryloxy, Arylthio, Aryl oder Heteroaryl gegebenenfalls mit C₁₋₃Alkyl, C₁₋₃Alkoxy, Halogen, (CH₂)ₙNR₁₀R₁₁ oder O(CH₂)₂₋₄NR₁₀R₁₁ substituiert ist;
oder R₉ ein Aryl oder Heteroaryl ist, wobei jedes Aryl oder Heteroaryl gegebenenfalls mit einer bis drei Gruppen substituiert ist, die ausgewählt sind aus gegebenenfalls mit Phenyl substituiertem C₁₋₃Alkyl, C₁₋₃Alkoxy, Halogen, (CH₂)ₙNR₁₀R₁₁, (CH₂)ₙCO₂R₁₂; (CH₂)ₙCONR₁₀R₁₁ und O(CH₂)₂₋₄NR₁₀R₁₁;
oder R₈ und R₉ zusammen einen gesättigten oder ungesättigten 6-gliedrigen aromatischen oder nichtaromatischen carbozyklischen Ring bilden, der gegebenenfalls mit einem oder zwei OH, Oxo oder (CH₂)ₙNR₁₀R₁₁ substituiert ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Aryl, ArylC₁₋₃alkyl und Heteroaryl; wobei besagte Alkyl, Cycloalkyl, Aryl, ArylC₁₋₃alkyl oder Heteroaryl gegebenenfalls mit OH, C₁₋₃Alkoxy, C₁₋₃Acyloxy, CO₂R₁₂, NR₁₃R₁₄, O(CH₂)₂₋₄NR₁₃R₁₄, Aryl oder Heteroaryl substituiert sind;
oder R₁₀ und R₁₁ zusammen eine 3- bis 7-gliedrige Alkylen-Kette bilden, welche über das N Atom, an dem sie angebracht sind, einen Ring vervollständigt; wobei besagte Alkylen-Kette gegebenenfalls von O, S(O)ₚ, und NR₁₃ unterbrochen ist; und wobei besagter Ring gegebenenfalls mit C₁₋₃Alkyl, C₁₋₃Alkoxy, OH oder -(CH₂)ₙNR₁₃R₁₄ substituiert ist;
R₁₂ ein H, C₁₋₆Alkyl oder C₃₋₈Cycloalkyl ist, worin jedes Alkyl oder Cycloalkyl gegebenenfalls mit Phenyl, OH, C₁₋₃Alkoxy oder NR₁₃R₁₄ substituiert ist; oder R₁₂ ein gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiertes Phenyl ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist;
m 1-4 ist, n 0-3 ist und p 0-2 ist; und
deren pharmazeutisch annehmbare Säure- oder Salzderivate.

13. Verbindung nach Anspruch 12, worin:
X ein NH oder N-CH₃ ist;
Rₐ ein H, HydroxyC₁₋₂alkyl, 2-Hydroxyethylaminomethyl, Methoxybenzylaminomethyl, ein gegebenenfalls halogeniertes Pyridinyl, ein Phenyl, 3-Hydroxy-2-oxo-propyl, Vinyl oder ein mit C₁₋₃Alkoxy oder Phenyl substituiertes C₃₋₅Alkinyl ist; und wobei Rₐ an der 4-Stellung gebunden ist;
R₁ und R₂ gleich oder verschieden sind und ausgewählt sind aus: Halogen, C₁₋₃Alkyl, wobei das C₁₋₃Alkyl gegebenenfalls teilweise oder vollständig halogeniert ist, NO₂, NR₁₃R₁₄;
R₃ ein H, Halogen, Methoxy oder Methyl ist;
R₄ und R₅ zusammen einen fusionierten Ring der Formel B vervollständigen;
R₈ ein H oder ein gegebenenfalls mit OH substituiertes C₁₋₃Alkyl ist; oder R₈ ein (CH₂)₂₋₃NR₁₀R₁₁ oder CO₂R₁₂ ist;
R₉ ein Methyl oder C₂₋₃Alkenyl ist, wovon jedes gegebenenfalls mit einem oder mehreren OH, CN, NR₁₀R₁₁, CONR₁₀R₁₁ oder CO₂R₁₂ substituiert ist;
oder R₉ ein Heteroaryl ist, das gegebenenfalls mit einer bis drei Gruppen substituiert ist, die ausgewählt sind aus gegebenenfalls mit Phenyl substituiertem C₁₋₃Alkyl, C₁₋₃Alkoxy, Halogen oder (CH₂)ₙNR₁₀R₁₁;
R₁₀ und R₁₁ gleich oder verschieden sein können und jedes unabhängig ausgewählt ist aus H, OH, C₁₋₃Alkoxy, verzweigtem oder unverzweigtem, gegebenenfalls mit OH, C₁₋₃Alkoxy, C₁₋₃Acyloxy, CO₂R₁₂, NR₁₃R₁₄, O(CH₂)₂₋₄NR₁₃R₁₄ oder Phenyl substituiertem C₁₋₃Alkyl;
oder R₁₀ und R₁₁ zusammen Morpholino, Pyrrolidinyl, Piperazinyl oder Piperidinyl bilden, wobei jedes gegebenenfalls mit C₁₋₃Alkyl, C₁₋₃Alkoxy oder OH substituiert ist;
R₁₂ ein H oder ein gegebenenfalls mit Phenyl, OH, C₁₋₃Alkoxy oder NR₁₃R₁₄ substituiertes C₁₋₃Alkyl ist;
R₁₃ und R₁₄je unabhängig aus H und C₁₋₃Alkyl, das gegebenenfalls mit C₁₋₃Alkoxy oder OH substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist.

14. Verbindung nach Anspruch 13, worin:
Rₐ ein H oder Hydroxymethyl ist;
R₁ und R₂ gleich oder verschieden sind und ausgewählt sind aus: Halogen, gegebenenfalls teilweise oder vollständig halogeniertem Methyl, NO₂ und NH₂;
R₃ ein H, Chloro, Fluoro, Bromo oder Methoxy ist;
R₁₀ und R₁₁ gleich oder verschieden sein können und jedes unabhängig ausgewählt ist aus H, OH, Methoxy, verzweigtem oder unverzweigtem, gegebenenfalls mit OH, NR₁₃R₁₄ oder Phenyl substituiertem C₁₋₃Alkyl;
oder R₁₀ und R₁₁ zusammen Morpholino, Pyrrolidinyl, Piperazinyl oder Piperidinyl bilden,
wobei jedes gegebenenfalls mit C₁₋₂Alkyl substituiert ist; und
R₁₂ ein gegebenenfalls mit Morpholino substituiertes C₁₋₃Alkyl ist.

15. Zwischenverbindung der Formel (III): 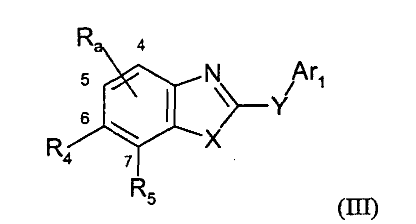 worin:
Ar₁ ein aromatischer oder nichtaromatischer Carbozyklus, Heteroaryl oder Heterozyklus ist; wobei besagter(s) Carbozyklus, Heteroaryl oder Heterozyklus gegebenenfalls mit einem oder mehreren R₁, R₂ und R₃ substituiert ist;
X ein NH, N-C₁₋₃Alkyl, N-Cyclopropyl, S oder O ist;
Y ein NR₁₅, S oder O ist;
Rₐ ein H, C₁₋₁₀Alkyl, C₂₋₁₀Alkenyl oder C₂₋₁₀Alkinyl ist, wovon jedes verzweigt oder zyklisch sein kann; oder Rₐ ein Aryl oder Heteroaryl ist;
wobei jedes Rₐ unabhängig gegebenenfalls mit einer oder mehreren C₁₋₃Alkyl, C₁₋₆Alkoxy, Halogen, OH, Oxo, NR₁₀R₁₁, Aryl oder Heteroaryl substituiert ist, wovon jedes Aryl oder Heteroaryl gegebenenfalls mit einer oder mehreren Gruppen ausgewählt aus Halogen, OH, C₁₋₃Alkyl, C₁₋₃Alkoxy, HydroxyC₁₋₃alkyl und (CH₂)ₘNR₁₀R₁₁ substituiert ist; und wobei Rₐ an der 4- oder 5- Stellung gebunden ist;
R₁ und R₂ gleich oder verschieden sind und ausgewählt sind aus H, Halogen, CN, NO₂, verzweigtem oder unverzweigtem, gesättigtem oder ungesättigtem C₁₋₁₀Alkyl, verzweigtem oder unverzweigtem C₁₋₁₀Alkoxy, verzweigtem oder unverzweigtem C₁₋₁₀Acyl, verzweigtem oder unverzweigtem C₁₋₁₀Acyloxy, verzweigtem oder unverzweigtem C₁₋₁₀Alkylthio, Aminosulfonyl, Di-(C₁₋₃)alkylaminosulfonyl, NR₁₀R₁₁, Aryl, Aroyl, Aryloxy, Arylsulfonyl, Heteroaryl und Heteroaryloxy; wobei die oben erwähnten R₁ und R₂ gegebenenfalls teilweise oder vollständig halogeniert oder gegebenenfalls mit einer bis drei unabhängig aus Oxo, OH, NR₁₀R₁₁, verzweigtem oder unverzweigtem C₁₋₆Alkyl, C₃₋₇Cycloalkyl, Phenyl, Naphthyl, Heteroaryl, Aminocarbonyl und Mono- oder Di(C₁₋₃)Alkylaminocarbonyl ausgewählten Gruppen substituiert sind;
R₃ ein H, Halogen, OH, (CH₂)ₙNR₁₀R₁₁, (CH₂)ₙCO₂R₁₂; ein gegebenenfalls mit OH substituiertes C₁₋₃Alkyl, ein gegebenenfalls halogeniertes C₁₋₃Alkoxy oder C₁₋₃ Alkylthio ist;
R₄ und R₅ zusammen mit den Atomen, an die sie gebunden sind, ein fusioniertes Ringsystem der Formel C vervollständigen: 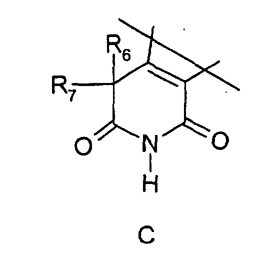
R₆ ein C₁₋₃Alkyl oder H ist;
R₇ ein verzweigtes oder unverzweigtes C₁₋₆Alkyl oder H ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Aryl, ArylC₁₋₃alkyl und Heteroaryl; wobei besagte Alkyl, Cycloalkyl, Aryl, ArylC₁₋₃alkyl oder Heteroaryl gegebenenfalls mit OH, C₁₋₃Alkoxy, C₁₋₃Acyloxy, CO₂R₁₂, NR₁₃R₁₄, O(CH₂)₂₋₄NR₁₃R₁₄, Aryl oder Heteroaryl substituiert sind;
oder R₁₀ und R₁₁ zusammen eine 3-7-gliedrige Alkylen-Kette bilden, welche über das N-Atom, an dem sie angebracht sind, einen Ring vervollständigt; wobei besagte Alkylen-Kette gegebenenfalls von O, S(O)ₚ und NR₁₃ unterbrochen ist; und wobei besagter Ring gegebenenfalls mit C₁₋₃Alkyl, C₁₋₃Alkoxy, OH oder -(CH₂)ₙNR₁₃R₁₄ substituiert ist;
R₁₂ ein H, C₁₋₆Alkyl oder C₃₋₈Cycloalkyl ist, wovon jedes Alkyl oder Cycloalkyl gegebenenfalls mit Phenyl, OH, C₁₋₃Alkoxy oder NR₁₃R₁₄ substituiert ist; oder R₁₂ ein gegebenenfalls mit einer bis drei aus C₁₋₃Alkyl, C₁₋₃Alkoxy, Halogen, (CH₂)ₘNR₁₀R₁₁, (CH₂)ₙCONR₁₀R₁₁ und O(CH₂)₂₋₄NR₁₀R₁₁ ausgewählten Gruppen substituiertes Phenyl ist;
R₁₃ und R₁₄ je unabhängig aus H und C₁₋₆Alkyl, das gegebenenfalls mit Alkoxy, OH oder Phenyl substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist; und
m 1-4 ist, n 0-3 ist und p 0-2 ist;
wobei sich der Begriff Heteroaryl auf ein stabiles 5- bis 8-gliedriges monozyklisches oder 8- bis 11-gliedriges bizyklisches aromatisches heterozyklisches Radikal bezieht, das aus Kohlenstoffatomen und 1 bis 4 aus Stickstoff, Sauerstoff und Schwefel ausgewählten Heteroatomen besteht;
sich der Begriff Heterozyklus auf ein stabiles 4- bis 8-gliedriges monozyklisches oder 8-bis 11-gliedriges bizyklisches heterozyklisches Radikal bezieht, das entweder gesättigt oder ungesättigt sein kann und nichtaromatisch ist und aus Kohlenstoffatomen sowie 1 bis 4 aus Stickstoff, Sauerstoff und Schwefel ausgewählten Heteroatomen besteht;
sich der Begriff Aryl auf einen 6- bis 10-gliedrigen aromatischen Carbozyklus bezieht; sich der Begriff Carbozyklus auf eine 3- bis 10-gliedrige aromatische oder nichtaromatische zyklische Kohlenstoffkette bezieht.

16. Verbindung nach Anspruch 15, worin
Ar₁ ist:
a) eine aus Cyclopropyl, Cyclobutyl, Cyclopentanyl, Cyclohexanyl, Cycloheptanyl ausgewählte Cycloalkyl-Gruppe;
b) eine aus Cyclopentenyl, Cyclohexenyl, Cycloheptenyl ausgewählte Cycloalkenyl-Gruppe;
c) Phenyl, Naphthyl; Indanyl, Indenyl, Dihydronaphthyl, Tetrahydronaphthyl, Fluorenyl;
d) ein aus Pyridinyl, Pyrimidinyl, Pyrazinyl, Pyridazinyl, Pyrrolyl, Imidazolyl, Pyrazolyl, Thienyl, Furyl, Isoxazolyl, Isothiazolyl, Oxazolyl, Oxadiazolyl, Thiazolyl, Thiadiazolyl, Chinolinyl, Isochinolinyl, Indolyl, Benzimidazolyl, Benzofuranyl, Benzoxazolyl, Benzisoxazolyl, Benzpyrazolyl, Benzothiofuranyl, Benzothiazolyl, Chinazolinyl und Indazolyl ausgewähltes Heteroaryl, oder ein aus Cyclopentenopyridin, Cyclohexanopyridin, Cyclopentanopyrimidin, Cyclohexanopyrimidin, Cyclopentanopyrazin, Cyclohexanopyrazin, Cyclopentanopyridazin, Cyclohexanopyridazin, Cyclopentanochinolin, Cyclohexanochinolin, Cyclopentanoisochinolin, Cyclohexanoisochinolin, Cyclopentanoindol, Cyclohexanoindol, Cyclopentanobenzimidazol, Cyclohexanobenzimidazol, Cyclopentanobenzoxazol, Cyclohexanobenzoxazol, Cyclopentanoimidazol, Cyclohexanoimidazol, Cyclopentanothiophen und Cyclohexanothiophen ausgewähltes fusioniertes Heteroaryl; oder
e) ein aus: Pyrrolinyl, Pyrrolidinyl, Pyrazolinyl, Pyrazolidinyl, Piperidinyl, Morpholinyl, Thiomorpholinyl, Pyranyl, Thiopyranyl, Piperazinyl und Indolinyl ausgewählter Heterozyklus;
wobei jedes der obigen Ar₁ gegebenenfalls mit einem oder mehreren R₁, R₂ und R₃ substituiert ist;
Rₐ ein H, C₁₋₆Alkyl, C₂₋₅Alkenyl, C₂₋₅Alkinyl, Phenyl oder Heteroaryl ist, das aus: Pyridinyl, Pyridazinyl, Pyrimidinyl, Pyrazinyl, Oxazolyl, Pyrazolyl, Imidazolyl, Furyl, Thiazolyl und Thienyl ausgewählt ist; wobei jedes Rₐ gegebenenfalls mit einer oder mehreren Phenyl, Halogen, C₁₋₃Alkyl, C₁₋₃Alkoxy, OH, Oxo oder NR₁₀R₁₁ substituiert ist; wobei sich Rₐ an der 4-Stellung befindet;
R₃ ein H, Halogen, Methyl, Methoxy, Hydroxymethyl oder OH ist;
R₆ ein C₁₋₃Alkyl oder H ist;
R₇ ein verzweigtes oder unverzweigtes C₁₋₆Alkyl oder H ist;
R₁₀ und R₁₁ gleich oder verschieden sein können und jedes unabhängig ausgewählt ist aus H, OH, C₁₋₃Alkoxy, verzweigtem oder unverzweigtem C₁₋₆Alkyl, C₃₋₈Cycloalkyl, Benzyl und Phenyl; wobei besagtes Alkyl, Cycloalkyl oder Phenyl gegebenenfalls mit OH, C₁₋₃Alkoxy, C₁₋₃Acyloxy, CO₂R₁₂, NR₁₃R₁₄, O(CH₂)₂₋₄NR₁₃R₁₄ oder Phenyl substituiert ist;
oder R₁₀ und R₁₁ zusammen Morpholino, Pyrrolidinyl, Piperazinyl oder Piperidinyl bilden, wovon jedes gegebenenfalls mit C₁₋₃Alkyl, C₁₋₃Alkoxy, OH oder -(CH₂)ₙNR₁₃R₁₄ substituiert ist;
R₁₂ ein H oder ein gegebenenfalls mit Phenyl, OH, C₁₋₃Alkoxy oder NR₁₃R₁₄ substituiertes C₁₋₆Alkyl ist;
R₁₃ und R₁₄ je unabhängig aus H und C₁₋₆Alkyl, das gegebenenfalls mit C₁₋₃Alkoxy, OH oder Phenyl substituiert ist, ausgewählt sind;
und
oder R₁₃ und R₁₄ zusammen eine ringschliessende Kette bilden, wobei besagte Kette (CH₂)₄₋₅ oder (CH₂)₂O(CH₂)₂ ist.

17. Verbindung nach Anspruch 16, worin:
Ar₁ ein Phenyl, oder Pyridyl ist;
X ein NH oder N-CH₃ ist;
Y ein NH ist und
Rₐ ein H, HydroxyC₁₋₂alkyl, 2-Hydroxyethylaminomethyl, Methoxybenzylaminomethyl, ein gegebenenfalls halogeniertes Pyridinyl, ein Phenyl, 3-Hydroxy-2-oxo-propyl, Vinyl oder ein mit C₁₋₃Alkoxy oder Phenyl substituiertes C₃₋₅ Alkinyl ist;
R₁ und R₂ gleich oder verschieden sind und ausgewählt sind aus: Halogen, C₁₋₃ Alkyl, wobei die C₁₋₃Alkyl gegebenenfalls teilweise oder vollständig halogeniert sind, NO₂, NR₁₃R₁₄;
R₃ ein H, Halogen, Methoxy oder Methyl ist;
R₁₀ und R₁₁ gleich oder verschieden sein können und jedes unabhängig ausgewählt ist aus H, OH, C₁₋₃Alkoxy, verzweigtem oder unverzweigtem, gegebenenfalls mit OH, C₁₋₃Alkoxy, C₁₋₃Acyloxy, CO₂R₁₂, NR₁₃R₁₄, O(CH₂)₂₋₄NR₁₃R₁₄ oder Phenyl substituiertem C₁₋₃Alkyl;
oder R₁₀ und R₁₁ zusammen Morpholino, Pyrrolidinyl, Piperazinyl oder Piperidinyl bilden, wobei jedes gegebenenfalls mit C₁₋₃ Alkyl, C₁₋₃Alkoxy oder OH substituiert ist;
R₁₂ ein H oder ein gegebenenfalls mit Phenyl, OH, C₁₋₃Alkoxy oder NR₁₃R₁₄ substituiertes C₁₋₃Alkyl ist;
R₁₃ und R₁₄ je unabhängig aus H und C₁₋₃Alkyl, das gegebenenfalls mit C₁₋₃Alkoxy oder OH substituiert ist, ausgewählt sind;
oder R₁₃ und R₁₄ zusammen eine ringschliessende Kette bilden, wobei besagte Kette (CH₂)₄₋₅ oder (CH₂)₂O(CH₂)₂ ist.

18. Verbindung nach Anspruch 17, worin:
Ar₁ ein Phenyl ist;
Rₐ ein H oder Hydroxymethyl ist;
R₁ und R₂ gleich oder verschieden sind und ausgewählt sind aus: Halogen, gegebenenfalls teilweise oder vollständig halogeniertem Methyl, NO₂ und NH₂;
R₃ ein H, Chloro, Fluoro, Bromo oder Methoxy ist;
R₁₀ und R₁₁ gleich oder verschieden sein können und jedes unabhängig ausgewählt ist aus H, OH, Methoxy, verzweigtem oder unverzweigtem, gegebenenfalls mit OH, NR₁₃R₁₄ oder Phenyl substituiertem C₁₋₃Alkyl; oder R₁₀ und R₁₁ zusammen Morpholino, Pyrrolidinyl, Piperazinyl oder Piperidinyl bilden, wobei jedes gegebenenfalls mit C₁₋₂Alkyl substituiert ist; und
R₁₂ ein gegebenenfalls mit Morpholino substituiertes C₁₋₃Alkyl ist.

19. Verbindung nach Anspruch 9, ausgewählt aus der Gruppe bestehend aus:
2-(2,6-Dichlorophenylamino)-3,5-dihydro-imidazo[4,5-i]phenanthridin-4-on;
2-(2,6-Dichlorophenylamino)-1,6,7-trimethyl-1,8-dihydro-imidazo[4,5-h]isochinolin-9-on;
2-(2,6-Dichlorophenylamino)-1,7-dimethyl-6-(2-hydroxyethyl)-1,8-dihydro-imidazo[4,5-h]isochinolin-9-on;
2-(2,6-Dichlorophenylamino)-1,7-dimethyl-9-oxo-1,8-dihydro-imidazo[4,5-h]isochinolin-6-carbonsäure-methylester;
3-[2-(2,6-Dichlorophenylamino)-1-methyl-9-oxo-8,9-dihydro-1H-imidazo[4,5-h]isochinolin-7-yl]-acrylsäure-methylester;
2-(2-Chloro-6-methylphenylamino)-1,6,7-trimethyl-1,8-dihydro-imidazo[4,5-h]isochinolin-9-on;
3-[2-(2,6-Dichlorophenylamino)-1-methyl-9-oxo-8,9-dihydro-1H-imidazo[4,5-h]isochinolin-7-yl]-N-methoxy-N-methylacrylamid;
2-(2-Chloro-6-nitrophenylamino)-1,6,7-trimethyl-1,8-dihydro-imidazo[4,5-h]isochinolin-9-on;
N-Benzyl-3-[2-(2,6-Dichlorophenylamino)-1-methyl-9-oxo-8,9-dihydro-1H-imidazo[4,5-h]isochinolin-7-yl]-acrylamid;
3-[2-(2,6-Dichlorophenylamino)-1-methyl-9-oxo-8,9-dihydro-1H-imidazo[4,5-h]isochinolin-7-yl]-acrylsäure-4-morpholinamid;
2-(2,6-Dichlorophenylamino)-4-hydroxymethyl-1,6,7-trimethyl-1,8-dihydro-imidazo[4,5-h]isochinolin-9-on;
2-(2,6-Dichlorophenylamino)-1,6-dimethyl-7-vinyl-1,8-dihydro-imidazo[4,5-h]isochinolin-9-on;
2-(2,6-Dichlorophenylamino)-1,7-dimethyl-9-oxo-1,8-dihydro-imidazo[4,5-h]isochinolin-6-carbonsäure-2-(4-moropholino)ethylester;
2-(2,6-Dichlorophenylamino)-1,6-dimethyl-7-(3-hydroxypropen-1-yl)-1,8-dihydro-imidazo[4,5-h]-isochinolin-9-on;
2-(2,6-Dichlorophenylamino)-1,6-dimethyl-7-oxazol-5-yl-1,8-dihydro-imidazo[4,5-h]isochinolin-9-on;
2-(2,6-Dichlorophenylamino)-1-methyl-7-vinyl-1,8-dihydro-imidazo[4,5-h]isochinolin-9-on;
2-(2,6-Dichlorophenylamino)-1,6-dimethyl-7-(3-morpholin-4-yl-propen-1-yl)-1,8-dihydro-imidazo[4,5-h]-isochinolin-9-on;
3-[2-(2,6-Dichlorophenylamino)-1,7-dimethyl-9-oxo-8,9-dihydro-1H-imidazo[4,5-h]isochinolin-7-yl]-acrylonitril;
2-(2-Chloro-6-methylphenylamino)-1,7-dimethyl-1,8-dihydro-imidazo[4,5-h]isochinolin-9-on;
2-(2,6-Dichlorophenylamino)-1-methyl-7-oxazol-5-yl-1,8-dihydro-imidazo[4,5-h]isochinolin-9-on;
2-(2,6-Dichlorophenylamino)-7-(3-hydroxypropen-1-yl)-1-methyl-1,8-dihydro-imidazo[4,5-h]-isochinolin-9-on;
2-(2-Chloro-6-methylphenylamino)-7-(3-hydroxypropen-1-yl)-1-methyl-1,8-dihydro-imidazo[4,5-h]-isochinolin-9-on;
2-(2,6-Dichlorophenylamino)-7-(3-diethylaminopropen-1-yl)-1,6-dimethyl-1,8-dihydro-imidazo[4,5-h]-isochinolin-9-on;
2-(2,6-Dichlorophenylamino)-1,6-dimethyl-7-(3-pyrrolidin-1-yl-propen-1-yl)-1,8-dihydro-imidazo[4,5-h]-isochinolin-9-on;
2-(2,6-Dichlorophenylamino)-7-(3-diethylaminopropen-1-yl)-1-methyl-1,8-dihydro-imidazo[4,5-h]-isochinolin-9-on;
2-(2,6-Dichlorophenylamino)-1,6-dimethyl-7-(4-methylpiperazin-1-yl-propen-1-yl)-1,8-dihydro-imidazo[4,5-h]-isochinolin-9-on;
2-(2,6-Dichlorophenylamino)-1,6-dimethyl-7-(3-piperidin-1-yl-propen-1-yl)-1,8-dihydro-imidazo[4,5-h]-isochinolin-9-on;
2-(2,6-Dichlorophenylamino)-1,6-dimethyl-7-{3-[ethyl(2-hydroxyethyl)amino]propen-1-yl}-1,8-dihydro-imidazo[4,5-h]-isochinolin-9-on;
7-(3-Diethylaminopropen-1-yl)-1,6-dimethyl-2-(2,6-dimethylphenylamino)-1,8-dihydro-imidazo[4,5-h]-isochinolin-9-on;
2-(2,6-Dichlorophenylamino)-7-{3-[(2-diethylaminoethyl)methylamino]-propen-1-yl}-1,6-dimethyl-1,8-dihydro-imidazo[4,5-h]-isochinolin-9-on;
7-(3-Diethylaminopropen-1-yl)-1,6-dimethyl-2-(2,4,6-trichlorophenylamino)-1,8-dihydro-imidazo[4,5-h]-isochinolin-9-on;
2-(2,6-Dichlorophenylamino)-6-methyl-7-oxazol-5-yl-1,8-dihydro-imidazo[4,5-h]isochinolin-9-on;
2-(2,6-Dichlorophenylamino)-1,6-dimethyl-7-[3-(2-pyrrolidin-1-ylmethylpyrrolidin-1-yl)-propen-1-yl]-1,8-dihydro-imidazo[4,5-*h*]-isochinolin-9-on;
7-[3-(2*S*-Aminomethylpyrrolidin-1-yl)-propen-1-yl]-2-(2,6-dichlorophenylamino)-1,6-dimethyl-1,8-dihydro-imidazo[4,5-*h*]-isochinolin-9-on;
1-{3-[2-(2,6-Dichlorophenylamino)-1,6-dimethyl-9-oxo-8,9-dihydro-1H-imidazo[4,5-h]isochinolin-7-yl]-propenyl}-*L*-prolin-carboxamid;
1- {3-[2-(2,6-dichlorophenylamino)-1,6-dimethyl-9-oxo-8,9-dihydro-1H-imidazo[4,5-h]isochinolin-7-yl]-propenyl}-piperidin-3-carboxamid;
2-(2,6-Dichlorophenylamino)-1,6-dimethyl-7-(methylhydrazonomethyl)-1,8-dihydro-imidazo[4,5-h]-isochinolin-9-on;
7-[3-(3-Aminopyrrolidin-1-yl)-propen-1-yl]-2-(2,6-dichlorophenylamino)-1,6-dimethyl-1,8-dihydro-imidazo[4,5-*h*]-isochinolin-9-on;
2-(2,6-Dichlorophenylamino)-1,6-dimethyl-7-[3-(3-acetamidopyrrolidin-1-yl)-propen-1-yl]-1,8-dihydro-imidazo[4,5-*h*]-isochinolin-9-on;
2-(2,6-Dichlorophenylamino)-1,6-dimethyl-7-[3-(3-dimethylaminopyrrolidin-1-yl)-propen-1-yl]-1,8-dihydro-imidazo[4,5-*h*]-isochinolin-9-on;
1-{3-[2-(2,6-Dichlorophenylamino)-1,6-dimethyl-9-oxo-8,9-dihydro-1H-imidazo[4,5-h]isochinolin-7-yl]-propenyl}-piperidin-2-carboxamid;
7-[3-(3-Aminomethylpiperidin-1-yl)-propen-1-yl]-2-(2,6-dichlorophenylamino)-1,6-dimethyl-1,8-dihydro-imidazo[4,5-*h*]-isochinolin-9-on;
1-{3-[2-(2,6-Dichlorophenylamino)-1,6-dimethyl-9-oxo-8,9-dihydro-1H-imidazo[4,5-h]isochinolin-7-yl]-propenyl}-piperidin-3-carbonsäure-diethylamid;
2-(2,6-Dichlorophenylamino)-1,6-dimethyl-7-ethinyl-1,8-dihydro-imidazo[4,5-h]-isochinolin-9-on;
1-{3-[2-(2,6-dichlorophenylamino)-1,6-dimethyl-9-oxo-8,9-dihydro-1H-imidazo[4,5-h]isochinolin-7-yl]-propenyl}-3-methyl-harnstoff;
Cyclohexan-carbonsäure-{3-[2-(2,6-dichlorophenylamino)-1,6-dimethyl-9-oxo-8,9-dihydro-1H-imidazo[4,5-h]isochinolin-7-yl]-propenyl}amid;
2-(2,6-Dichlorophenylamino)-1-methyl-7-phenyl-1,8-dihydro-imidazo[4,5-h]isochinolin-9-on;
*N*-{3-[2-(2,6-Dichlorophenylamino)-1,6-dimethyl-9-oxo-8,9-dihydro-1*H*-imidazo[4,5-h]isochinolin-7-yl]-propenyl}-methansulfonamid;
3-[2-(2,6-dichlorophenylamino)-1,6-dimethyl-9-oxo-8,9-dihydro-1H-imidazo[4,5-h]isochinolin-7-yl]-propenyl-harnstoff;
1-Cyclohexyl-3- {3-[2-(2,6-dichlorophenylamino)-1,6-dimethyl-9-oxo-8,9-dihydro-1Himidazo[4,5-h]isochinolin-7-yl]-propenyl}-harnstoff;
*N*-{3-[2-(2,6-Dichlorophenylamino)-1,6-dimethyl-9-oxo-8,9-dihydro-1*H*-imidazo[4,5-h]isochinolin-7-yl]-propenyl}-benzolsulfonamid;
2-(2,6-Dichlorophenylamino)-1,6-dimethyl-7-(3-ethylaminopropen-1-yl)-1,8-dihydroimidazo[4,5-h]-isochinolin-9-on;
*N*-{3-[2-(2,6-Dichlorophenylamino)-1,6-dimethyl-9-oxo-8,9-dihydro-1*H*-imidazo[4,5-h]isochinolin-7-yl]-propenyl}-guanidin;
Piperidin-3-carbonsäure-{3-[2-(2,6-dichlorophenylamino)-1,6-dimethyl-9-oxo-8,9-dihydro-1H-imidazo[4,5-h]isochinolin-7-yl]-propenyl}amid;
*L*-Prolin-{3-[2-(2,6-dichlorophenylamino)-1,6-dimethyl-9-oxo-8,9-dihydro-1Himidazo[4,5-h]isochinolin-7-yl]-propenyl}amid;
D-Prolin- {3-[2-(2,6-dichlorophenylamino)-1,6-dimethyl-9-oxo-8,9-dihydro-1Himidazo[4,5-h]isochinolin-7-yl]-propenyl}amid;
3-[2-(2,6-Dichlorophenylamino)-1,6-dimethyl-9-oxo-8,9-dihydro-1H-imidazo[4,5-h]isochinolin-7-yl]-benzamid;
*L*-Azetidin-2-carbonsäure-{3-[2-(2,6-dichlorophenylamino)-1,6-dimethyl-9-oxo-8,9-dihydro-1H-imidazo[4,5-h]isochinolin-7-yl]-propenyl}amid;
Piperidin-2-carbonsäure-{3-[2-(2,6-dichlorophenylamino)-1,6-dimethyl-9-oxo-8,9-dihydro-1H-imidazo[4,5-h]isochinolin-7-yl]-propenyl}amid; und
die pharmazeutisch annehmbaren Derivate davon.

20. Verbindung nach Anspruch 19, ausgewählt aus der Gruppe bestehend aus:
2-(2,6-Dichlorophenylamino)-3,5-dihydro-imidazo[4,5-i]phenanthridin-4-on;
2-(2,6-Dichlorophenylamino)-1,6,7-trimethyl-1,8-dihydro-imidazo[4,5-h]isochinolin-9-on;
2-(2,6-Dichlorophenylamino)-1,7-dimethyl-6-(2-hydroxyethyl)-1,8-dihydro-imidazo[4,5-h]isochinolin-9-on;
2-(2,6-Dichlorophenylamino)-1,7-dimethyl-9-oxo-1,8-dihydro-imidazo[4,5-h]isochinolin-6-carbonsäure-methylester;
3-[2-(2,6-Dichlorophenylamino)-1-methyl-9-oxo-8,9-dihydro-1H-imidazo[4,5-h]isochinolin-7-yl]-acrylsäure-methylester;
2-(2-Chloro-6-methylphenylamino)-1,6,7-trimethyl-1,8-dihydro-imidazo[4,5-h]isochinolin-9-on;
3-[2-(2,6-Dichlorophenylamino)-1-methyl-9-oxo-8,9-dihydro-1H-imidazo[4,5-h]isochinolin-7-yl]-N-methoxy-N-methylacrylamid;
2-(2-Chloro-6-nitrophenylamino)-1,6,7-trimethyl-1,8-dihydro-imidazo[4,5-h]isochinolin-9-on;
N-Benzyl-3-[2-(2,6-Dichlorophenylamino)-1-methyl-9-oxo-8,9-dihydro-1H-imidazo[4,5-h]isochinolin-7-yl]-acrylamid;
3-[2-(2,6-Dichlorophenylamino)-1-methyl-9-oxo-8,9-dihydro-1H-imidazo[4,5-h]isochinolin-7-yl]-acrylsäure-4-morpholinamid;
2-(2,6-Dichlorophenylamino)-4-hydroxymethyl-1,6,7-trimethyl-1,8-dihydro-imidazo[4,5-h]isochinolin-9-on;
2-(2,6-Dichlorophenylamino)-1,6-dimethyl-7-vinyl-1,8-dihydro-imidazo[4,5-h]isochinolin-9-on;
2-(2,6-Dichlorophenylamino)-1,7-dimethyl-9-oxo-1,8-dihydro-imidazo[4,5-h]isochinolin-6-carbonsäure-2-(4-moropholino)ethylester;
2-(2,6-Dichlorophenylamino)-1,6-dimethyl-7-(3-hydroxypropen-1-yl)-1,8-dihydro-imidazo[4,5-h]-isochinolin-9-on;
2-(2,6-Dichlorophenylamino)-1,6-dimethyl-7-oxazol-5-yl-1,8-dihydro-imidazo[4,5-h]isochinolin-9-on;
2-(2,6-Dichlorophenylamino)-1-methyl-7-vinyl-1,8-dihydro-imidazo[4,5-h]isochinolin-9-on;
2-(2,6-Dichlorophenylamino)-1,6-dimethyl-7-(3-morpholin-4-yl-propen-1-yl)-1,8-dihydro-imidazo[4,5-h]-isochinolin-9-on;
3-[2-(2,6-Dichlorophenylamino)-1,7-dimethyl-9-oxo-8,9-dihydro-1H-imidazo[4,5-h]isochinolin-7-yl]-acrylonitril;
2-(2-Chloro-6-methylphenylamino)-1,7-dimethyl-1,8-dihydro-imidazo[4,5-h]isochinolin-9-on;
2-(2,6-Dichlorophenylamino)-1-methyl-7-oxazol-5-yl-1,8-dihydro-imidazo[4,5-h]isochinolin-9-on;
2-(2,6-Dichlorophenylamino)-7-(3-hydroxypropen-1-yl)-1-methyl-1,8-dihydro-imidazo[4,5-h]-isochinolin-9-on;
2-(2-Chloro-6-methylphenylamino)-7-(3-hydroxypropen-1-yl)-1-methyl-1,8-dihydro-imidazo[4,5-h]-isochinolin-9-on;
2-(2,6-Dichlorophenylamino)-7-(3-diethylaminopropen-1-yl)-1,6-dimethyl-1,8-dihydro-imidazo[4,5-h]-isochinolin-9-on;
2-(2,6-Dichlorophenylamino)-1,6-dimethyl-7-(3-pyrrolidin-1-yl-propen-1-yl)-1,8-dihydro-imidazo[4,5-h]-isochinolin-9-on;
2-(2,6-Dichlorophenylamino)-7-(3-diethylaminopropen-1-yl}-1-methyl-1,8-dihydro-imidazo[4,5-h]-isochinolin-9-on;
2-(2,6-Dichlorophenylamino)-1,6-dimethyl-7-(4-methylpiperazin-1-yl-propen-1-yl)-1,8-dihydro-imidazo[4,5-h]-isochinolin-9-on;
2-(2,6-Dichlorophenylamino)-1,6-dimethyl-7-(3-piperidin-1-yl-propen-1-yl)-1,8-dihydro-imidazo[4,5-h]-isochinolin-9-on;
2-(2,6-Dichlorophenylamino)-1,6-dimethyl-7-{3-[ethyl(2-hydroxyethyl)amino]propen-1-yl}-1,8-dihydro-imidazo[4,5-h]-isochinolin-9-on;
7-(3-Diethylaminopropen-1-yl)-1,6-dimethyl-2-(2,6-dimethylphenylamino)-1,8-dihydro-imidazo[4,5-h]-isochinolin-9-on;
2-(2,6-Dichlorophenylamino)-7- {3-[(2-diethylaminoethyl)methylamino]-propen-1-yl}-1,6-dimethyl-1,8-dihydro-imidazo[4,5-h]-isochinolin-9-on; und
die pharmazeutisch annehmbaren Derivate davon.

21. Pharmazeutische Zusammensetzung enthaltend eine therapeutisch wirksame Menge einer Verbindung nach den Ansprüchen 1, 9, 15 oder 19.

22. Verwendung einer Verbindung nach den Ansprüchen 1, 9, 15 oder 19 zur Herstellung eines Medikamentes für die Behandlung einer Autoimmunerkrankung oder Krebserkrankung.

23. Verwendung nach Anspruch 22, wobei die Autoimmunerkrankung ausgewählt ist aus rheumatoider Arthritis, multipler Sklerose, Guillain-Barre-Syndrom, Morbus Crohn, Colitis ulzerosa, Psoriasis, eine Graft-versus-host-Reaktion, systemischer Lupus erythematosus, insulin-abhängiger Diabetes mellitus und Asthma.

24. Verwendung nach Anspruch 22, wobei die Krebserkrankung aus einem src-abhängigen Tumor oder einem PDGF-abhängigen Tumor ausgewählt ist.

25. Verwendung nach Anspruch 24, wobei der src-abhängige Tumor aus Mammakarzinom, Kolonkarzinom, Melanom und Sarkom ausgewählt ist.

26. Verwendung nach Anspruch 24, wobei der PDGF-abhängige Tumor aus Ovarialkarzinom, Prostatakarzinom und Glioblastom ausgewählt ist.

27. Verwendung einer Verbindung nach Anspruch 1, 9, 15, oder 19 zur Herstellung eines Medikamentes für die Behandlung einer aus Osteoporose, Morbus Paget, Knochenentzündung, und Gelenkentzündung ausgewählten Erkrankung.

28. Verwendung einer Verbindung nach Anspruch 1, 9, 15, oder 19 zur Herstellung eines Medikamentes für die Behandlung einer aus fibrotischen Erkrankungen, Restenose und Atherosklerose ausgewählten Erkrankung.

29. Verwendung einer Verbindung nach Anspruch 1, 9, 15, oder 19 zur Herstellung eines Medikamentes für die Verstärkung oder Potenzierung der Wirksamkeit einer Strahlentherapie.

30. Verfahren zur Herstellung einer Verbindung der Formel (I) 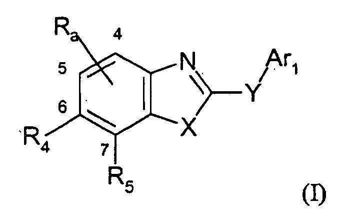 worin X ein N-R₁₅ ist und Ar₁, R₄, R₅, R₁₅ und Rₐ wie in Anspruch 1 definiert sind, wobei das besagte Verfahren umfasst:
a) Umsetzung einer Verbindung der Formel (II) mit Ar₁NCS in einem geeigneten Lösungsmittel bei ungefähr Raumtemperatur bis Refluxtemperatur während ungefähr 3 bis 24 h unter Bildung einer Verbindung der Formel (III); 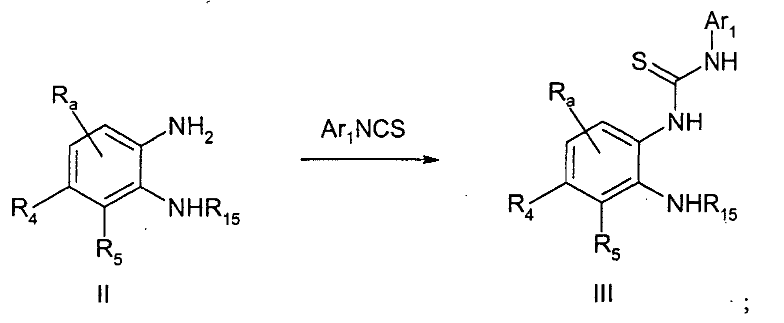
b) Umsetzung des Produktes (II) der Stufe a) mit einem aus 1,3-Dicyclohexylcarbodiimid (DCC) und Quecksilberoxid ausgewählten geeigneten Aktivierungsmittel in einem geeigneten Lösungsmittel bei ungefähr Raumtemperatur bis Refluxtemperatur unter Bildung einer Verbindung der Formel (I) wie oben angegeben oder deren Vorläufer.

31. Verfahren zur Herstellung einer Verbindung der Formel (I) 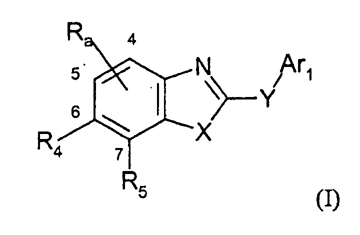 worin X ein S ist, Y ein NH ist und Ar₁, R₄, R₅ und Rₐ wie in Anspruch 1 definiert sind,
wobei das besagte Verfahren umfasst:
a) Umsetzung einer Verbindung der Formel (IV) mit Ar₁NCS in einem geeigneten Lösungsmittel bei ungefähr Raumtemperatur bis Refluxtemperatur während ungefähr 3 bis 24 h unter Bildung einer Verbindung der Formel (V); 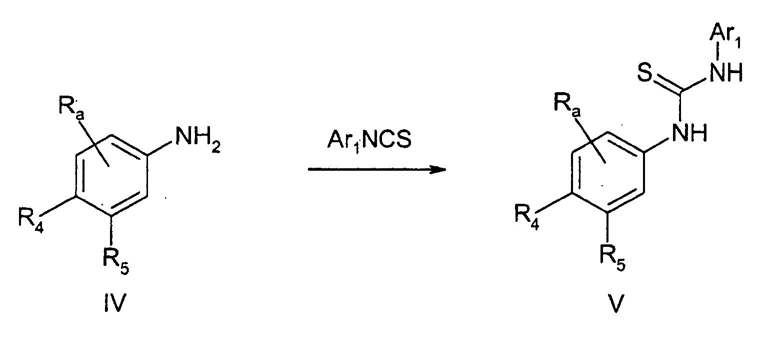
b) Umsetzung des Produktes (V) der Stufe a) unter Zyklisierungs-Bedingungen in einem geeigneten Lösungsmittel bei ungefähr Refluxtemperatur unter Bildung einer Verbindung der Formel (I) oder eines Vorläufers derselben.

32. Verfahren zur Herstellung einer Verbindung der Formel (XI), worin R₁₅, R₈ und R₉ wie in Anspruch 1 definiert sind: 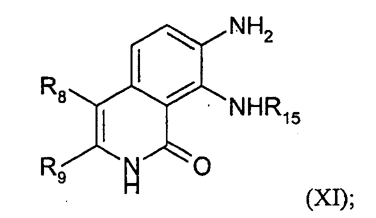 wobei besagtes Verfahren umfasst:
a) Umsetzung einer Verbindung der Formel (VI) mit NHR₁₅ in einem geeigneten Lösungsmittel, gegebenenfalls in einem Druckkolben und bei ungefähr 0 bis 80°C unter Bildung von VII, und anschliessendes Umsetzung der Verbindung VII mit dem Ketoester VIII in Gegenwart einer geeigneten Base in einem geeigneten Lösungsmittel bei ungefähr Raumtemperatur unter Bildung einer Verbindung der Formel (IX): 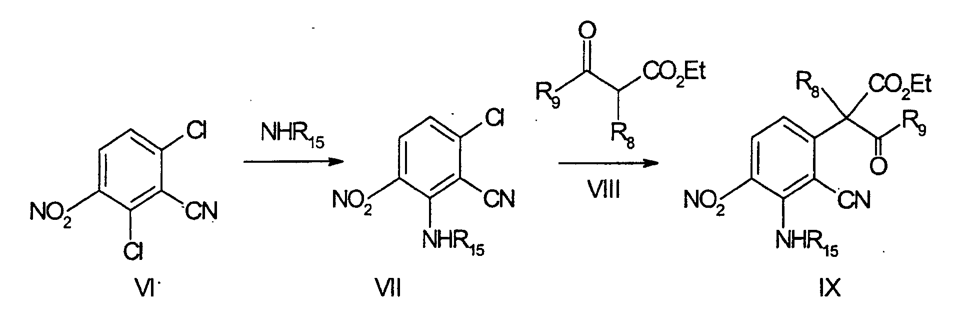
b) Hydrolysieren des Produktes der Stufe a) durch Umsetzung mit wässriger Säure, und Zyklisieren bei ungefähr Refluxtemperatur; gefolgt von anschliessender Reduktion des zyklischen Produktes in einem geeigneten Lösungsmittel unter Bildung einer Verbindung der Formel (XI).

33. Verfahren zur Herstellung einer Verbindung der Formel: 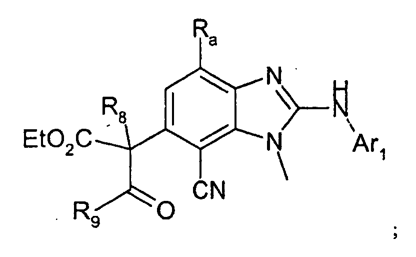 worin Rₐ, R₈, R₉ und Ar₁ wie in Anspruch 1 definiert sind;
wobei besagtes Verfahren umfasst:
a) Umsetzung einer Verbindung der Formel (XII) mit Brom in einem geeigneten Lösungsmittel bei Raumtemperatur unter Bildung von XIII. 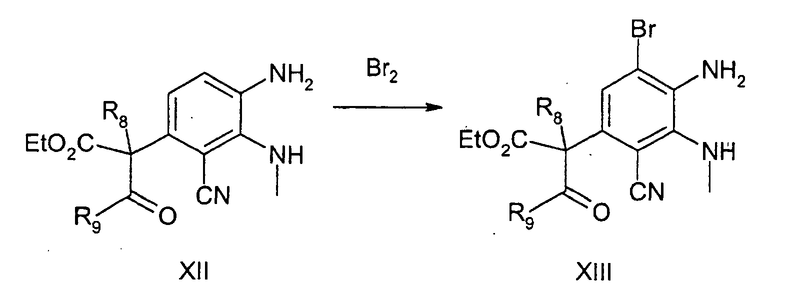
b) Umsetzung einer Verbindung der Formel (XIII) mit Ar₁NCS in einem geeigneten Lösungsmittel bei ungefähr Raumtemperatur bis Refluxtemperatur während ungefähr 3 bis 24 h und anschliessendes Umsetzung des Produktes mit einem geeigneten aus 1,3-Dicyclohexylcarbodiimid (DCC) und Quecksilberoxid ausgewählen Aktivierungsmittel in einem geeigneten Lösungsmittel bei ungefähr Raumtemperatur bis Refluxtemperatur unter Bildung einer Verbindung der Formel (XIV);
c) Durchführen einer Kreuzkupplungsreaktion zur Einführung von Rₐ anstelle von Brom in Gegenwart eines geeigneten Katalysators in einem geeigneten Lösungsmittel bei ungefähr 100°C, unter Bildung der unten angegebenen Produkt-Verbindung:

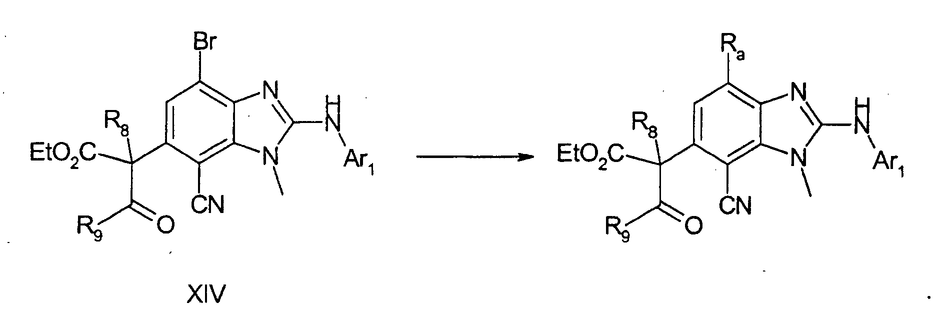

## Revendications

1. Composé de formule (I): 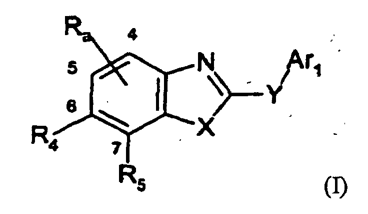 dans laquelle:
Ar₁ est un carbocycle aromatique ou non aromatique, un hétéroaryle ou un hétérocycle; ledit carbocycle, hétéroaryle ou hétérocycle étant éventuellement substitué par un ou plusieurs R₁, R₂ et R₃;
X est NH, N-alkyle en C₁-C₃, N-cyclopropyle, S ou O;
Y est NR₁₅, S ou O;
Rₐ est H, alkyle en C₁-C₁₀, alcényle en C₂-C₁₀ ou alcynyle en C₂-C₁₀ pouvant chacun être ramifié ou cyclique; ou Rₐ est aryle ou hétéroaryle; chaque Rₐ étant indépendamment éventuellement substitué par un ou plusieurs groupes alkyle en C₁-C₆, alcoxy en C₁-C₆, halogéno, OH, oxo, NR₁₀R₁₁, aryle ou hétéroaryle, chaque aryle ou hétéroaryle étant éventuellement substitué par un ou plusieurs groupes choisis parmi halogéno, OH, alkyle en C₁-C₃, alcoxy en C₁-C₃, hydroxyalkyle en C₁-C₃ et (CH₂)ₘNR₁₀R₁₁; et Rₐ étant lié en position 4 ou 5;
R₁ et R₂ sont identiques ou différents et choisis parmi H, halogéno, CN, NO₂, alkyle en C₁-C₁₀ linéaire ou ramifié, saturé ou insaturé, alcoxy en C₁-C₁₀ linéaire ou ramifié, acyle en C₁-C₁₀ linéaire ou ramifié, acyloxy en C₁-C₁₀ linéaire ou ramifié, alkylthio en C₁-C₁₀ linéaire ou ramifié, aminosulfonyle, di(alkyl en C₁-C₃)aminosulfonyle, NR₁₀R₁₁, aryle, aroyle, aryloxy, arylsulfonyle, hétéroaryle et hétéroaryloxy; les R₁ et R₂ précités étant éventuellement partiellement ou entièrement halogénés ou éventuellement substitués par 1 à 3 groupes choisis indépendamment parmi oxo, OH, NR₁₀R₁₁, alkyle en C₁-C₆ linéaire ou ramifié, cycloalkyle en C₃-C₇, phényle, naphtyle, hétéroaryle, aminocarbonyle et mono- ou di(alkyl en C₁-C₃)aminocarbonyle;
R₃ est H, halogéno, OH, (CH₂)ₙNR₁₀R₁₁, CONR₁₀R₁₁, (CH₂)ₙCO₂R₁₂; alkyle en C₁-C₃ éventuellement substitué par OH, alcoxy en C₁-C₃ éventuellement halogéné ou alkylthio en C₁-C₃;
R₄ et R₅, avec les atomes auxquels ils sont liés, complètent un système cyclique condensé des formules A ou B: 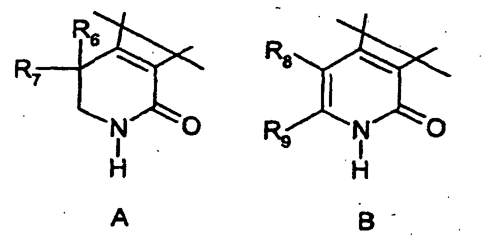
R₆ est alkyle en C₁-C₃ ou H;
R₇ est alkyle en C₁-C₆ linéaire ou ramifié ou H;
R₈ est H, alkyle en C₁-C₆ linéaire ou ramifié, saturé ou insaturé, éventuellement substitué par phényle, OH ou alcoxy en C₁-C₃; ou R₈ est (CH₂)ₘNR₁₀R₁₁, (CH₂)ₘNR₁₀COR₁₂, (CH₂)ₙCO₂R₁₂, (CH₂)ₙCONR₁₀R₁₁; ou R₈ est phényle ou hétéroaryle, chacun étant éventuellement substitué par alkyle en C₁-C₃, alcoxy en C₁-C₃, OH, -SO₃H ou halogéno;
R₉ est H, CN ou CONR₁₀R₁₁; ou R₉ est alkyle en C₁-C₁₀ linéaire ou ramifié, cycloalkyle en C₃-C₁₀, cycloalcényle en C₅-C₇, alcényle en C₂-C₆, alcynyle en C₂-C₆, chacun étant éventuellement substitué par un ou plusieurs groupes cycloalkyle en C₃-C₁₀, cycloalkylidène en C₃-C₁₀, cycloalcényle en C₅-C₇, halogéno, OH, oxo, CN, alcoxy en C₁-C₃, acyloxy en C₁-C₃, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryle ou hétéroaryle; chaque aryloxy, arylthio, aryle ou hétéroaryle étant éventuellement substitué par alkyle en C₁-C₃, alcoxy en C₁-C₃, halogéno, (CH₂)ₙNR₁₀R₁₁ ou O(CH₂)₂₋₄NR₁₀R₁₁;
ou R₉ est aryle, hétéroaryle ou hétérocycle, chacun des aryle, hétéroaryle ou hétérocycle étant éventuellement substitué par 1 à 3 groupes choisis parmi alkyle en C₁-C₃ éventuellement substitué par phényle ou NR₁₀C(=NR₁₀)NR₁₀R₁₁, alcoxy en C₁-C₃, halogéno, CN, oxo, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 5 ou 6 chaînons saturé ou insaturé, aromatique ou non aromatique, éventuellement substitué par 1 ou 2 groupes alkyle en C₁-C₃, OH, oxo ou (CH₂)ₙNR₁₀R₁₁, ou éventuellement condensé en spiro à un groupe 1,3-dioxolane ou groupe 1,3-dithiolane, chaque groupe 1,3-dioxolane ou groupe 1,3-dithiolane étant éventuellement substitué par alkyle en C₁-C₆, alcoxy en C₁-C₆, OH ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, aryle, aryl-(alkyle en C₁-C₃) et hétéroaryle; lesdits alkyle, cycloalkyle, aryle, aryl-(alkyle en C₁-C₃) ou hétéroaryle étant éventuellement substitués par OH, alcoxy en C₁-C₃, CN, NO₂, acyloxy en C₁-C₃, CO₂R₁₂, NR₁₃R₁₄, O(CH₂)₂₋₄NR₁₃R₁₄, aryle ou hétéroaryle;
ou R₁₀ et R₁₁ forment ensemble une chaîne alkylène de 3-7 chaînons complétant un cycle avec l'atome d'azote auquel ils sont liés; ladite chaîne alkylène étant éventuellement interrompue par O, S(O)ₚ et NR₁₃; et ledit cycle étant éventuellement substitué par alkyle en C₁-C₃, alcoxy en C₁-C₃, OH, -(CH₂)ₙNR₁₃R₁₄, CONR₁₃R₁₄ ou NR₁₃COR₁₄;
R₁₂ est H, alkyle en C₁-C₆ ou cycloalkyle en C₃-C₈, chaque alkyle ou cycloalkyle étant éventuellement substitué par phényle, OH, alcoxy en C₁-C₃ ou NR₁₃R₁₄; ou R₁₂ est un phényle ou hétérocycle, éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂;
R₁₅ est H ou alkyle en C₁-C₃;
m est 1-4; n est 0-3 et p est 0-2;
où le terme hétéroaryle désigne un radical hétérocyclique aromatique monocyclique de 5-8 chaînons ou bicyclique de 8-11 chaînons stable consistant en atomes de carbone et de 1 à 4 hétéroatomes choisis parmi l'azote, l'oxygène et le soufre;
le terme hétérocycle désigne un radical hétérocyclique monocyclique de 4-8 chaînons ou bicyclique de 8-11 chaînons stable pouvant être saturé ou insaturé, et qui est non aromatique, consistant en atomes de carbone et de 1 à 4 hétéroatomes choisis parmi l'azote, l'oxygène et le soufre;
le terme aryle désigne un carbocycle aromatique de 6-10 chaînons;
le terme carbocycle désigne une chaîne carbonée cyclique aromatique ou non aromatique de 3-10 chaînons; et
leurs dérivés acide ou sel acceptables d'un point de vue pharmaceutique.

2. Composé selon la revendication 1, dans lequel
Ar₁ est
a) un groupe cycloalkyle choisi parmi cyclopropyle, cyclobutyle, cyclopentyle, cyclohexyle, cycloheptyle;
b) un groupe cycloalcényle choisi parmi cyclopentényle, cyclohexényle, cycloheptényle;
c) phényle, naphtyle, indanyle, indényle, dihydronaphtyle, tétrahydronaphtyle, fluorényle;
d) hétéroaryle choisi parmi pyridinyle, pyrimidinyle, pyrazinyle, pyridazinyle, pyrrolyle, imidazolyle, pyrazolyle, thiényle, furyle, isoxazolyle, isothiazolyle, oxazolyle, oxadiazolyle, thiazolyle, thiadiazolyle, quinoléinyle, isoquinoléinyle, indolyle, benzimidazolyle, benzofuranyle, benzoxazolyle, benzisoxazolyle, benzopyrazolyle, benzothiofuranyle, benzothiazolyle, quinazolinyle, et indazolyle, ou hétéroaryle condensé choisi parmi cyclopenténopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoléine, cyclohexanoquinoléine, cyclopentanoisoquinoléine, cyclohexanoisoquinoléine, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophène et cyclohexanothiophène; ou
e) un hétérocycle choisi parmi pyrrolinyle, pyrrolidinyle, pyrazolinyle, pyrazolidinyle, pipéridinyle, morpholinyle, thiomorpholinyle, pyranyle, thiopyranyle, pipérazinyle et indolinyle; chacun des Ar₁ ci-dessus étant éventuellement substitué par un ou plusieurs R₁, R₂ et R₃;
Rₐ est H, alkyle en C₁-C₆, alcényle en C₂-C₅, alcynyle en C₂-C₅, phényle ou hétéroaryle choisi parmi pyridinyle, pyridazinyle, pyrimidinyle, pyrazinyle, oxazolyle, pyrazolyle, imidazolyle, furyle, thiazolyle et thiényle; chaque Rₐ étant éventuellement substitué par un ou plusieurs groupes phényle, halogéno, alkyle en C₁-C₃, alcoxy en C₁-C₃, OH, oxo ou NR₁₀R₁₁; Rₐ étant en position 4;
R₁ et R₂ ont la définition donnée ci-dessus;
R₃ est H, halogène, méthyle, méthoxy, hydroxyméthyle ou OH;
R₈ est H, alkyle en C₁-C₃ linéaire ou ramifié, saturé ou insaturé, éventuellement substitué par OH; ou R₈ est (CH₂)₂₋₃NR₁₀R₁₁, (CH₂)ₙCO₂R₁₂ ou (CH₂)ₙCONR₁₀R₁₁;
R₉ est CN ou CONR₁₀R₁₁; ou R₉ est alkyle en C₁-C₃ linéaire ou ramifié, alcényle en C₂-C₄, alcynyle en C₂-C₄, chacun étant éventuellement substitué par un ou plusieurs groupes cycloalkyle en C₅-C₇, cycloalkylidène en C₅-C₇, cycloalcényle en C₅-C₇, OH, CN, acyloxy en C₁-C₃, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, CONR₁₀R₁₁, CO₂R₁₂, C(R₁₀)=NNR₁₀R₁₁, C(R₁₀)=NNR₁₀CONR₁₀R₁₁, aryle ou hétéroaryle; chaque aryle ou hétéroaryle étant éventuellement substitué par alkyle en C₁-C₃, alcoxy en C₁-C₃, halogéno, (CH₂)ₙNR₁₀R₁₁ ou O(CH₂)₂₋₄NR₁₀R₁₁;
ou R₉ est aryle, hétéroaryle ou hétérocycle, chacun étant éventuellement substitué par 1 à 3 groupes choisis parmi alkyle en C₁-C₃ éventuellement substitué par phényle ou NR₁₀C(=NR₁₀)NR₁₀R₁₁, alcoxy en C₁-C₃, halogéno, CN, oxo, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 5 ou 6 chaînons saturé ou insaturé, aromatique ou non aromatique, éventuellement substitué par alkyle en C₁-C₃ ou OH, ou éventuellement condensé en spiro à un groupe 1,3-dioxolane ou groupe 1,3-dithiolane, chaque groupe 1,3-dioxolane ou groupe 1,3-dithiolane étant éventuellement substitué par alkyle en C₁-C₃, alcoxy en C₁-C₃, OH ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, benzyle et phényle; ledit alkyle, cycloalkyle, benzyle ou phényle étant éventuellement substitué par OH, alcoxy en C₁-C₃, acyloxy en C₁-C₃, CN, NO₂, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃, OH, -(CH₂)ₙNR₁₃R₁₄, CONR₁₃R₁₄ ou NR₁₃COR₁₄;
R₁₂ est H, alkyle en C₁-C₆ ou cycloalkyle en C₅-C₇, chacun étant éventuellement substitué par phényle, OH, alcoxy en C₁-C₃ ou NR₁₃R₁₄; ou R₁₂ est phényle ou hétérocycle, éventuellement substitué chacun par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂; et
R₁₅ est H;
où le terme hétéroaryle désigne un radical hétérocyclique aromatique monocyclique de 5-8 chaînons ou bicyclique de 8-11 chaînons stable consistant en atomes de carbone et de 1 à 4 hétéroatomes choisis parmi l'azote, l'oxygène et le soufre;
le terme hétérocycle désigne un radical hétérocyclique monocyclique de 4-8 chaînons ou bicyclique de 8-11 chaînons stable pouvant être saturé ou insaturé, et qui est non aromatique, consistant en atomes de carbone et de 1 à 4 hétéroatomes choisis parmi l'azote, l'oxygène et le soufre;
le terme aryle désigne un carbocycle aromatique de 6-10 chaînons;
le terme carbocycle désigne une chaîne carbonée cyclique aromatique ou non aromatique de 3-10 chaînons.

3. Composé selon la revendication 2, dans lequel:
Ar₁ est phényle ou pyridyle, chacun étant éventuellement substitué par un ou plusieurs R₁, R₂ et R₃ tels que définis ci-dessous;
X est NH ou N-CH₃;
Y est NH et
Rₐ est H, hydroxyalkyle en C₁-C₂, 2-hydroxyéthylaminométhyle, méthoxybenzylaminométhyle, pyridinyle éventuellement halogéné, phényle, 3-hydroxy-2-oxopropyle, vinyle ou alcynyle en C₃-C₅ substitué par alcoxy en C₁-C₃ ou phényle;
R₁ et R₂ sont identiques ou différents et sont choisis parmi H, halogéno, alkyle en C₁-C₃, le groupe alkyle en C₁-C₃ étant éventuellement partiellement ou entièrement halogéné, NO₂, NR₁₃R₁₄;
R₃ est H, halogéno, méthoxy ou méthyle;
R₄ et R₅ complètent ensemble un cycle condensé de formule B;
R₈ est H, alkyle en C₁-C₃ éventuellement substitué par OH; ou R₈ est (CH₂)₂₋₃NR₁₀R₁₁ ou CO₂R₁₂;
R₉ est CN; ou R₉ est méthyle, alcényle en C₂-C₃ ou alcynyle en C₂-C₃, chacun étant éventuellement substitué par un où plusieurs groupes cycloalkylidène en C₅-C₇, cycloalcényle en C₅-C₇, OH, CN, NR₁₀R₁₁, NR₁₀CONR₁₀R₁₁, NR₁₀COR₁₂, NR₁₀S(O)ₚR₁₂, CONR₁₀R₁₁, CO₂R₁₂, C(R₁₀)=NNR₁₀R₁₁ ou hétéroaryle;
ou R₉ est aryle ou hétéroaryle éventuellement substitué par 1 à 3 groupes choisis parmi alkyle en C₁-C₃ éventuellement substitué par phényle, alcoxy en C₁-C₃, halogéno, amino ou CONH₂;
ou R₈ et R₉ forment ensemble un cycle cyclopentène condensé en spiro à un groupe 1,3-dioxolane, ledit groupe 1,3-dioxolane étant éventuellement substitué par alkyle en C₁-C₃, alcoxy en C₁-C₃, OH ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₃ linéaire ou ramifié, cycloalkyle en C₅-C₇ ou phényle; ledit alkyle, cycloalkyle ou phényle étant éventuellement substitué par OH, alcoxy en C₁-C₃, acyloxy en C₁-C₃, NO₂, CO₂R₁₂, NR₁₃R₁₄, O(CH₂)₂₋₄NR₁₃R₁₄ ou phényle;
ou R₁₀ et R₁₁ forment ensemble un groupe morpholino, pyrrolidinyle, pipérazinyle ou pipéridinyle, chacun étant éventuellement substitué par alkyle en C₁-C₃, alcoxy en C₁-C₃, OH, -(CH₂)ₙNR₁₃R₁₄, CONR₁₃R₁₄ ou NR₁₃COR₁₄;
R₁₂ est H, alkyle en C₁-C₃ ou cycloalkyle en C₅-C₇, chacun étant éventuellement substitué par phényle, OH, alcoxy en C₁-C₃ ou NR₁₃R₁₄; ou R₁₂ est phényle ou un hétérocycle azoté saturé de 4 à 6 chaînons, éventuellement substitués chacun par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₃ éventuellement substitué par alcoxy en C₁-C₃ ou OH;
ou R₁₃ et R₁₄ forment ensemble une chaîne complétant un cycle, ladite chaîne étant (CH₂)₄₋₅ ou (CH₂)₂O(CH₂)₂.

4. Composé selon la revendication 3, dans lequel
Ar₁ est phényle;
Rₐ est H ou hydroxyméthyle;
R₁ et R₂ sont identiques ou différents et sont choisis parmi halogéno, méthyle éventuellement partiellement ou entièrement halogéné, NO₂ ou NH₂;
R₃ est H, chloro, fluoro, bromo ou méthoxy;
R₁₀ et R₁₁ peuvent être identiques ou différents et sont choisis chacun indépendamment parmi H, OH, méthoxy, alkyle en C₁-C₃ linéaire ou ramifié ou cycloalkyle en C₅-C₇; ledit alkyle ou cycloalkyle étant éventuellement substitué par OH, NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₂, NR₁₃R₁₄, CONR₁₃R₁₄ ou NR₁₃COR₁₄; et
R₁₂ est alkyle en C₁-C₃ éventuellement substitué par morpholino; ou R₁₂ est phényle ou est azétidinyle, pyrrolidinyle ou pipéridinyle, éventuellement substitués chacun par 1 à 3 groupes choisis parmi alkyle en C₁-C₃, alcoxy en C₁-C₃ et halogéno.

5. Composé selon la revendication 1, dans lequel:
Ar₁ est un carbocycle aromatique ou non aromatique, un hétéroaryle ou un hétérocycle; ledit carbocycle, hétéroaryle ou hétérocycle étant éventuellement substitué par un ou plusieurs R₁, R₂ et R₃;
X est NH, N-alkyle en C₁-C₃, N-cyclopropyle, S ou O;
Y est NR₁₅, S ou O;
Rₐ est H, alkyle en C₁-C₁₀, alcényle en C₂-C₁₀ ou alcynyle en C₂-C₁₀, chacun pouvant être ramifié ou cyclique; ou Rₐ est aryle ou hétéroaryle; chaque Rₐ étant indépendamment éventuellement substitué par un ou plusieurs groupes alkyle en C₁-C₃, alcoxy en C₁-C₆, halogéno, OH, oxo, NR₁₀R₁₁, aryle ou hétéroaryle, chaque aryle ou hétéroaryle étant éventuellement substitué par un ou plusieurs groupes choisis parmi halogéno, OH, alkyle en C₁-C₃, alcoxy en C₁-C₃, hydroxyalkyle en C₁-C₃ et (CH₂)ₘNR₁₀R₁₁, et où Rₐ est lié en position 4 ou 5;
R₁ et R₂ sont identiques ou différents et choisis parmi H, halogéno, CN, NO₂, alkyle en C₁-C₁₀ linéaire ou ramifié, saturé ou insaturé, alcoxy en C₁-C₁₀ linéaire où ramifié, acyle en C₁-C₁₀ linéaire ou ramifié, acyloxy en C₁-C₁₀ linéaire ou ramifié, alkylthio en C₁-C₁₀ linéaire ou ramifié, aminosulfonyle, di(alkyl en C₁-C₃)aminosulfonyle, NR₁₀R₁₁, aryle, aroyle, aryloxy, arylsulfonyle, hétéroaryle et hétéroaryloxy; les R₁ et R₂ précités étant éventuellement partiellement ou entièrement halogénés ou éventuellement substitués par 1 à 3 groupes choisis indépendamment parmi oxo, OH, NR₁₀R₁₁, alkyle en C₁-C₆ linéaire ou ramifié, cycloalkyle en C₃-C₇, phényle, naphtyle, hétéroaryle, aminocarbonyle et mono- ou di(alkyl en C₁-C₃)aminocarbonyle;
R₃ est H, halogéno, OH, (CH₂)ₙNR₁₀R₁₁, (CH₂)ₙCO₂R₁₂; alkyle en C₁-C₃ éventuellement substitué par OH, alcoxy en C₁-C₃ éventuellement halogéné ou alkylthio en C₁-C₃;
R₄ et R₅, avec les atomes auxquels ils sont liés, complètent un système cyclique condensé des formules A ou B: 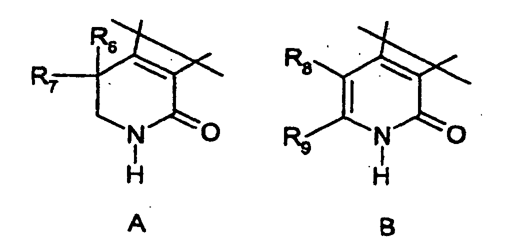
R₆ est alkyle en C₁-C₃ ou H;
R₇ est alkyle en C₁-C₆ linéaire ou ramifié ou H;
R₈ est H, alkyle en C₁-C₆ linéaire ou ramifié, saturé ou insaturé; éventuellement substitué par phényle, OH ou alcoxy en C₁-C₃; ou R₈ est (CH₂)ₘNR₁₀R₁₁, (CH₂)ₘNR₁₀COR₁₂, (CH₂)ₙCO₂R₁₂, (CH₂)ₙCONR₁₀R₁₁; ou R₈ est phényle ou hétéroaryle, chacun étant éventuellement substitué par alkyle en C₁-C₃, alcoxy en C₁-C₃, OH, -SO₃H ou halogéno;
R₉ est H; ou R₉ est alkyle en C₁-C₁₀ linéaire ou ramifié, cycloalkyle en C₃-C₁₀, alcényle en C₂-C₆, alcynyle en C₂-C₆, chacun étant éventuellement substitué par un ou plusieurs groupes halogéno, OH, oxo, CN, alcoxy en C₁-C₃, NR₁₀R₁₁, NR₁₀COR₁₂, SR₁₂, CONR₁₀R₁₁, CO₂R₁₂, aryloxy, arylthio, aryle ou hétéroaryle; chaque aryloxy, arylthio, aryle ou hétéroaryle étant éventuellement substitué par alkyle en C₁-C₃, alcoxy en C₁-C₃, halogéno, (CH₂)ₙNR₁₀R₁₁ ou O(CH₂)₂₋₄NR₁₀R₁₁;
ou R₉ est aryle ou hétéroaryle, chaque aryle ou hétéroaryle étant éventuellement substitué par 1 à 3 groupes choisis parmi alkyle en C₁-C₃ éventuellement substitué par phényle, alcoxy en C₁-C₃, halogéno, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 6 chaînons saturé ou insaturé, aromatique ou non aromatique, éventuellement substitué par 1 ou 2 OH, oxo ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, aryle, aryl-(alkyle en C₁-C₃) et hétéroaryle; ledit alkyle, cycloalkyle, aryle, aryl-(alkyle en C₁-C₃) ou hétéroaryle étant éventuellement substitué par OH, alcoxy en C₁-C₃, acyloxy en C₁-C₃, CO₂R₁₂, NR₁₃R₁₄, O(CH₂)₂₋₄NR₁₃R₁₄, aryle ou hétéroaryle;
ou R₁₀ et R₁₁ forment ensemble une chaîne alkylène de 3-7 chaînons complétant un cycle avec l'atome d'azote auquel ils sont liés; ladite chaîne alkylène étant éventuellement interrompue par O, S(O)ₚ et NR₁₃; et ledit cycle étant éventuellement substitué par alkyle en C₁-C₃, alcoxy en C₁-C₃, OH ou -(CH₂)ₙNR₁₃R₁₄;
R₁₂ est H, alkyle en C₁-C₆ ou cycloalkyle en C₃-C₈, chaque alkyle ou cycloalkyle étant éventuellement substitué par phényle, OH, alcoxy en C₁-C₃ ou NR₁₃R₁₄; ou R₁₂ est phényle éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂;
R₁₅ est H ou alkyle en C₁-C₃;
m est 1-4; n est 0-3 et p est 0-2; et
leurs dérivés acide ou sels acceptables d'un point de vue pharmaceutique.

6. Composé selon la revendication 5, dans lequel:
Ar₁ est
a) un groupe cycloalkyle choisi parmi cyclopropyle, cyclobutyle, cyclopentyle, cyclohexyle, cycloheptyle;
b) un groupe cycloalcényle choisi parmi cyclopentényle, cyclohexényle, cycloheptényle;
c) phényle, naphtyle, indanyle, indényle, dihydronaphtyle, tétrahydronaphtyle, fluorényle;
d) hétéroaryle choisi parmi pyridinyle, pyrimidinyle, pyrazinyle, pyridazinyle, pyrrolyle, imidazolyle, pyrazolyle, thiényle, furyle, isoxazolyle, isothiazolyle, oxazolyle, oxadiazolyle, thiazolyle, thiadiazolyle, quinoléinyle, isoquinoléinyle, indolyle, benzimidazolyle, benzofuranyle, benzoxazolyle, benzisoxazolyle, benzopyrazolyle, benzothiofuranyle, benzothiazolyle, quinazolinyle, et indazolyle, ou hétéroaryle condensé choisi parmi cyclopenténopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoléine, cyclohexanoquinoléine, cyclopentanoisoquinoléine, cyclohexanoisoquinoléine, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophène et cyclohexanothiophène; ou
e) un hétérocycle choisi parmi pyrrolinyle, pyrrolidinyle, pyrazolinyle, pyrazolidinyle, pipéridinyle, morpholinyle, thiomorpholinyle, pyranyle, thiopyranyle, pipérazinyle et indolinyle;
chacun des Ar₁ ci-dessus étant éventuellement substitué par un ou plusieurs R₁, R₂ et R₃ tels que définis ci-dessus;
Rₐ est H, alkyle en C₁-C₆, alcényle en C₂-C₅, alcynyle en C₂-C₅, phényle ou hétéroaryle choisi parmi pyridinyle, pyridazinyle, pyrimidinyle, pyrazinyle, oxazolyle, pyrazolyle, imidazolyle, furyle, thiazolyle et thiényle; chaque Rₐ étant éventuellement substitué par un ou plusieurs groupes phényle, halogéno, alkyle en C₁-C₃, alcoxy en C₁-C₃, OH, oxo ou NR₁₀R₁₁; Rₐ étant en position 4;
R₃ est H, halogéno, méthyle, méthoxy, hydroxyméthyle ou OH;
R₈ est H, alkyle en C₁-C₃ linéaire ou ramifié, saturé ou insaturé, éventuellement substitué par OH; ou R₈ est (CH₂)₂₋₃NR₁₀R₁₁, (CH₂)ₙCO₂R₁₂ ou (CH₂)ₙCONR₁₀R₁₁;
R₉ est alkyle en C₁-C₃ linéaire ou ramifié, alcényle en C₂-C₄, alcynyle en C₂-C₄, chacun étant éventuellement substitué par un ou plusieurs groupes OH, CN, NR₁₀R₁₁, CONR₁₀R₁₁, CO₂R₁₂, aryle ou hétéroaryle; chaque aryle ou hétéroaryle étant éventuellement substitué par alkyle en C₁-C₃, alcoxy en C₁-C₃, halogéno, (CH₂)ₙNR₁₀R₁₁ ou O(CH₂)₂₋₄NR₁₀R₁₁;
ou R₉ est aryle ou hétéroaryle éventuellement substitué par 1 à 3 groupes choisis parmi alkyle en C₁-C₃ éventuellement substitué par phényle, alcoxy en C₁-C₃, halogéno, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 6 chaînons saturé ou insaturé, aromatique ou non aromatique, éventuellement substitué par OH;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, benzyle et phényle; ledit alkyle, cycloalkyle, benzyle ou phényle-étant éventuellement substitué par OH, alcoxy en C₁-C₃, acyloxy en C₁-C₃, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃, OH, ou -(CH₂)ₙNR₁₃R₁₄;
R₁₂ est H ou alkyle en C₁-C₆ éventuellement substitué par phényle, OH, alcoxy en C₁-C₃ ou NR₁₃R₁₄;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂;
R₁₅ est H.

7. Composé selon la revendication 6, dans lequel:
Ar₁ est phényle ou pyridyle;
X est NH ou N-CH₃;
Y est NH et
Rₐ est H, hydroxyalkyle en C₁-C₂, 2-hydroxyéthylaminométhyle, méthoxybenzylaminométhyle, pyridinyle éventuellement halogéné, phényle, 3-hydroxy-2-oxopropyle, vinyle ou alcynyle en C₃-C₅ substitué par alcoxy en C₁-C₃ ou phényle;
R₁ et R₂ sont identiques ou différents et sont choisis parmi halogéno, alkyle en C₁-C₃, le groupe alkyle en C₁-C₃ étant éventuellement partiellement ou entièrement halogéné, NO₂, NR₁₃R₁₄;
R₃ est H, halogéno, méthoxy ou méthyle;
R₄ et R₅ complètent ensemble un cycle condensé de formule B;
R₈ est H, alkyle en C₁-C₃ éventuellement substitué par OH; ou R₈ est (CH₂)₂₋₃NR₁₀R₁₁ ou CO₂R₁₂;
R₉ est méthyle ou alcényle en C₂-C₃, chacun étant éventuellement substitué par un ou plusieurs groupes OH, CN, NR₁₀R₁₁, CONR₁₀R₁₁ ou CO₂R₁₂;
ou R₉ est hétéroaryle éventuellement substitué par 1 à 3 groupes choisis parmi alkyle en C₁-C₃ éventuellement substitué par phényle, alcoxy en C₁-C₃, halogéno ou amino;
R₁₀ et R₁₁ peuvent être identiques ou différents et sont choisis chacun indépendamment parmi H, OH, alcoxy en C₁-C₃, alkyle en C₁-C₃ linéaire ou ramifié, éventuellement substitué par OH, alcoxy en C₁-C₃, acyloxy en C₁-C₃, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃ ou OH;
R₁₂ est H ou alkyle en C₁-C₃ éventuellement substitué par phényle, OH, alcoxy en C₁-C₃ ou NR₁₃R₁₄;
R₁₃ et R₁₄ sont choisis chacun indépendamment parmi H et alkyle en C₁-C₃ éventuellement substitué par alcoxy en C₁-C₃ ou OH;
ou R₁₃ et R₁₄ forment ensemble une chaîne complétant un cycle, ladite chaîne étant (CH₂)₄₋₅ ou (CH₂)₂O(CH₂)₂.

8. Composé selon la revendication 7, dans lequel
Ar₁ est phényle;
Rₐ est H ou hydroxyméthyle;
R₁ et R₂ sont identiques ou différents et sont choisis parmi halogéno, méthyle éventuellement partiellement ou entièrement halogéné, NO₂ et NH₂;
R₃ est H, chloro, fluoro, bromo ou méthoxy;
R₁₀ et R₁₁ peuvent être identiques ou différents et sont choisis chacun indépendamment parmi H, OH, méthoxy, alkyle en C₁-C₃ linéaire ou ramifié, éventuellement substitué par OH, NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₂; et
R₁₂ est alkyle en C₁-C₃ éventuellement substitué par morpholino.

9. Composé selon la revendication 1, de formule (Ia): 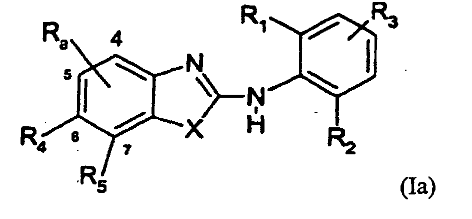 dans laquelle:
X est NH, N-alkyle en C₁-C₃, N-cyclopropyle, S ou O;
Rₐ est H, alkyle en C₁-C₁₀, alcényle en C₂-C₁₀ ou alcynyle en C₂-C₁₀ pouvant chacun être ramifié ou cyclique; ou Rₐ est aryle ou hétéroaryle; chaque Rₐ étant indépendamment éventuellement substitué par un ou plusieurs groupes alkyle en C₁-C₆, alcoxy en C₁-C₆, halogéno, OH, oxo, NR₁₀R₁₁, aryle ou hétéroaryle, chaque aryle ou hétéroaryle étant éventuellement substitué par un ou plusieurs groupes choisis parmi halogéno, OH, alkyle en C₁-C₃, alcoxy en C₁-C₃, hydroxyalkyle en C₁-C₃ et (CH₂)ₘNR₁₀R₁₁; et Rₐ est en position 4 ou 5;
R₁ et R₂ sont identiques ou différents et choisis parmi H, halogéno, CN, NO₂, alkyle en C₁-C₁₀ linéaire ou ramifié, saturé ou insaturé, alcoxy en C₁-C₁₀ linéaire ou ramifié, acyle en C₁-C₁₀ linéaire ou ramifié, acyloxy en C₁-C₁₀ linéaire ou ramifié, alkylthio en C₁-C₁₀ linéaire ou ramifié, aminosulfonyle, di(alkyl en C₁-C₃)aminosulfonyle, NR₁₀R₁₁, aryle, aroyle, aryloxy, arylsulfonyle, hétéroaryle et hétéroaryloxy; les R₁ et R₂ précités étant éventuellement partiellement ou entièrement halogénés ou éventuellement substitués par 1 à 3 groupes choisis indépendamment parmi oxo, OH, NR₁₀R₁₁, alkyle en C₁-C₆ linéaire ou ramifié, cycloalkyle en C₃-C₇, phényle, naphtyle, hétéroaryle, aminocarbonyle et mono- ou di(alkyl en C₁-C₃)aminocarbonyle;
R₃ est H, halogéno, OH, (CH₂)ₙNR₁₀R₁₁, CONR₁₀R₁₁, (CH₂)ₙCO₂R₁₂; alkyle en C₁-C₃ éventuellement substitué par OH, alcoxy en C₁-C₃ éventuellement halogéné ou alkylthio en C₁-C₃;
R₄ et R₅, avec les atomes àuxquels ils sont liés, complètent un, système cyclique condensé des formules A ou B: 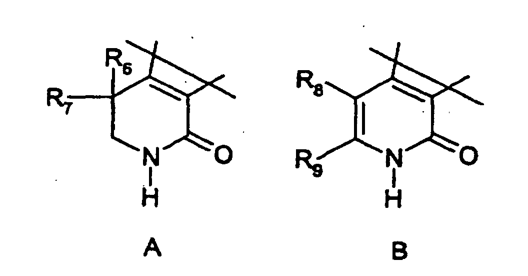
R₆ est alkyle en C₁-C₃ ou H;
R₇ est alkyle en C₁-C₆ linéaire ou ramifié ou H;
R₈ est H, alkyle en C₁-C₆ linéaire ou ramifié, saturé ou insaturé, éventuellement substitué par phényle, OH ou alcoxy en C₁-C₃; ou R₈ est (CH₂)ₘNR₁₀R₁₁, (CH₂)ₘNR₁₀COR₁₂, (CH₂)ₙCO₂R₁₂, (CH₂)ₙCONR₁₀R₁₁; ou R₈ est phényle ou hétéroaryle, chacun étant éventuellement substitué par alkyle en C₁-C₃, alcoxy en C₁-C₃, OH, -SO₃H ou halogéno;
R₉ est H, CN ou CONR₁₀R₁₁; ou R₉ est alkyle en C₁-C₁₀ linéaire ou ramifié, cycloalkyle en C₃-C₁₀, cycloalcényle en C₅-C₇, alcényle en C₂-C₆, alcynyle en C₂-C₆, chacun étant éventuellement substitué par un ou plusieurs groupes cycloalkyle en C₃-C₁₀, cycloalkylidène en C₃-C₁₀, cycloalcényle en C₅-C₇, halogéno, OH, oxo, CN, alcoxy en C₁-C₃, acyloxy en C₁-C₃, NR₁₀R₁₁, NR₁₀CONR₁₀R₁₁, NR₁₀C(=NR₁₀)NR₁₀R₁₁, NR₁₀COR₁₂, NR₁₀S(O)ₚR₁₂, SR₁₂, CONR₁₀R₁₁, CO₂R₁₂, C(R₁₀)=NNR₁₀R₁₁, C(R₁₀)=NNR₁₀CONR₁₀R₁₁, aryloxy, arylthio, aryle ou hétéroaryle; chaque aryloxy, arylthio, aryle ou hétéroaryle étant éventuellement substitué par alkyle en C₁-C₃, alcoxy en C₁-C₃, halogéno, (CH₂)ₙNR₁₀R₁₁ ou O(CH₂)₂₋₄NR₁₀R₁₁;
ou R₉ est aryle, hétéroaryle ou hétérocycle, chacun des aryle, hétéroaryle ou hétérocycle étant éventuellement substitué par 1 à 3 groupes choisis parmi alkyle en C₁-C₃ éventuellement substitué par phényle ou NR₁₀C(=NR₁₀)NR₁₀R₁₁, alcoxy en C₁-C₃, halogéno, CN, oxo, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 5 ou 6 chaînons saturé ou insaturé, aromatique ou non aromatique, éventuellement substitué par 1 ou 2 groupes alkyle en C₁-C₃, OH, oxo ou (CH₂)ₙNR₁₀R₁₁, ou éventuellement condensé en spiro à un groupe 1,3-dioxolane ou groupe 1,3-dithiolane, chaque groupe 1,3-dioxolane ou groupe 1,3 groupe-dithiolane étant éventuellement substitué par alkyle en C₁-C₆, alcoxy en C₁-C₆, OH ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, aryle, aryl-(alkyle en C₁-C₃) et hétéroaryle; ledit alkyle, cycloalkyle, aryle, aryl-(alkyle en C₁-C₃) ou hétéroaryle étant éventuellement substitué par OH, alcoxy en C₁-C₃, CN, NO₂, acyloxy en C₁-C₃, CO₂R₁₂, NR₁₃R₁₄, O(CH₂)₂₋₄NR₁₃R₁₄, aryle ou hétéroaryle;
ou R₁₀ et R₁₁ forment ensemble une chaîne alkylène de 3-7 chaînons complétant un cycle avec l'atome d'azote auquel ils sont liés; ladite chaîne alkylène étant éventuellement interrompue par O, S(O)ₚ et NR₁₃; et ledit cycle étant éventuellement substitué par alkyle en C₁-C₃, alcoxy en C₁-C₃, OH, -(CH₂)ₙNR₁₃R₁₄, CONR₁₃R₁₄ ou NR₁₃COR₁₄;
R₁₂ est H, alkyle en C₁-C₆ ou cycloalkyle en C₃-C₈, chaque alkyle ou cycloalkyle étant éventuellement substitué par phényle, OH, alcoxy en C₁-C₃ ou NR₁₃R₁₄; ou R₁₂ est phényle ou hétérocycle, éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂;
m est 1-4; n est 0-3 et p est 0-2; et
leurs dérivés acide ou sel acceptables d'un point de vue pharmaceutique.

10. Composé selon la revendication 9, dans lequel
X est NH ou N-CH₃;
Rₐ est H, hydroxyalkyle en C₁-C₂, 2-hydroxyéthylaminométhyle, méthoxybenzylaminométhyle, pyridinyle éventuellement halogéné, phényle, 3-hydroxy-2-oxopropyle, vinyle ou alcynyle en C₃-C₅ substitué par alcoxy en C₁-C₃ ou phényle; et où Rₐ est lié en position 4 ;
R₁ et R₂ sont identiques ou différents et sont choisis parmi H, halogéno, alkyle en C₁-C₃, le groupe alkyle en C₁-C₃ étant éventuellement partiellement ou entièrement halogéné, NO₂, NR₁₃R₁₄;
R₃ est H, halogéno, méthoxy ou méthyle;
R₄ et R₅ complètent ensemble un cycle condensé de formule B;
R₈ est H, alkyle en C₁-C₃ éventuellement substitué par OH; ou R₈ est (CH₂)₂₋₃NR₁₀R₁₁ ou CO₂R₁₂;
R₉ est CN; ou R₉ est méthyle, alcényle en C₂-C₃ ou alcynyle en C₂-C₃, chacun étant éventuellement substitué par un ou plusieurs groupes cycloalkylidène en C₅-C₇, cycloalcényle en C₅-C₇, OH, CN, NR₁₀R₁₁, NR₁₀CONR₁₀R₁₁, NR₁₀COR₁₂, NR₁₀S(O)ₚR₁₂, CONR₁₀R₁₁, CO₂R₁₂, C(R₁₀)=NNR₁₀R₁₁ ou hétéroaryle;
ou R₉ est aryle ou hétéroaryle éventuellement substitué par 1 à 3 groupes choisis parmi alkyle en C₁-C₃ éventuellement substitué par phényle, alcoxy en C₁-C₃, halogéno, amino ou CONH₂;
ou R₈ et R₉ forment ensemble un cycle cyclopentène condensé en spiro à un groupe 1,3-dioxolane, ledit groupe 1,3-dioxolane étant éventuellement substitué par alkyle en C₁-C₃, alcoxy en C₁-C₃, OH ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₃ linéaire ou ramifié, cycloalkyle en C₅-C₇ ou phényle; ledit alkyle, cycloalkyle ou phényle étant éventuellement substitué par OH, alcoxy en C₁-C₃, acyloxy en C₁-C₃, NO₂, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃, OH, -(CH₂)ₙNR₁₃R₁₄, CONR₁₃R₁₄ ou NR₁₃COR₁₄;
R₁₂ est H, alkyle en C₁-C₃ ou cycloalkyle en C₅-C₇, chacun étant éventuellement substitué par phényle, OH, alcoxy en C₁-C₃ ou NR₁₃R₁₄; ou R₁₂ est phényle ou un hétérocycle azoté saturé de 4 à 6 chaînons, chacun étant éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₃ éventuellement substitué par alcoxy en C₁-C₃ ou OH;
ou R₁₃ et R₁₄ forment ensemble une chaîne complétant un cycle, ladite chaîne étant (CH₂)₄₋₅ ou (CH₂)₂O(CH₂)₂.

11. Composé selon la revendication 10, dans lequel
Rₐ est H ou hydroxyméthyle;
R₁ et R₂ sont identiques ou différents et sont choisis parmi halogéno, méthyle éventuellement partiellement ou entièrement halogéné, NO₂ et NH₂;
R₃ est H, chloro, fluoro, bromo ou méthoxy;
R₁₀ et R₁₁ peuvent être identiques ou différents et sont choisis chacun indépendamment parmi H, OH, méthoxy, alkyle en C₁-C₃ linéaire ou ramifié ou cycloalkyle en C₅-C₇; ledit alkyle ou cycloalkyle étant éventuellement substitué par OH, NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₂, NR₁₃R₁₄, CONR₁₃R₁₄ ou NR₁₃COR₁₄; et
R₁₂ est alkyle en C₁-C₃ éventuellement substitué par morpholino; ou R₁₂ est phényle ou est azétidinyle, pyrrolidinyle ou pipéridinyle, éventuellement substitués chacun par 1 à 3 groupes choisis parmi les groupes alkyle en C₁-C₃, alcoxy en C₁-C₃ et halogéno.

12. Composé selon la revendication 9, dans lequel
X est NH, N-alkyle en C₁-C₃, N-cyclopropyle, S ou O;
Rₐ est H, alkyle en C₁-C₁₀, alcényle en C₂-C₁₀ ou alcynyle en C₂-C₁₀, pouvant chacun être ramifié ou cyclique; ou Rₐ est aryle ou hétéroaryle; chaque Rₐ étant indépendamment éventuellement substitué par un ou plusieurs groupes alcoxy en C₁-C₆, halogéno, OH, oxo, NR₁₀R₁₁, aryle ou hétéroaryle, chaque aryle ou hétéroaryle étant éventuellement substitué par un ou plusieurs groupes choisis parmi halogéno, OH, alkyle en C₁-C₃, alcoxy en C₁-C₃, hydroxyalkyle en C₁-C₃ et (CH₂)ₘNR₁₀R₁₁, et Rₐ est lié en position 4 ou 5;
R₁ et R₂ sont identiques ou différents et choisis parmi H, halogéno, CN, NO₂, alkyle en C₁-C₁₀ linéaire ou ramifié, saturé ou insaturé, alcoxy en C₁-C₁₀ linéaire ou ramifié, acyle en C₁-C₁₀ linéaire ou ramifié, acyloxy en C₁-C₁₀ linéaire ou ramifié, alkylthio en C₁-C₁₀ linéaire ou ramifié, aminosulfonyle, di(alkyl en C₁-C₃)aminosulfonyle, NR₁₀R₁₁, aryle, aroyle, aryloxy, arylsulfonyle, hétéroaryle et hétéroaryloxy; les R₁ et R₂ précités étant éventuellement partiellement ou entièrement halogénés ou éventuellement substitués par 1 à 3 groupes choisis indépendamment parmi oxo, OH, NR₁₀R₁₁, alkyle en C₁-C₆ linéaire ou ramifié, cycloalkyle en C₃-C₇, phényle, naphtyle, hétéroaryle, aminocarbonyle et mono- ou di(alkyl en C₁-C₃)aminocarbonyle;
R₃ est H, halogéno, OH, (CH₂)ₙNR₁₀R₁₁, (CH₂)ₙCO₂R₁₂; alkyle en C₁-C₃ éventuellement substitué par OH, alcoxy en C₁-C₃ éventuellement halogéné ou alkylthio en C₁-C₃;
R₄ et R₅, avec les atomes auxquels ils sont liés, complètent un système cyclique condensé des formules A ou B: 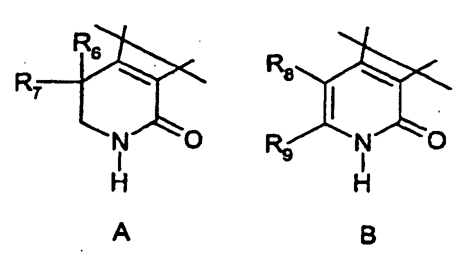
R₆ est alkyle en C₁-C₃ ou H;
R₇ est alkyle en C₁-C₆ linéaire ou ramifié ou H;
R₈ est H, alkyle en C₁-C₆ linéaire ou ramifié, saturé ou insaturé, éventuellement substitué par phényle, OH ou alcoxy en C₁-C₃; ou R₈ est (CH₂)ₘNR₁₀R₁₁, (CH₂)ₘNR₁₀COR₁₂, (CH₂)ₙCO₂R₁₂, (CH₂)ₙCONR₁₀R₁₁; ou R₈ est phényle ou hétéroaryle, chacun étant éventuellement substitué par alkyle en C₁-C₃, alcoxy en C₁-C₃, OH, -SO₃H ou halogéno;
R₉ est H; ou R₉ est alkyle en C₁-C₁₀ linéaire ou ramifié, cycloalkyle en C₃-C₁₀, alcényle en C₂-C₆, alcynyle en C₂-C₆, chacun étant éventuellement substitué par un ou plusieurs groupes halogéno, OH, oxo, CN, alcoxy en C₁-C₃, NR₁₀R₁₁, NR₁₀COR₁₂, SR₁₂, CONR₁₀R₁₁, CO₂R₁₂, aryloxy, arylthio, aryle ou hétéroaryle; chaque aryloxy, arylthio, aryle ou hétéroaryle étant éventuellement substitué par alkyle en C₁-C₃, alcoxy en C₁-C₃, halogéno, (CH₂)ₙNR₁₀R₁₁ ou O(CH₂)₂-₄NR₁₀R₁₁;
ou R₉ est aryle ou hétéroaryle, chaque aryle ou hétéroaryle étant éventuellement substitué par 1 à 3 groupes choisis parmi alkyle en C₁-C₃ éventuellement substitué par phényle, alcoxy en C₁-C₃, halogéno, (CH₂)ₙNR₁₀R₁₁, (CH₂)ₙCO₂R₁₂, (CH₂)ₙCONR₁₀R₁₁ et O(CH₂)₂₋₄NR₁₀R₁₁;
ou R₈ et R₉ forment ensemble un cycle carbocyclique de 6 chaînons saturé ou insaturé, aromatique ou non aromatique, éventuellement substitué par 1 ou 2 groupes OH, oxo ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, aryle, aryl-(alkyle en C₁-C₃) et hétéroaryle; ledit alkyle, cycloalkyle, aryle, aryl-(alkyle en C₁-C₃) ou hétéroaryle étant éventuellement substitué par OH, alcoxy en C₁-C₃, acyloxy en C₁-C₃, CO₂R₁₂, NR₁₃R₁₄, O(CH₂)₂₋₄NR₁₃R₁₄, aryle ou hétéroaryle;
ou R₁₀ et R₁₁ forment ensemble une chaîne alkylène de 3-7 chaînons complétant un cycle avec l'atome d'azote auquel ils sont liés; ladite chaîne alkylène étant éventuellement interrompue par O, S(O)ₚ et NR₁₃; et ledit cycle étant éventuellement substitué par alkyle en C₁-C₃, alcoxy en C₁-C₃, OH ou -(CH₂)ₙNR₁₃R₁₄;
R₁₂ est H, alkyle en C₁-C₆ ou cycloalkyle en C₃-C₈, chaque alkyle ou cycloalkyle étant éventuellement substitué par phényle, OH, alcoxy en C₁-C₃ ou NR₁₃R₁₄; ou R₁₂ est phényle, éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂;
m est 1-4; n est 0-3 et p est 0-2; et
leurs dérivés acide ou sel acceptables d'un point de vue pharmaceutique.

13. Composé selon la revendication 12, dans lequel
X est NH ou N-CH₃;
Rₐ est H, hydroxyalkyle en C₁-C₂, 2-hydroxyéthylaminométhyle, méthoxybenzylaminométhyle, pyridinyle éventuellement halogéné, phényle, 3-hydroxy-2-oxopropyle, vinyle ou alcynyle en C₃-C₅ substitué par alcoxy en C₁-C₃ ou phényle; et R₄ est lié en position 4;
R₁ et R₂ sont identiques ou différents et sont choisis parmi halogéno, alkyle en C₁-C₃, le groupe alkyle en C₁-C₃ étant éventuellement partiellement ou entièrement halogéné, NO₂, NR₁₃R₁₄;
R₃ est H, halogéno, méthoxy ou méthyle;
R₄ et R₅ complètent ensemble un cycle condensé de formule B;
R₈ est H, alkyle en C₁-C₃ éventuellement substitué par OH; ou R₈ est (CH₂)₂₋₃NR₁₀R₁₁ ou CO₂R₁₂;
R₉ est méthyle ou alcényle en C₂-C₃, chacun étant éventuellement substitué par un ou plusieurs groupes OH, CN, NR₁₀R₁₁, CONR₁₀R₁₁ ou CO₂R₁₂;
ou R₉ est hétéroaryle éventuellement substitué par 1 à 3 groupes choisis parmi alkyle en C₁-C₃ éventuellement substitué par phényle, alcoxy en C₁-C₃, halogéno ou (CH₂)ₙNR₁₀R₁₁;
R₁₀ et R₁₁ peuvent être identiques ou différents et sont choisis chacun indépendamment parmi H, OH, alcoxy en C₁-C₃, alkyle en C₁-C₃ linéaire ou ramifié, éventuellement substitué par OH, alcoxy en C₁-C₃, acyloxy en C₁-C₃, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃ ou OH;
R₁₂ est H ou alkyle en C₁-C₃ éventuellement substitué par phényle, OH, alcoxy en C₁-C₃ ou NR₁₃R₁₄;
R₁₃ et R₁₄ sont choisis chacun indépendamment parmi H et alkyle en C₁-C₃ éventuellement substitué par alcoxy en C₁-C₃ ou OH;
ou R₁₃ et R₁₄ forment ensemble une chaîne complétant un cycle, ladite chaîne étant (CH₂)₄₋₅ ou (CH₂)₂O(CH₂)₂.

14. Composé selon la revendication 13, dans lequel
Rₐ est H ou hydroxyméthyle;
R₁ et R₂ sont identiques ou différents et sont choisis parmi halogéno, méthyle éventuellement partiellement ou entièrement halogéné, NO₂ et NH₂;
R₃ est H, chloro, fluoro, bromo ou méthoxy;
R₁₀ et R₁₁ peuvent être identiques ou différents et sont choisis chacun indépendamment parmi H, OH, méthoxy, alkyle en C₁-C₃ linéaire ou ramifié, éventuellement substitué par OH, NR₁₃R₁₄ ou phényle;
ou R₁₀ et R₁₁ forment ensemble morpholino, pyrrolidinyle, pipérazinyle ou pipéridinyle, chacun éventuellement substitué par alkyle en C₁-C₂; et
R₁₂ est alkyle en C₁-C₃ éventuellement substitué par morpholino.

15. Composé intermédiaire de formule (III) 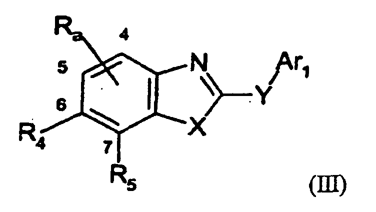 dans laquelle:
Ar₁ est un carbocycle aromatique ou non aromatique, un hétéroaryle ou un hétérocycle; ledit carbocycle, hétéroaryle ou hétérocycle étant éventuellement substitué par un ou plusieurs R₁, R₂ et R₃;
X est NH, N-alkyle en C₁-C₃, N-cyclopropyle, S ou O;
Y est NR₁₅, S ou O;
Rₐ est H, alkyle en C₁-C₁₀, alcényle en C₂-C₁₀ ou alcynyle en C₂-C₁₀, pouvant chacun être ramifié où cyclique; ou Rₐ est aryle ou hétéroaryle; chaque Rₐ étant indépendamment éventuellement substitué par un ou plusieurs groupes alkyle en C₁-C₃, alcoxy en C₁-C₆, halogéno, OH, oxo, NR₁₀R₁₁, aryle ou hétéroaryle, chaque aryle ou hétéroaryle étant éventuellement substitué par un ou plusieurs groupes choisis parmi halogéno, OH, alkyle en C₁-C₃, alcoxy en C₁-C₃, hydroxyalkyle en C₁-C₃ et (CH₂)ₘNR₁₀R₁₁, et où Rₐ est lié en position 4 ou 5;
R₁ et R₂ sont identiques ou différents et choisis parmi H, halogéno, CN, NO₂, alkyle en C₁-C₁₀ linéaire ou ramifié, saturé ou insaturé, alcoxy en C₁-C₁₀ linéaire ou ramifié, acyle en C₁-C₁₀ linéaire ou ramifié, acyloxy en C₁-C₁₀ linéaire ou ramifié, alkylthio en C₁-C₁₀ linéaire ou ramifié, aminosulfonyle, di(alkyl en C₁-C₃)aminosulfonyle, NR₁₀R₁₁, aryle, aroyle, aryloxy, arylsulfonyle, hétéroaryle et hétéroaryloxy; les R₁ et R₂ précités étant éventuellement partiellement ou entièrement halogénés ou éventuellement substitués par 1 à 3 groupes choisis indépendamment parmi oxo, OH, NR₁₀R₁₁, alkyle en C₁-C₆ linéaire ou ramifié, cycloalkyle en C₃-C₇, phényle, naphtyle, hétéroaryle, aminocarbonyle et mono- ou di(alkyl en C₁-C₃)aminocarbonyle;
R₃ est H, halogéno, OH, (CH₂)ₙNR₁₀R₁₁, (CH₂)ₙCO₂R₁₂; alkyle en C₁-C₃ éventuellement substitué par OH, alcoxy en C₁-C₃ éventuellement halogéné ou alkylthio en C₁-C₃;
R₄ et R₅, avec les atomes auxquels ils sont liés, complètent un système cyclique condensé de formule C: 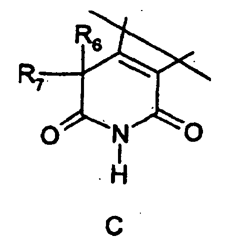
R₆ est alkyle en C₁-C₃ ou H;
R₇ est alkyle en C₁-C₆ linéaire ou ramifié ou H;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, aryle, aryl-(alkyle en C₁-C₃) et hétéroaryle; lesdits alkyle, cycloalkyle, aryle, aryl-(alkyle en C₁-C₃) ou hétéroaryle étant éventuellement substitués par OH, alcoxy en C₁-C₃, acyloxy en C₁-C₃, CO₂R₁₂, NR₁₃R₁₄, O(CH₂)₂₋₄NR₁₃R₁₄, aryle ou hétéroaryle;
ou R₁₀ et R₁₁ forment ensemble une chaîne alkylène de 3-7 chaînons complétant un cycle avec l'atome d'azote auquel ils sont liés; ladite chaîne alkylène étant éventuellement interrompue par O, S(O)ₚ et NR₁₃; et ledit cycle étant éventuellement substitué par alkyle en C₁-C₃, alcoxy en C₁-C₃, OH ou -(CH₂)ₙNR₁₃R₁₄;
R₁₂ est H, alkyle en C₁-C₆ ou cycloalkyle en C₃-C₈, chaque alkyle ou cycloalkyle étant éventuellement substitué par phényle, OH, alcoxy en C₁-C₃ ou NR₁₃R₁₄; ou R₁₂ est phényle éventuellement substitué par 1 à 3 groupes choisis parmi alkyle en C₁-C₃, alcoxy en C₁-C₃, halogéno, (CH₂)ₘNR₁₀R₁₁, (CH₂)ₙCONR₁₀R₁₁ et O(CH₂)₂₋₄NR₁₀R₁₁;
R₁₃ et R₁₄ sont choisis chacun indépendamment parmi H et alkyle en C₁-C₆ éventuellement substitué par alcoxy, OH ou phényle;
ou R₁₃ et R₁₄ forment ensemble une chaîne complétant un cycle, ladite chaîne étant (CH₂)₄₋₅ ou (CH₂)₂O(CH₂)₂; et
m est 1-4; n est 0-3 et p est 0-2;
où le terme hétéroaryle désigne un radical hétérocyclique aromatique monocyclique de 5-8 chaînons ou bicyclique de 8-11 chaînons stable consistant en atomes de carbone et de 1 à 4 hétéroatomes choisis parmi l'azote, l'oxygène et le soufre;
le terme hétérocycle désigne un radical hétérocyclique monocyclique de 4-8 chaînons ou bicyclique de 8-11 chaînons stable pouvant être saturé ou insaturé, et qui est non aromatique, consistant en atomes de carbone et de 1 à 4 hétéroatomes choisis parmi l'azote, l'oxygène et le soufre;
le terme aryle désigne un carbocycle aromatique de 6-10 chaînons;
le terme carbocycle désigne une chaîne carbonée cyclique aromatique ou non aromatique de 3-10 chaînons.

16. Composé selon la revendication 15, dans lequel
Ar₁ est
a) un groupe cycloalkyle choisi parmi cyclopropyle, cyclobutyle, cyclopentyle, cyclohexyle, cycloheptyle;
b) un groupe cycloalcényle choisi parmi cyclopentényle, cyclohexényle, cycloheptényle;
c) phényle, naphtyle, indanyle, indényle, dihydronaphtyle, tétrahydronaphtyle, fluorényle;
d) hétéroaryle choisi parmi pyridinyle, pyrimidinyle, pyrazinyle, pyridazinyle, pyrrolyle, imidazolyle, pyrazolyle, thiényle, furyle, isoxazolyle, isothiazolyle, oxazolyle, oxadiazolyle, thiazolyle, thiadiazolyle, quinoléinyle, isoquinoléinyle, indolyle, benzimidazolyle, benzofuranyle, benzoxazolyle, benzisoxazolyle, benzopyrazolyle, benzothiofuranyle, benzothiazolyle, quinazolinyle, et indazolyle, ou hétéroaryle condensé choisi parmi les groupes cyclopenténopyridine, cyclohexanopyridine, cyclopentanopyrimidine, cyclohexanopyrimidine, cyclopentanopyrazine, cyclohexanopyrazine, cyclopentanopyridazine, cyclohexanopyridazine, cyclopentanoquinoléine, cyclohexanoquinoléine, cyclopentanoisoquinoléine, cyclohexanoisoquinoléine, cyclopentanoindole, cyclohexanoindole, cyclopentanobenzimidazole, cyclohexanobenzimidazole, cyclopentanobenzoxazole, cyclohexanobenzoxazole, cyclopentanoimidazole, cyclohexanoimidazole, cyclopentanothiophène et cyclohexanothiophène; ou
e) un hétérocycle choisi parmi pyrrolinyle, pyrrolidinyle, pyrazolinyle, pyrazolidinyle, pipéridinyle, morpholinyle, thiomorpholinyle, pyranyle, thiopyranyle, pipérazinyle et indolinyle;
chacun des Ar₁ ci-dessus étant éventuellement substitué par un ou plusieurs R₁, R₂ et R₃ tels que définis ci-dessus;
Rₐ est H, alkyle en C₁-C₆, alcényle en C₂-C₅, alcynyle en C₂-C₅, phényle ou hétéroaryle choisi parmi pyridinyle, pyridazinyle, pyrimidinyle, pyrazinyle, oxazolyle, pyrazolyle, imidazolyle, furyle, thiazolyle et thiényle; chaque Rₐ étant éventuellement substitué par un ou plusieurs groupes phényle, halogéno, alkyle en C₁-C₃, alcoxy en C₁-C₃, OH, oxo ou NR₁₀R₁₁; Rₐ étant en position 4;
R₃ est H, halogéno, méthyle, méthoxy, hydroxyméthyle ou OH;
R₆ est alkyle en C₁-C₃ ou H;
R₇ est alkyle en C₁-C₆ linéaire ou ramifié ou H;
R₁₀ et R₁₁ peuvent être identiques ou différents et sont choisis chacun indépendamment parmi H, OH, alcoxy en C₁-C₃, alkyle en C₁-C₆ linéaire ou ramifié, cycloalkyle en C₃-C₈, benzyle et phényle; ledit alkyle, cycloalkyle ou phényle étant éventuellement substitué par OH, alcoxy en C₁-C₃, acyloxy en C₁-C₃, CO₂R₁₂, NR₁₃R₁₄, O(CH₂)₂₋₄NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃, OH ou -(CH₂)ₙNR₁₃R₁₄;
R₁₂ est H ou alkyle en C₁-C₆ éventuellement substitué par phényle, OH, alcoxy en C₁-C₃ ou NR₁₃R₁₄;
R₁₃ et R₁₄ sont choisis chacun indépendamment parmi H et alkyle en C₁-C₆ éventuellement substitué par alcoxy en C₁-C₃, OH ou phényle;
ou R₁₃ et R₁₄ forment ensemble une chaîne complétant un cycle, ladite chaîne étant (CH₂)₄₋₅ ou (CH₂)₂O(CH₂)₂.

17. Composé selon la revendication 16, dans lequel:
Ar₁ est phényle ou pyridyle;
X est NH ou N-CH₃;
Y est NH et
Rₐ est H, hydroxyalkyle en C₁-C₂, 2-hydroxyéthylaminométhyle, méthoxybenzylaminométhyle, pyridinyle éventuellement halogéné, phényle, 3-hydroxy-2-oxopropyle, vinyle ou alcynyle en C₃-C₅ substitué par alcoxy en C₁-C₃ ou phényle;
R₁ et R₂ sont identiques ou différents et sont choisis parmi halogéno, alkyle en C₁-C₃, le groupe alkyle en C₁-C₃ étant éventuellement partiellement ou entièrement halogéné, NO₂, NR₁₃R₁₄;
R₃ est H, halogéno, méthoxy ou méthyle;
R₁₀ et R₁₁ peuvent être identiques ou différents et sont choisis chacun indépendamment parmi H, OH, alcoxy en C₁-C₃, alkyle en C₁-C₃ linéaire ou ramifié, éventuellement substitué par OH, alcoxy en C₁-C₃, acyloxy én C₁-C₃, CO₂R₁₂, NR₁₃R₁₄, O(CH₂)₂₋₄NR₁₃R₁₄ ou phényle; ,
ou R₁₀ et R₁₁ forment ensemble morpholino, pyrrolidinyle, pipérazinyle ou pipéridinyle, chacun étant éventuellement substitué par alkyle en C₁-C₃, alcoxy en C₁-C₃ ou OH;
R₁₂ est H ou alkyle en C₁-C₃ éventuellement substitué par phényle, OH, alcoxy en C₁-C₃ ou NR₁₃R₁₄;
R₁₃ et R₁₄ sont choisis chacun indépendamment parmi H et alkyle en C₁-C₃ éventuellement substitué par alcoxy en C₁-C₃ ou OH;
ou R₁₃ et R₁₄ forment ensemble une chaîne complétant un cycle, ladite chaîne étant (CH₂)₄₋₅ ou (CH₂)₂O(CH₂)₂.

18. Composé selon la revendication 17, dans lequel
Ar₁ est phényle;
Rₐ est H ou hydroxyméthyle;
R₁ et R₂ sont identiques ou différents et sont choisis parmi halogéno, méthyle éventuellement partiellement ou entièrement halogéné, NO₂ et NH₂;
R₃ est H, chloro, fluoro, bromo ou méthoxy;
R₁₀ et R₁₁ peuvent être identiques ou différents et sont choisis chacun indépendamment parmi H, OH, méthoxy, alkyle en C₁-C₃ linéaire ou ramifié, éventuellement substitué par OH, NR₁₃R₁₄ ou phényle;
ou R₁₀ et R₁₁ forment ensemble morpholino, pyrrolidinyle, pipérazinyle ou pipéridinyle, chacun étant éventuellement substitué par alkyle en C₁-C₂; et
R₁₂ est alkyle en C₁-C₃ éventuellement substitué par morpholino.

19. Composé selon la revendication 9, choisi dans le groupe consistant en:
la 2-(2,6-dichlorophénylamino)-3,5-dihydroimidazo[4,5-i]phénanthridin-4-one;
la 2-(2,6-dichlorophénylamino)-1,6,7-triméthyl-1,8-dihydroimidazo[4,5-h]isoquinoléine-9-one;
la 2-(2,6-dichlorophénylamino)-1,7-diméthyl-6-(2-hydroxyéthyl)-1,8-dihydroimidazo[4,5-h]isoquinoléine-9-one;
le 2-(2,6-dichlorophénylamino)-1,7-diméthyl-9-oxo-1,8-dihydroimidazo[4,5-h]isoquinoléine-6-carboxylate de méthyle;
le 3-[2-(2,6-dichlorophénylamino)-1-méthyl-9-oxo-8,9-dihydro-1H-imidazo[4,5-h]isoquinoléin-7-yl]acrylate de méthyle;
la 2-(2-chloro-6-méthylphénylamino)-1,6,7-triméthyl-1,8-dihydroimidazo[4,5-h]isoquinoléine-9-one;
le 3-[2-(2,6-dichlorophénylamino)-1-méthyl-9-oxo-8,9-dihydro-1H-imidazo[4,5-h]isoquinoléin-7-yl]-N-méthoxy-N-méthylacrylamide;
la 2-(2-chloro-6-nitrophénylamino)-1,6,7-triméthyl-1,8-dihydroimidazo[4,5-h]isoquinoléine-9-one;
le N-benzyl-3-[2-(2,6-dichlorophénylamino)-1-méthyl-9-oxo-8,9-dihydro-1H-imidazo[4,5-h]isoquinoléin-7-yl]acrylamide;
le 4-morpholinamide de l'acide 3-[2-(2,6-dichlorophénylamino)-1-méthyl-9-oxo-8,9-dihydro-1H-imidazo[4,5-h]isoquinoléin-7-yl]acrylique;
la 2-(2,6-dichlorophénylamino)-4-hydroxyméthyl-1,6,7-triméthyl-1,8-dihydroimidazo[4,5-h]isoquinoléine-9-one;
la 2-(2,6-dichlorophénylamino)-1,6-diméthyl-7-vinyl-1,8-dihydroimidazo[4,5-h]isoquinoléine-9-one;
le 2-(2,6-dichlorophénylamino)-1,7-diméthyl-9-oxo-1,8-dihydroimidazo[4,5-h]isoquinoléine-6-carboxylate de 2-(4-morpholino)éthyle,
la 2-(2,6-dichlorophénylamino)-1,6-diméthyl-7-(3-hydroxypropén-1-yl)-1,8-dihydroimidazo[4,5-h]-isoquinoléin-9-one;
la 2-(2,6-dichlorophénylamino)-1,6-diméthyl-7-oxazol-5-yl-1,8-dihydroimidazo[4,5-h]isoquinoléin-9-one;
la 2-(2,6-dichlorophénylamino)-1-méthyl-7-vinyl-1,8-dihydroimidazo[4,5-h]isoquinoléine-9-one;
la 2-(2,6-dichlorophénylamino)-1,6-diméthyl-7-(3-morpholin-4-ylpropén-1-yl)-1,8-dihydroimidazo[4,5-h]isoquinoléin-9-one;
le 3-[2-(2,6-dichlorophénylamino)-1,7-diméthyl-9-oxo-8,9-dihydro-1H-imidazo[4,5-h]isoquinoléin-7-yl]acrylonitrile;
la 2-(2-chloro-6-méthylphénylamino)-1,7-diméthyl-1,8-dihydroimidazo[4,5-h]isoquinoléine-9-one;
la 2-(2,6-dichlorophénylamino)-1-méthyl-7-oxazol-5-yl-1,8-dihydroimidazo[4,5-h]isoquinoléin-9-one;
la 2-(2,6-dichlorophénylamino)-7-(3-hydroxypropén-1-yl)-1-méthyl-1,8-dihydroimidazo[4,5-h]isoquinoléin-9-one;
la 2-(2-chloro-6-méthylphénylamino)-7-(3-hydroxypropén-1-yl)-1-méthyl-1,8-dihydroimidazo[4,5-h]isoquinoléin-9-one;
la 2-(2,6-dichlorophénylamino)-7-(3-diéthylaminopropén-1-yl)-1,6-diméthyl-1,8-dihydroimidazo[4,5-h]isoquinoléin-9-one;
la 2-(2,6-dichlorophénylamino)-1,6-diméthyl-7-(3-pyrrolidin-1-ylpropén-1-yl)-1,8-dihydroimidazo[4,5-h]isoquinoléin-9-one;
la 2-(2,6-dichlorophénylamino)-7-(3-diéthylaminopropén-1-yl)-1-méthyl-1,8-dihydroimidazo[4,5-h]isoquinoléin-9-one;
la 2-(2,6-dichlorophénylamino)-1,6-diméthyl-7-(4-méthylpipérazin-1-yl-propén-1-yl)-1,8-dihydroimidazo[4,5-h]isoquinoléin-9-one;
la 2-(2,6-dichlorophénylamino)-1,6-diméthyl-7-(3-pipéridin-1-yl-propén-1-yl)-1,8-dihydroimidazo[4,5-h]isoquinoléin-9-one;
la 2-(2,6-dichlorophénylamino)-1,6-diméthyl-7-{3-[éthyl(2-hydroxyéthyl)amino]-propén-1-yl}-1,8-dihydroimidazo[4,5-h]isoquinoléin-9-one;
la 7-(3-diéthylaminopropén-1-yl)-1,6-diméthyl-2-(2,6-diméthylphénylamino)-1,8-dihydroimidazo[4,5-h]isoquinoléin-9-one;
la 2-(2,6-dichlorophénylamino)-7-{3-[(2-diéthylaminoéthyl)méthylamino]propén-1-yl}-1,6-diméthyl-1,8-dihydro-imidazo[4,5-h]isoquinoléin-9-one,
la 7-(3-diéthylaminopropén-1-yl)-1,6-diméthyl-2-(2,4,6-trichlorophénylamino)-1,8-dihydroimidazo[4,5-h]isoquinoléin-9-one;
la 2-(2,6-dichlorophénylamino)-6-méthyl-7-oxazol-5-yl-1,8-dihydroimidazo[4,5-h]isoquinoléin-9-one;
la 2-(2,6-dichlorophénylamino)-1,6-diméthyl-7-[3-(2-pyrrolidin-1-ylméthylpyrrolidin-1-yl)propén-1-yl]-1,8-dihydroimidazo[4,5-h]isoquinoléin-9-one;
la 7-[3-(2S-aminométhylpyrrolidin-1-yl)propén-1-yl]-2-(2,6-dichlorophénylamino)-1,6-diméthyl-1,8-dihydroimidazo[4,5-h]isoquinoléin-9-one;
le 1-{3-[2-(2,6-dichlorophénylamino)-1,6-diméthyl-9-oxo-8,9-dihydro-1H-imidazo[4,5-h]isoquinoléin-7-yl]propényl}-L-proline-carboxamide;
le 1-{3-[2-(2,6-dichlorophénylamino)-1,6-diméthyl-9-oxo-8,9-dihydro-1H-imidazo[4,5-h]isoquinoléin-7-yl]propényl)pipéridine-3-carboxamide;
la 2-(2,6-dichlorophénylamino)-1,6-diméthyl-7-(méthylhydrazonométhyl)-1,8-dihydroimidazo[4,5-h]isoquinoléin-9-one;
la 7-[3-(3-aminopyrrolidin-1-yl)-propén-1-yl]-2-(2,6-dichlorophénylamino)-1,6-diméthyl-1,8-dihydroimidazo[4,5-h]isoquinoléin-9-one;
la 2-(2,6-dichlorophénylamino)-1,6-diméthyl-7-[3-(3-acétamidopyrrolidin-1-yl)-propén-1-yl]-1,8-dihydroimidazo[4,5-h]isoquinoléin-9-one;
la 2-(2,6-dichlorophénylamino)-1,6-diméthyl-7-[3-(3-diméthylaminopyrrolidin-1-yl)-propén-1-yl]-1,8-dihydroimidazo[4,5-h]isoquinoléin-9-one;
le 1-{3-[2-(2,6-dichlorophénylamino)-1,6-diméthyl-9-oxo-8,9-dihydro-1H-imidazo[4,5-h]isoquinoléin-7-yl]propényl}pipéridine-2-carboxamide;
la 7-[3-(3-aminométhylpipéhdin-1-yl)propén-1-yl]-2-(2,6-dichlorophénylamino)-1,6-diméthyl-1,8-dihydroimidazo[4,5-h]-isoquinoléin-9-one;
le diéthylamide de l'acide 1-{3-[2-(2,6-dichlorophénylamino)-1,6-diméthyl-9-oxo-8,9-dihydro-1H-imidazo[4,5-h]isoquinoléin-7-yl]propényl}pipéridine-3-carboxylique;
la 2-(2,6-dichlorophénylamino)-1,6-diméthyl-7-éthynyl-1,8-dihydroimidazo[4,5-h]-isoquinoléin-9-one;
la 1-{3-[2-(2,6-dichlorophénylamino)-1,6-diméthyl-9-oxo-8,9-dihydro-1H-imidazo[4,5-h]isoquinoléin-7-yl]propényl}-3-méthylurée;
le {3-[2-(2,6-dichlorophénylamino)-1,6-diméthyl-9-oxo-8,9-dihydro-1H-imidazo[4,5-h]isoquinoléin-7-yl]propényl)amide de l'acide cyclohexanecarboxylique; la 2-(2,6-dichlorophénylamino)-1-méthyl-7-phényl-1,8-dihydroimidazo[4,5-h]isoquinoléin-9-one;
le N-{3-[2-(2,6-dichlorophénylamino)-1,6-diméthyl-9-oxo-8,9-dihydro-1H-imidazo[4,5-h]isoquinoléin-7-yl]propényl}méthanesulfonamide;
la 3-[2-(2,6-dichlorophénylamino)-1,6-diméthyl-9-oxo-8,9-dihydro-1H-imidazo[4,5-h]isoquinoléin-7-yl]propénylurée;
la 1-cyclohexyl-3-{3-[2-(2,6-dichlorophénylamino)-1,6-diméthyl-9-oxo-8,9-dihydro-1H-imidazo[4,5-h]isoquinoléin-7-yl]propényl}urée;
le N-{3-[2-(2,6-dichlorophénylamino)-1,6-diméthyl-9-oxo-8,9-dihydro-1H-imidazo[4,5-h]isoquinoléin-7-yl]propényl}benzènesulfonamide;
la 2-(2,6-dichlorophénylamino)-1,6-diméthyl-7-(3-éthylaminopropén-1-yl)-1,8-dihydroimidazo[4,5-h]isoquinoléin-9-one;
la N-{3-[2-(2,6-dichlorophénylamino)-1,6-diméthyl-9-oxo-8,9-dihydro-1H-imidazo[4,5-h]isoquinoléin-7-yl]propényl}guanidine;
le {3-[2-(2,6-dichlorophénylamino)-1,6-diméthyl-9-oxo-8,9-dihydro-1H-imidazo[4,5-h]isoquinoléin-7-yl]propényl}amide de l'acide pipéridine-3-carboxylique;
le {3-[2-(2,6-dichlorophénylamino)-1,6-diméthyl-9-oxo-8,9-dihydro-1H-imidazo[4,5-h]isoquinoléin-7-yl]propényl}amide de L-proline;
le {3-[2-(2,6-dichlorophénylamino)-1,6-diméthyl-9-oxo-8,9-dihydro-1H-imidazo[4,5-h]isoquinoléin-7-yl]-propényl}amide de D-proline;
le 3-[2-(2,6-dichlorophénylamino)-1,6-diméthyl-9-oxo-8,9-dihydro-1H-imidazo[4,5-h]isoquinoléin-7-yl]benzamide;
le {3-[2-(2,6-dichlorophénylamino)-1,6-diméthyl-9-oxo-8,9-dihydro-1H-imidazo[4,5-h]isoquinoléin-7-yl]-propényl}amide de l'acide L-azétidine-2-carboxylique;
le {3-[2-(2,6-dichlorophénylamino)-1,6-diméthyl-9-oxo-8,9-dihydro-1H-imidazo[4,5-h]isoquinoléin-7-yl]propényl}amide de l'acide pipéridine-2-carboxylique; et
leurs dérivés acceptables d'un point de vue pharmaceutique.

20. Composé selon la revendication 19, choisi dans le groupe consistant en :
la 2-(2,6-dichlorophénylamino)-3,5-dihydroimidazo[4,5-i]phénanthridin-4-one;
la 2-(2,6-dichlorophénylamino)-1,6,7-triméthyl-1,8-dihydroimidazo[4,5-h]isoquinoléine-9-one;
la 2-(2,6-dichlorophénylamino)-1,7-diméthyl-6-(2-hydroxyéthyl)-1,8-dihydroimidazo[4,5-h]isoquinoléine-9-one;
le 2-(2,6-dichlorophénylamino)-1,7-diméthyl-9-oxo-1,8-dihydro-imidazo[4,5-h]isoquinoléine-6-carboxylate de méthyle;
le 3-[2-(2,6-dichlorophénylamino)-1-méthyl-9-oxo-8,9-dihydro-1H-imidazo[4,5-h]isoquinoléin-7-yl]acrylate de méthyle;
la 2-(2-chloro-6-méthylphénylamino)-1,6,7-triméthyl-1,8-dihydroimidazo[4,5-h]isoquinoléine-9-one;
le 3-[2-(2,6-dichlorophénylamino)-1-méthyl-9-oxo-8,9-dihydro-1H-imidazo[4,5-h]isoquinoléin-7-yl]-N-méthoxy-N-méthylacrylamide;
la 2-(2-chloro-6-nitrophénylamino)-1,6,7-triméthyl-1,8-dihydroimidazo[4,5-h]isoquinoléine-9-one;
le N-benzyl-3-[2-(2,6-dichlorophénylamino)-1-méthyl-9-oxo-8,9-dihydro-1H-imidazo[4,5-h]isoquinoléin-7-yl]acrylamide;
le 4-morpholinamide de l'acide 3-[2-(2,6-dichlorophénylamino)-1-méthyl-9-oxo-8,9-dihydro-1H-imidazo[4,5-h]isoquinoléin-7-yl]acrylique;
la 2-(2,6-dichlorophénylamino)-4-hydroxyméthyl-1,6,7-triméthyl-1,8-dihydroimidazo[4,5-h]isoquinoléine-9-one;
la 2-(2,6-dichlorophénylamino)-1,6-diméthyl-7-vinyl-1,8-dihydroimidazo[4,5-h]isoquinoléine-9-one;
le 2-(2,6-dichlorophénylamino)-1,7-diméthyl-9-oxo-1,8-dihydroimidazo[4,5-h]isoquinoléine-6-carboxylate de 2-(4-morpholino)éthyle;
la 2-(2,6-dichlorophénylamino)-1,6-diméthyl-7-(3-hydroxypropén-1-yl)-1,8-dihydroimidazo[4,5-h]isoquinoléin-9-one;
la 2-(2,6-dichlorophénylamino)-1,6-diméthyl-7-oxazol-5-yl-1,8-dihydroimidazo[4,5-h]isoquinoléin-9-one;
la 2-(2,6-dichlorophénylamino)-1-méthyl-7-vinyl-1,8-dihydroimidazo[4,5-h]isoquinoléine-9-one;
la 2-(2,6-dichlorophénylamino)-1,6-diméthyl-7-(3-morpholin-4-ylpropén-1-yl)-1,8-dihydroimidazo[4,5-h]isoquinoléin-9-one;
le 3-[2-(2,6-dichlorophénylamino)-1,7-diméthyl-9-oxo-8,9-dihydro-1H-imidazo[4,5-h]isoquinoléin-7-yl]acrylonitrile;
la 2-(2-chloro-6-méthylphénylamino)-1,7-diméthyl-1,8-dihydroimidazo[4,5-h]isoquinoléine-9-one;
la 2-(2,6-dichlorophénylamino)-1-méthyl-7-oxazol-5-yl-1,8-dihydroimidazo[4,5-h]isoquinoléin-9-one;
la 2-(2,6-dichlorophénylamino)-7-(3-hydroxypropén-1-yl)-1-méthyl-1,8-dihydroimidazo[4,5-h]isoquinoléin-9-one;
la 2-(2-chloro-6-méthylphénylamino)-7-(3-hydroxypropén-1-yl)-1-méthyl-1,8-dihydroimidazo[4,5-h]isoquinoléin-9-one;
la 2-(2,6-dichlorophénylamino)-7-(3-diéthylaminopropén-1-yl)-1,6-diméthyl-1,8-dihydroimidazo[4,5-h]isoquinoléin-9-one;
la 2-(2,6-dichlorophénylamino)-1,6-diméthyl-7-(3-pyrrolidin-1-ylpropén-1-yl)-1,8-dihydroimidazo[4,5-h]isoquinoléin-9-one;
la 2-(2,6-dichlorophénylamino)-7-(3-diéthylaminopropén-1-yl)-1-méthyl-1,8-dihydroimidazo[4,5-h]isoquinoléin-9-one;
la 2-(2,6-dichlorophénylamino)-1,6-diméthyl-7-(4-méthylpipérazin-1-ylpropén-1-yl)-1,8-dihydroimidazo[4,5-h]isoquinoléin-9-one;
la 2-(2,6-dichlorophénylamino)-1,6-diméthyl-7-(3-pipéridin-1-ylpropén-1-yl)-1,8-dihydroimidazo[4,5-h]isoquinoléin-9-one;
la 2-(2,6-dichlorophénylamino)-1,6-diméthyl-7-{3-[éthyl(2-hydroxyéthyl)amino]-propén-1-yl}-1,8-dihydroimidazo[4,5-h]isoquinoléin-9-one;
la 7-(3-diéthylaminopropén-1-yl)-1,6-diméthyl-2-(2,6-diméthylphénylamino)-1,8-dihydroimidazo[4,5-h]isoquinoléin-9-one;
la 2-(2,6-dichlorophénylamino)-7-{3-[(2-diéthylaminoéthyl)méthylamino]propén-1-yl}-1,6-diméthyl-1,8-dihydroimidazo[4,5-h]isoquinoléin-9-one; et
leurs dérivés acceptables d'un point de vue pharmaceutique.

21. Composition pharmaceutique comprenant une quantité thérapeutiquement efficace d'un composé selon les revendications 1, 9, 15 ou 19.

22. Utilisation d'un composé selon les revendications 1, 9, 15 ou 19 pour la préparation d'un médicament destiné au traitement d'une maladie auto-immune ou d'un cancer.

23. Utilisation selon la revendication 22, dans laquelle la maladie auto-immune est choisie parmi la polyarthrite rhumatoïde, la sclérose en plaques, le syndrome de Guillain-Barré, la maladie de Crohn, la colite ulcéreuse, le psoriasis, le maladie du greffon contre l'hôte, le lupus érythémateux aigu disséminé, le diabète sucré insulinodépendant et l'asthme.

24. Utilisation selon la revendication 22, dans laquelle le cancer est choisi parmi une tumeur src-dépendante ou une tumeur PDGF-dépendante.

25. Utilisation selon la revendication 24, dans laquelle la tumeur src-dépendante est choisie parmi le cancer du sein, le cancer du côlon, le mélanome et le sarcome.

26. Utilisation selon la revendication 24, dans laquelle la tumeur PDGF-dépendante est choisie parmi le cancer de l'ovaire, le cancer de la prostate et le glioblastome.

27. Utilisation d'un composé selon les revendications 1, 9, 15 ou 19 pour la préparation d'un médicament destiné au traitement d'une maladie choisie parmi l'ostéoporose, la maladie de Paget, l'inflammation des os et l'inflammation des articulations.

28. Utilisation d'un composé selon les revendications 1, 9, 15 ou 19 pour la préparation d'un médicament destiné au traitement d'une maladie choisie parmi les maladies fibreuses, la resténose et l'athérosclérose.

29. Utilisation d'un composé selon les revendications 1, 9, 15 ou 19 pour la préparation d'un médicament destiné à renforcer ou à potentialiser l'efficacité d'une radiothérapie.

30. Procédé de préparation d'un composé de formule (I) 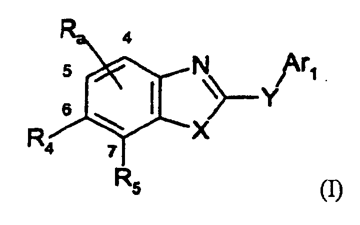 dans laquelle X est N-R₁₅ et Ar₁, R₄, R₅, R₁₅ et Rₐ ont la définition donnée dans la revendication 1, ledit procédé comprenant:
a) la réaction d'un composé de formule (II) avec Ar₁NCS dans un solvant approprié à une température d'environ la température ambiante à la température de reflux pendant environ 3 à 24 heures pour l'obtention d'un composé de formule (III) 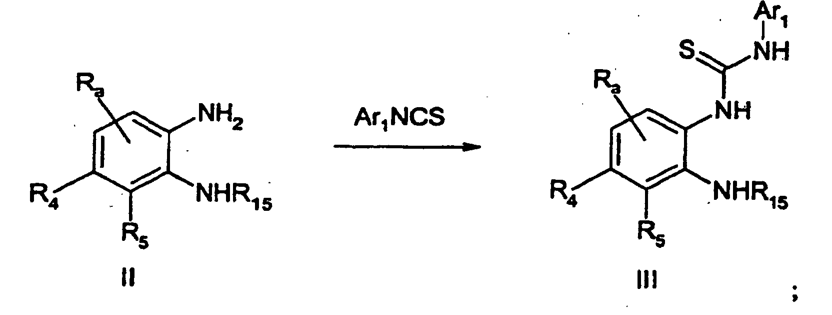
b) la réaction du produit (II) de l'étape a) avec un agent d'activation approprié choisi parmi le 1,3-dicyclohexylcarbodiimide (DCC) et l'oxyde mercurique dans un solvant approprié à une température d'environ la température ambiante à la température de reflux, pour former un composé de formule (I) tel que représenté ci-dessus ou des précurseurs de ce composé.

31. Procédé de préparation d'un composé de formule (I) 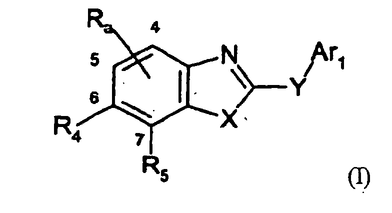 dans laquelle X est S, Y est NH et Ar₁, R₄, R₅ et Rₐ ont la définition donnée dans la revendication 1, ledit procédé comprenant:
a) la réaction d'un composé de formule (IV) avec Ar₁NCS dans un solvant approprié à une température d'environ la température ambiante à la température de reflux pendant environ 3 à 24 heures pour former un composé de formule (V); 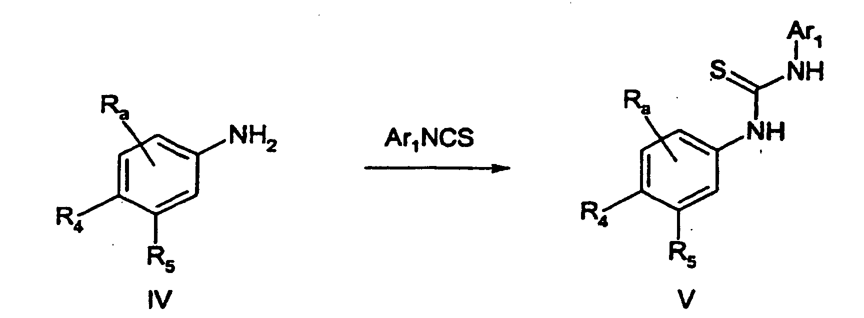
b) la réaction du produit (V) de l'étape a) dans des conditions de cyclisation dans un solvant approprié à environ la température de reflux pour former un composé de formule (I) ou un de ses précurseurs.

32. Procédé de préparation d'un composé de formule (XI) 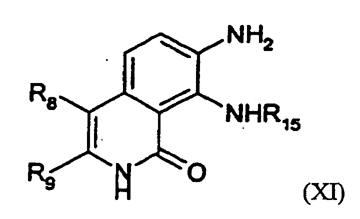 dans laquelle R₁₅, R₈ et R₉ sont tels que décrits dans la revendication 1, ledit procédé comprenant:
a) la réaction d'un composé de formule (VI) avec NHR₁₅ dans un solvant approprié éventuellement dans un récipient sous pression et à une température d'environ 0 à 80°C, pour l'obtention de VII, puis la réaction du composé VII avec un cétoester VIII en présence d'une base appropriée dans un solvant approprié, à environ la température ambiante, pour former un composé de formule (IX): 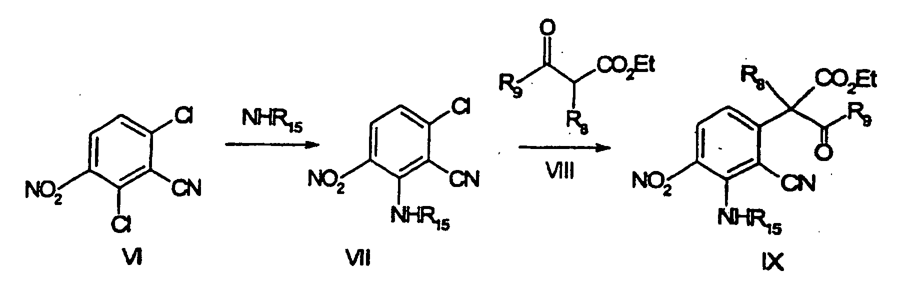
b) l'hydrolyse du produit de l'étape a) par réaction avec un acide aqueux, et la cyclisation à environ la température de reflux; suivie de la réduction du produit cyclisé dans un solvant approprié pour former un composé de formule (XI).

33. Procédé de préparation d'un composé de formule 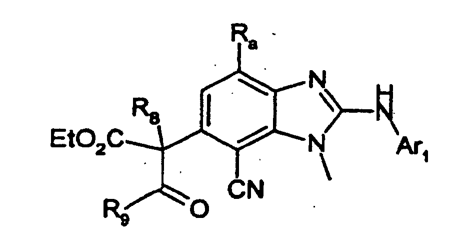 dans laquelle Rₐ, R₈, R₉ et Ar₁ sont tels que décrits dans la revendication 1; ledit procédé comprenant:
a) la réaction d'un composé de formule (XII) avec du brome dans un solvant approprié à la température ambiante pour l'obtention de XIII: 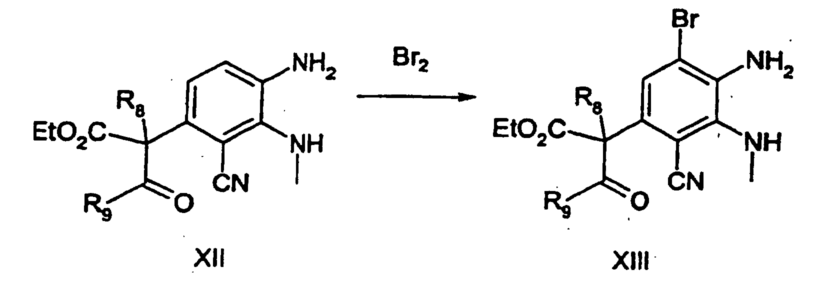
b) la réaction d'un composé de formule (XIII) avec Ar₁NCS dans un solvant approprié à une température d'environ la température ambiante à la température de reflux pendant environ 3 à 24 heures, puis la réaction du produit avec un agent d'activation approprié choisi parmi le 1,3-dicyclohexylcarbodiimide (DCC) et l'oxyde mercurique dans un solvant approprié à une température d'environ la température ambiante à la température de reflux pour former un composé de formule (XIV);
c) un couplage croisé pour l'introduction de Rₐ à la place du brome en présence d'un catalyseur approprié dans un solvant approprié à environ 100°C, pour former le composé produit présenté ci-dessous:
